(19) Europäisches Patentamt
European Patent Office
Office européen des brevets

(11) EP 2 261 213 A1

(12) EUROPEAN PATENT APPLICATION
published in accordance with Art. 153(4) EPC

(43) Date of publication:
**15.12.2010 Bulletin 2010/50**

(21) Application number: **09729180.1**

(22) Date of filing: **31.03.2009**

(51) Int Cl.:
*C07D 215/38* (2006.01) *A61K 31/382* (2006.01)
*A61K 31/47* (2006.01) *A61K 31/4709* (2006.01)
*A61P 3/04* (2006.01) *A61P 43/00* (2006.01)
*C07D 335/02* (2006.01) *C07D 401/12* (2006.01)
*C07D 409/12* (2006.01) *C07D 417/12* (2006.01)

(86) International application number:
**PCT/JP2009/056663**

(87) International publication number:
**WO 2009/123194 (08.10.2009 Gazette 2009/41)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **01.04.2008 JP 2008094805**

(71) Applicant: **Takeda Pharmaceutical Company Limited**
**Osaka 541-0045 (JP)**

(72) Inventors:
- **MURATA, Toshiki**
**Osaka-shi**
**Osaka 532-8686 (JP)**
- **TAKAMI, Kazuaki**
**Osaka-shi**
**Osaka 532-8686 (JP)**
- **KAMAURA, Masahiro**
**Osaka-shi**
**Osaka 532-8686 (JP)**
- **OKAWA, Tomohiro**
**Osaka-shi, Osaka 532-8686 (JP)**

(74) Representative: **Jones, Nicholas Andrew et al**
**Withers & Rogers LLP**
**Goldings House**
**2 Hays Lane**
**London**
**SE1 2HW (GB)**

# (54) HETEROCYCLIC COMPOUND

(57) The present invention provides to a compound having melanin-concentrating hormone receptor antagonistic action and low toxicity, and useful as a agent for the prophylaxis or treatment of obesity and the like.

The present invention relates to a compound represented by the formula (I):

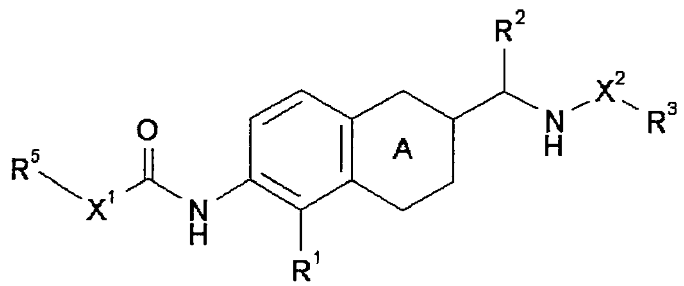

(I)

wherein each symbol is as defined in the specification, or a salt thereof.

EP 2 261 213 A1

## Description

## Technical Field

The present invention relates to a heterocyclic compound having a melanin-concentrating hormone (hereinafter sometimes abbreviated as MCH) receptor antagonistic action, and useful as an agent for the prophylaxis or treatment of obesity and the like.

(Background of the Invention)

Melanin-concentrating hormone is derived from hypothalamus and is known to show an appetite stimulating action. Moreover, it has been reported that MCH knockout mouse shows normal behavior but eats significantly less and weighs light as compared to normal mouse (Nature, vol. 396, page 670, 1998). Therefore, an MCH receptor antagonist is expected to provide a superior anorexigenic agent or antiobesity drug.
The following compounds are known as compounds having MCH receptor antagonistic action. 1) Patent document 1 (WO01/21577) discloses a compound represented by the formula:
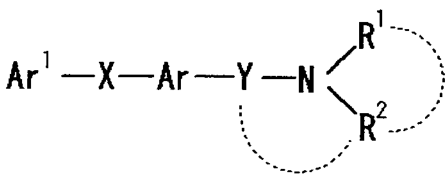

wherein

Ar$^1$ is a cyclic group optionally having substituent(s);
X is a spacer having 1 to 6 atoms in the main chain;
Y is a bond or a spacer having 1 to 6 atoms in the main chain;
Ar is a monocyclic aromatic ring optionally condensed with a 4 to 8 membered non-aromatic ring and optionally additional substituent(s); and
R$^1$ and R$^2$ are the same or different and each is a hydrogen atom or a hydrocarbon group optionally having substituent (s); or
R$^1$ and R$^2$ optionally form, together with the adjacent nitrogen atom, a nitrogen-containing heterocycle optionally having substituent(s); or
R$^2$ optionally forms a spiro ring together with Ar; or
R$^2$ optionally forms, together with the adjacent nitrogen atom and Y, a nitrogen-containing heterocycle optionally having substituent(s)

or a salt thereof.
2) Patent document 2 (WO01/82925) discloses a compound represented by the formula:
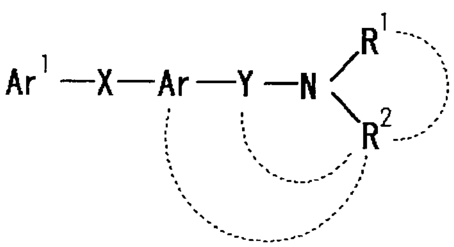

(I)

wherein Ar$^1$ is an optionally substituted cyclic group;

X and Y are each independently spacer having 1 to 6 atoms in the main chain;
Ar is an optionally substituted fused polycyclic aromatic ring; and
$R^1$ and $R^2$ are each independently a hydrogen atom or an optionally substituted hydrocarbon group; or
$R^1$ and $R^2$ are optionally bonded to form an optionally substituted nitrogen-containing heterocycle; or
$R^2$ and Y are optionally bonded to form an optionally substituted nitrogen-containing heterocycle; or
$R^2$ and Ar are optionally bonded to form an optionally substituted nitrogen-containing fused ring,

or a salt thereof.

3) Patent document 3 (WO01/087834) discloses a compound represented by the formula:

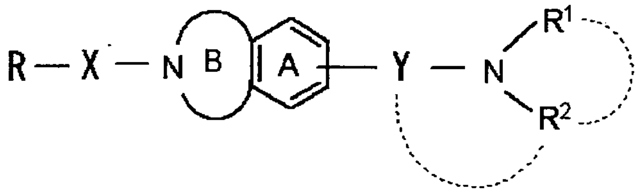

(I)

wherein

R is a hydrogen atom, a halogen atom or a cyclic group optionally having substituent(s);
X is a bond or a spacer having 1 to 10 atoms in the main chain;
Y is a spacer having 1 to 6 atoms in the main chain;
ring A is a benzene ring optionally having additional substituent(s);
ring B is a 5- to 9-membered nitrogen-containing non-aromatic heterocycle optionally having additional substituent (s); and
$R^1$ and $R^2$ are the same or different and each is a hydrogen atom, a hydrocarbon group optionally having substituent (s) or a heterocyclic group optionally having substituent(s); or
$R^1$ and $R^2$ optionally form, together with the adjacent nitrogen atom, a nitrogen-containing heterocycle optionally having substituent(s); or
$R^2$ optionally forms, together with the adjacent nitrogen atom and Y, a nitrogen-containing heterocycle optionally having substituent(s),

or a salt thereof or a prodrug thereof.

4) Patent document 4 (WO03/035624) discloses a compound represented by the formula:

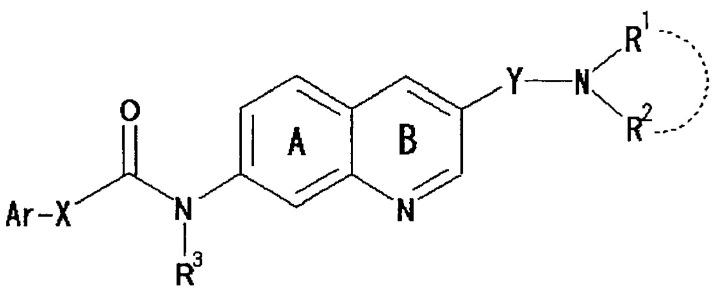

(I)

wherein

Ar is an optionally substituted cyclic group;
X is a bond or a spacer having 1 to 6 atoms in the main chain;
$R^1$ and $R^2$ are each independently a hydrogen atom or an optionally substituted hydrocarbon group;
$R^1$ and $R^2$ are optionally bonded to form an optionally substituted nitrogen-containing heterocycle;
Y is an optionally substituted divalent hydrocarbon group (excluding CO);

$R^3$ is a hydrogen atom or an optionally substituted hydrocarbon group; and
ring A and ring B are each independently optionally having additional substituent(s) (the additional substituent for ring B is optionally bonded to $R^1$ to form a ring),

or a salt thereof or a prodrug thereof.

5) Patent document 5 (WO2006/118320) discloses a compound represented by the formula:

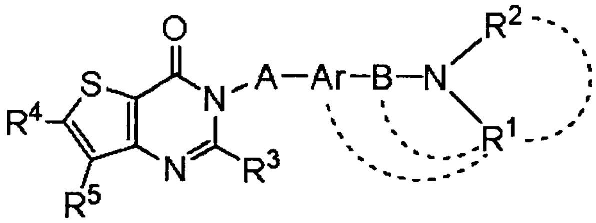

(I)

wherein

Ar is an optionally substituted ring;
A is a spacer having 1 to 4 atoms in the main chain;
B is a bond, a $C_{1-10}$ alkylene group or an oxygen atom;
$R^3$ and $R^5$ are each independently a hydrogen atom or a substituent;
$R^4$ is an optionally substituted cyclic group or an optionally substituted $C_{1-10}$ alkyl group; and
$R^1$ and $R^2$ are each independently a hydrogen atom or a substituent, or
$R^1$ is optionally bonded to $R^2$ or B to form an optionally substituted nitrogen-containing heterocycle, or
$R^1$ is optionally bonded to Ar to form an optionally substituted nitrogen-containing fused heterocycle,

or a salt thereof or a prodrug thereof.

Patent Document 1: WO01/21577
Patent Document 2: WO01/82925
Patent Document 3: WO01/087834
Patent Document 4: WO03/035624
Patent Document 5: WO2006/118320

## Disclosure of the Invention

## Problems to be Solved by the Invention

There is a demand for the development of a compound showing a melanin-concentrating hormone receptor antagonistic action and low toxicity, which is useful as an agent for the prophylaxis or treatment of obesity and the like.

## Means of Solving the Problems

The present inventors have conducted intensive studies of a compound having an MCH receptor antagonistic action, and showing low toxicity (particularly, cardiotoxicity (e.g., human ether-a-go-go related gene (HERG) inhibitory activity), phospholipidosis (PLsis) and the like often causing problems in the drug discovery) and found that a compound represented by the formula (I):

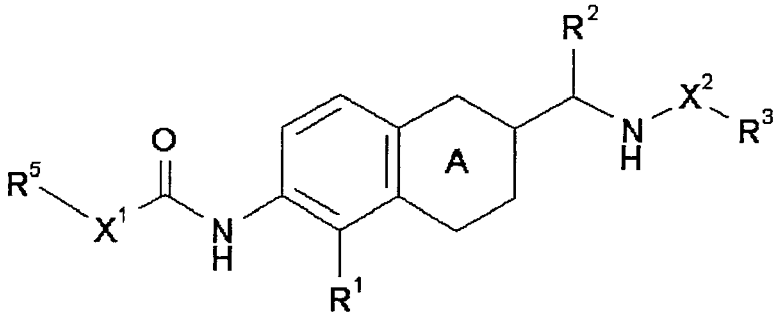

(I)

wherein

ring A is an optionally further substituted 6-membered ring;
$R^1$ is a hydrogen atom, a halogen atom or a $C_{1-6}$ alkyl group;
$R^2$ is a hydrogen atom or a $C_{1-6}$ alkyl group;
$R^3$ is
a group represented by the formula: $-Y-S(O)_{m1}-R^{4a}$
wherein

Y is a bond or NH;
m1 is an integer of 1 or 2; and
$R^{4a}$ is a $C_{1-6}$ alkyl group optionally substituted by 1 to 3 halogen atoms, or

a cyclic group represented by the formula:

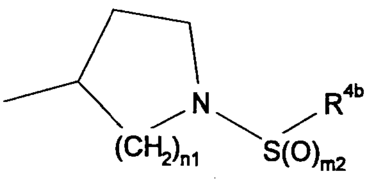 , 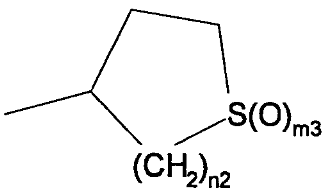 , or 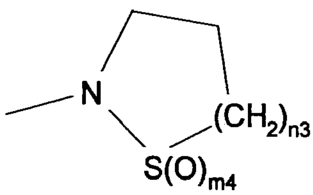

wherein

m2, m3, m4, n1, n2 and n3 are each independently an integer of 1 or 2; and
$R^{4b}$ is a $C_{1-6}$ alkyl group optionally substituted by 1 to 3 halogen atoms
(the ring moiety of the cyclic group is optionally further substituted);

$R^5$ is an optionally further substituted 5- or 6-membered cyclic group;
$X^1$ is a bond or a $C_{1-6}$ alkylene group; and
$X^2$ is a bond or a $C_{1-6}$ alkylene group,
or a salt thereof (sometimes to be abbreviated as "compound (I)" in the present specification) has a superior MCH receptor antagonistic action, and shows low toxicity such as cardiotoxicity (e.g., HERG inhibitory activity), PLsis and the like, which resulted in the completion of the present invention.

Accordingly, the present invention relates to

[1] compound (I);
[2] the compound of the above-mentioned [1], wherein ring A is an optionally further substituted 6-membered aromatic heterocycle;
[3] the compound of the above-mentioned [1], wherein $R^3$ is a group represented by the formula: $-Y-S(O)_{m1}-R^{4a}$ wherein Y is a bond; m1 is an integer of 1 or 2; and $R^{4a}$ is a $C_{1-6}$ alkyl group optionally substituted by 1 to 3 halogen

atoms, or a cyclic group represented by the formula:

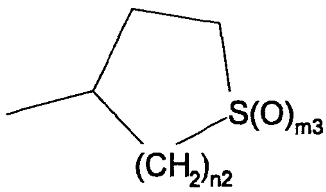

wherein m3 and n2 are each independently an integer of 1 or 2 (the ring moiety of the cyclic group is optionally further substituted);

[4] the compound of the above-mentioned [1], wherein $R^5$ is an optionally further substituted phenyl group;
[5] the compound of the above-mentioned [1], wherein $X^1$ is a bond;
[6] the compound of the above-mentioned [1], wherein
ring A is an optionally further substituted 6-membered aromatic heterocycle;
$R^1$ is a hydrogen atom, a halogen atom or a $C_{1-6}$ alkyl group;
$R^2$ is a hydrogen atom or a $C_{1-6}$ alkyl group;
$R^3$ is a group represented by the formula: $-Y-S(O)_{m1}-R^{4a}$ wherein
Y is a bond; m1 is an integer of 1 or 2; and $R^{4a}$ is a $C_{1-6}$ alkyl group optionally substituted by 1 to 3 halogen atoms, or a cyclic group represented by the formula:

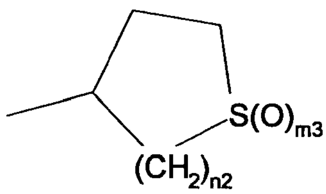

wherein m3 and n2 are each independently an integer of 1 or 2 (the ring moiety of the cyclic group is optionally further substituted);
$R^5$ is an optionally further substituted phenyl group;
$X^1$ is a bond; and
$X^2$ is a bond or a $C_{1-6}$ alkylene group;

[7] 4-(cyclopropylmethoxy)-N-(3-{[(1,1-dioxidotetrahydro-2H-thiopyran-4-yl)amino]methyl}-8-methylquinolin-7-yl) benzamide or a salt thereof (Example 31);
[8] 4-(cyclopropylmethoxy)-N-(8-methyl-3-{[(trans-1-oxidotetrahydro-2H-thiopyran-4-yl)amino]methyl}quinolin-7-yl) benzamide or a salt thereof (Example 32);
[9] 4-(cyclopropylmethoxy)-N-(3-{[(1,1-dioxidotetrahydro-2H-thiopyran-4-yl)amino]methyl}-8-methylquinolin-7-yl)-2-fluorobenzamide or a salt thereof (Example 34);
[10] 4-(cyclopropylmethoxy)-2-fluoro-N-(8-methyl-3-{[(trans-1-oxidotetrahydro-2H-thiopyran-4-yl)amino]methyl} quinolin-7-yl)benzamide or a salt thereof (Example 35);
[11] 4-(cyclopropylethynyl)-N-(3-{[(1,1-dioxidotetrahydro-2H-thiopyran-4-yl)amino]methyl}-8-methylquinolin-7-yl) benzamide or a salt thereof (Example 41);
[12] a prodrug of the compound of the above-mentioned [1];
[13] a pharmaceutical agent comprising the compound of the above-mentioned [1] or a prodrug thereof;
[14] the pharmaceutical agent of the above-mentioned [13], which is a melanin-concentrating hormone receptor antagonist;

[15] the pharmaceutical agent of the above-mentioned [13], which is an anorexigenic agent;
[16] the pharmaceutical agent of the above-mentioned [13], which is an agent for the prophylaxis or treatment of obesity;
[17] a method for the prophylaxis or treatment of obesity in a mammal, which comprises administering an effective amount of the compound of the above-mentioned [1] or a prodrug thereof to the mammal;
[18] use of the compound of the above-mentioned [1] or a prodrug thereof for the production of an agent for the prophylaxis or treatment of obesity;

and the like.

## Effect of the Invention

Compound (I) has a melanin-concentrating hormone receptor antagonistic action, and shows low toxicity such as cardiotoxicity (e.g., HERG inhibitory activity), PLsis and the like. Therefore, compound (I) is very useful as an agent for the prophylaxis or treatment of obesity and the like.

(Detailed Description of the Invention)

The definition of each symbol in the formula (I) is explained in detail in the following.
The "halogen atom" in the present specification means, unless otherwise specified, a fluorine atom, a chlorine atom, a bromine atom or an iodine atom.
The "$C_{1-6}$ alkyl group" in the present specification means, unless otherwise specified, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like.
The "$C_{2-6}$ alkenyl group" in the present specification means, unless otherwise specified, ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl or the like.
The "$C_{2-6}$ alkynyl group" in the present specification means, unless otherwise specified, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl or the like.
The "$C_{3-10}$ cycloalkyl group" in the present specification means, unless otherwise specified, cyclopropyl, cyclobutyl, cyclopentyl, cyclohexyl, cycloheptyl, cyclooctyl or the like. Of these, $C_{3-6}$ cycloalkyl group is preferable.
The "$C_{1-3}$ alkylenedioxy group" in the present specification means, unless otherwise specified, methylenedioxy, ethylenedioxy, trimethylendioxy or the like.
The "$C_{1-3}$ alkylenedioxy group" in the present specification means, unless otherwise specified, methylenedioxy, ethylenedioxy, trimethylenedioxy or the like.
The "$C_{1-6}$ alkyl group" in the present specification means, unless otherwise specified, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like.
The "$C_{1-6}$ alkoxy group" in the present specification means, unless otherwise specified, methoxy, ethoxy, propoxy, isopropoxy, butoxy, isobutoxy, sec-butoxy, tert-butoxy or the like.
The "$C_{1-6}$ alkoxy-carbonyl group" in the present specification means, unless otherwise specified, methoxycarbonyl, ethoxycarbonyl, propoxycarbonyl, tert-butoxycarbonyl or the like.
The "$C_{1-6}$ alkyl-carbonyl group" in the present specification means, unless otherwise specified, acetyl, propanoyl, butanoyl, isobutanoyl, pentaoyl, isopentanoyl, hexanoyl or the like.
Ring A is an optionally further substituted 6-membered ring.
Examples of the "6-membered ring" of the "optionally further substituted 6-membered ring" for ring A include benzene, cyclohexane, cyclohexene, a cyclohexadiene, a 6-membered aromatic heterocycle and a 6-membered non-aromatic heterocycle.
Specific examples of the cyclohexadiene include 2,4-cyclohexadiene and 2,5-cyclohexadiene.
Examples of the 6-membered aromatic heterocycle include a 6-membered aromatic heterocycle containing, as a ring-constituting atom besides carbon atom, 1 to 4 hetero atoms selected from an oxygen atom, a sulfur atom (the sulfur atom is optionally oxidized) and a nitrogen atom.
Preferable specific examples of the 6-membered aromatic heterocycle include pyridine, pyrazine, pyrimidine and pyridazine.
Examples of the 6-membered non-aromatic heterocycle include a 6-membered non-aromatic heterocycle containing, as a ring-constituting atom besides carbon atom, 1 to 4 hetero atoms selected from an oxygen atom, a sulfur atom (the sulfur atom is optionally oxidized) and a nitrogen atom.

Preferable specific examples of the 6-membered non-aromatic heterocycle include pyran, piperidine, piperazine, morpholine and thiomorpholine.
As to the "6-membered ring" of the "optionally further substituted 6-membered ring" for ring A, the moiety represented by
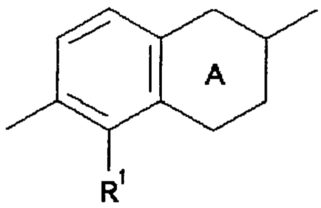

(wherein each symbol is as defined above) of the formula (I) means a group derived from a bicycle wherein formed by ring A and a benzene ring having one common bond (that is, they are condensed). The bond multiplicity for ring A and that for the benzene ring, involved in the bicycle formation, are the same. For example, when the moiety represented by
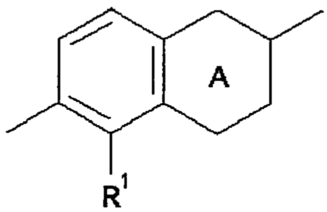

(wherein each symbol is as defined above) is the moiety represented by
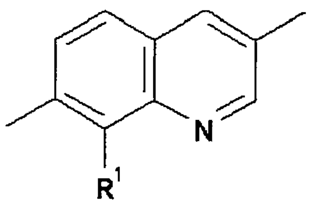

,

then ring A is "pyridine".
The "6-membered ring" of the "optionally further substituted 6-membered ring" for ring A is preferably

(1) a 6-membered aromatic heterocycle (preferably pyridine),
(2) a 6-membered non-aromatic heterocycle (preferably pyran),
(3) benzene,
(4) cyclohexene, or
(5) a cyclohexadiene (e.g., 2,4-cyclohexadiene),

more preferably a 6-membered aromatic heterocycle (preferably pyridine).
The "6-membered ring" of the "optionally further substituted 6-membered ring" for ring A optionally has 1 or 2 substituents at substitutable positions. Examples of the substituent include

(1) a $C_{3-10}$ cycloalkyl group (e.g., cyclopropyl, cyclohexyl);
(2) a $C_{6-14}$ aryl group (e.g., phenyl, naphthyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group optionally substituted by 1 to 3 halogen atoms, and
(d) a halogen atom;

(3) an aromatic heterocyclic group (e.g., thienyl, furyl, pyridyl, pyrazolyl, imidazolyl, tetrazolyl, oxazolyl, thiazolyl, oxadiazolyl, thiadiazolyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group optionally substituted by 1 to 3 halogen atoms, and
(d) a halogen atom;

(4) a non-aromatic heterocyclic group (e.g., tetrahydrofuryl, morpholinyl, thiomorpholinyl, piperidinyl, pyrrolidinyl, piperazinyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group optionally substituted by 1 to 3 halogen atoms, and
(d) a halogen atom;

(5) an amino group optionally mono- or di-substituted by substituent(s) selected from

(a) a $C_{1-6}$ alkyl group optionally substituted by 1 to 3 halogen atoms,
(b) a $C_{1-6}$ alkyl-carbonyl group optionally substituted by 1 to 3 halogen atoms, and
(c) a $C_{1-6}$ alkoxy-carbonyl group optionally substituted by 1 to 3 halogen atoms;

(6) a $C_{1-6}$ alkyl-carbonyl group optionally substituted by 1 to 3 halogen atoms;
(7) a $C_{1-6}$ alkoxy-carbonyl group optionally substituted by 1 to 3 substituents selected from

(a) a halogen atom, and
(b) a $C_{1-6}$ alkoxy group;

(8) a $C_{1-6}$ alkylsulfonyl group (e.g., methylsulfonyl, ethylsulfonyl, isopropylsulfonyl) optionally substituted by 1 to 3 halogen atoms;
(9) a carbamoyl group optionally mono- or di-substituted by $C_{1-6}$ alkyl group(s) optionally substituted by 1 to 3 halogen atoms;
(10) a thiocarbamoyl group optionally mono- or di-substituted by $C_{1-6}$ alkyl group(s) optionally substituted by 1 to 3 halogen atoms;
(11) a sulfamoyl group optionally mono- or di-substituted by $C_{1-6}$ alkyl group(s) optionally substituted by 1 to 3 halogen atoms;
(12) a carboxy group;
(13) a hydroxy group;
(14) a $C_{1-6}$ alkoxy group optionally substituted by 1 to 3 substituents selected from

(a) a halogen atom,
(b) a carboxy group,
(c) a $C_{1-6}$ alkoxy group,
(d) a $C_{1-6}$ alkoxy-carbonyl group,
(e) an amino group optionally mono- or di-substituted by substituent(s) selected from a $C_{1-6}$ alkyl group and a $C_{1-6}$ alkoxy-carbonyl group,
(f) a $C_{6-14}$ aryl group (e.g., phenyl),
(g) a $C_{3-10}$ cycloalkyl group (e.g., cyclopropyl, cyclobutyl), and
(h) an aromatic heterocyclic group (e.g., thienyl, furyl);

(15) a $C_{2-6}$ alkenyloxy group (e.g., ethenyloxy) optionally substituted by 1 to 3 halogen atoms;
(16) a $C_{6-14}$ aryloxy group (e.g., phenyloxy, naphthyloxy);
(17) a $C_{1-6}$ alkyl-carbonyloxy group (e.g., acetyloxy, tert-butylcarbonyloxy);

(18) a $C_{6-14}$ aryl-carbonyl group (e.g., benzoyl) optionally substituted by 1 to 3 substituents selected from

(a) a halogen atom, and
(b) a $C_{1-6}$ alkyl group optionally substituted by 1 to 3 halogen atoms;

(19) a non-aromatic heterocyclylcarbonyl group (e.g., pyrrolidinylcarbonyl, morpholinylcarbonyl, 1,1-dioxidothiomorpholinylcarbonyl) optionally substituted by 1 to 3 substituents selected from a $C_{1-6}$ alkyl group optionally substituted by 1 to 3 halogen atoms;
(20) a mercapto group;
(21) a $C_{1-6}$ alkylthio group (e.g., methylthio, ethylthio) optionally substituted by 1 to 3 halogen atoms;
(22) a $C_{7-13}$ aralkylthio group (e.g., benzylthio);
(23) a $C_{6-14}$ arylthio group (e.g., phenylthio, naphthylthio);
(24) a cyano group;
(25) a nitro group;
(26) a halogen atom;
(27) a $C_{1-3}$ alkylenedioxy group;
(28) an aromatic heterocyclylcarbonyl group (e.g., pyrazolylcarbonyl, pyrazinylcarbonyl, isoxazolylcarbonyl, pyridylcarbonyl, thiazolylcarbonyl) optionally substituted by 1 to 3 $C_{1-6}$ alkyl groups optionally substituted by 1 to 3 halogen atoms ;
(29) a hydroxyimino group optionally substituted by $C_{1-6}$ alkyl group(s) optionally substituted by 1 to 3 $C_{6-14}$ aryl groups (e.g., phenyl);
(30) a $C_{1-6}$ alkyl group optionally substituted by 1 to 3 substituents selected from

(a) a halogen atom,
(b) a carboxy group,
(c) a hydroxy group,
(d) a $C_{1-6}$ alkoxy-carbonyl group,
(e) a $C_{1-6}$ alkoxy group,
(f) an amino group optionally mono- or di-substituted by $C_{1-6}$ alkyl group(s), and
(g) a $C_{3-10}$ cycloalkyloxy group (preferably cyclopropyloxy);

(31) a $C_{2-6}$ alkenyl group (e.g., ethenyl) optionally substituted by 1 to 3 substituents selected from

(a) a halogen atom,
(b) a carboxy group,
(c) a hydroxy group,
(d) a $C_{1-6}$ alkoxy-carbonyl group,
(e) a $C_{1-6}$ alkoxy group,
(f) an amino group optionally mono- or di-substituted by $C_{1-6}$ alkyl group(s), and
(g) a $C_{3-10}$ cycloalkyl group (e.g., cyclopropyl);

(32) a $C_{2-6}$ alkynyl group (e.g., ethynyl) optionally substituted by 1 to 3 substituents selected from

(a) a $C_{3-10}$ cycloalkyl group (e.g., cyclopropyl, cyclobutyl);

(33) a $C_{7-13}$ aralkyl group (e.g., benzyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group, and
(d) a halogen atom;

and the like.

When the "6-membered ring" of the "optionally further substituted 6-membered ring" for ring A is a non-aromatic ring, it optionally has, as a substituent, an oxo group besides the above-mentioned substituents.
When the number of the substituents is not less than 2, the respective substituents may be the same or different.
When the "6-membered ring" of the "optionally further substituted 6-membered ring" for ring A is an aromatic

ring, preferable examples of the substituent include

(1) a $C_{1-6}$ alkyl group

and the like.
When the "6-membered ring" of the "optionally further substituted 6-membered ring" for ring A is a non-aromatic ring, preferable examples of the substituent include

(1) a $C_{1-6}$ alkyl group,
(2) an oxo group

and the like.
Ring A is preferably

(1) an optionally further substituted 6-membered aromatic heterocycle (preferably pyridine),
(2) an optionally further substituted 6-membered non-aromatic heterocycle (preferably pyran),
(3) an optionally further substituted benzene,
(4) an optionally further substituted cyclohexene,
(5) an optionally further substituted cyclohexadiene (preferably 2,4-cyclohexadiene),

or the like (of these, an optionally further substituted 6-membered aromatic heterocycle (preferably pyridine) is preferable), more preferably

(1) a 6-membered aromatic heterocycle (preferably pyridine) optionally substituted by 1 or 2 substituents selected from

(a) a $C_{1-6}$ alkyl group,

(2) a 6-membered non-aromatic heterocycle (preferably pyran) optionally substituted by 1 or 2 substituents selected from

(a) a $C_{1-6}$ alkyl group, and
(b) an oxo group,

(3) benzene optionally substituted by 1 or 2 substituents selected from

(a) a $C_{1-6}$ alkyl group,

(4) cyclohexene optionally substituted by 1 or 2 substituents selected from

(a) a $C_{1-6}$ alkyl group, or

(5) a cyclohexadiene (preferably 2,4-cyclohexadiene) optionally substituted by 1 or 2 substituents selected from

(a) a $C_{1-6}$ alkyl group

(of these,

(1) a 6-membered aromatic heterocycle (preferably pyridine) optionally substituted by 1 or 2 substituents selected from

(a) a $C_{1-6}$ alkyl group

is preferable).

Ring A is particularly preferably unsubstituted pyridine.
$R^1$ is a hydrogen atom, a halogen atom (preferably a fluorine atom) or a $C_{1-6}$ alkyl group (preferably methyl).
$R^2$ is a hydrogen atom or a $C_{1-6}$ alkyl group (preferably methyl).

$R^3$ is
a group represented by the formula: $-Y-S(O)_{m1}-R^{4a}$
wherein
Y is a bond or NH;
m1 is an integer of 1 or 2; and
$R^{4a}$ is a $C_{1-6}$ alkyl group (preferably methyl) optionally substituted by 1 to 3 halogen atoms (preferably fluorine),
or
a cyclic group represented by the formula:
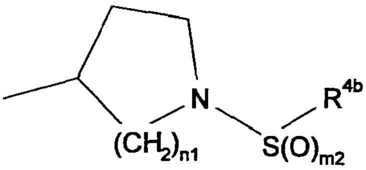
,
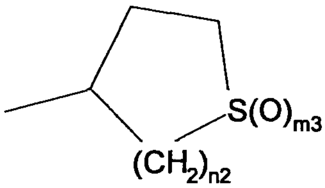
, or
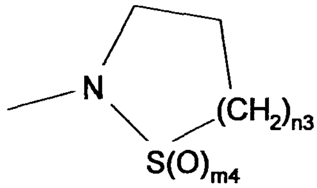

wherein m2, m3, m4, n1, n2 and n3 are each independently an integer of 1 or 2; and
$R^{4b}$ is a $C_{1-6}$ alkyl group (preferably methyl) optionally substituted by 1 to 3 halogen atoms (preferably fluorine) (the ring moiety of the cyclic group is optionally further substituted).

The "$C_{1-6}$ alkyl group optionally substituted by 1 to 3 halogen atoms" for $R^{4a}$ is preferably methyl, trifluoromethyl or the like.
The "$C_{1-6}$ alkyl group optionally substituted by 1 to 3 halogen atoms" for $R^{4b}$ is preferably methyl, trifluoromethyl or the like.
The ring moiety of the cyclic group represented by the formula:
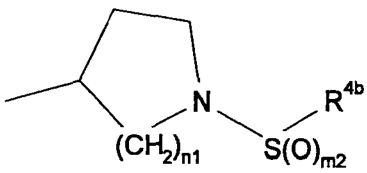
,
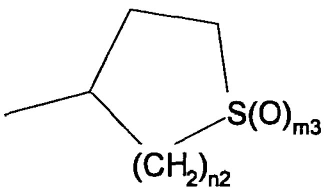
, or
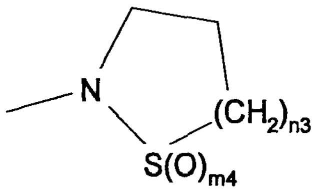

wherein each symbol is as defined above, for $R^3$, optionally has additional 1 to 3 substituents at substitutable positions. Examples of the additional substituent include those similar to the substituent that the aforementioned "6-membered ring" of the "optionally further substituted 6-membered ring" for ring A optionally has.
When the number of the substituents is not less than 2, the respective substituents may be the same or different.
The substituent for the ring moiety is preferably a hydroxy group.
The cyclic group for $R^3$ is preferably a group represented by the formula:
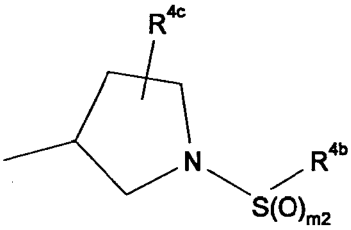
,
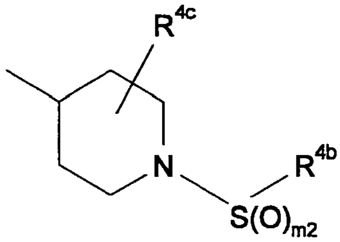
,
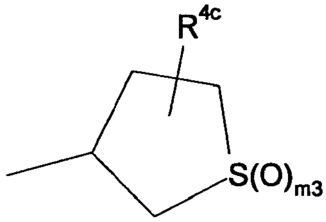
,

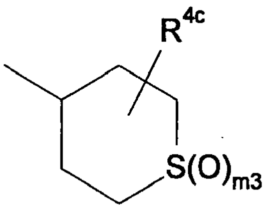,
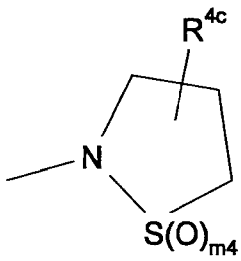, or
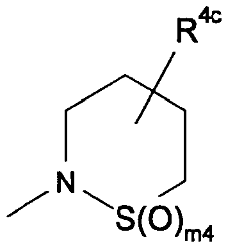

wherein m2, m3, m4 and $R^{4b}$ is as defined above, and $R^{4c}$ is a hydrogen atom or a substituent, more preferably a group represented by the formula:

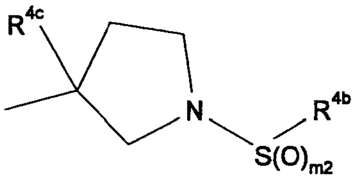,
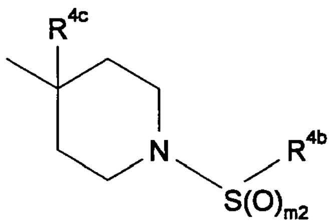,
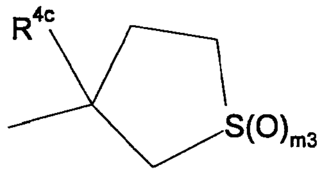,

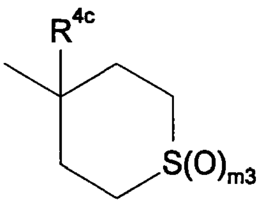,
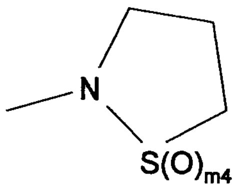, or
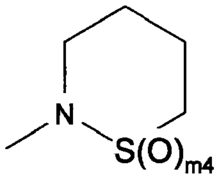

wherein each symbol is as defined above.

$R^3$ is preferably

a group represented by the formula: $-Y-S(O)_{m1}-R^{4a}$ wherein

Y is a bond;

m1 is an integer of 1 or 2; and

$R^{4a}$ is a $C_{1-6}$ alkyl group optionally substituted by 1 to 3 halogen atoms, or

a cyclic group represented by the formula:

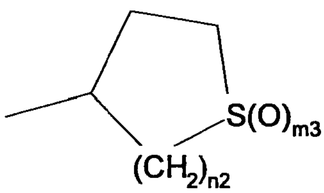

wherein

m3 and n2 are each independently an integer of 1 or 2

(the ring moiety of the cyclic group is optionally further substituted).

$R^5$ is optionally further substituted 5- or 6-membered cyclic group.
Examples of the "5- or 6-membered cyclic group" of the "optionally further substituted 5- or 6-membered cyclic group" for $R^5$ include phenyl, a $C_{5-6}$ cycloalkyl group, a $C_{5-6}$ cycloalkenyl group, a $C_{5-6}$ cycloalkadienyl group, a 5- or 6-membered aromatic heterocyclic group, a 5- or 6-membered non-aromatic heterocyclic group and the like.
Examples of the $C_{5-6}$ cycloalkyl group include cyclopentyl and cyclohexyl.
Examples of the $C_{5-6}$ cycloalkenyl group include 2-cyclopenten-1-yl, 3-cyclopenten-1-yl, 2-cyclohexen-1-yl, 3-cyclohexen-1-yl and the like.
Examples of the $C_{5-6}$ cycloalkadienyl group include 2,4-cyclopentadien-1-yl, 2,4-cyclohexadien-1-yl, 2,5-cyclohexadien-1-yl and the like.
Examples of the 5- or 6-membered aromatic heterocyclic group include a 5- or 6-membered aromatic heterocyclic group containing, as a ring-constituting atom besides carbon atom, 1 to 4 hetero atoms selected from an oxygen atom, a sulfur atom (the sulfur atom is optionally oxidized) and a nitrogen atom.
Preferable specific examples of the 5- or 6-membered aromatic heterocyclic group include furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 4-isothiazolyl), oxazolyl (e.g., 2-oxazolyl, 4-oxazolyl, 5-oxazolyl), isoxazolyl, oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl, 1,3,5-triazin-1-yl) and the like.
Examples of the 5- or 6-membered non-aromatic heterocycle include a 5- or 6-membered non-aromatic heterocyclic group containing, as a ring-constituting atom besides carbon atom, 1 to 4 hetero atoms selected from an oxygen atom, a sulfur atom (the sulfur atom is optionally oxidized) and a nitrogen atom.
Preferable specific examples of the 5- or 6-membered non-aromatic heterocyclic group include tetrahydrofuryl (e.g., 2-tetrahydrofuryl), dihydropyrrolyl (e.g., 2,3-dihydro-1H-pyrrol-1-yl), pyrrolidinyl (e.g., 1-pyrrolidinyl), 1,1-dioxidotetrahydrothienyl (e.g., 1,1-dioxidotetrahydro-3-thienyl), piperidinyl (e.g., piperidino), morpholinyl (e.g., morpholino), thiomorpholinyl (e.g., thiomorpholino), 1,1-dioxidothiomorpholinyl (e.g., 1,1-dioxidothiomorpholino), piperazinyl (e.g., 1-piperazinyl), hexamethyleniminyl (e.g., hexamethylenimin-1-yl), oxazolinyl (e.g., 2,5-dihydrooxazol-3-yl, 3,4-dihydrooxazol-3-yl), thiazolinyl (e.g., 2,5-dihydrothiazol-3-yl, 3,4-dihydrothiazol-3-yl), imidazolinyl (e.g., 2-imidazolin-3-yl), oxazolidinyl (e.g., oxazolidin-3-yl), thiazolidinyl (e.g., thiazolidin-3-yl), imidazolidinyl (e.g., imidazolidin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), thioxooxazolidinyl (e.g., 2-thioxo-1,3-oxazolidin-5-yl), tetrahydropyranyl (e.g., 4-tetrahydropyranyl), tetrahydrothiopyranyl (e.g., 4-tetrahydrothiopyranyl), 1,1-dioxidotetrahydrothiopyranyl (e.g., 1,1-dioxidotetrahydrothiopyran-4-yl), pyrazolinyl (e.g., pyrazolin-3-yl), pyrazolidinyl (e.g., pyrazolidin-1-yl), oxotetrahydropyridazinyl (e.g., 3-oxo-2,3,4,5-tetrahydropyridazin-4-yl) and the like.
The "5- or 6-membered cyclic group" of the "optionally further substituted 5- or 6-membered cyclic group" for $R^5$ is preferably a 6-membered cyclic group, more preferably

(1) a phenyl group,
(2) a 6-membered non-aromatic heterocyclic group (preferably piperidinyl),
(3) a cyclohexyl group

or the like, further more preferably a phenyl group.
The "5- or 6-membered cyclic group" of the "optionally further substituted 5- or 6-membered cyclic group" for $R^5$ optionally has additional 1 to 3 substituents at substitutable positions. Examples of the additional substituent include those similar to the substituent that the aforementioned "6-membered ring" of the "optionally further substituted 6-membered ring" for ring A optionally has.
When the number of the substituents is not less than 2, the respective substituents may be the same or different.
When the "5- or 6-membered cyclic group" of the "optionally further substituted 5- or 6-membered cyclic group" for $R^5$ is an aromatic ring group, preferable examples of the substituent include

(1) a halogen atom (preferably fluorine, chlorine);
(2) a $C_{1-6}$ alkyl group (preferably methyl) optionally substituted by 1 to 3 substituents selected from

(a) a halogen atom (preferably fluorine), and
(b) a $C_{3-10}$ cycloalkyloxy group (preferably cyclopropyloxy);

(3) a $C_{2-6}$ alkynyl group (preferably ethynyl) optionally substituted by 1 to 3 substituents selected from

(a) a $C_{3-10}$ cycloalkyl group (preferably cyclopropyl, cyclobutyl);

(4) a $C_{1-6}$ alkoxy group (preferably methoxy, ethoxy, propoxy) optionally substituted by 1 to 3 substituents selected from

(a) a halogen atom (preferably fluorine),
(b) a $C_{3-10}$ cycloalkyl group (preferably cyclopropyl, cyclobutyl), and
(c) an aromatic heterocyclic group (preferably thienyl, furyl);

(5) a $C_{2-6}$ alkenyl group (preferably ethenyl) optionally substituted by 1 to 3 substituents selected from (a) a $C_{3-10}$ cycloalkyl group (preferably cyclopropyl);

and the like.

When the "5- or 6-membered cyclic group" of the "optionally further substituted 5- or 6-membered cyclic group" for $R^5$ is a non-aromatic ring group, preferable examples of the substituent include

(1) a hydroxy group;
(2) a phenyl group optionally substituted by 1 to 3 substituents selected from

(a) a halogen atom (preferably fluorine, chlorine), and
(b) a $C_{1-6}$ alkyl group (preferably methyl) optionally substituted by 1 to 3 halogen atoms (preferably fluorine)

and the like.

$R^5$ is preferably

(1) an optionally further substituted phenyl group,
(2) an optionally further substituted 6-membered non-aromatic heterocyclic group (preferably piperidinyl), or
(3) an optionally further substituted cyclohexyl group, (of these, an optionally further substituted phenyl group is preferable),

more preferably
a group represented by the formula:
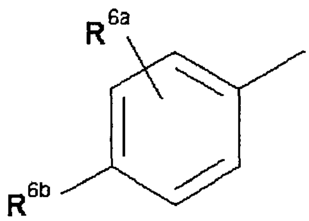

wherein

$R^{6a}$ is

(1) a hydrogen atom,
(2) a halogen atom (preferably fluorine, chlorine), or
(3) a $C_{1-6}$ alkyl group (preferably methyl) optionally substituted by 1 to 3 halogen atoms (preferably fluorine); and

$R^{6b}$ is

(1) a hydrogen atom;
(2) a $C_{2-6}$ alkynyl group (preferably ethynyl) optionally substituted by 1 to 3 substituents selected from

(a) a $C_{3-10}$ cycloalkyl group (preferably cyclopropyl, cyclobutyl),

(3) a $C_{1-6}$ alkoxy group (preferably methoxy, ethoxy, propoxy) optionally substituted by 1 to 3 substituents selected from

(a) a halogen atom (preferably fluorine),
(b) a $C_{3-10}$ cycloalkyl group (preferably cyclopropyl, cyclobutyl), and
(c) an aromatic heterocyclic group (preferably thienyl, furyl),

(4) a $C_{1-6}$ alkyl group (preferably methyl) optionally substituted by 1 to 3 substituents selected from

(a) a $C_{3-10}$ cycloalkyloxy group (preferably cyclopropyloxy), or

(5) a $C_{2-6}$ alkenyl group (preferably ethenyl) optionally substituted by 1 to 3 substituents selected from

(a) a $C_{3-10}$ cycloalkyl group (preferably cyclopropyl), or a group represented by the formula:

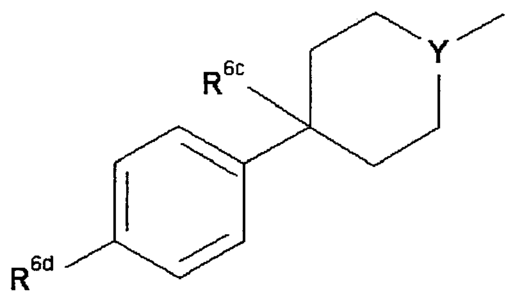

wherein

Y is CH or N;
$R^{6c}$ is

(1) a hydrogen atom, or
(2) a hydroxy group; and

$R^{6d}$ is

(1) a hydrogen atom,
(2) a halogen atom (preferably fluorine, chlorine), or
(3) a $C_{1-6}$ alkyl group (preferably methyl) optionally substituted by 1 to 3 halogen atoms (preferably fluorine).

$R^5$ is more preferably a group represented by the formula:
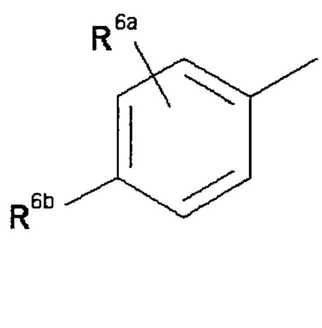

wherein

$R^{6a}$ is

(1) a hydrogen atom,
(2) a halogen atom (preferably fluorine, chlorine), or
(3) a $C_{1-6}$ alkyl group (preferably methyl) optionally substituted by 1 to 3 halogen atoms (preferably fluorine); and

$R^{6b}$ is

(1) a hydrogen atom;
(2) a $C_{2-6}$ alkynyl group (preferably ethynyl) optionally substituted by 1 to 3 substituents selected from

(a) a $C_{3-10}$ cycloalkyl group (preferably cyclopropyl, cyclobutyl),

(3) a $C_{1-6}$ alkoxy group (preferably methoxy, ethoxy, propoxy) optionally substituted by 1 to 3 substituents selected from

(a) a halogen atom (preferably fluorine),
(b) a $C_{3-10}$ cycloalkyl group (preferably cyclopropyl, cyclobutyl), and
(c) an aromatic heterocyclic group (preferably thienyl, furyl),

(4) a $C_{1-6}$ alkyl group (preferably methyl) optionally substituted by 1 to 3 substituents selected from

(a) a $C_{3-10}$ cycloalkyloxy group (preferably cyclopropyloxy), or

(5) a $C_{2-6}$ alkenyl group (preferably ethenyl) optionally substituted by 1 to 3 substituents selected from

(a) a $C_{3-10}$ cycloalkyl group (preferably cyclopropyl).

$X^1$ is a bond or a $C_{1-6}$ alkylene group.
Examples of the "$C_{1-6}$ alkylene group" for $X^1$ include $-CH_2-$, $-(CH_2)_2-$, $-(CH_2)_3-$, $-(CH_2)_4-$, $-(CH_2)_5-$, $-(CH_2)_6-$, $-CH(CH_3)-$, $-CH(C_2H5)-$, $-CH(C_3H_7)-$, $-CH(i\text{-}C_3H_7)-$, $-CH(CH_3)-CH_2-$, $-CH_2-CH(CH_3)-$, $-CH(CH_3)-(CH_2)_2-$, $-(CH_2)_2-CH(CH_3)-$, $-CH_2-CH(CH_3)-CH_2-$, $-C(CH_3)_2-$. $-(CH(CH_3))_2-$, $-CH_2-CH(CH_3)-$, $-CH_2-C(CH_3)_2-$, $-CH_2-C(CH_3)_2-CH_2-$ and the like.
$X^1$ is preferably a bond.
$X^2$ is a bond or a $C_{1-6}$ alkylene group.
Examples of the "$C_{1-6}$ alkylene group" for $X^2$ include those similar to the aforementioned "$C_{1-6}$ alkylene group" for $X^1$.
The $C_{1-6}$ alkylene group for $X^2$ is preferably $-CH_2-$, $-(CH_2)_2-$, $-(CH_2)_3-$, $-(CH_2)_4-$ or $-CH_2-C(CH_3)_2-CH_2-$.
Compound (I) is preferably the following compound.

[Compound (A)]

Compound (I) wherein

ring A is

(1) an optionally further substituted 6-membered aromatic heterocycle (preferably pyridine),
(2) an optionally further substituted 6-membered non-aromatic heterocycle (preferably pyran),
(3) an optionally further substituted benzene,
(4) an optionally further substituted cyclohexene, or
(5) an optionally further substituted cyclohexadiene (preferably 2,4-cyclohexadiene);

$R^1$ is a hydrogen atom, a halogen atom (preferably a fluorine atom) or a $C_{1-6}$ alkyl group (preferably methyl);
$R^2$ is a hydrogen atom or a $C_{1-6}$ alkyl group (preferably methyl); $R^3$ is
a group represented by the formula: $-Y-S(O)_{m1}-R^{4a}$
wherein

Y is a bond or NH;
m1 is an integer of 1 or 2; and

$R^{4a}$ is a $C_{1-6}$ alkyl group (preferably methyl) optionally substituted by 1 to 3 halogen atoms (preferably fluorine),

or
a group represented by the formula:

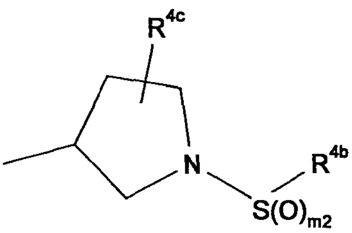
,
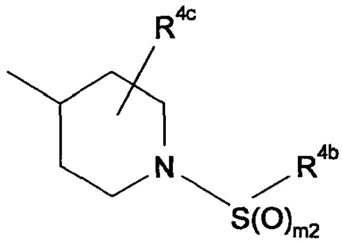
,
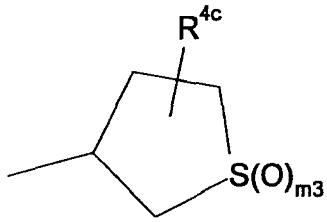
,

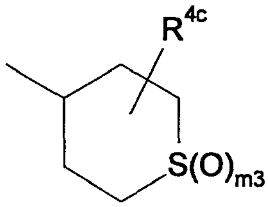
,
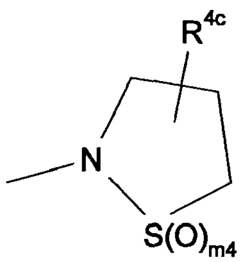
, or
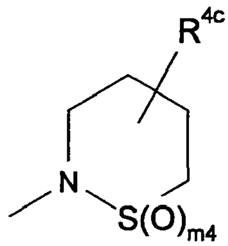

wherein

m2, m3 and m4 are each independently an integer of 1 or 2;
$R^{4b}$ is a $C_{1-6}$ alkyl group (preferably methyl) optionally substituted by 1 to 3 halogen atoms (preferably fluorine); and
$R^{4c}$ is a hydrogen atom or a substituent;

$R^5$ is

(1) an optionally further substituted phenyl group,
(2) an optionally further substituted 6-membered non-aromatic heterocyclic group (preferably piperidinyl), or
(3) an optionally further substituted cyclohexyl group;

$X^1$ is a bond; and
$X^2$ is a bond or a $C_{1-6}$ alkylene group (preferably $-CH_2-$, $-(CH_2)_2-$, $-(CH_2)_3-$, $-(CH_2)_4-$, $-CH_2-C(CH_3)_2-CH_2-$.

[Compound (B)]

Compound (I) wherein

ring A is

(1) a 6-membered aromatic heterocycle (preferably pyridine) optionally substituted by 1 or 2 substituents selected from

(a) a $C_{1-6}$ alkyl group,

(2) a 6-membered non-aromatic heterocycle (preferably pyran) optionally substituted by 1 or 2 substituents selected from

(a) a $C_{1-6}$ alkyl group, and
(b) an oxo group,

(3) benzene optionally substituted by 1 or 2 substituents selected from

(a) a $C_{1-6}$ alkyl group,

(4) cyclohexene optionally substituted by 1 or 2 substituents selected from

(a) a $C_{1-6}$ alkyl group, or

(5) a cyclohexadiene (preferably 2,4-cyclohexadiene) optionally substituted by 1 or 2 substituents selected from

(a) a $C_{1-6}$ alkyl group;

$R^1$ is a hydrogen atom, a halogen atom (preferably a fluorine atom) or a $C_{1-6}$ alkyl group (preferably methyl);
$R^2$ is a hydrogen atom or a $C_{1-6}$ alkyl group (preferably methyl);
$R^3$ is
a group represented by the formula: $-Y-S(O)_{m1}-R^{4a}$
wherein

Y is a bond or NH;
m1 is an integer of 1 or 2; and
$R^{4a}$ is a $C_{1-6}$ alkyl group (preferably methyl) optionally substituted by 1 to 3 halogen atoms (preferably fluorine), or,

a group represented by the formula:

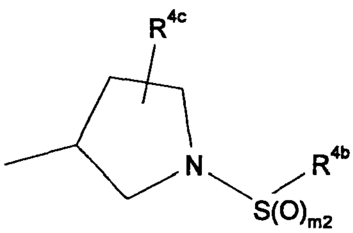,
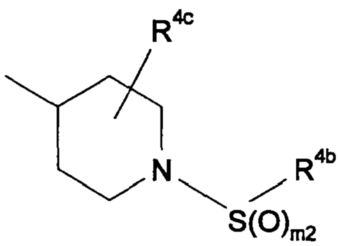,
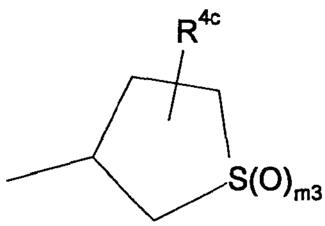,

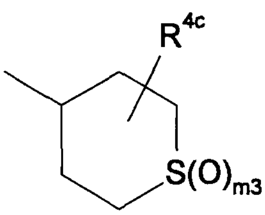,
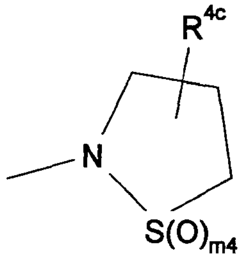, or
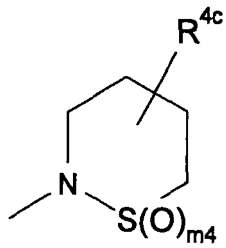

wherein

m2, m3 and m4 are each independently an integer of 1 or 2;
$R^{4b}$ is a $C_{1-6}$ alkyl group (preferably methyl) optionally substituted by 1 to 3 halogen atoms (preferably fluorine); and
$R^{4c}$ is a hydrogen atom or a hydroxy group;

$R^5$ is

a group represented by the formula:
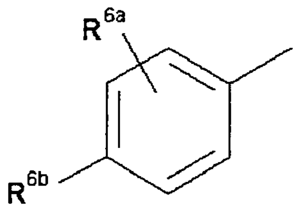

wherein

$R^{6a}$ is

(1) a hydrogen atom,
(2) a halogen atom (preferably fluorine, chlorine), or
(3) a $C_{1-6}$ alkyl group (preferably methyl) optionally substituted by 1 to 3 halogen atoms (preferably fluorine); and

$R^{6b}$ is

(1) a hydrogen atom;
(2) a $C_{2-6}$ alkynyl group (preferably ethynyl) optionally substituted by 1 to 3 substituents selected from

(a) a $C_{3-10}$ cycloalkyl group (preferably cyclopropyl, cyclobutyl),

(3) a $C_{1-6}$ alkoxy group (preferably methoxy, ethoxy, propoxy) optionally substituted by 1 to 3 substituents selected from

(a) a halogen atom (preferably fluorine),
(b) a $C_{3-10}$ cycloalkyl group (preferably cyclopropyl, cyclobutyl), and
(c) an aromatic heterocyclic group (preferably thienyl, furyl),

(4) a $C_{1-6}$ alkyl group (preferably methyl) optionally substituted by 1 to 3 substituents selected from

(a) a $C_{3-10}$ cycloalkyloxy group (preferably cyclopropyloxy), or

(5) a $C_{2-6}$ alkenyl group (preferably ethenyl) optionally substituted by 1 to 3 substituents selected from

(a) a $C_{3-10}$ cycloalkyl group (preferably cyclopropyl), or a group represented by the formula:

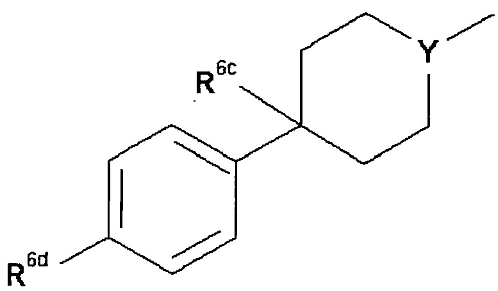

wherein

Y is CH or N;
$R^{6c}$ is

(1) a hydrogen atom, or
(2) a hydroxy group; and

$R^{6d}$ is

(1) a hydrogen atom,
(2) a halogen atom (preferably fluorine, chlorine), or
(3) a $C_{1-6}$ alkyl group (preferably methyl) optionally substituted by 1 to 3 halogen atoms (preferably fluorine);

$X^1$ is a bond; and
$X^2$ is a bond or a $C_{1-6}$ alkylene group (preferably $-CH_2-$, $-(CH_2)_2-$. $-(CH_2)_3-$, $-(CH_2)_4-$, $-CH_2-C(CH_3)_2-CH_2-$).

[Compound (C)]

ring A is an optionally further substituted 6-membered aromatic heterocycle (preferably pyridine);
$R^1$ is a hydrogen atom, a halogen atom (preferably a fluorine atom) or a $C_{1-6}$ alkyl group (preferably methyl);
$R^2$ is a hydrogen atom or a $C_{1-6}$ alkyl group (preferably methyl);
$R^3$ is a group represented by formula: $-Y-S(O)_{m1}-R^{4a}$

wherein Y is a bond;
m1 is an integer of 1 or 2; and
$R^{4a}$ is a $C_{1-6}$ alkyl group optionally substituted by 1 to 3 halogen atoms, or

a cyclic group represented by the formula:

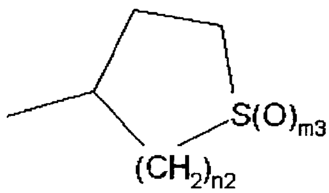

wherein

m3 and n2 are each independently an integer of 1 or 2
(the ring moiety of the cyclic group is optionally further substituted);

$R^5$ is an optionally further substituted phenyl group;
$X^1$ is a bond; and
$X^2$ is a bond or a $C_{1-6}$ alkylene group (preferably $-CH_2-$, $-(CH_2)_2-$. $-(CH_2)_3-$ , $-(CH_2)_4-$, $-CH_2-C(CH_3)_2-CH_2-$).

[Compound (D)]

ring A is pyridine;
$R^1$ is a hydrogen atom, a fluorine atom or methyl;
$R^2$ is a hydrogen atom or methyl;
$R^3$ is a group represented by the formula: $-Y-S(O)_{m1}-R^{4a}$

wherein

Y is a bond;
m1 is an integer of 1 or 2; and
$R^{4a}$ is a $C_{1-6}$ alkyl group optionally substituted by 1 to 3 halogen atoms, or

a cyclic group represented by the formula:

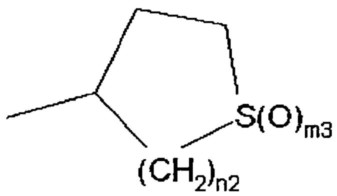

wherein

m3 and n2 are each independently an integer of 1 or 2; $R^5$ is a group represented by the formula:

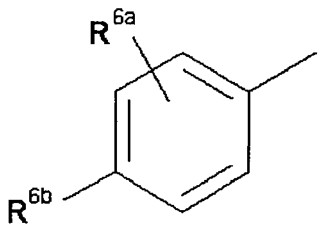

wherein

$R^{6a}$ is

(1) a hydrogen atom,
(2) a halogen atom (preferably fluorine, chlorine), or
(3) a $C_{1-6}$ alkyl group (preferably methyl) optionally substituted by 1 to 3 halogen atoms (preferably fluorine); and

$R^{6b}$ is

(1) a hydrogen atom;
(2) a $C_{2-6}$ alkynyl group (preferably ethynyl) optionally substituted by 1 to 3 substituents selected from

(a) a $C_{3-10}$ cycloalkyl group (preferably cyclopropyl, cyclobutyl),

(3) a $C_{1-6}$ alkoxy group (preferably methoxy, ethoxy, propoxy) optionally substituted by 1 to 3 substituents selected from

(a) a halogen atom (preferably fluorine),
(b) a $C_{3-10}$ cycloalkyl group (preferably cyclopropyl, cyclobutyl), and
(c) an aromatic heterocyclic group (preferably thienyl, furyl),

(4) a $C_{1-6}$ alkyl group (preferably methyl) optionally substituted by 1 to 3 substituents selected from

(a) a $C_{3-10}$ cycloalkyloxy group (preferably cyclopropyloxy), or

(5) a $C_{2-6}$ alkenyl group (preferably ethenyl) optionally substituted by 1 to 3 substituents selected from

(a) a $C_{3-10}$ cycloalkyl group (preferably cyclopropyl);

$X^1$is a bond; and
$X^2$ is a bond or a $C_{1-6}$ alkylene group (preferably $-CH_2-$, $-(CH_2)_2-$, $-(CH_2)_3-$, $-(CH_2)_4-$, $-CH_2-C(CH_3)_2-CH_2-$).

When compound (I) is in the form of a salt, examples thereof include salts with inorganic bases, ammonium salts, salts with organic bases, salts with inorganic acids, salts with organic acids, salts with basic or acidic amino acids and the like.
Preferable examples of the salts with inorganic bases include alkali metal salts such as sodium salt, potassium salt and the like; alkaline earth metal salts such as calcium salts, magnesium salts, barium salts and the like; aluminum salts, and the like.
Preferable examples of the salts with organic bases include salts with trimethylamine, triethylamine, pyridine, picoline, ethanolamine, diethanolamine, triethanolamine, dicyclohexylamine, N,N'-dibenzylethylenediamine and the like.
Preferable examples of the salts with inorganic acids include salts with hydrochloric acid, hydrobromic acid, nitric acid, sulfuric acid, phosphoric acid and the like.
Preferable examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.
Preferable examples of the salts with basic amino acids include salts with arginine, lysine, ornithine and the like.
Preferable examples of the salts with acidic amino acids include salts with aspartic acid, glutamic acid and the like.
Of these, pharmaceutically acceptable salts are preferable.
Compound (I) may be any of a non-solvate (e.g., anhydride) and a solvate (e.g., hydrate).
Moreover, compound (I) may be labeled with an isotope (e.g., $^{3}H$, $^{14}C$, $^{35}S$, $^{125}I$).
In addition, compound (I) may also a deuterium conversion form wherein $^{1}H$ has been converted to $^{2}H$(D).
When compound (I) contains an optical isomer, a stereoisomer, a regioisomer or a rotamer, these are also encompassed in compound (I), and can be obtained as a single product according to synthesis and separation methods known *per* se. For example, when compound (I) has an optical isomer, an optical isomer resolved from this compound is also encompassed in compound (I).
The optical isomer can be produced according to a method known per se.
A prodrug of compound (I) means a compound which is converted to compound (I) with a reaction due to an enzyme, an gastric acid, etc. under the physiological condition in the living body, that is, a compound which is converted to compound (I) by enzymatic oxidation, reduction, hydrolysis, etc; a compound which is converted to compound (I) by hydrolysis etc. due to gastric acid, etc. A prodrug for compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl) methoxycarbonylation, tetrahydrofuranylation, pyrrolidylmethylation, pivaloyloxymethylation or tert-butylation, etc.); a compound obtained by subjecting a hydroxy group in compound (I) to an acylation, alkylation, phosphorylation or boration (e.g., a compound obtained by subjecting a hydroxy group in compound (I) to an acetylation, palmitoylation, propanoylation, pivaloylation, succinylation, fumarylation, alanylation or dimethylaminomethylcarbonylation, etc.); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation, etc.) and the like. Any of these compounds can be produced from compound (I) by a method known *per se.*
A prodrug of compound (I) may also be one which is converted into compound (I) under a physiological condition, such as those described in IYAKUHIN no KAIHATSU (Development of Pharmaceuticals), Vol. 7, Design of Molecules, p.163-198, published by HIROKAWA SHOTEN (1990).
Compound (I) can be produced according to [Production Method 1] to [Production Method 6], which are described in detail below, or an analogous method thereto.
The compounds for the starting compound may be used in the form of a salt, respectively. As such salt, those exemplified as the salt of the aforementioned compound (I) can be used.
In the following [Production Method 1] to [Production Method 6], when alkylation reaction, hydrolysis reaction, amination reaction, amidation reaction, esterification reaction, etherification reaction, oxidation reaction, reduction reaction etc. are to be conducted, these reactions are carried out according to methods known *per se*, for example, those described in

Organic Functional Group Preparations, 2nd Ed., Academic Press Inc., 1989; Comprehensive Organic Transformations, VCH Publishers Inc., 1989; and the like.
The solvent used for each reaction is explained in the following.
Examples of the "alcohol solvent" include methanol, ethanol, propanol, 2-propanol, butanol, isobutanol, tert-butanol and the like.
Examples of the "ester solvent" include methyl acetate, ethyl acetate, n-butyl acetate, tert-butyl acetate and the like.
Examples of the "ether solvent" include diethyl ether, diisopropyl ether, tert-butyl methyl ether, tetrahydrofuran (THF), 1,4-dioxane, 1,2-dimethoxyethane and the like.
Examples of the "halogenated hydrocarbon solvent" include dichloromethane, chloroform, 1,2-dichloroethane, carbon tetrachloride and the like.
Examples of the "aromatic solvent" include benzene, toluene, xylene, pyridine and the like.
Examples of the "nitrile solvent" include acetonitrile, propionitrile and the like.
Examples of the "amide solvent" include N,N-dimethylformamide (DMF), N,N-dimethylacetamide (DMA), 1-methyl-2-pyrrolidinone (NMP) and the like.
Examples of the "ketone solvent" include acetone, methyl ethyl ketone and the like.
Examples of the "sulfoxide solvent" include dimethyl sulfoxide (DMSO) and the like.

[Production Method 1]

Compound (I) can be produced, for example, by subjecting compound (II) to the following amination reaction with compound (III).

(Amination Reaction)

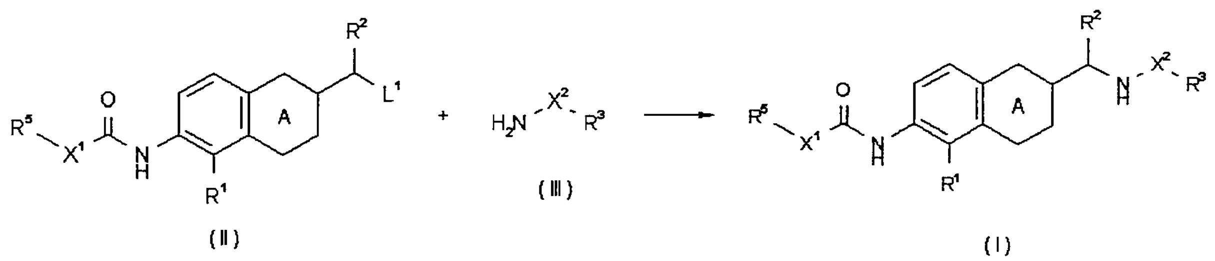

wherein $L^1$ is a leaving group, and other symbols are as defined above.
Examples of the "leaving group" for $L^1$ include a halogen atom (e.g., chlorine, bromine, iodine), $C_{1-6}$ alkylsulfonyloxy optionally substituted by 1 to 3 halogens (e.g., methanesulfonyloxy, ethanesulfonyloxy, trifluoromethanesulfonyloxy), optionally substituted $C_{6-10}$ arylsulfonyloxy, hydroxy and the like.
Examples of the "substituent" of the "optionally substituted $C_{6-10}$ arylsulfonyloxy" include a halogen atom (e.g., chlorine, bromine, iodine), $C_{1-6}$ alkyl optionally substituted by 1 to 3 halogens, $C_{1-6}$ alkoxy optionally substituted by 1 to 3 halogens and the like. The number of substituent is, for example, 1 to 3. Specific examples of the "optionally substituted $C_{6-10}$ arylsulfonyloxy" include benzenesulfonyloxy, p-toluenesulfonyloxy, 1-naphthalenesulfonyloxy, 2-naphthalenesulfonyloxy and the like.
$L^1$is preferably a halogen atom, methanesulfonyloxy, trifluoromethanesulfonyloxy, p-toluenesulfonyloxy or the like.
This reaction is generally carried out in an inert solvent.
Examples of the "inert solvent" include alcohol solvents, ester solvents, ether solvents, halogenated hydrocarbon solvents, aromatic solvents, nitrile solvents, amide solvents, ketone solvents, sulfoxide solvents, water and the like. These solvents may be used in a mixture of two or more kinds thereof at an appropriate ratio. Of these, acetonitrile, N,N-dimethylformamide (DMF), 1-methyl-2-pyrrolidinone (NMP), N,N-dimethylacetamide (DMA), acetone, ethanol, pyridine and the like are preferable.
The amount of compound (III) to be used is generally 1 equivalent to 100 equivalents, relative to compound (II). Alternatively, an excess amount of compound (III) may be used as a reaction solvent.
The reaction temperature is generally about -20°C to 200°C, preferably room temperature to 100°C. The reaction time is, for example, about 0.5 hr to 1 day.
This reaction may be carried out in the copresence of a base as necessary.
Examples of the "base" include the following:

1) strong bases such as alkali metal or alkaline earth metal hydrides (e.g., lithium hydride, sodium hydride, potassium hydride, calcium hydride), alkali metal or alkaline earth metal amides (e.g., lithium amide, sodium amide, lithium diisopropylamide, lithium dicyclohexylamide, lithium hexamethyldisilazide, sodium hexamethyldisilazide, potassium hexamethyldisilazide), alkali metal or alkaline earth metal $C_{1-6}$ alkoxides (e.g., sodium methoxide, sodium ethoxide, potassium tert-butoxide), and the like;
2) inorganic bases such as alkali metal or alkaline earth metal hydroxides (e.g., sodium hydroxide, potassium hydroxide, lithium hydroxide, barium hydroxide), alkali metal or alkaline earth metal carbonates (e.g., sodium carbonate, potassium carbonate, cesium carbonate), alkali metal hydrogen carbonates (e.g., sodium hydrogen carbonate, potassium hydrogen carbonate) and the like;
3) organic bases such as amines (e.g., triethylamine, N,N-diisopropylethylamine, N-methylmorpholine, 4-dimethylaminopyridine, DBU (1,8-diazabicyclo[5.4.0]undec-7-ene), DBN (1,5-diazabicyclo[4.3.0]non-5-ene)), basic heterocyclic compounds (e.g., pyridine, imidazole, 2,6-lutidine) and the like.

Of the above-mentioned bases, sodium carbonate, potassium carbonate, cesium carbonate, sodium hydrogen carbonate, triethylamine, N,N-diisopropylethylamine, pyridine and the like are preferable.
The amount of the base to be used is generally 0.1 to 100 equivalents, preferably 1 to 10 equivalents, relative to compound (II).
The aforementioned compound (II) can be produced according to a method known per se, for example, the method described in WO 2001/082925, WO 2003/035624 or the like.
The aforementioned compound (III) can be produced according to a method known per se.

[Production Method 2]

Compound (I) can also be produced, for example, by subjecting compound (IIa) to the following amination reaction of with compound (IV) (Step 1A), and then subjecting the resulting compound to deprotection reaction (Step 1B).

(Amination Reaction, Deprotection Reaction)

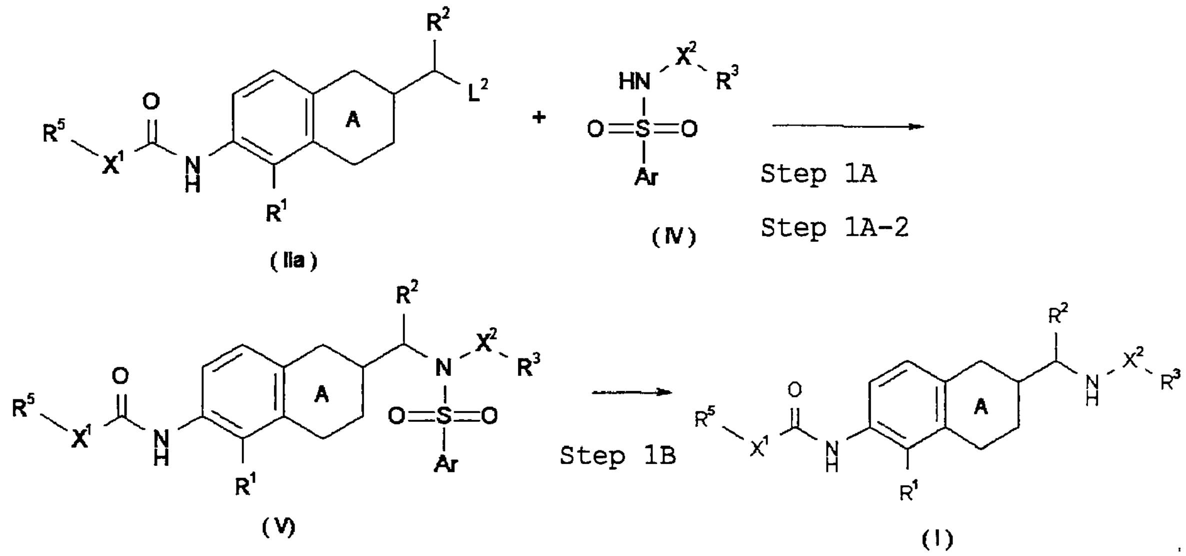

(IIa) (IV) (V) (I)

wherein $L^2$ is a leaving group, Ar is an optionally further substituted aromatic ring, and other symbols are as defined above.
Examples of the "leaving group" for $L^2$ include those similar to the above-mentioned "leaving group" for $L^1$.
$L^2$ is preferably a halogen atom, methanesulfonyloxy, trifluoromethanesulfonyloxy, p-toluenesulfonyloxy, hydroxy or the like.
Specific examples of the "aromatic ring" of the "optionally further substituted aromatic ring" for Ar include a $C_{6-14}$ aromatic hydrocarbon (e.g., benzene, naphthalene), a 5- or 6-membered aromatic heterocycle (e.g., pyrrole, imidazole, pyrazole, pyridine) and the like. Of these, benzene is preferable.

Examples of the "substituent" of the "optionally further substituted aromatic ring" include those similar to the substituent for aforementioned "optionally further substituted 6-membered ring" for ring A. Of these, nitro, cyano, a halogen atom and the like are preferable. The number of the substituent is, for example, 1 to 5. When the number of the substituents is not less than 2, the respective substituents may be the same or different.
The "optionally substituted aromatic ring" is preferably 2-nitrobenzene, 2,4-dinitrobenzene or the like.

<Step 1A> (Amination Reaction)

This reaction is generally carried out in an inert solvent.
Examples of the "inert solvent" include those similar to the solvent used in the aforementioned Production Method 1. Of these, acetonitrile, N,N-dimethylformamide (DMF), 1-methyl-2-pyrrolidinone (NMP), N,N-dimethylacetamide (DMA), tetrahydrofuran (THF), acetone, ethanol, pyridine and the like are preferable.
The amount of compound (IV) to be used is generally 1 equivalent to 100 equivalents, relative to compound (IIa).
The reaction temperature is generally about -20°C to 200°C, preferably room temperature to 100°C. The reaction time is, for example, about 0.5 hr to 1 day.
This reaction may be carried out in the copresence of a base as necessary.
Examples of the "base" include those similar to the base used in the aforementioned Production Method 1. Of these, sodium carbonate, potassium carbonate, cesium carbonate, sodium hydrogen carbonate, triethylamine, N,N-diisopropylethylamine, pyridine and the like are preferable.
The amount of the base to be used is generally 0.1 to 100 equivalents, preferably 1 to 10 equivalents, relative to compound (IIa).

<Step 1A-2> (Amination Reaction)

When the "leaving group" for $L^1$ of the aforementioned compound (IIa) is hydroxy, the aforementioned compound (V) can also produced by subjecting the aforementioned compound (IIa) to the "Mitsunobu reaction" (e.g., Synthesis), pages 1-27, 1981) with compound (IV).
The "Mitsunobu reaction" can be carried out, for example, by reacting compound (IIa) with 0.5 to 10 equivalents (preferably 1 to 2 equivalents) of compound (IV) in an inert solvent, in the presence of an azodicarboxamide or azodicarboxylate and a trialkylphosphine or triarylphosphine.
Examples of the "inert solvent" include those similar to the solvent used in the aforementioned Production Method 1. Of these, acetonitrile, N,N-dimethylformamide (DMF), 1-methyl-2-pyrrolidinone (NMP), N,N-dimethylacetamide (DMA), acetone, tetrahydrofuran (THF) and the like are preferable.
Examples of the "azodicarboxamide or azodicarboxylate" include diisopropyl azodicarboxylate, diethyl azodicarboxylate, 1,1'-(azodicarbonyl)dipiperidine and the like. The amount thereof to be used is generally 1 to 20 equivalents, preferably 1 to 3 equivalents, relative to compound (IIa).
Examples of the "trialkylphosphine or triarylphosphine" include triphenylphosphine, tributylphosphine and the like. The amount thereof to be used is generally 1 to 20 equivalents, preferably 1 to 3 equivalents, relative to compound (IIa).
The reaction temperature is generally about -20°C to 100°C, preferably 0°C to 50°C. The reaction time is, for example, about 0.5 hr to 1 week, preferably 3 hr to 1 day.
The aforementioned compound (IIa) can be produced according to a method known per se, for example, the method described in WO 2001/082925, WO 2003/035624 or the like, and the like.
The aforementioned compound (IV) can be produced according to a method known per se.

<Step 1B> (Deprotection Reaction)

The "deprotection reaction" can be carried out, for example, by reacting compound (V) with a base in an inert solvent. This reaction may be carried out in the copresence of a thiol as necessary.
Examples of the "inert solvent" include those similar to the solvent used in the aforementioned Production Method 1. Of these, acetonitrile, N,N-dimethylformamide (DMF), 1-methyl-2-pyrrolidinone (NMP), N,N-dimethylacetamide (DMA), acetone, tetrahydrofuran (THF) and the like are preferable.
Examples of the "base" include those similar to the base used in the aforementioned Production Method 1. Of these, sodium hydroxide, potassium hydroxide, lithium hydroxide and the like are preferable.
The amount of the base to be used is generally 1 to 100 equivalents, preferably 1 to 20 equivalents, relative to compound (V).
Examples of the "thiol" include mercaptoacetic acid, thiophenol and the like. The amount of the thiol to be used is generally 1 to 100 equivalents, preferably 1 to 20 equivalents, relative to compound (V).
The reaction temperature is generally -20°C to 150°C, preferably 0°C to 30°C. The reaction time is generally 0.5 hr to

48 hr, preferably 1 hr to 24 hr.

[Production Method 3]

Compound (I) can also be produced, for example, by subjecting compound (IIa) to the following amination reaction with compound (IVa) (Step 2A), followed by deprotection reaction (Step 2B), alkylation reaction (Step 2C) and deprotection reaction (Step 2D).

(Amination reaction, Deprotection Reaction, Alkylation reaction)

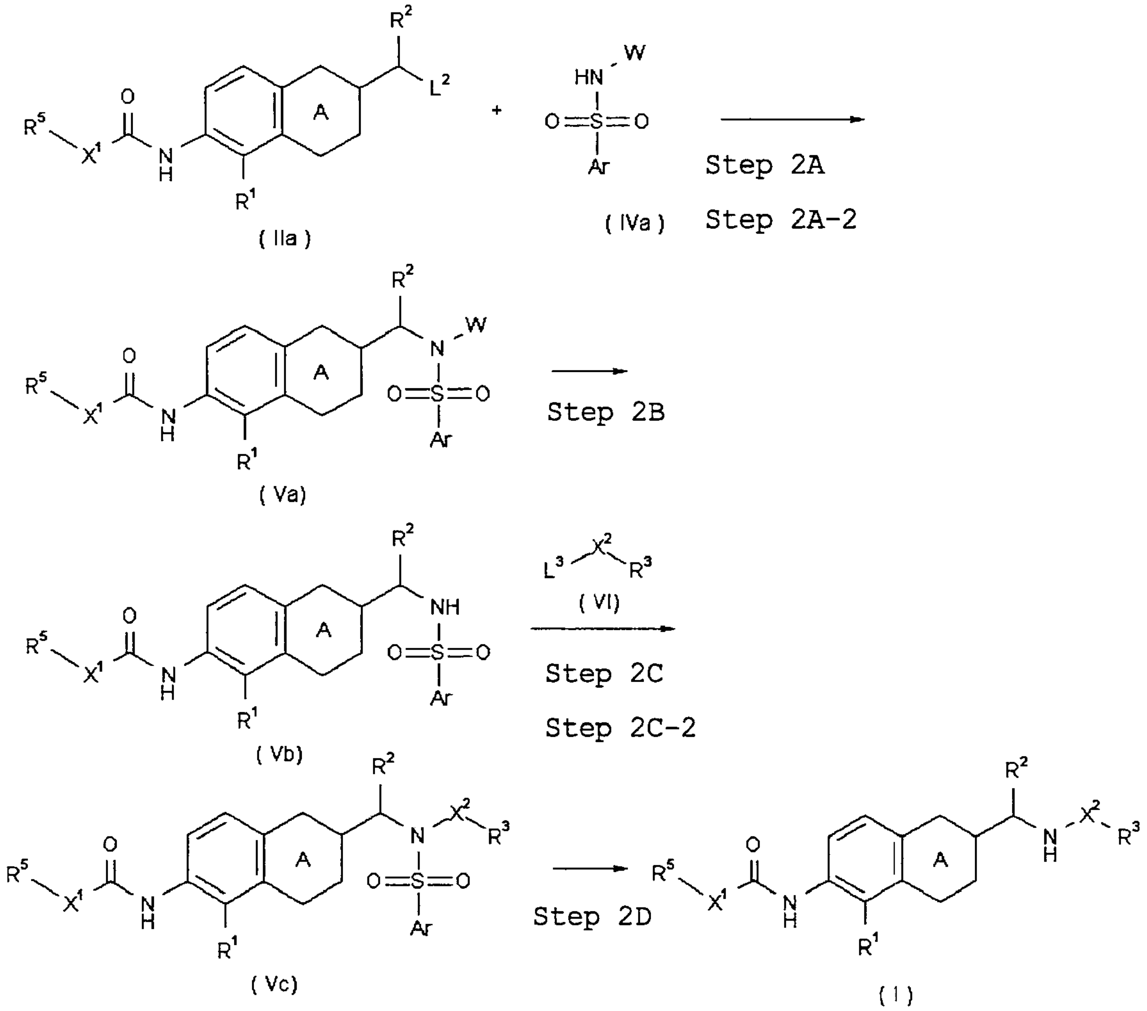


wherein W is an amino-protecting group, $L^3$ is a leaving group, and other symbols are as defined above. Examples of the "amino-protecting group" for W include those similar to the amino-protecting group mentioned below. W is preferably tert-butoxycarbonyl, benzyloxycarbonyl or the like.
Examples of the "leaving group" for $L^3$ include those similar to the above-mentioned "leaving group" for $L^1$.
$L^3$ is preferably a halogen atom, methanesulfonyloxy, trifluoromethanesulfonyloxy, p-toluenesulfonyloxy, hydroxy or the like.

<Step 2A> (Amination Reaction)

This reaction is generally carried out in an inert solvent.
Examples of the "inert solvent" include those similar to the solvent used in the aforementioned Production Method 1. Of

these, acetonitrile, N,N-dimethylformamide (DMF), 1-methyl-2-pyrrolidinone (NMP), N,N-dimethylacetamide (DMA), tetrahydrofuran (THF), acetone, ethanol, pyridine and the like are preferable.
The amount of compound (IVa) to be used is generally 1 equivalent to 100 equivalents, relative to compound (IIa).
The reaction temperature is generally about -20°C to 200°C, preferably room temperature to 100°C. The reaction time is, for example, about 0.5 hr to 1 day.
This reaction may be carried out in the copresence of a base as necessary.
Examples of the "base" include those similar to the base used in the aforementioned Production Method 1.
Of the above-mentioned base, triethylamine, N,N-diisopropylethylamine, pyridine and the like are preferable.
The amount of the base to be used is generally 0.1 to 100 equivalents, preferably 1 to 10 equivalents, relative to compound (IIa).

<Step 2A-2> (Amination Reaction)

When the "leaving group" for $L^2$ of the aforementioned compound (IIa) is hydroxy, the aforementioned compound (Va) can be also produced by subjecting compound (IIa) to the "Mitsunobu reaction" (e.g., Synthesis, pages 1-27, 1981) with the aforementioned compound (IVa).
The "Mitsunobu reaction" can be carried out, for example, by reacting compound (IIa) with 0.5 to 10 equivalents (preferably 1 to 2 equivalents) of compound (IVa) in an inert solvent, in the presence of an azodicarboxamide or azodicarboxylate and a trialkylphosphine or triarylphosphine.
Examples of the "inert solvent" include those similar to the solvent used in the aforementioned Production Method 1. Of these, acetonitrile, N,N-dimethylformamide (DMF), 1-methyl-2-pyrrolidinone (NMP), N,N-dimethylacetamide (DMA), acetone, tetrahydrofuran (THF) and the like are preferable.
Examples of the "azodicarboxamide or azodicarboxylate" include diisopropyl azodicarboxylate, diethyl azodicarboxylate, 1,1'-(azodicarbonyl)dipiperidine and the like. The amount thereof to be used is generally 1 to 20 equivalents, preferably 1 to 3 equivalents, relative to compound (IIa).
Examples of the "trialkylphosphine or triarylphosphine" include triphenylphosphine, tributylphosphine and the like. The amount thereof to be used is generally 1 to 20 equivalents, preferably 1 to 3 equivalents, relative to compound (IIa).
The reaction temperature is generally about -20°C to 100°C, preferably 0°C to 50°C. The reaction time is, for example, about 0.5 hr to 1 week, preferably 3 hr to 1 day.
The aforementioned compound (IIa) can be produced according to a method known per se, for example, the method described in WO 2001/082925, WO 2003/035624 or the like, and the like.
The aforementioned compound (IVa) can be produced according to a method known per se.

<Step 2B> (Deprotection Reaction)

The "deprotection reaction" can be carried out according to a method known per se, for example, the method described in Protective Groups in Organic Synthesis, John Wiley and Sons (1980) or the like. For example, a method using acid, base, ultraviolet rays, hydrazine, phenylhydrazine, sodium N-methyldithiocarbamate, tetrabutylammonium fluoride, palladium acetate, a trialkylsilyl halide (e.g., trimethylsilyl iodide, trimethylsilyl bromide and the like) and the like, a reduction method and the like can be employed.
When W is a tert-butoxycarbonyl group or the like, the "deprotection reaction" of compound (Va) can be carried out, for example, in the copresence of an acid such as a mineral acid (e.g., hydrochloric acid, sulfuric acid, hydrobromic acid, iodine acid, periodic acid), an organic acid (e.g., trifluoroacetic acid, formic acid, acetic acid, methanesulfonic acid, benzenesulfonic acid, trifluoromethanesulfonic acid) and the like, in an inert solvent.
Examples of the "inert solvent" include those similar to the solvent used in the aforementioned Production Method 1. Of these, ethyl acetate, 1,4-dioxane, dichloroethane and the like are preferable.
The amount of the acid to be used is generally 1 to 100 equivalents, preferably 1 to 40 equivalents, relative to compound (Va). The strength of mineral acid is generally 0.1N to 18N, preferably 1N to 12N. When an organic acid is used, an excess amount of organic acid may be used as a reaction solvent.
The reaction temperature is generally -20°C to 200°C, preferably 0°C to 60°C. The reaction time is generally 0.5 hr to 48 hr, preferably 1 hr to 24 hr.

<Step 2C> (Alkylation Reaction)

The "alkylation reaction" can be carried out, for example, by reacting compound (Vb) with 1 to 50 equivalents (preferably 1 to 5 equivalents) of compound (VI) in the presence of a base in an inert solvent.
Examples of the "inert solvent" include those similar to the solvent used in the aforementioned Production Method 1. Of these, acetonitrile, N,N-dimethylformamide (DMF), 1-methyl-2-pyrrolidinone (NMP), dimethylacetamide (DMA), tetrahydrofuran (THF), acetone, ethanol, pyridine and the like are preferable.
The amount of compound (VI) to be used is generally 1 equivalent to 100 equivalents, relative to compound (Vb).
The reaction temperature is generally about -20°C to 200°C, preferably room temperature to 100°C. The reaction time is, for example, about 0.5 hr to 1 day.
This reaction may be carried out in the copresence of a base as necessary.
Examples of the "base" include those similar to the base used in the aforementioned Production Method 1. Of these, triethylamine, N,N-diisopropylethylamine, pyridine and the like are preferable.
The amount of the base to be used is generally 0.1 to 100 equivalents, preferably 1 to 10 equivalents, relative to compound (Vb).

<Step 2C-2> (Alkylation Reaction)

When the "leaving group" for $L^3$ of the aforementioned compound (VI) is hydroxy, the aforementioned compound (Vc) can also be produced by subjecting compound (Vb) to the "Mitsunobu reaction" (e.g., (Synthesis, pages 1-27, 1981) with the aforementioned compound (VI).
The "Mitsunobu reaction" can be carried out, for example, by reacting compound (Vb) with 0.5 to 10 equivalents (preferably 1 to 2 equivalents) of compound (VI) in an inert solvent, in the presence of an azodicarboxamide or azodicarboxylate and a trialkylphosphine or triarylphosphine.
Examples of the "inert solvent" include those similar to the solvent used in the aforementioned Production Method 1. Of these, acetonitrile, N,N-dimethylformamide (DMF), 1-methyl-2-pyrrolidinone (NMP), N,N-dimethylacetamide (DMA), acetone, tetrahydrofuran (THF) and the like are preferable.
Examples of the "azodicarboxamide or azodicarboxylate" include diisopropyl azodicarboxylate, diethyl azodicarboxylate, l,l'-(azodicarbonyl)dipiperidine and the like. The amount thereof to be used is generally 1 to 20 equivalents, preferably 1 to 3 equivalents, relative to compound (Vb).
Examples of the "trialkylphosphine or triarylphosphine" include triphenylphosphine, tributylphosphine and the like. The amount thereof to be used is generally 1 to 20 equivalents, preferably 1 to 3 equivalents, relative to compound (Vb).
The reaction temperature is generally about -20°C to 100°C, preferably 0°C to 50°C. The reaction time is, for example, about 0.5 hr to 1 week, preferably 3 hr to 1 day.
The aforementioned compound (VI) can be produced according to a method known per se.

<Step 2D> (Deprotection Reaction)

The "deprotection reaction" can be carried out, for example, by reacting compound (Vc) with a base in an inert solvent. This reaction may be carried out in the copresence of a thiol as necessary.
Examples of the "inert solvent" include those similar to the solvent used in the aforementioned Production Method 1. Of these, acetonitrile, N,N-dimethylformamide (DMF), 1-methyl-2-pyrrolidinone (NMP), N,N-dimethylacetamide (DMA), acetone, tetrahydrofuran (THF) and the like are preferable.
Examples of the "base" include those similar to the base used in the aforementioned Production Method 1. Of these, sodium hydroxide, potassium hydroxide, lithium hydroxide and the like are preferable.
The amount of the base to be used is generally 1 to 100 equivalents, preferably 1 to 20 equivalents, relative to compound (Vc).
Examples of the "thiol" include mercaptoacetic acid, thiophenol and the like. The amount of the thiol to be used is generally 1 to 100 equivalents, preferably 1 to 20 equivalents, relative to compound (Vc).
The reaction temperature is generally -20°C to 150°C, preferably 0°C to 30°C. The reaction time is generally 0.5 hr to 48 hr, preferably 1 hr to 24 hr.

[Production Method 4]

Compound (I) can also be produced, for example, by the following reductive N-alkylation reaction of compound (VII) with compound (IIIa).

(Reductive N-Alkylation Reaction)

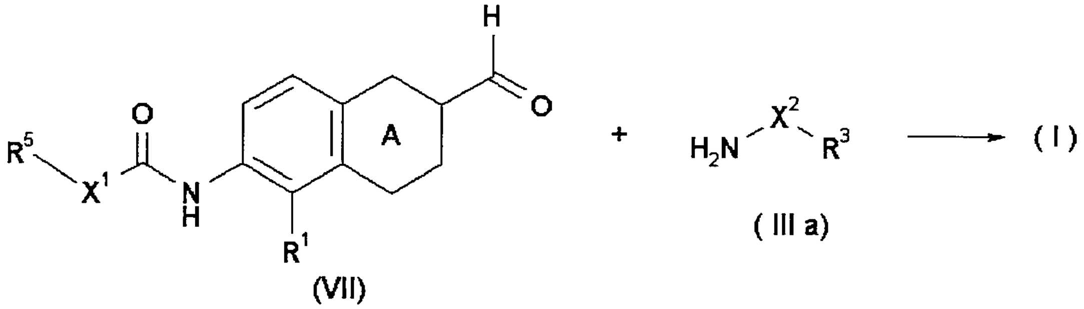

wherein each symbol is as defined above.

The "reductive N-alkylation reaction" can be carried out, for example, by reacting compound (VII) with 1 to 50 equivalents (preferably 1 to 5 equivalents) of compound (IIIa) in the presence of a reducing agent, in an inert solvent.

Examples of the "inert solvent" include those similar to the solvent used in the aforementioned Production Method 1. Of these, acetonitrile, N,N-dimethylformamide (DMF), 1-methyl-2-pyrrolidinone (NMP), N,N-dimethylacetamide (DMA), 1,2-dichloroethane, acetic acid and the like are preferable.

Examples of the reducing agent include sodium borohydride, sodium triacetoxyborohydride, sodium cyanoborohydride and the like.

The amount of the reducing agent to be used is generally 1 to 20 equivalents, preferably 1 to 5 equivalents, relative to compound (VII).

The reaction temperature is generally -20°C to 150°C, preferably 0°C to 60°C. The reaction time is generally 5 min to 40 hr, preferably 1 to 24 hr.

This reaction can also be carried out in the presence of an acid. Examples of the acid to be used include organic acids such as acetic acid, methanesulfonic acid and the like; inorganic acids such as hydrochloric acid, sulfuric acid and the like, and the like.

The amount of the acid to be used is generally 0.01 equivalent to 0.1 equivalent relative to compound (VII) in the case of an inorganic acid, or generally 0.01 equivalent to 100 equivalents relative to compound (VII) in the case of an organic acid. When organic acid is used, an excess amount of organic acid may be used as a reaction solvent.

The aforementioned compound (VII) can be produced according to a method known per se, for example, the method described in WO 2001/082925, WO 2003/035624 or the like, and the like.

The aforementioned compound (IIIa) can be produced according to a method known per se.

[Production Method 5]

Of compound (I), compound (Ia) can also be produced, for example, by the following reductive N-alkylation reaction of compound (VIII) with compound (IX).

(Reductive N-Alkylation Reaction)

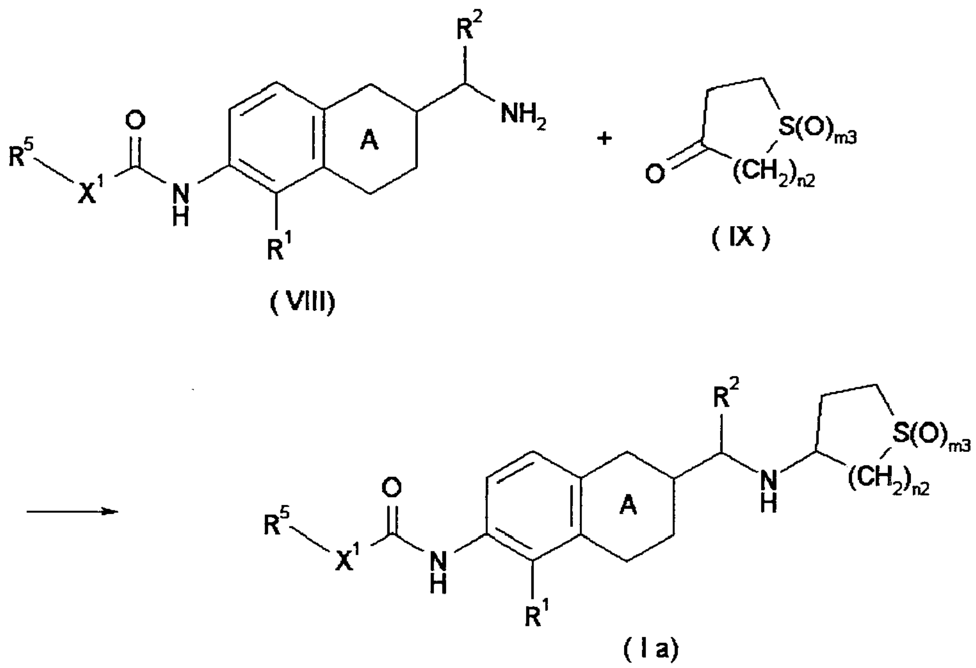

wherein each symbol is as defined above.
The "reductive N-alkylation reaction can be carried out, for example, by reacting compound (VIII) with 0.1 to 1.5 equivalents (preferably 0.2 to 1 equivalent) of compound (IX) in the presence of an reducing agent, in an inert solvent.
Examples of the "inert solvent" include those similar to the solvent used in the aforementioned Production Method 1. Of these, acetonitrile, N,N-dimethylformamide (DMF), 1-methyl-2-pyrrolidinone (NMP), N,N-dimethylacetamide (DMA), 1,2-dichloroethane, acetic acid and the like are preferable.
Examples of the reducing agent include sodium borohydride, sodium triacetoxyborohydride, sodium cyanoborohydride and the like.
The amount of the reducing agent to be used is generally 1 to 20 equivalents, preferably 1 to 5 equivalents, relative to compound (VIII).
The reaction temperature is generally -20°C to 150°C, preferably 0°C to 60°C. The reaction time is generally 5 min to 40 hr, preferably 1 to 24 hr.
This reaction can also be carried out in the presence of an acid. Examples of the acid to be used include organic acids such as acetic acid, methanesulfonic acid and the like; inorganic acids such as hydrochloric acid, sulfuric acid and the like, and the like.
The amount of the acid to be used is generally 0.01 equivalent to 0.1 equivalent relative to compound (VIII) in the case of inorganic acid, or generally 0.01 equivalent to 100 equivalents relative to compound (VIII) in the case of organic acid. When organic acid is used, an excess amount of organic acid may be used as a reaction solvent.
The aforementioned compound (VIII) can be produced according to a method known per se, for example, the method described in WO 2001/082925, WO 2003/035624 or the like, and the like.
The aforementioned compound (IX) can be produced according to a method known per se.

[Production Method 6]

Compound (I) can also be produced, for example, by the following oxidation reaction of compound (Ib).

(Oxidation Reaction)

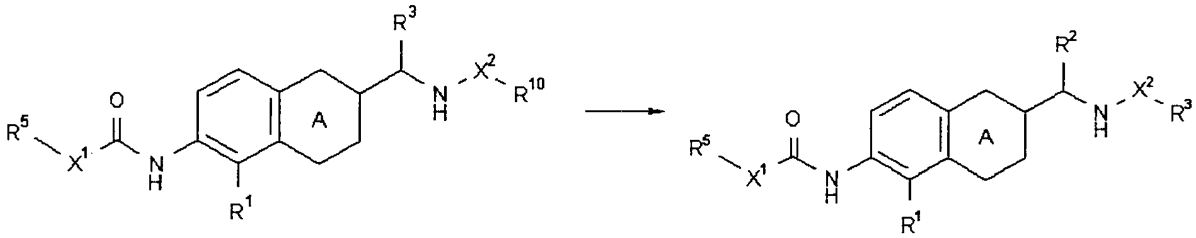

(Ib) (I)

wherein $R^{10}$ is

a group represented by the formula: $-Y-S(O)_{m1a}-R^{4a}$ wherein m1a is an integer of 0 or 1, and other symbols are as defined above), or
a cyclic group represented by the formula:

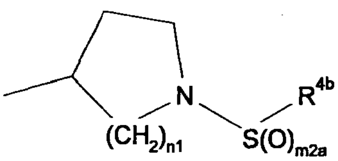 , 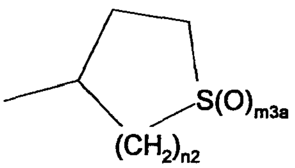 , or 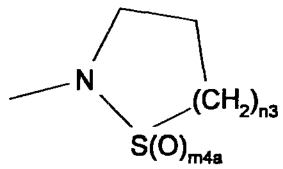

wherein m2a, m3a and m4a are each independently an integer of 0 or 1, and other symbols are as defined above) (the ring moiety of the cyclic group is optionally further substituted),
and other symbols are as defined above.
The "oxidation reaction" can be carried out, for example, by reacting compound (Ib) with an oxidant in an inert solvent. Examples of the "inert solvent" include those similar to the solvent used in the aforementioned Production Method 1. Of these, methanol, acetonitrile, N,N-dimethylformamide (DMF), 1-methyl-2-pyrrolidinone (NMP), N,N-dimethylacetamide (DMA), dimethyl sulfoxide(DMSO), acetone, water and the like are preferable.
Examples of the oxidant include oxone, sodium periodate, osmic acid and the like.
The amount of the oxidant to be used is generally 0.5 to 30 equivalents, preferably 1 to 3 equivalents, relative to compound (Ib).
The reaction temperature is generally -20°C to 150°C, preferably 0°C to 30°C. The reaction time is generally 5 min to 40 hr, preferably 1 to 24 hr.
The aforementioned compound (Ib) can be produced according to a method known per se, or the aforementioned [Production Method 1] to [Production Method 5].

In compound (I) thus obtained, the functional group in a molecule can also be converted to the object functional group by combining chemical reactions known per se. Examples of such chemical reaction include oxidation reaction, reduction reaction, alkylation reaction, hydrolysis reaction, amination reaction, amidation reaction, esterification reaction, aryl-coupling reaction, deprotection reaction and the like.

In each of the above-mentioned reactions, when the starting compound has an amino group, a carboxyl group, a hydroxy group, a carbonyl group or a mercapto group as a substituent, a protecting group generally used in peptide chemistry and the like may be introduced into these groups. By removing the protecting group as necessary after the reaction, the objective compound can be obtained.

Examples of the amino-protecting group include a formyl group, a $C_{1-6}$ alkyl-carbonyl group (e.g., acetyl, propionyl), a $C_{1-6}$ alkoxy-carbonyl group (e.g., methoxycarbonyl, ethoxycarbonyl, tert-butoxycarbonyl), a benzoyl group, a $C_{7-10}$ aralkyl-carbonyl group (e.g., benzylcarbonyl), a $C_{7-14}$ aralkyloxy-carbonyl group (e.g., benzyloxycarbonyl, a 9-fluorenylmethoxycarbonyl), a trityl group, a phthaloyl group, an N,N-dimethylaminomethylene group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like. These groups are optionally substituted by 1 to 3 substituents selected from a halogen

atom, a $C_{1-6}$ alkoxy group and a nitro group.

Examples of the carboxyl-protecting group include a $C_{1-6}$ alkyl group, a $C_{7-10}$ aralkyl group (e.g., benzyl), a phenyl group, a trityl group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like. These groups are optionally substituted by 1 to 3 substituents selected from a halogen atom, a $C_{1-6}$ alkoxy group and a nitro group.

Examples of the hydroxy-protecting group include a $C_{1-6}$ alkyl group, a phenyl group, a trityl group, a $C_{7-10}$ aralkyl group (e.g., benzyl), a formyl group, a $C_{1-6}$ alkyl-carbonyl group, a benzoyl group, a $C_{7-10}$ aralkyl-carbonyl group (e.g., benzylcarbonyl), a 2-tetrahydropyranyl group, a 2-tetrahydrofuranyl group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like. These groups are optionally substituted by 1 to 3 substituents selected from a halogen atom, a $C_{1-6}$ alkyl group, a $C_{1-6}$ alkoxy group and a nitro group.

Examples of the carbonyl-protecting group include a cyclic acetal (e.g., 1,3-dioxane), an acyclic acetal (e.g., di-$C_{1-6}$ alkyl acetal) and the like.

Examples of the mercapto-protecting group include a $C_{1-6}$ alkyl group, a phenyl group, a trityl group, a $C_{7-10}$ aralkyl group (e.g., benzyl), a $C_{1-6}$ alkyl-carbonyl group, a benzoyl group, a $C_{7-10}$ aralkyl-carbonyl group (e.g., benzylcarbonyl), a $C_{1-6}$ alkoxy-carbonyl group, a $C_{6-14}$ aryloxy-carbonyl group (e.g., phenyloxycarbonyl), a $C_{7-14}$ aralkyloxycarbonyl group (e.g., benzyloxycarbonyl, 9-fluorenylmethoxycarbonyl), a 2-tetrahydropyranyl group, a $C_{1-6}$ alkylaminocarbonyl group (e.g., methylaminocarbonyl, ethylaminocarbonyl) and the like. These groups are optionally substituted by 1 to 3 substituents selected from a halogen atom, a $C_{1-6}$ alkyl group, a $C_{1-6}$ alkoxy group and a nitro group.

The compound (I) can be isolated and purified by methods known per se such as solvent extraction, changing of liquid properties, transdissolution, crystallization, recrystallized from, chromatography and the like. It is also possible to isolate and purify the starting compounds of compound (I), or their salts using the same known methods as above, but they can also be used as starting materials in the next process as a reaction mixture without being isolated.

Inasmuch as compound (I) and a prodrug thereof (hereinafter abbreviated as the compound of the present invention) has a superior MCH receptor antagonistic action, it is useful as an agent for the prophylaxis or treatment of diseases caused by MCH.

In addition, the compound of the present invention also shows low toxicity (e.g., cardiotoxicity (e.g., human ether-ago-go related gene (HERG) inhibitory activity), phospholipidosis (PLsis), acute toxicity, chronic toxicity, genetic toxicity, reproductive toxicity, drug interaction, carcinogenicity, phototoxicity).

Moreover, the compound of the present invention is superior in oral absorbability.

Furthermore, the compound of the present invention is superior in intracerebral transferability.

Accordingly, the compound of the present invention is safely administered as an agent for the prophylaxis or treatment of diseases caused by MCH to mammals (e.g., rat, mouse, guinea pig, rabbit, sheep, horse, pig, cow, monkey, human).

The diseases caused by MCH include obesity [e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity and the like], hyperphagia, emotional disorder, sexual dysfunction, depression, anxiety and the like.

The compound of the present invention is useful as an agent for the prophylaxis or treatment of a lifestyle-related disease such as diabetes (e.g., type 1 diabetes, type 2 diabetes, gestational diabetes, obese diabetes, borderline type diabetes), impaired glucose tolerance (IGT (Impaired Glucose Tolerance)), diabetic complications (e.g., diabetic retinopathy, diabetic neuropathy, diabetic nephropathy), hyperlipidemia (e.g., hypertriglyceridemia, hypercholesterolemia, high LDL-cholesterolemia, low HDL-cholesterolemia, postprandial hyperlipemia), arteriosclerosis, knee arthritis, metabolic syndrome and the like.

Furthermore, the compound of the present invention is also useful as an anorexigenic agent.

The compound of the present invention can also be concurrently used with diet therapy (e.g., diet therapy for diabetes), or an exercise therapy.

The compound of the present invention can be used for the prophylaxis or treatment of pigmentation disorder based on abnormality of melanin or melanocyte. Examples of the pigmentation disorder include pigment proliferation, pigment decrease and the like. Examples of the pigment proliferation include drug pigmentation caused by antitumor agent and the like; chromatosis and incompetence of pigment associated with diseases such as endocrine metabolism disorder (e.g., Addison's disease), genetic diseases, chronic hepatopathy, kidney failure, acanthosis nigricans, systemic scleroderma and the like; and the like. Examples of the pigment decrease include phenylketonuria, systemic or localized albinism, foliaceous leukoderma or leukoderma vulgaris associated with tuberous sclerosis; depigmentation associated with systemic scleroderma and the like.

The compound of the present invention can be used for the prophylaxis or treatment of depigmentation due to chloasma, ephelides, sunburn and the like; and further, hyperpigmentation or hypopigmentation for cosmetic purposes.

The compound of the present invention can be used directly or as a pharmaceutical composition prepared together with a pharmacologically acceptable carrier by a means known per se, for example, the method described in the Japanese Pharmacopoeia.

Examples of the pharmacologically acceptable carrier include various organic or inorganic carrier substances conventionally used as a preparation material, for example, excipient, lubricant, binder and disintegrant for solid preparations; solvent, solubilizing agent, suspending agent, isotonicity agent, buffer and soothing agent for liquid preparations and the like. Where necessary, an additive for pharmaceutical preparations such as preservative, antioxidant, colorant, sweetening agent, adsorbent, wetting agent and the like can be used.

Examples of the excipient include lactose, sucrose, D-mannitol, starch, cornstarch, crystalline cellulose, light anhydrous silicic acid.

Examples of the lubricant include magnesium stearate, calcium stearate, talc, colloidal silica.

Examples of the binder include crystalline cellulose, sucrose, D-mannitol, dextrin, hydroxypropylcellulose, hydroxypropylmethylcellulose, polyvinylpyrrolidone, starch, sucrose, gelatin, methylcellulose, sodium carboxymethylcellulose.

Examples of the disintegrant include starch, carboxymethylcellulose, calcium carboxymethylcellulose, croscarmellose sodium, sodium carboxymethyl starch, low-substituted hydroxypropylcellulose (L-HPC).

Examples of the solvent include water for injection, alcohol, propylene glycol, macrogol, sesame oil, corn oil.

Examples of the solubilizing agent include polyethylene glycol, propylene glycol, D-mannitol, benzyl benzoate, ethanol, trisaminomethane, cholesterol, triethanolamine, sodium carbonate and sodium citrate.

Examples of the suspending agent include surfactant such as stearyltriethanolamine, sodium lauryl sulfate, lauryl aminopropionate, lecithin, benzalkonium chloride, benzethonium chloride, glyceryl monostearate and the like; hydrophilic polymer such as polyvinyl alcohol, polyvinylpyrrolidone, sodium carboxymethylcellulose, methylcellulose, hydroxymethylcellulose, hydroxyethylcellulose, hydroxypropylcellulose and the like.

Examples of the isotonicity agent include glucose, D-sorbitol, sodium chloride, glycerol and D-mannitol.

Examples of the buffer include buffers such as phosphate, acetate, carbonate, citrate and the like.

Examples of the soothing agent include benzyl alcohol.

Examples of the preservative include p-hydroxybenzoates, chlorobutanol, benzyl alcohol, phenethyl alcohol, dehydroacetic acid and sorbic acid.

Examples of the antioxidant include sulfite and ascorbic acid salt.

Examples of the colorant include water-soluble food tar color (e.g., food colors such as Food Color Red No. 2 and No. 3, Food Color Yellow No. 4 and No. 5, Food Color Blue No. 1 and No. 2 and the like), water-insoluble lake dye (e.g., aluminum salt of the aforementioned water-soluble food tar color), natural dye (e.g., β-carotene, chlorophyll and ferric oxide red).

Examples of the sweetening agent include saccharin sodium, dipotassium glycyrrhizinate, aspartame and stevia.

Examples of the adsorbent include porous starch, calcium silicate (trade name: Florite RE), magnesium alumino metasilicate (trade name: Neusilin), light anhydrous silicic acid (trade name: Sylysia).

Examples of the wetting agent include propylene glycol monostearate, sorbitan monooleate, diethylene glycol monolaurate, polyoxyethylenelauryl ether.

Examples of the dosage form of the aforementioned pharmaceutical composition include oral preparations such as tablet (including sugar-coated tablet, film-coated tablet, sublingual tablet, orally disintegrable tablet), powder, granule, capsule (including soft capsule, microcapsule), troche, syrup, emulsion, suspension and the like; and parenteral preparations such as injection (e.g., subcutaneous injection, intravenous injection, intramuscular injection, intraperitoneal injection etc.), external preparation (e.g., preparations for nasal administration, dermal preparation, ointment), suppository (e.g., rectal suppository, vaginal suppository), pellet, nasal preparations, pulmonary preparation (inhalant), drip infusion and the like. These preparations can be safely administered orally or parenterally (e.g., topical, rectal, intravenous administration).

In addition, these preparations may be controlled-release preparation (e.g., sustained-release microcapsule) such as immediate-release preparation, sustained-release preparation and the like.

The content of the compound of the present invention in the pharmaceutical composition is, for example, about 0.1 to 100 wt% of the pharmaceutical composition.

The dose of the compound of the present invention is appropriately determined according to the subject of administration, administration route, disease and the like.

For example, the daily dose of the compound of the present invention for oral administration to an adult patient (body weight about 60 kg) with obesity is about 0.1 to about 500 mg, preferably about 1 to about 100 mg, more preferably about 5 to about 100 mg. This amount can be administered at once or in several portions for one day.

The compound of the present invention can be used in combination with a concomitant drug that does not adversely influence the compound of the present invention for the purpose of, for example, enhancing the action of the compound of the present invention (treatment effects of obesity, diabetes, depression, anxiety and the like), reduction

of the amount of the compound of the present invention to be used, and the like. Examples of the concomitant drug include "agent for treating diabetes", "agent for treating diabetic complication", "antiobesity agent", "agent for treating hypertension", "agent for treating hyperlipidemia", "agent for treating arthritis", "antianxiety agent", "antidepressant", "sleep-inducing drug" and the like. Two or more kinds of these concomitant drugs may be used in combination at an appropriate ratio.

Examples of the above-mentioned "agent for treating diabetes" include insulin preparations (e.g., animal insulin preparations extracted from pancreas of bovine and swine; human insulin preparations genetically synthesized using *Escherichia coli* or yeast; zinc insulin; protamine zinc insulin; fragment or derivative of insulin (e.g., INS-1), oral insulin preparation and the like), insulin sensitizers (e.g., pioglitazone or a salt thereof (preferably hydrochloride), rosiglitazone or a salt thereof (preferably maleate), Tesaglitazar, Ragaglitazar, Muraglitazar, Edaglitazone, Metaglidasen, Naveglitazar, AMG-131, THR-0921, TAK-379), α-glucosidase inhibitors (e.g., voglibose, acarbose, miglitol, emiglitate), biguanides (e.g., metformin, buformin or a salt thereof (e.g., hydrochloride, fumarate, succinate)), insulin secretagogues [sulfonylureas (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or a calcium salt hydrate thereof], dipeptidyl peptidase IV inhibitors (e.g., Alogliptin or a salt thereof (preferably benzoate), Vildagliptin, Sitagliptin, Saxagliptin, T-6666, TS-021), β3 agonists (e.g., AJ-9677), GPR40 agonists, GLP-1 receptor agonists [e.g., GLP-1, GLP-1MR agent, NN-2211, AC-2993 (exendin-4), BIM-51077, Aib(8,35)hGLP-1(7,37)$NH_2$, CJC-1131], amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists), SGLUT (sodium-glucose cotransporter) inhibitors (e.g., T-1095), 11β-hydroxysteroid dehydrogenase inhibitors (e.g., BVT-3498), adiponectin or a agonist thereof, IKK inhibitors (e.g., AS-2868), leptin resistance improving drugs, somatostatin receptor agonists, glucokinase activators (e.g., Ro-28-1675), GIP (Glucose-dependent insulinotropic peptide), glucose dependency insulin secretagogues (e.g., TAK-875) and the like.

Examples of the above-mentioned "agent for treating diabetic complication" include aldose reductase inhibitors (e.g., tolrestat, epalrestat, zenarestat, zopolrestat, minalrestat, fidarestat, CT-112, ranirestat (AS-3201)), neurotrophic factors and enhancers thereof (e.g., NGF, NT-3, BDNF, neurotrophin production/secretion promoting agents described in WO01/14372 (e.g., 4-(4-chlorophenyl)-2-(2-methyl-1-imidazolyl)-5-[3-(2-methylphenoxy)propyl]oxazole), TAK-583), PKC inhibitors (e.g., ruboxistaurin mesylate), AGE inhibitors (e.g., ALT946, pimagedine, N-phenacylthiazolium bromide (ALT766), EXO-226, Pyridorin, pyridoxamine), active oxygen scavengers (e.g., thioctic acid), cerebral vasodilators (e.g., tiapride, mexiletine), somatostatin receptor agonists (e.g., BIM23190), apoptosis signal regulating kinase-1 (ASK-1) inhibitors and the like.

Examples of the above-mentioned "antiobesity agent" include central nervous system antiobesity agents [e.g., dexfenfluramine, fenfluramine, phentermine, sibutramine, anfepramone, dexamphetamine, mazindol, phenylpropanolamine, clobenzorex; neuropeptide Y antagonists (e.g., CP-422935); cannabinoid receptor antagonists (e.g., SR-141716, SR-147778); ghrelin antagonists; 11β-hydroxysteroid dehydrogenase inhibitors (e.g., BVT-3498)], pancreatic lipase inhibitors (e.g., orlistat, cetilistat), β3 agonists (e.g., AJ-9677), anorectic peptides (e.g., leptin, CNTF (ciliary neurotrophic factor)), cholecystokinin agonists (e.g., lintitript, FPL-15849), anorexigenic agents (e.g., P-57) and the like.

Examples of the above-mentioned "agent for treating hypertension" include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid, TAK-491), calcium antagonists (e.g., manidipine, nifedipine, amlodipine, efonidipine, nicardipine), potassium channel openers (e.g., levcromakalim, L-27152, AL 0671, NIP-121), clonidine and the like.

Examples of the above-mentioned "agent for treating hyperlipidemia" include HMG-CoA reductase inhibitors (e.g., pravastatin, simvastatin, lovastatin, atorvastatin, fluvastatin, rosuvastatin, pitavastatin or a salt thereof (e.g., sodium salt, calcium salt)), squalene synthase inhibitors (e.g., compound described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), ACAT inhibitors (e.g., Avasimibe, Eflucimibe), anion exchange resins (e.g., colestyramine), probucol, nicotinic acid drugs (e.g., nicomol, niceritrol), ethyl icosapentate, phytosterols (e.g., soysterol, γ-oryzanol) and the like.

Examples of the above-mentioned "agent for treating arthritis" include ibuprofen and the like.

Examples of the above-mentioned "antianxiety agent" include chlordiazepoxide, diazepam, oxazolam, medazepam, cloxazolam, bromazepam, lorazepam, alprazolam, fludiazepam and the like.

Examples of the above-mentioned "antidepressant" include fluoxetine, fluvoxamine, imipramine, paroxetine, sertraline, desipramine, amitriptyline and the like.

Examples of the above-mentioned "sleep-inducing drug" include Ramelteon, GABA-type sleep-inducing drugs (e.g., brotizolam, estazolam, flurazepam, nitrazepam, triazolam, Flunitrazepam, Lormetazepam, rilmazafone, Quazepam, zopiclone, eszopiclone, zolpidem, Zaleplon, indiplon, gaboxadol); non-GABA type sleep-inducing drugs (e.g., eplivanserin,

Pruvanserin, diphenhydramine, trazodone, doxepin) and the like.

The administration time of the aforementioned concomitant drug is not limited, and the compound of the present invention and the concomitant drug can be administered to an administration subject simultaneously, or may be administered at staggered times. The dosage of the concomitant drug may be determined according to the dose clinically used, and can be appropriately selected depending on an administration subject, administration route, disease, combination and the like. The administration mode of the concomitant drug is not particularly limited, and the compound of the present invention and the concomitant drug only need to be combined on administration. Examples of such administration mode include the following:

(1) administration of a single preparation obtained by simultaneously processing the compound of the present invention and the concomitant drug, (2) simultaneous administration of two kinds of preparations of the compound of the present invention and the concomitant drug, which have been separately produced, by the same administration route, (3) administration of two kinds of preparations of the compound of the present invention and the concomitant drug, which have been separately produced, by the same administration route in a staggered manner, (4) simultaneous administration of two kinds of preparations of the compound of the present invention and the concomitant drug, which have been separately produced, by different administration routes, (5) administration of two kinds of preparations of the compound of the present invention and the concomitant drug, which have been separately produced, by different administration routes in a staggered manner (e.g., administration in the order of the compound of the present invention and the concomitant drug, or in the reverse order) and the like.

The compounding ratio of the compound of the present invention to the concomitant drug can be appropriately selected depending on the administration subject, administration route, diseases and the like.

Example

The present invention is described in detail by way of the following Reference Examples, Examples, Formulation Example and Experimental Example. These are not intended to restrict the present invention, and may be modified within the range not deviating from the scope of this invention.

The "room temperature" in the following Reference Examples and Examples means a temperature of 15°C to 30°C. For drying an organic layer, anhydrous magnesium sulfate or anhydrous sodium sulfate was employed. Unless otherwise specifically indicated, "%" means percent by weight.

FABMS(pos) is mass spectrum measured by the (+) method in the Fast Atom Bombardment Mass Spectrometry.

The abbreviations used in the present specification mean the following.

Ac: acetyl
Me: methyl
s: singlet
d: doublet
t: triplet
q: quartet
dd: double doublet
dt: double triplet
m: multiplet
br: broad
J: coupling constant
Hz: Hertz
$CDCl_3$: deuterated chloroform
DMA: dimethylacetamide
THF: tetrahydrofuran
NMP: 1-methyl-2-pyrrolidinone
DMF: N,N-dimethylformamide
DMSO: dimethyl sulfoxide
$^1$H-NMR: proton nuclear magnetic resonance

Reference Example 1

4-cyclopropylmethoxybenzoic acid

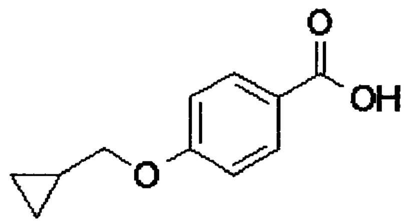

Cyclopropylmethyl bromide (50 mL) was added dropwise to a solution of methyl 4-hydroxybenzoate (55 g) and potassium carbonate (69 g) in acetonitrile (300 mL) at room temperature, and the mixture was stirred at 70°C for 2 hr. The reaction solution was concentrated, water (150 mL) was added to the residue, and the precipitated methyl 4-cyclopropylmethoxybenzoate was collected by filtration using glass filter. The crystals were suspended in water (100 mL), 8N aqueous sodium hydroxide solution (200 mL) was added thereto, and the mixture was stirred with heating at 90°C for 2 hr. The reaction solution was cooled under ice bath, and neutralized with concentrated hydrochloric acid. Ethyl acetate was added thereto to dissolve the precipitated crystals, and the solution was extracted with ethyl acetate. The organic layer was washed with saturated brine, and dried over anhydrous magnesium sulfate, and the solvent was evaporated under reduced pressure to give the title compound (68 g, yield 98%) as a white solid.
$^1$H NMR (300 MHz, $CDCl_3$) δ: 0.37-0.42(m,2 H),0.64-0.72(m,2H),1.26-1.34(m,1H),3.87(d,J=7.OHz,2H),6.96(d,J=7.0Hz, 2H),8.05(d,J=7.0H z,2H),12.6(s,1H).

Reference Example 2

4-(2-cyclopropylethoxy)benzoic acid

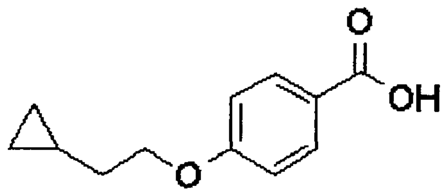

2-Cyclopropylethanol (22.0 g), triphenylphosphine (65.3 g) and methyl 4-hydroxybenzoate (34.4 g) were dissolved in tetrahydrofuran (300 mL), and 40% diethyl azodicarboxylate toluene solution (118.0 g) was added dropwise thereto. The mixture was stirred at room temperature for 3 hr, and the reaction mixture was concentrated. The precipitate was filtered through glass filter, and washed with a mixed solvent of ethyl acetate and hexane. The filtrate was concentrated, and the residue was purified by silica gel column chromatography [eluent; hexane:ethyl acetate=3:1 (volume ratio)] to give methyl 4-(2-cyclopropylethoxy)benzoate as an oil.
This oil was suspended in water (200 mL), 8N aqueous sodium hydroxide solution (100 mL) was added thereto, and the mixture was stirred with heating at 90°C for 3 hr. The reaction solution was cooled under ice bath, and neutralized with 6N aqueous hydrochloric acid solution to allow precipitation of the resultant product. The mixture was extracted with ethyl acetate. The organic layer was washed with saturated brine, and dried over anhydrous magnesium sulfate, and the solvent was evaporated under reduced pressure. The obtained solid was washed with hexane to give the title compound (43.2 g, yield 93%) as a white solid.
$^1$H NMR (300 MHz, CDCl3) δ: 0.08-0.18(m,2 H),0.43-0.61(m,2H),0.77-0.99(m,1H),1.71(q,J=6.6Hz,2 H)4.11(t,J=6.6Hz, 2H),6.95(d,J=9.1Hz,2H),8.06(d,J=9.1Hz,2H),12. 6(s,1H).

Reference Example 3

4-(cyclopropylmethoxy)-N-[3-formyl-8-methylquinolin-7-yl]benzamide

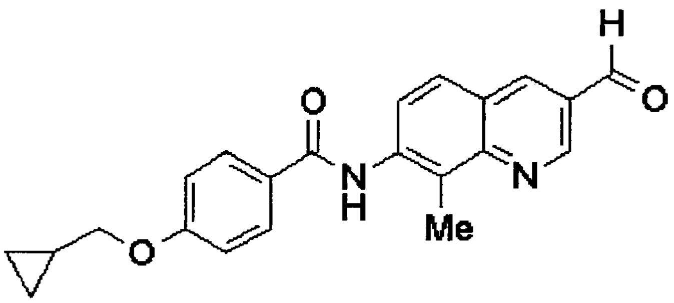

4-Cyclopropylmethoxybenzoic acid (11.4 g) obtained in Reference Example 1, oxalyl dichloride (15.5 mL) and N,N-dimethylformamide (one drop) were mixed in tetrahydrofuran (100 mL), and the mixture was stirred at room temperature for 6 hr. The reaction mixture was concentrated under reduced pressure, and pyridine (100 mL) was added to the concentrated residue. To this mixture was added a solution of 7-amino-8-methylquinoline-3-carbaldehyde (10.0 g) in pyridine (100 mL) under ice-cooling, and the mixture was stirred at room temperature for 24 hr. The reaction mixture was concentrated under reduced pressure, and 1N aqueous sodium hydroxide solution (200 mL) was added to the concentrated residue. The mixture was extracted with chloroform (3×200 mL), and the organic layer was concentrated under reduced pressure. The precipitated crystals were washed with diethyl ether while stirring, collected by filtration, and dried to give the title compound (18.9 g, yield 97%) as pale-yellow crystals.
$^1$H NMR (300 MHz, DMSO-$d_6$) δ: 0.37 (2 H, m) 0.60 (2 H, m) 1.27 (1 H, m) 2.69 (3 H, s) 3.93 (2 H, d, J=7.0 Hz) 7.08 (2 H, d, J=8.7 Hz) 7.82 (1 H, d, J=8.7 Hz) 8.03 (2 H, d, J=8.7 Hz) 8.06 (1 H, m) 8.93 (1 H, d, J=1.9 Hz) 9.31 (1 H, d, J=2.1 Hz) 10.17 (1 H, s) 10.26 (1 H, s).

Reference Example 4

4-(cyclopropylmethoxy)-N-[3-(hydroxymethyl)-8-methylquinolin-7-yl]benzamide

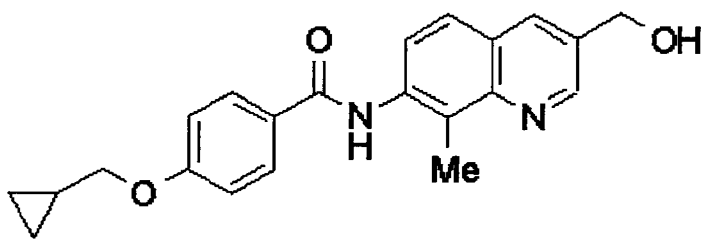

4-(Cyclopropylmethoxy)-N-(3-formyl-8-methylquinolin-7-yl)benzamide (12.00 g) obtained in Reference Example 3 and sodium borohydride (2.80 g) were suspended in THF (100 mL). Methanol (100 mL) was added gradually thereto at 0°C, and the mixture was stirred for 2 hr. The reaction was quenched with 1N aqueous hydrochloric acid solution, and the mixture was basified with 8N aqueous sodium hydroxide solution. The obtained colorless suspension was filtered to collect the solid, and the solid was washed with water, and dried under reduced pressure to give the title compound (11.82 g, yield 98%) as a colorless powder.
$^1$H NMR (300 MHz, $CDCl_3$) δ: 0.40 (2 H, q, J=4.9 Hz), 0.65-0.74 (2 H, m), 1.25-1.38 (1 H, m), 1.80-1.90 (1 H, m), 2.82 (3 H, s), 3.90 (2 H, d, J=7.0 Hz), 4.93 (2 H, d, J=5.3 Hz), 7.02 (2 H, d, J=8.7 Hz), 7.73 (1 H, d, J=8.7 Hz), 7.87-7.95 (3 H, m, J=8.7 Hz), 8.09-8.14 (1 H, m), 8.30 (1 H, d, J=8.9 Hz), 8.92 (1 H, d, J=1.9 Hz).

Reference Example 5

N-[3-(chloromethyl)-8-methylquinolin-7-yl]-4-(cyclopropylmethoxy)benzamide hydrochloride

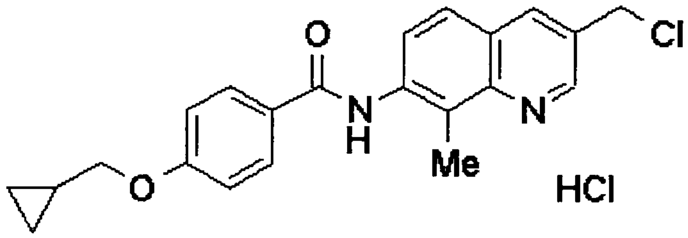

To thionyl chloride (100 mL) cooled to -78°C was added 4-(cyclopropylmethoxy)-N-[3-(hydroxymethyl)-8-methylquinolin-7-yl]benzamide (20.00 g) obtained in Reference Example 4 under nitrogen stream. While allowed to warm to room temperature, the mixture was stirred for 4 hr, and the diethyl ether (200 mL) was added thereto. The obtained yellow suspension was filtered to collect the solid, and the solid was washed with diethyl ether and toluene, and dried under reduced pressure to give the title compound (21.21 g, yield 92%) as a colorless powder.
$^1$H NMR (300 MHz, $CDCl_3$) δ: 0.32-0.40 (2 H, m), 0.55-0.65 (2 H, m), 1.17-1.36 (1 H, m), 2.65 (3 H, s), 3.93 (2 H, d, J=7.2 Hz), 5.04 (2 H, s), 7.02-7.12 (2 H, m), 7.68 (1 H, d, J=8.9 Hz), 7.86 (1 H, d, J=8.7 Hz), 7.98-8.07 (2 H, m), 8.44 (1 H, d, J=2.3 Hz), 9.00 (1 H, d, J=2.3 Hz), 10.10 (1 H, s).

Reference Example 6

4-(cyclopropylmethoxy)-N-[3-(1-hydroxyethyl)-8-methylquinolin-7-yl]benzamide

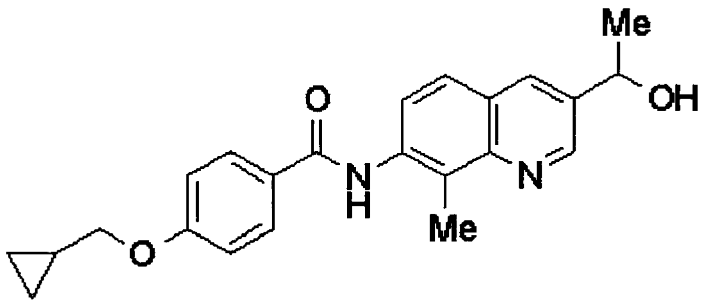

4-(Cyclopropylmethoxy)-N-(3-formyl-8-methylquinolin-7-yl)benzamide (18.88 g) obtained in Reference Example 3 was dissolved in THF (200 mL), and the solution was added dropwise over 1 hr to a mixture (cooled to 5°C) of 3N methylmagnesium bromide ether solution (87.3 mL) and THF (200 mL) under a nitrogen atmosphere while stirring. The mixture was allowed to warm to room temperature, and stirred for 3 hr. The reaction mixture was extracted with 10% aqueous ammonium chloride solution, and the organic layer was separated. The aqueous layer was extracted with ethyl acetate. The obtained organic layers were combined, and concentrated under reduced pressure. The precipitated solid was collected by filtration, washed with ethyl acetate, and dried under reduced pressure to give the title compound (16.67 g, yield 85%) as a pale-yellow solid. $^1$H NMR (300 MHz, DMSO-$d_6$) δ: 0.33 - 0.39 (2 H, m), 0.56 - 0.64 (2 H, m), 1.21 - 1.33 (1 H, m), 1.48 (3 H, d, J=6.4 Hz), 2.64 (3 H, s), 3.92 (2 H, d, J=7.0 Hz), 4.94 - 5.04 (1 H, m), 5.49 (1 H, d, J=4.1 Hz), 7.07 (2 H, d, J=8.9 Hz), 7.59 (1 H, d, J=8.9 Hz), 7.81 (1 H, d, J=8.7 Hz), 8.02 (2 H, d, J=8.9 Hz), 8.23 (1 H, d, J=2.1 Hz), 8.94 (1 H, d, J=2.3 Hz), 10.06 (1 H, s).

Reference Example 7

N-[3-(1-chloroethyl)-8-methylquinolin-7-yl]-4-(cyclopropylmethoxy)benzamide hydrochloride

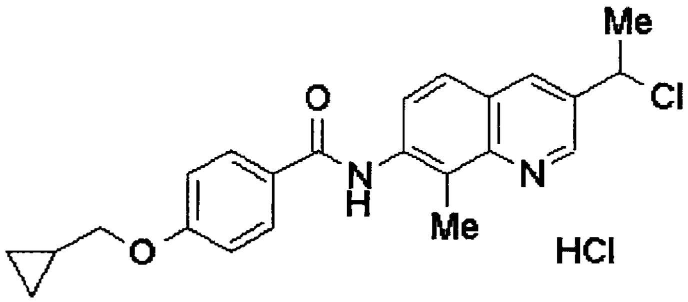

To thionyl chloride (120 mL) cooled to -78°C was added 4-(cyclopropylmethoxy)-N-[3-(1-hydroxyethyl)-8-methylquinolin-7-yl]benzamide (20.00 g) obtained in Reference Example 6 under nitrogen stream. While allowed to warm to room temperature, the mixture was stirred for 7 hr, and diethyl ether was added thereto. The mixture was stirred for 22 hr, and concentrated under reduced pressure. The obtained residue was suspended in 1N aqueous hydrochloric acid solution (500 mL) and acetone (50 mL), and the suspension was stirred at room temperature for 14 hr. The precipitated solid was collected by filtration, washed with ether, and dried under reduced pressure to give the title compound (22.80 g, yield 99.5%) as a pale-yellow solid.
$^1$H NMR (300 MHz, $CDCl_3$) δ: 0.36-0.44 (2 H, m), 0.65-0.74 (2 H, m), 1.27-1.38 (1 H, m), 2.01 (3 H, d, J=6.8 Hz), 3.12 (3 H, s), 3.91 (2 H, d, J=7.0 Hz), 5.33 (1 H, q, J=6.8 Hz), 7.05 (2 H, d, J=8.9 Hz), 7.97 (1 H, d, J=9.0 Hz), 8.06 (2 H, d, J=8.7 Hz), 8.70-8.82 (3 H, m), 9.25 (1 H, d, J=2.1 Hz).

Reference Example 8

4-(2-cyclopropylethoxy)-N-(3-formyl-8-methylquinolin-7-yl)benzamide

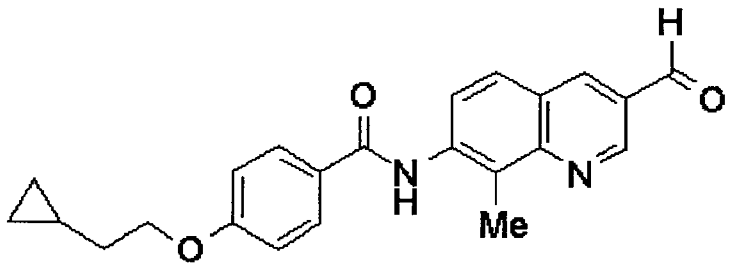

To a suspension of 4-(2-cyclopropylethoxy)benzoic acid (3.323 g) obtained in Reference Example 2 in methylene chloride (100 mL) were added oxalyl dichloride (2.76 mL) and DMF (one drop) at room temperature, and the mixture was stirred for 1 hr. The solvent was removed under reduced pressure, and subjected to azeotropic removal with toluene.
The residue was diluted with pyridine (20 mL), and 7-amino-8-methylquinoline-3-carbaldehyde (3.000 g) was added thereto.
The mixture was stirred at room temperature for 16 hr, and water and chloroform were successively added thereto. The mixture was partitioned. The separated organic layer was dried over sodium sulfate, and concentrated under reduced pressure. The obtained residue was purified by silica gel column chromatography [eluent; hexane:ethyl acetate=2:1 (volume ratio)→ethyl acetate] to give the title compound (2.536 g, yield 44%) as a yellow powder.
$^1$H NMR (300 MHz, DMSO-$d_6$) δ: 0.11-0.19 (2 H, m), 0.40-0.51 (2 H, m), 0.79-0.93 (1 H, m), 1.67 (2 H, q, J=6.6 Hz), 2.69 (3 H, s), 4.14 (2 H, t, J=6.7 Hz), 7.10 (2 H, d, J=8.9 Hz), 7.82 (1 H, d, J=8.7 Hz), 7.97-8.11 (3 H, m), 8.94 (1 H, d,

J=2.1 Hz), 9.32 (1 H, d, J=2.1 Hz), 10.17 (1 H, s), 10.26 (1 H, s).

Reference Example 9

4-(2-cyclopropylethoxy)-N-[3-(hydroxymethyl)-8-methylquinolin-7-yl]benzamide

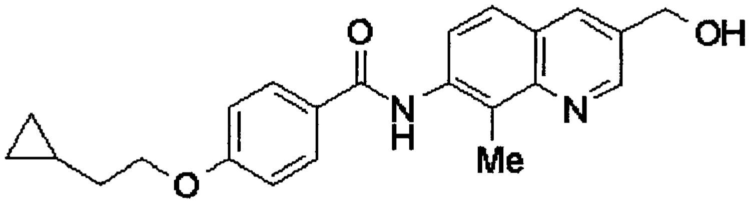

4-(2-Cyclopropylethoxy)-N-(3-formyl-8-methylquinolin-7-yl)benzamide (1.223 g) obtained in Reference Example 8 and sodium borohydride (purity 90%, 0.275 g) were suspended in THF (15 mL), methanol (3 mL) was gradually added thereto at 0°C, and the mixture was stirred for 2 hr. The solvent was evaporated under reduced pressure, and the mixture was acidified with 1N hydrochloric acid, and diluted with water. The mixture was basified with 1N aqueous sodium hydroxide solution, and the resulting solid was collected by filtration, washed with water, and dried in air to give the title compound (1.217 g, yield 99%) as a yellow powder.
$^1$H NMR (300 MHz, $CDCl_3$) δ: 0.15 (2 H, q, J=5.0 Hz), 0.42-0.59 (2 H, m), 0.78-0.96 (1 H, m), 1.73 (2 H, q, J=6.8 Hz), 1.86 (1 H, t, J=5.7 Hz), 2.82 (3 H, s), 4.13 (2 H, t, J=6.1 Hz), 4.93 (2 H, d, J=5.7 Hz), 7.03 (2 H, d, J=8.7 Hz), 7.73 (1 H, d, J=8.7 Hz), 7.84-7.97 (3 H, m), 8.11 (1 H, s), 8.30 (1 H, d, J=9.1 Hz), 8.92 (1 H, d, J=2.3 Hz).

Reference Example 10

N-[3-(chloromethyl)-8-methylquinolin-7-yl]-4-(2-cyclopropylethoxy)benzamide hydrochloride

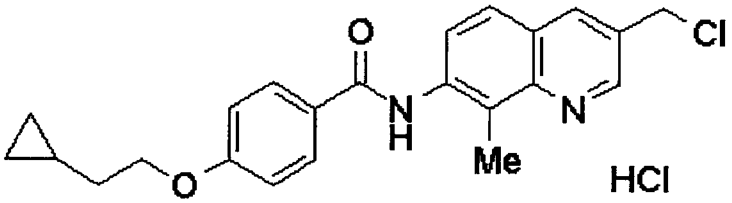

To thionyl chloride (100 mL) cooled to -78°C was added 4-(2-cyclopropylethoxy)-N-[3-(hydroxymethyl)-8-methylquinolin-7-yl]benzamide (1.217 g) obtained in Reference Example 9 under nitrogen stream. While allowed to warm to room temperature, the mixture was stirred for 3 hr, and diethyl ether was added thereto. The obtained yellow suspension was filtered to collect solid, and the solid was washed with diethyl ether and toluene, and dried under reduced pressure to give the title compound (1.263 g, yield 88%) as a yellow powder.
$^1$H NMR (300 MHz, DMSO-$d_6$) δ: 0.10-0.19 (2 H, m), 0.41-0.50 (2 H, m), 0.79-0.92 (1 H, m), 1.67 (2 H, q, J=6.8 Hz), 2.65 (3 H, s), 4.13 (2 H, t, J=6.6 Hz), 5.05 (2 H, s), 7.09 (2 H, d, J=8.7 Hz), 7.70 (1 H, d, J=8.7 Hz), 7.88 (1 H, d, J=9.1 Hz), 8.03 (2 H, d, J=8.7 Hz), 8.49 (1 H, d, J=2.3 Hz), 9.01 (1 H, d, J=2.3 Hz), 10.14 (1 H, s).

Reference Example 11

4-(2-cyclopropylethoxy)-N-[3-(1-hydroxyethyl)-8-methylquinolin-7-yl]benzamide

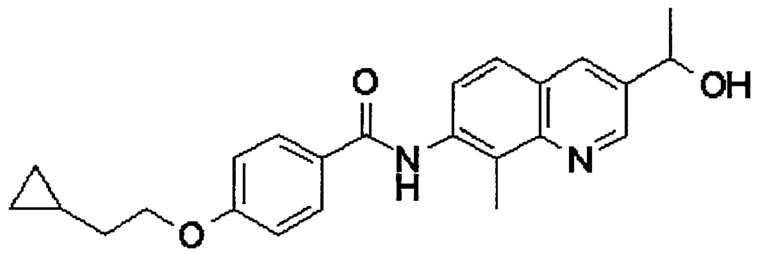

4-(2-Cyclopropylethoxy)-N-(3-formyl-8-methylquinolin-7-yl)benzamide (1.13 g) obtained in Reference Example 8 was suspended in THF (15 mL), methylmagnesium bromide (1M THF solution, 10.1 mL) was added thereto at 0°C, and the mixture was stirred at room temperature for 1.5 hr. To the reaction solution was added water, and the mixture was concentrated under reduced pressure. The precipitate was collected by filtration, washed with water, and dried under reduced pressure to give the title compound (965 mg, yield 73%) as a yellow solid.
$^1$H NMR (300 MHz, $CDCl_3$) δ ppm 0.15 (2 H, q, J=4.7 Hz), 0.44 - 0.56 (2 H, m), 0.79 - 0.94 (1 H, m), 1.64 (3 H, d, J=6.4 Hz), 1.73 (2 H, q, J=6.6 Hz), 1.96 (1 H, br. s.), 2.82 (3 H, s), 4.13 (2 H, t, J=6.6 Hz), 5.08 - 5.22 (1 H, m), 7.02 (2 H, d, J=8.7 Hz), 7.73 (1 H, d, J=8.7 Hz), 7.84 - 7.97 (3 H, m), 8.11 (1 H, d, J=2.7 Hz), 8.30 (1 H, d, J=9.1 Hz), 8.95 (1 H, d, J=2.3 Hz).

Reference Example 12

N-[3-(1-chloroethyl)-8-methylquinolin-7-yl]-4-(2-cyclopropylethoxy)benzamide hydrochloride

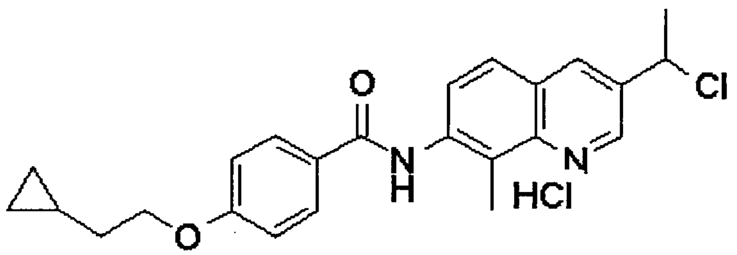

4-(2-Cyclopropylethoxy)-N-[3-(1-hydroxyethyl)-8-methylquinolin-7-yl]benzamide (963 mg) obtained in Reference Example 11 was slowly added to thionyl chloride (10 mL) at - 78°C. While allowed to warm to room temperature, the mixture was stirred for 3 hr, and diethyl ether was added to the reaction solution. The precipitate was collected by filtration, washed with diethyl ether and toluene, and dried under reduced pressure to give the title compound (1.096 g, yield 100%) as a yellow solid.
$^1$H NMR (300 MHz, DMSO-$d_6$) δ ppm 0.11 - 0.19 (2 H, m), 0.40 - 0.51 (2 H, m), 0.75 - 0.96 (1 H, m), 1.67 (2 H, q, J=6.7 Hz), 1.96 (3 H, d, J=6.8 Hz), 2.65 (3 H, s), 4.13 (2 H, t, J=6.6 Hz), 5.58 - 5.72 (1 H, m), 7.09 (2 H, d, J=8.7 Hz), 7.67 (1 H, d, J=8.7 Hz), 7.86 (1 H, d, J=8.7 Hz), 7.97 - 8.10 (2 H, m), 8.45 (1 H, d, J=2.7 Hz), 9.05 (1 H, d, J=2.3 Hz), 10.10 (1 H, s).

Reference Example 13

tert-butyl [2,2-dimethyl-3-(methylthio)propyl]carbamate

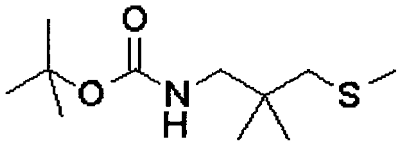

3-Amino-2,2-dimethylpropan-1-ol (25.0 g) was diluted with THF (250 mL), 0.5N aqueous sodium hydroxide solution (250 mL) and ditert-butyl dicarbonate (58.2 g) were added thereto, and the mixture was stirred at room temperature for 106 hr. The mixture was cooled to 0°C, and 1N hydrochloric acid (250 mL) and ethyl acetate (1200 mL) were added thereto. The organic layer was washed with saturated brine, dried over sodium sulfate, and concentrated under reduced pressure. The residue was dissolved in THF (500 mL), and triethylamine (67.5 mL) and methanesulfonyl chloride (41.6 g) were added thereto at 0°C, and the mixture was stirred at the same temperature for 1 hr. The mixture was diluted with ethyl acetate (1000 mL), washed with aqueous sodium hydrogen carbonate solution (500 mL) and saturated brine, dried over sodium sulfate, and concentrated under reduced pressure. The residue was diluted with DMF (360 mL), sodium methanethiolate (70.1 g) was added thereto, and the mixture was stirred at 80°C for 14 hr. The mixture was allowed to cool to room temperature, ethyl acetate (1500 mL) was added thereto, and the mixture was washed successively with water (twice) and saturated brine, dried over sodium sulfate, and concentrated under reduced pressure. The residue was purified by silica gel column chromatography [eluent; hexane:ethyl acetate=4:1 (volume ratio)] to give the title compound (46.0 g, yield 81%) as a yellow oil.
$^1$H NMR (300 MHz, $CDCl_3$) δ: 0.97 (6 H, s), 1.45 (9 H, s), 2.12 (3 H, s), 2.45 (2 H, s), 3.05 (2 H, d, J=6.6 Hz), 4.67 (1 H, br).

Reference Example 14

tert-butyl [2,2-dimethyl-3-(methylsulfonyl)propyl]carbamate

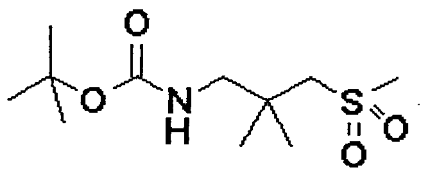

tert-Butyl [2,2-dimethyl-3-(methylthio)propyl]carbamate (46.0 g) obtained in Reference Example 13 was dissolved in ethyl acetate (500 mL), m-chloroperbenzoic acid (65%, 105 g) was added thereto at 0°C, and the mixture was stirred at the same temperature for 17 hr. An aqueous solution (300 mL) of sodium thiosulfate pentahydrate (51.4 g) was added thereto at 0°C, and the mixture was stirred at room temperature for 30 min. Ethyl acetate (300 mL) and water (300 mL) were added thereto. The organic layer was washed with 1N aqueous sodium hydroxide solution (500 mL), water (300 mL) and saturated brine (200 mL), dried over sodium sulfate, filtered through NH silica gel, and concentrated under reduced pressure to give the title compound (49.5 g, yield 95%) as a white solid.
$^1$H NMR (300 MHz, $CDCl_3$) δ: 1.14-1.25 (6 H, m), 1.45 (9 H, s), 2.94 (3 H, s), 3.00 (2 H, s), 3.23 (2 H, d, J=6.8 Hz), 5.09 (1 H, br).

Reference Example 15

2,2-dimethyl-3-(methylsulfonyl)propan-1-amine hydrochloride

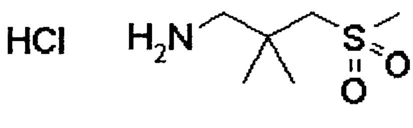

tert-Butyl [2,2-dimethyl-3-(methylsulfonyl)propyl]carbamate (49.5 g) obtained in Reference Example 14 was dissolved in THF (500 mL), concentrated hydrochloric acid (150 mL) was added thereto, and the mixture was stirred at 60°C for 3.5 hr. The reaction solution was concentrated under reduced pressure. To the residue were added toluene and ethanol, and the mixture was concentrated. The residue was washed with diisopropyl ether to give the title compound (36.4 g, yield 97%) as white crystals.
$^1$H NMR (300 MHz, $CDCl_3$) δ: 1.17 (6 H, s), 2.95 (2 H, q, J=5.7 Hz), 3.01 (3 H, s), 3.42 (2 H, s), 8.14 (3 H, s).

Reference Example 16

tert-butyl {2-[(methylsulfonyl)amino]ethyl}carbamate

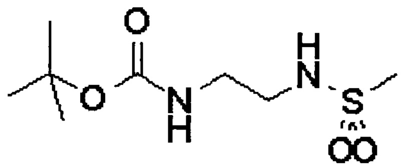

tert-Butyl (2-aminoethyl)carbamate (3.51 g) and triethylamine (4.6 mL) were mixed in THF (100 mL), methanesulfonyl chloride (2.5 mL) was added thereto at 0°C, and the mixture was stirred for 2 hr. The reaction solution was concentrated under reduced pressure, and the residue was diluted with ethyl acetate. The solution was washed with water, aqueous sodium hydrogen carbonate solution and saturated brine, dried over sodium sulfate, and concentrated under reduced pressure. The residue was recrystallized from ethyl acetate to give the title compound (4.45 g, yield 85%) as white crystals. $^1$H NMR (300 MHz, $CDCl_3$) δ: 1.45 (9 H, s), 2.97 (3 H, s), 3.22-3.36 (4 H, m), 4.92 (2 H, br. s.).

Reference Example 17

N-(2-aminoethyl)methanesulfonamide hydrochloride

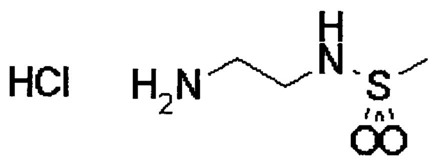

tert-Butyl {2-[(methylsulfonyl)amino]ethyl}carbamate (4.44 g) obtained in Reference Example 16 was dissolved in ethyl acetate (40 mL)-THF (20 mL), hydrogen chloride-ethyl acetate (4M, 23 mL) was added thereto at room temperature, and the mixture was stirred for 23 hr. The reaction solution was concentrated under reduced pressure, and the residue was washed with ethyl acetate to give the title compound (3.02 g yield 93%) as white crystals.
$^1$H NMR (300 MHz, DMSO-$d_6$) δ: 2.90 (2 H, t, J=6.4 Hz), 2.96 (3 H, s), 3.20 (2 H, t, J=6.2 Hz), 7.35 (1 H, br. s.), 8.06 (3 H, s).

Reference Example 18

1-(methylsulfonyl)piperidin-4-amine hydrochloride

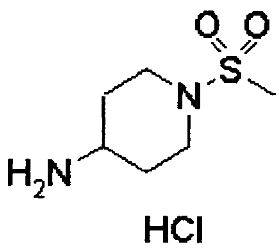

To a solution of tert-butyl piperidin-4-ylcarbamate (10.59 g) and triethylamine (9.76 mL) in THF (220 mL) was slowly added methanesulfonyl chloride (6.76 g) at 0°C, and the mixture was stirred overnight at room temperature. To the reaction solution was added ethyl acetate, and the mixture was washed with water and saturated brine, dried over

sodium sulfate, and concentrated under reduced pressure. The residue was dissolved in ethyl acetate (250 mL), 4N hydrogen chloride-ethyl acetate solution (50 mL) was added thereto, and the mixture was stirred overnight at room temperature. The solvent was evaporated under reduced pressure to give the title compound (9.80 g, yield 86%) as a white solid.
$^1$H NMR (300 MHz, DMSO-$d_6$) δ: 1.57-1.69 (2 H, m), 2.00-2.04 (2 H, m), 2.77-2.86 (2 H, m), 2.88 (3 H, s),3.11-3.16 (1 H, m), 3.56-3.60 (2 H, m), 8.44 (3 H, br).

Reference Example 19

2-(1,1-dioxidoisothiazolidin-2-yl)ethanamine hydrochloride

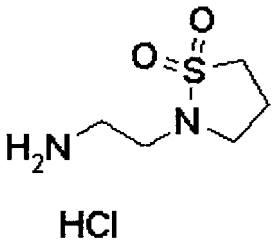

To a solution of tert-butyl (2-aminoethyl)carbamate (22.9 g) and diisopropylethylamine (21.97 g) in THF (250 mL) was slowly added 3-chloropropane-1-sulfonyl chloride (26.8 g) at 0°C, and the mixture was stirred overnight at room temperature. To the reaction solution was added ethyl acetate, and the mixture was washed with water and saturated brine, dried over sodium sulfate, and concentrated under reduced pressure. The residue was dissolved in DMF (300 mL), sodium hydride (6.80 g) was slowly added thereto at 0°C, and the mixture was stirred at room temperature for 3 hr. To the reaction solution was added ethyl acetate, and the mixture was washed with water and saturated brine, dried over sodium sulfate, and concentrated under reduced pressure. The residue was dissolved in ethyl acetate (300 mL), 4N hydrogen chloride-ethyl acetate solution (100 mL) was added thereto, and the mixture was stirred overnight at room temperature. The solvent was evaporated under reduced pressure to give the title compound (20.0 g, yield 70%) as a white solid.
$^1$H NMR (300 MHz, DMSO-$d_6$) δ: 1.59 (6 H, s), 2.48-2.53 (4 H, m), 4. 66 (3 H, br).

Reference Example 20

N-(2-aminoethyl)-1,1,1-trifluoromethanesulfonamide hydrochloride

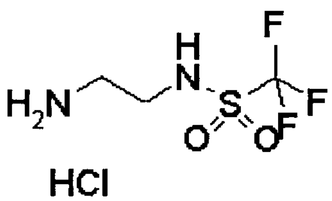

To a solution of tert-butyl (2-aminoethyl)carbamate (5.00 g) and triethylamine (6.27 mL) in THF (200 mL) was slowly added trifluoromethanesulfonyl chloride (6.00 g) at 0°C, and the mixture was stirred overnight at room temperature. To the reaction solution was added ethyl acetate, and the mixture was washed with water and saturated brine, dried over sodium sulfate, and concentrated under reduced pressure. The residue was dissolved in ethyl acetate (100 mL), 4N hydrogen chloride-ethyl acetate solution (10 mL) was added thereto, and the mixture was stirred overnight at room temperature. The solvent was evaporated under reduced pressure to give the title compound (4.27 g, yield 60%) as a white solid.
$^1$H NMR (300 MHz, DMSO-$d_6$) δ: 2.95 (2 H, br), 3.45 (2 H, t, J=6.6 Hz), 8.34 (3 H, br), 10.02 (1 H, br).

Reference Example 21

1-[(trifluoromethyl)sulfonyl]piperidin-4-amine hydrochloride

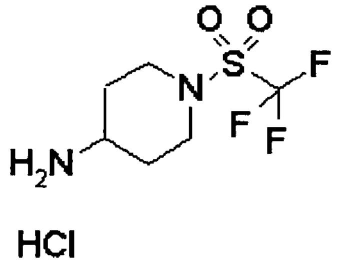

To a solution of tert-butyl piperidin-4-ylcarbamate (7.62 g) and triethylamine (6.97 mL) in THF (150 mL) was slowly added trifluoromethanesulfonyl chloride (6.74 g) at 0°C, and the mixture was stirred overnight at room temperature. To the reaction solution was added ethyl acetate, and the mixture was washed with water and saturated brine, dried over sodium sulfate, and concentrated under reduced pressure. The residue was dissolved in ethyl acetate (250 mL), 4N hydrogen chloride-ethyl acetate solution (50 mL) was added thereto, and the mixture was stirred overnight at room temperature. The solvent was evaporated under reduced pressure to give the title compound (7.00 g, yield 69%) as a white solid.
$^1$H NMR (300 MHz, DMSO-$d_6$) δ: 1.58-1.71 (1 H, m), 1.78-1.92 (1 H, m), 2.09-2.13 (2 H, m), 2.95-3.04 (1 H, m), 3.29-3.36 (2 H, m), 3.83-3.87 (2 H, m), 8.57 (3 H, br).

Reference Example 22

1-(methylsulfonyl)pyrrolidin-3-amine hydrochloride

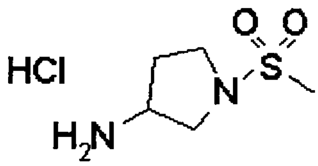

To a solution of tert-butyl pyrrolidin-3-ylcarbamate (10.3 g) and triethylamine (9.06 mL) in THF (150 mL) was slowly added methanesulfonyl chloride (6.53 g) at 0°C, and the mixture was stirred overnight at room temperature. To the reaction solution was added ethyl acetate, and the mixture was washed with water and saturated brine, dried over sodium sulfate, and concentrated under reduced pressure. The residue was dissolved in ethyl acetate (250 mL), 4N hydrogen chloride-ethyl acetate solution (50 mL) was added thereto, and the mixture was stirred overnight at room temperature. The solvent was evaporated under reduced pressure to give the title compound (10.03 g, yield 90%) as a white solid.
$^1$H NMR (300 MHz, DMSO-$d_6$) δ: 1.98-2.07 (1 H, m), 2.17-2.27 (1 H, m), 2.97 (3 H, s), 3.27-3.37 (2 H, m), 3.43-3.57 (2 H, m), 3.834 (1 H, br), 8.62 (3 H, br).

Reference Example 23

N-(3-aminopropyl)methanesulfonamide hydrochloride

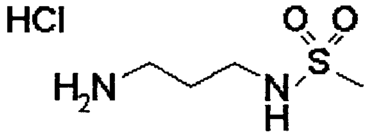

To a solution of tert-butyl (3-aminopropyl)carbamate (5.00 g) and triethylamine (6.27 mL) in THF (200 mL) was slowly added methanesulfonyl chloride (3.44 g) at 0°C, and the mixture was stirred overnight at room temperature. To the reaction solution was added ethyl acetate, and the mixture was washed with water and saturated brine, dried over sodium sulfate, and concentrated under reduced pressure. The residue was dissolved in ethyl acetate (100 mL), 4N hydrogen chloride-ethyl acetate solution (25 mL) was added thereto, and the mixture was stirred overnight at room temperature. The solvent was evaporated under reduced pressure to give the title compound (4.22 g, yield 78%) as a white solid.
$^1$H NMR (300 MHz, DMSO-$d_6$) δ: 1.74-1.82 (2 H, m), 2.78-2.85 (2 H, m), 2.91 (3 H, s), 2.99-3.06 (2 H, m), 7.20 (1 H, t, J=6.0 Hz), 8.17 (3 H, br).

Reference Example 24

1-[1-(methylsulfonyl)piperidin-4-yl]methanamine hydrochloride

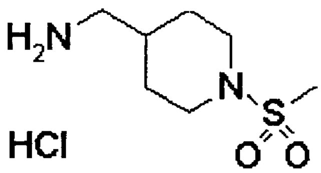

To a solution of tert-butyl (piperidin-4-ylmethyl)carbamate (5.00 g) and triethylamine (6.27 mL) in THF (200 mL) was slowly added methanesulfonyl chloride (2.86 g) at 0°C, and the mixture was stirred overnight at room temperature. To the reaction solution was added ethyl acetate, and the mixture was washed with water and saturated brine, dried over sodium sulfate, and concentrated under reduced pressure. The residue was dissolved in ethyl acetate (100 mL), 4N hydrogen chloride-ethyl acetate solution (25 mL) was added thereto, and the mixture was stirred overnight at room temperature. The solvent was evaporated under reduced pressure to give the title compound (4.17 g, yield 78%) as a white solid.
$^1$H NMR (300 MHz, DMSO-$d_6$) δ: 1.17-1.29 (2 H, m), 1.69-1.76 (1 H, m), 1.81-1.85 (2 H, m),2.63-2.74 (4 H, m),2.86 (3 H, s), 3.53-3.57 (2 H, m), 8.16 (3 H, br).

Reference Example 25

4-(aminomethyl)tetrahydro-2H-thiopyran-4-ol

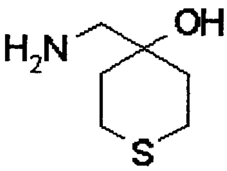

Tetrahydro-4H-thiopyran-4-one (5.50 g) was dissolved in toluene (30 mL), trimethylsilyl cyanide (5.97 mL) and zinc iodide (1.51 g) were added thereto, and the mixture was stirred at room temperature for 2 hr. The reaction solution was concentrated under reduced pressure, and the residue was diluted with THF (50 mL). Lithium aluminum hydride

(2.47 g) was added thereto at 0°C, and the mixture was stirred at room temperature for 16 hr. The mixture was cooled to 0°C, and sodium sulfate decahydrate (16.75 g) was added thereto. The mixture was filtered, and the filtrate was concentrated under reduced pressure to give the title compound (7.25 g, yield 100%) as a colorless transparent oil.
$^1$H NMR (300 MHz, $CDCl_3$) δ: 1.53-1.66 (2 H, m), 1.77-1.90 (2 H, m), 2.36-2.48 (2 H, m), 2.57 (2 H, t, J=6.5 Hz), 2.97-3.11 (2 H, m).

Reference Example 26

tert-butyl [(4-hydroxytetrahydro-2H-thiopyran-4-yl)methyl]carbamate

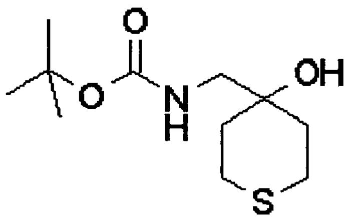

4-(Aminomethyl)tetrahydro-2H-thiopyran-4-ol (5.004 g) obtained in Reference Example 25 was dissolved in THF (80 mL), a solution of di-tert-butyl dicarbonate (7.89 mL) in THF (20 mL) was added dropwise over 30 min at 0°C. The mixture was stirred at room temperature for 24 hr, and the reaction solution was filtered. The filtrate was concentrated under reduced pressure, the residue was diluted with a mixed solution of toluene-ethyl acetate, and the mixture was concentrated under reduced pressure. The residue was diluted with hexane-ethyl acetate, and the precipitate was collected by filtration, and dried to give the title compound (3.675 g, yield 44%) as a white solid. The filtrate was purified by silica gel column chromatography [eluent; hexane:ethyl acetate=9:1 (volume ratio)→hexane:ethyl acetate=9:11 (volume ratio)] to give the title compound (2.529 g, yield 30%) as a white solid.
$^1$H NMR (300 MHz, $CDCl_3$) δ: 1.45 (9 H, s), 1.61-1.74 (2 H, m), 1.82-1.94 (2 H, m), 2.39-2.55 (3 H, m), 2.89-3.03 (2 H, m), 3.11 (2 H, d, J=6.4 Hz), 4.84 (1 H, br. s.).

Reference Example 27

tert-butyl [(4-hydroxy-1,1-dioxidotetrahydro-2H-thiopyran-4-yl)methyl]carbamate

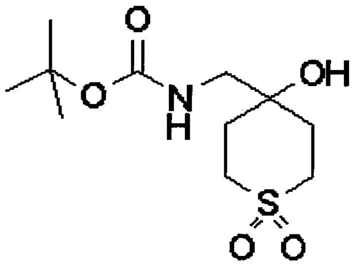

tert-Butyl [(4-hydroxytetrahydro-2H-thiopyran-4-yl)methyl]carbamate (1.000 g) obtained in Reference Example 26 was dissolved in acetone (40 mL), a solution of oxone (3.728 g) in water (40 mL) was added over 30 min at 0°C, and the mixture was stirred at the same temperature for 2 hr. The reaction solution was filtered, and the filtrate was concentrated under reduced pressure. The residue was extracted with ethyl acetate. The extract was purified by silica gel column chromatography [eluent; ethyl acetate], and concentrated under reduced pressure to give the title compound (933 mg, yield 83%) as a white solid.
$^1$H NMR (300 MHz, $CDCl_3$) δ: 1.46 (9 H, s), 1.99-2.10 (2 H, m), 2.81-2.94 (2 H, m), 3.18 (2 H, d, J=6.1 Hz), 3.35-3.55 (4 H, m), 4.94 (1 H, br. s.).

Reference Example 28

4-(aminomethyl)tetrahydro-2H-thiopyran-4-ol 1,1-dioxide hydrochloride

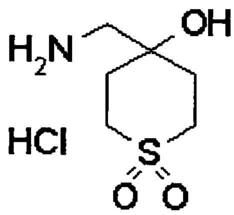

tert-Butyl [(4-hydroxy-1,1-dioxidotetrahydro-2H-thiopyran-4-yl)methyl]carbamate (932 mg) obtained in Reference Example 27 was dissolved in methanol (30 mL), 4N hydrogen chloride-ethyl acetate solution (8 mL) was added thereto, and the mixture was stirred for 24 hr. The reaction solution was concentrated under reduced pressure, and the residue was suspended in ethyl acetate. The precipitate was collected by filtration, washed with ethyl acetate, and concentrated under reduced pressure to give the title compound (707 mg, yield 98%) as a white solid.
$^1$H NMR (300 MHz, DMSO-$d_6$) δ: 1.86 - 2.13 (4 H, m) 3.00 - 3.11 (2 H, m) 3.12 - 3.25 (2 H, m) 3.32 (1 H, s) 5.53 (1 H, s) 7.96 (3 H, br. s.).

Reference Example 29

tetrahydro-2H-thiopyran-4-carbonitrile

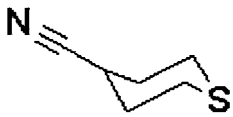

Tetrahydro-4H-thiopyran-4-one (10.00 g) and p-toluenesulfonylmethyl isocyanide (18.49 g) were dissolved in DME (400 mL). A solution of potassium tert-butoxide (19.32 g) in tert-butanol (150 mL) was added thereto at 0°C, and the mixture was stirred at room temperature for 3 hr. The mixture was diluted with diethyl ether, washed successively with saturated aqueous sodium hydrogen carbonate solution and saturated brine (twice), dried over sodium sulfate, and concentrated under reduced pressure. The residue was purified by silica gel column chromatography [eluent; ethyl acetate] to give the title compound (10.93 g, yield 100%) as a brown transparent oil.
$^1$H NMR (300 MHz, $CDCl_3$) δ: 2.03-2.22 (4 H, m), 2.52-2.65 (2 H, m), 2.77-2.93 (3 H, m).

Reference Example 30

1-(tetrahydro-2H-thiopyran-4-yl)methanamine hydrochloride

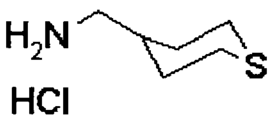

To a suspension of lithium aluminum hydride (80%, 2.797 g) in THF (150 mL) was added dropwise a solution

of tetrahydro-2H-thiopyran-4-carbonitrile (5.00 g) obtained in Reference Example 29 in THF (50 mL) at 0°C, and the mixture was stirred at room temperature for 2 hr. The reaction solution was cooled to 0°C, sodium sulfate decahydrate (19.00 g) was slowly added thereto, and the mixture was stirred at room temperature for 2 hr. The mixture was filtered, and the filtrate was concentrated under reduced pressure. The residue was diluted with ethyl acetate, and hydrogen chloride-ethyl acetate (4M, 10 mL) was added dropwise thereto. The mixture was stirred at room temperature for 2 hr, and the precipitate was collected by filtration, washed with ethyl acetate, and dried under reduced pressure to give the title compound (4.85 g, yield 73%) as a brown solid.
$^1$H NMR (300 MHz, DMSO-$d_6$) δ: 1.20-1.38 (2 H, m), 1.52-1.71 (2 H, m), 2.00 (2 H, dq, J=13.3, 3.5, 3.2 Hz), 2.54-2.76 (5 H, m), 7.82 (3 H, br. s.).

Reference Example 31

4-(cyclopropylmethoxy)-N-(8-methyl-3-{[(tetrahydro-2H-thiopyran-4-ylmethyl)amino]methyl}quinolin-7-yl)benzamide

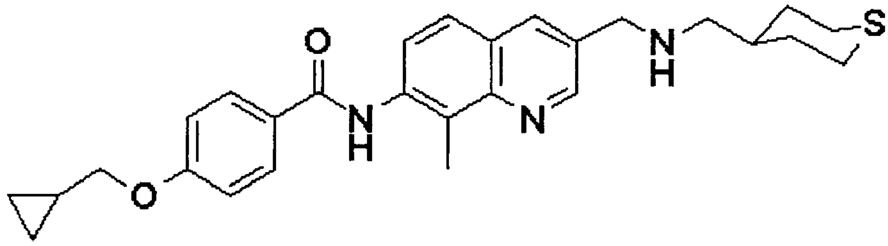

4-(Cyclopropylmethoxy)-N-(3-formyl-8-methylquinolin-7-yl)benzamide (800 mg) obtained in Reference Example 3 and 1-(tetrahydro-2H-thiopyran-4-yl)methanamine hydrochloride (558 mg) obtained in Reference Example 30 were dissolved in a mixed solvent of NMP (16 mL) and acetic acid (2.0 mL), and the mixture was stirred at room temperature for 10 hr. Sodium triacetoxyborohydride (941 mg) was added thereto, and the mixture was stirred at room temperature for 24 hr. The reaction solution was diluted with ethyl acetate, and aqueous sodium hydroxide solution (2M, 50 mL) was added dropwise thereto at 0°C to basify the mixture. The organic layer was washed saturated brine (twice), dried over sodium sulfate, and concentrated under reduced pressure. The residue was purified by silica gel column chromatography [eluent; ethyl acetate→ethyl acetate:methanol=3:1 (volume ratio)], and the obtained solid was recrystallized from diisopropyl ether-ethyl acetate to give the title compound (820 mg, yield 78%) as a pale-yellow solid.
$^1$H NMR (300 MHz, $CDCl_3$) δ: 0.35-0.43 (2 H, m), 0.64-0.74 (2 H, m), 1.21-1.47 (4 H, m), 2.04-2.15 (2 H, m), 2.55 (2 H, d, J=6.4 Hz), 2.57-2.76 (4 H, m), 2.81 (3 H, s), 3.90 (2 H, d, J=7.0 Hz), 3.97 (2 H, s), 7.02 (2 H, d, J=8.9 Hz), 7.70 (1 H, d, J=9.2 Hz), 7.86-7.95 (3 H, m), 8.04 (1 H, d, J=2.1 Hz), 8.27 (1 H, d, J=8.9 Hz), 8.89 (1 H, d, J=2.3 Hz).

Reference Example 32

4-(2-cyclopropylethoxy)-N-[8-methyl-3-({[3-(methylthio)propyl]amino}methyl)quinolin-7-yl]benzamide

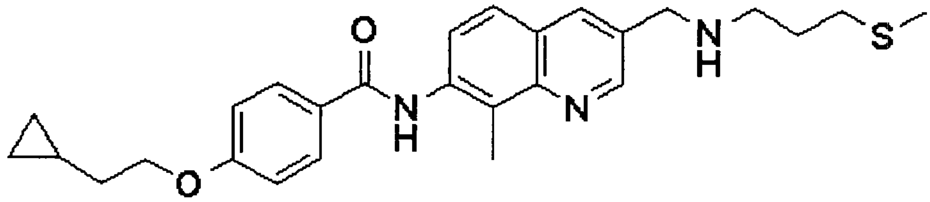

N-[3-(Chloromethyl)-8-methylquinolin-7-yl]-4-(2-cyclopropylethoxy)benzamide hydrochloride (1.00 g) obtained in Reference Example 10 was suspended in NMP (10 mL), ethyldiisopropylamine (1.59 mL) and 3-methylthiopropylamine (1.04 mL) were added thereto, and the mixture was stirred at 40°C for 17.5 hr. To the reaction solution were added water and ethyl acetate, and the organic layer was washed with water and saturated brine. The organic layer was purified

successively by NH silica gel column chromatography [eluent; ethyl acetate→ethyl acetate:methanol=97:3 (volume ratio)], silica gel column chromatography [eluent; ethyl acetate→ethyl acetate:methanol=1:9 (volume ratio)] and NH silica gel column chromatography [eluent; ethyl acetate:methanol=4:1 (volume ratio)→ethyl acetate:methanol=1:9 (volume ratio)], and the obtained solid was recrystallized from ethyl acetate-diisopropyl ether to give the title compound (399 mg, yield 37%) as a white solid.
$^1$H NMR (300 MHz, $CDCl_3$) δ: 0.47-0.56 (2 H, m), 0.82-0.94 (1 H, m), 1.73 (2 H, q, J=6.7 Hz), 1.79-1.91 (2 H, m), 2.10 (3 H, s), 2.59 (2 H, t, J=7.2 Hz), 2.80 (2 H, t, J=7.0 Hz), 2.81 (3 H, s), 4.00 (2 H, s), 4.13 (2 H, t, J=6.7 Hz), 7.02 (2 H, d, J=8.9 Hz), 7.70 (1 H, d, J=8.9 Hz), 7.87-7.95 (3 H, m), 8.06 (1 H, d, J=2.1 Hz), 8.26 (1 H, d, J=8.9 Hz), 8.89 (1 H, d, J=2.3 Hz).

Reference Example 33

4-(2-cyclopropylethoxy)-N-[8-methyl-3-(1-{[3-(methylthio)propyl]amino}ethyl)quinolin-7-yl]benzamide

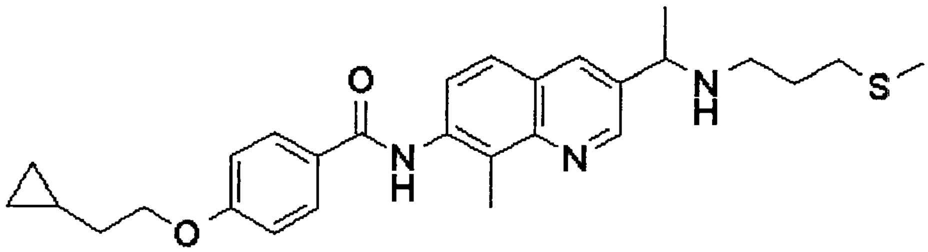

N-[3-(1-Chloroethyl)-8-methylquinolin-7-yl]-4-(2-cyclopropylethoxy)benzamide hydrochloride (1.50 g) obtained in Reference Example 12 was suspended in NMP (10 mL), ethyldiisopropylamine (2.30 mL) and 3-methylthiopropylamine (1.51 mL) were added thereto, and the mixture was stirred at 70°C for 18.5 hr. To the reaction solution were added water and ethyl acetate, and the organic layer was washed with water and saturated brine. The organic layer was purified successively by NH silica gel column chromatography [eluent; ethyl acetate→ethyl acetate:methanol=99:1 (volume ratio)], silica gel column chromatography [eluent; ethyl acetate→ethyl acetate:methanol=1:9 (volume ratio)] and NH silica gel column chromatography [eluent; ethyl acetate:methanol=17:3 (volume ratio)→ethyl acetate:methanol=1:4 (volume ratio)], and the obtained solid was recrystallized from ethyl acetate-diisopropyl ether to give the title compound (376 mg, yield 23%) as a white solid.
$^1$H NMR (300 MHz, $CDCl_3$) δ: 0.10-0.20 (2 H, m), 0.47-0.58 (2 H, m), 0.79-0.95 (1 H, m), 1.47 (3 H, d, J=6.8 Hz), 1.68-1.86 (4 H, m), 2.07 (3 H, s), 2.44-2.63 (3 H, m), 2.69 (1 H, dt, J=11.5, 6.8, 6.6 Hz), 2.82 (3 H, s), 4.00 (1 H, q, J=6.6 Hz), 4.13 (2 H, t, J=6.6 Hz), 7.02 (2 H, d, J=8.7 Hz), 7.71 (1 H, d, J=9.1 Hz), 7.86-7.97 (3 H, m), 8.03 (1 H, d, J=1.9 Hz), 8.27 (1 H, d, J=9.1 Hz), 8.91 (1 H, d, J=2.3 Hz).

Reference Example 34

tetrahydro-4H-thiopyran-4-one oxime

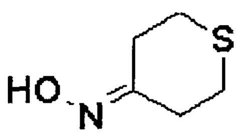

Tetrahydro-4H-thiopyran-4-one (50.0 g), sodium acetate (176.5 g) and hydroxyamine hydrochloride (149.5 g) were suspended in a mixed solvent of ethanol (1000 mL) and water (70 mL), and the suspension was heated under reflux for 13 hr. The reaction solution was allowed to cool to room temperature, and concentrated under reduced pressure. To the residue was added water, and the mixture was extracted three times with ethyl acetate. The extract was washed with water and saturated brine, dried over sodium sulfate, and concentrated under reduced pressure to give the title compound (55.8 g, yield 99%) as pale-yellow crystals.

$^1H$ NMR (300 MHz, $CDCl_3$) δ: 2.52-2.60 (2 H, m), 2.70-2.84 (4 H, m), 2.84-2.91 (2 H, m), 7.65 (1 H, br. s.).

Reference Example 35

tetrahydro-2H-thiopyran-4-amine hydrochloride

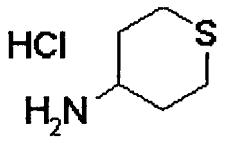

To a suspension of lithium aluminum hydride (21.2 g) in THF (770 mL) was added dropwise a solution of tetrahydro-4H-thiopyran-4-one oxime (27.9 g) obtained in Reference Example 34 in THF (80 mL) at 0°C, and the mixture was heated under reflux for 13 hr. The reaction solution was cooled to 0°C, and diluted with THF (300 mL), and sodium sulfate decahydrate (144 g) was slowly added thereto. The obtained suspension was filtered, and the filtrate was concentrated under reduced pressure. The residue was diluted with ethyl acetate, and hydrogen chloride-ethyl acetate solution (4M, 59 mL) was added dropwise thereto at room temperature. The mixture was stirred at the same temperature for 1 hr, and filtered. The filtrate was washed with ethyl acetate, and dried under reduced pressure to give the title compound (26.9 g, yield 82%) as a pale-pink solid.
$^1H$ NMR (300 MHz, DMSO-$d_6$) δ: 1.53-1.70 (2 H, m), 2.14-2.26 (2 H, m), 2.60-2.75 (4 H, m), 3.03 (1 H, tt, J=11.4, 3.6 Hz), 8.14 (3 H, br. s.).

Reference Example 36

tert-butyl tetrahydro-2H-thiopyran-4-ylcarbamate

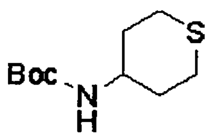

To a suspension of lithium aluminum hydride (13.5 g) in THF (110 mL) was slowly added tetrahydro-4H-thiopyran-4-one oxime (7.48 g) obtained in Reference Example 34 at 0°C, and the mixture was heated under reflux for 7 hr. The reaction solution was cooled to 0°C, and sodium sulfate decahydrate (91.9 g) was slowly added thereto. The obtained suspension was filtered, and the filtrate was concentrated under reduced pressure. The residue was diluted with THF (100 mL), and triethylamine (17.5 mL) and ditert-butyl dicarbonate (13.8 mL) were added thereto, and the mixture was stirred at room temperature for 4 days. The reaction solution was concentrated under reduced pressure, and the residue was partitioned between ethyl acetate and 1N hydrochloric acid. The organic layer was washed with water and saturated brine, dried over sodium sulfate, and concentrated under reduced pressure. The residue was purified by silica gel column chromatography to give the title compound (9.41 g, yield 76%) as a white solid.
$^1H$ NMR (300 MHz, $CDCl_3$) δ: 1.44 (9 H, s), 1.45-1.60 (2 H, m), 2.14-2.30 (2 H, m), 2.56-2.80 (4 H, m), 3.33-3.57 (1 H, m), 4. 46 (1 H, br. s.).

Reference Example 37

tert-butyl (1,1-dioxidotetrahydro-2H-thiopyran-4-yl)carbamate

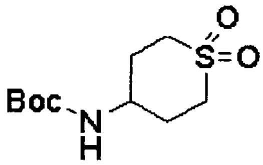

tert-Butyl tetrahydro-2H-thiopyran-4-ylcarbamate (3.01 g) obtained in Reference Example 36 was dissolved in acetone (100 mL), and a solution of oxone (12.7 g) in water (100 mL) was added dropwise thereto at 0°C. The reaction solution was stirred at the same temperature for 2 hr, and concentrated under reduced pressure. The residue was partitioned between ethyl acetate and water, and the organic layer was washed with water and saturated brine, dried over sodium sulfate, and concentrated under reduced pressure to give the title compound (3.14 g, yield 91%) as a white solid.
$^1$H NMR (300 MHz, $CDCl_3$) δ: 1.45 (9 H, s), 2.00-2.19 (2 H, m), 2.23-2.38 (2 H, m), 3.00-3.11 (4 H, m), 3.65-3.83 (1 H, m), 4.50 (1 H, br. d, J=6.1 Hz).

Reference Example 38

tetrahydro-2H-thiopyran-4-amine 1,1-dioxide hydrochloride

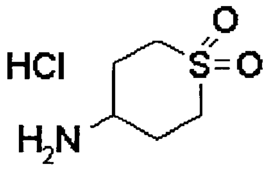

tert-Butyl (1,1-dioxidotetrahydro-2H-thiopyran-4-yl)carbamate (3.14 g) obtained in Reference Example 37 was dissolved in ethyl acetate (110 mL) at 50°C, hydrogen chloride-ethyl acetate solution (4M, 30 mL) was added thereto, and the mixture was stirred at room temperature for 19 hr. The crystals were collected by filtration, washed with ethyl acetate, and dried under reduced pressure to give the title compound (2.39 g, yield 100%) as white crystals.
$^1$H NMR (300 MHz, DMSO-$d_6$) δ: 1. 93-2.11 (2 H, m), 2.23 (2 H, d, J=14.4 Hz), 3.08-3.21 (2 H, m), 3.21-3.46 (3 H, m), 8.10 (3 H, br. s.).

Reference Example 39

4-(2-cyclopropylethoxy)-N-{8-methyl-3-[(tetrahydro-2H-thiopyran-4-ylamino)methyl]quinolin-7-yl}benzamide

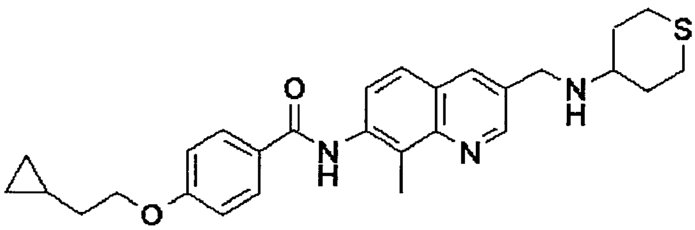

4-(2-Cyclopropylethoxy)-N-(3-formyl-8-methylquinolin-7-yl)benzamide (1.00 g) obtained in Reference Example 8, tetrahydro-2H-thiopyran-4-amine hydrochloride (821 mg) obtained in Reference Example 35 and diisopropylethylamine (0.91 mL) were dissolved in a mixed solvent of NMP (15 mL) and acetic acid (6.0 mL), and the mixture was stirred at room temperature for 3.5 hr. Sodium triacetoxyborohydride (1.70 g) was added thereto, the mixture was stirred at the same temperature for 6.5 hr, and 8N aqueous sodium hydroxide solution was added thereto. The mixture was extracted with ethyl acetate, and the organic layer was washed with water, dried over sodium sulfate, and concentrated under

reduced pressure. The residue was purified successively by silica gel column chromatography [eluent; ethyl acetate→ethyl acetate:methanol=9:1 (volume ratio)] and NH silica gel column chromatography [eluent; hexane:ethyl acetate=1:1 (volume ratio)→ethyl acetate] to give the title compound (795 mg, yield 63%) as yellow crystals.
$^1$H NMR (300 MHz, $CDCl_3$) δ: 0.11-0.19 (2 H, m), 0.47-0.56 (2 H, m), 0.79-0.95 (1 H, m), 1.55-1.68 (2 H, m), 1.73 (2 H, q, J=6.8 Hz), 2.18-2.30 (2 H, m), 2.53-2.78 (5 H, m), 2.81 (3 H, s), 3.49 (1 H, br. s.), 4.02 (2 H, s), 4.13 (2 H, t, J=6.6 Hz), 7.02 (2 H, d, J=8.7 Hz), 7.70 (1 H, d, J=8.7 Hz), 7.86-7.96 (3 H, m), 8.05 (1 H, d, J=2.3 Hz), 8.27 (1 H, d, J=8.7 Hz), 8.89 (1 H, d, J=2.3 Hz).

Reference Example 40

4-(2-cyclopropylethoxy)-2-fluorobenzoic acid

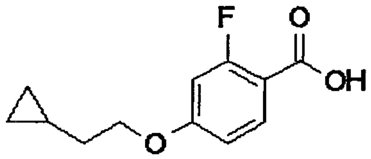

Methyl 2-fluoro-4-hydroxybenzoate (30.6 g), 2-cyclopropylethanol (2.0 g) and triphenylphosphine (5.55 g) were dissolved in THF (25 mL), and a solution of diisopropyl azodicarboxylate (90%, 4.9 mL) in THF (25 mL) was added dropwise thereto over 30 min at 0°C. The mixture was stirred at the same temperature for 1 hr, and to the reaction solution was added water. The mixture was extracted with hexane-ethyl acetate, and the organic layer was washed with water and saturated brine, dried over sodium sulfate, and concentrated under reduced pressure. The residue was purified by silica gel column chromatography [eluent; hexane→hexane:ethyl acetate=9:1 (volume ratio)] to give an orange transparent oil. The oil was dissolved in methanol (50 mL), 1N aqueous sodium hydroxide solution (35 mL) was added thereto, and the mixture was stirred at 50°C for 1 hr. The mixture was allowed to cool to room temperature, and concentrated under reduced pressure to remove methanol. The remaining aqueous solution was washed with hexane-ethyl acetate, and acidified with 1N hydrochloric acid. The precipitate was collected by filtration, washed with water, and dried under reduced pressure to give the title compound (3.87 g, yield 96%) as white crystals.
$^1$H NMR (300 MHz, DMSO-$d_6$) δ: 0.06 - 0.18 (2 H, m), 0.35 - 0.50 (2 H, m), 0.68 - 0.93 (1 H, m), 1.63 (2 H, q, J=6.7 Hz), 4.11 (2 H, t, J=6.6 Hz), 6.77 - 6.96 (2 H, m), 7.70 - 7.89 (1 H, m), 12.84 (1 H, br. s.).

Reference Example 41

4-(2-cyclopropylethoxy)-2-fluoro-N-(3-formyl-8-methylquinolin-7-yl)benzamide

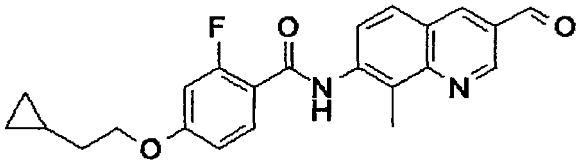

4-(2-Cyclopropylethoxy)-2-fluorobenzoic acid (3.87 g) obtained in Reference Example 40 was dissolved in THF (50 mL), oxalyl dichloride (2.22 mL) and DMF (one drop) were added thereto, and the mixture was stirred at room temperature for 2 hr. The reaction solution was concentrated under reduced pressure, and the residue was azeotropically concentrated with toluene (three times). The residue was dissolved in pyridine (100 mL), 7-amino-8-methylquinoline-3-carbaldehyde (3.21 g) was added thereto, and the mixture was stirred overnight at room temperature. 1N aqueous sodium hydroxide solution was added thereto, and the mixture was filtered. The obtained solid was washed with water, and dried under reduced pressure to give the title compound (6.03 g, yield 89%) as yellow crystals.
$^1$H NMR (300 MHz, DMSO-$d_6$) δ: 0.11-0.18 (2 H, m), 0.41-0.50 (2 H, m), 0.76-0.95 (1 H, m), 1.66 (2 H, q, J=6.8 Hz),

2.72 (3 H, s), 4.14 (2 H, t, J=6.5 Hz), 6.95 (1 H, dd, J=8.4, 2.4 Hz), 7.02 (1 H, dd, J=13.0, 2.3 Hz), 7.78 (1 H, t, J=8.6 Hz), 7.99 (1 H, d, J=9.0 Hz), 8.08 (1 H, d, J=8.4 Hz), 8.94 (1 H, d, J=2.1 Hz), 9.31 (1 H, d, J=2.1 Hz), 10.06 (1 H, d, J=3.4 Hz), 10.25 (1 H, s).

Reference Example 42

4-(2-cyclopropylethoxy)-2-fluoro-N-[3-(hydroxymethyl)-8-methylquinolin-7-yl]benzamide

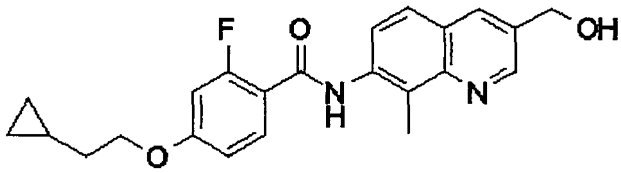

4-(2-Cyclopropylethoxy)-2-fluoro-N-(3-formyl-8-methylquinolin-7-yl)benzamide (3.03 g) obtained in Reference Example 41 and sodium borohydride (90%,652 mg) were suspended in THF (40 mL), and methanol (20 mL) was added dropwise thereto at 0°C. The mixture was stirred at room temperature for 4 hr, and water was added thereto. The mixture was acidified with 1N hydrochloric acid, and then basified with 1N aqueous sodium hydroxide solution. The mixture was filtered, and the obtained solid was washed with water, and dried under reduced pressure to give the title compound (3.03 g, yield 100%) as yellow crystals.
$^1$H NMR (300 MHz, DMSO-$d_6$) δ: 0.10-0.19 (2 H, m), 0.41-0.51 (2 H, m), 0.75-0.94 (1 H, m), 1.66 (2 H, q, J=6.5 Hz), 2.68 (3 H, s), 4.13 (2 H, t, J=6.5 Hz), 4.73 (2 H, d, J=5.5 Hz), 5.47 (1 H, t, J=5.6 Hz), 6.93 (1 H, dd, J=8.7, 2.3 Hz), 7.00 (1 H, dd, J=12.5, 2.2 Hz), 7.68-7.85 (2 H, m), 8.21 (1 H, s), 8.89 (1 H, d, J=2.1 Hz), 9.92 (1 H, d, J=2.6 Hz).

Reference Example 43

N-[3-(chloromethyl)-8-methylquinolin-7-yl]-4-(2-cyclopropylethoxy)-2-fluorobenzamide hydrochloride

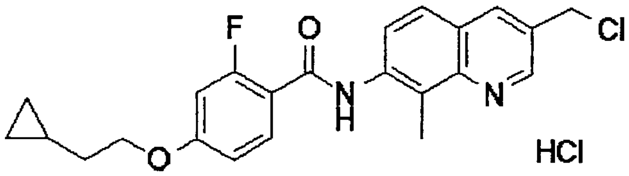

4-(2-Cyclopropylethoxy)-2-fluoro-N-[3-(hydroxymethyl)-8-methylquinolin-7-yl]benzamide (3.03 g) obtained in Reference Example 42 was slowly added at -78°C to thionyl chloride (10 mL), and the mixture was stirred for 2.5 hr while allowed to warm to 0°C. To the reaction solution was added diethyl ether, and the resulting crystals were collected by filtration, washed with toluene and diethyl ether, and dried under reduced pressure to give the title compound (3.33 g, yield 96%) as yellow crystals.
$^1$H NMR (300 MHz, DMSO-$d_6$) δ: 0.11-0.18 (2 H, m), 0.42-0.49 (2 H, m), 0.73-0.96 (1 H, m), 1.66 (2 H, q, J=6.5 Hz), 2.68 (3 H, s), 4.14 (2 H, t, J=6.6 Hz), 5.03 (2 H, s), 6.94 (1 H, dd, J=8.9, 2.4 Hz), 7.00 (1 H, dd, J=13.2, 2.3 Hz), 7.71-7.90 (3 H, m), 8.42 (1 H, d, J=2.4 Hz), 8.99 (1 H, d, J=2.3 Hz), 9.97 (1 H, d, J=2.1 Hz).

Reference Example 44

4-(2-cyclopropylethoxy)-2-fluoro-N-{8-methyl-3-[(tetrahydro-2H-thiopyran-4-ylamino)methyl]quinolin-7-yl}benzamide

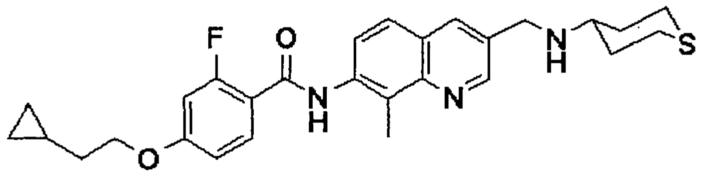

4-(2-Cyclopropylethoxy)-2-fluoro-N-(3-formyl-8-methylquinolin-7-yl)benzamide (1.05 g) obtained in Reference Example 41, tetrahydro-2H-thiopyran-4-amine hydrochloride (821 mg) obtained in Reference Example 35 and diisopropylethylamine (0.91 mL) were dissolved in a mixed solvent of NMP (15 mL) and acetic acid (6.0 mL), and the mixture was stirred at room temperature for 3.5 hr. Sodium triacetoxyborohydride (1.70 g) was added thereto, the mixture was stirred at the same temperature for 6.5 hr, and 8N aqueous sodium hydroxide solution was added thereto. The mixture was extracted with ethyl acetate, and the organic layer was washed with water, dried over sodium sulfate, and concentrated under reduced pressure. The residue was purified successively by silica gel column chromatography [eluent; ethyl acetate→ethyl acetate:methanol=47:3 (volume ratio)] and NH silica gel column chromatography [eluent; hexane:ethyl acetate=7:3 (volume ratio)→ethyl acetate] to give the title compound (901 mg, yield 68%) as a pale-brown crystals.
$^1$H NMR (300 MHz, $CDCl_3$) δ: 0.11-0.19 (2 H, m), 0.48-0.57 (2 H, m), 0.79-0.95 (1 H, m), 1.53-1.65 (2 H, m), 1.73 (2 H, q, J=6.7 Hz), 2.18-2.30 (2 H, m), 2.52-2.79 (5 H, m), 2.82 (3 H, s), 3.49 (1 H, br. s.), 4.02 (2 H, s), 4.12 (2 H, t, J=6.6 Hz), 6.73 (1 H, dd, J=14.6, 2.5 Hz), 6.88 (1 H, dd, J=8.7, 2.3 Hz), 7.70 (1 H, d, J=9.1 Hz), 8.05 (1 H, d, J=1.9 Hz), 8.19 (1 H, t, J=9.3 Hz), 8.39 (1 H, d, J=9.1 Hz), 8.65 (1 H, d, J=17.4 Hz), 8.89 (1 H, d, J=2.3 Hz).

Reference Example 45

tert-butyl [(7-{[4-(cyclopropylmethoxy)benzoyl]amino}-8-methylquinolin-3-yl)methyl][(2-nitrophenyl)sulfonyl]carbamate

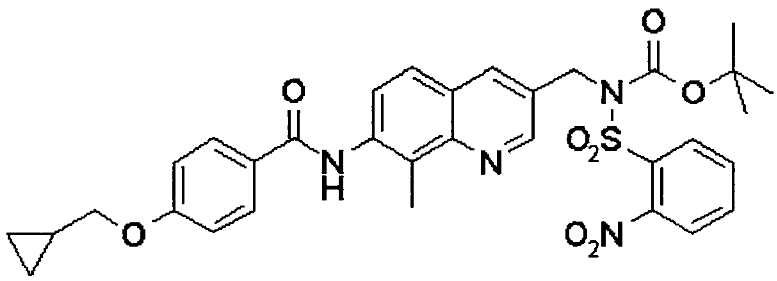

4-(Cyclopropylmethoxy)-N-[3-(hydroxymethyl)-8-methylquinolin-7-yl]benzamide (10.0 g) obtained in Reference Example 4, tert-butyl [(2-nitrophenyl)sulfonyl]carbamate (10.8 g) and triphenylphosphine (10.1 g) were suspended in THF (100 mL), and a solution of diisopropyl azodicarboxylate (8.45 mL) in THF (20 mL) was added dropwise thereto at 0°C. While allowed to warm to room temperature, the reaction solution was stirred for 24 hr, and concentrated under reduced pressure to remove about 2/3 of solvent. The residue was filtered, the filtrate was concentrated, and the residue was purified by silica gel column chromatography [eluent; hexane:ethyl acetate=7:3 (volume ratio)→hexane:ethyl acetate=1:9 (volume ratio)] to give the title compound (14.4 g, yield 80%) as a yellow amorphous solid.
$^1$H NMR (300 MHz, $CDCl_3$) δ: 0.36-0.44 (2 H, m), 0.64-0.74 (2 H, m), 1.24-1.34 (10 H, m), 2.83 (3 H, s), 3.90 (2 H, d, J=7.0 Hz), 5.15 (2 H, s), 7.02 (2 H, d, J=8.9 Hz), 7.72-7.86 (4 H, m), 7.87-7.96 (3 H, m), 8.22 (1 H, d, J=2.3 Hz), 8.30 (1 H, d, J=9.0 Hz), 8.34-8.39 (1 H, m), 9.02 (1 H, d, J=2.4 Hz).

Reference Example 46

4-(cyclopropylmethoxy)-N-[8-methyl-3-({[(2-nitrophenyl)sulfonyl]amino}methyl)quinolin-7-yl]benzamide

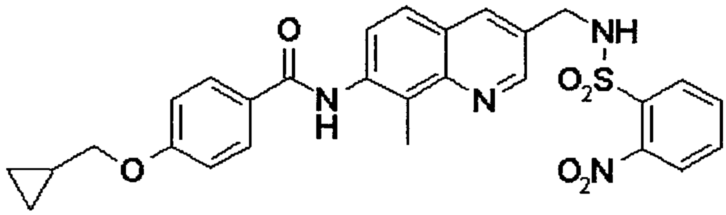

To a solution of tert-butyl [(7-{[4-(cyclopropylmethoxy)benzoyl]amino}-8-methylquinolin-3-yl)methyl][(2-nitrophenyl)sulfonyl]carbamate (14.4 g) obtained in Reference Example 45 in DMF (70 mL) was added hydrogen chloride-ethyl acetate solution (4M, 56 mL), and the mixture was stirred at 60°C for 62 hr. The reaction solution was allowed to cool to room temperature, and 1N aqueous sodium hydroxide solution (400 mL) was added thereto. The organic layer was washed with water (X4), and concentrated under reduced pressure, and the residue was collected by filtration, and dried to give the title compound (10.3 g, yield 84%) as a white solid.
$^1$H NMR (300 MHz, $CDCl_3$) δ: 0.36-0.43 (2 H, m), 0.65-0.73 (2 H, m), 1.27-1.38 (1 H, m), 2.75 (3 H, s), 3.90 (2 H, d, J=6.8 Hz), 4.55 (2 H, d, J=6.4 Hz), 5.90 (1 H, t, J=6.4 Hz), 7.02 (2 H, d, J=8.9 Hz), 7.48-7.56 (1 H, m), 7.58-7.67 (2 H, m), 7.81 (1 H, dd, J=7.9, 1.3 Hz), 7.87-7.94 (3 H, m), 7.96 (1 H, dd, J=7.8, 1.4 Hz), 7.99 (1 H, d, J=2.1 Hz), 8.29 (1 H, d, J=8.9 Hz), 8.75 (1 H, d, J=2.4 Hz).

Reference Example 47

4-(cyclopropylmethoxy)-N-[8-methyl-3-({[4-(methylthio)butyl][(2-nitrophenyl)sulfonyl]amino}methyl)quinolin-7-yl]benzamide

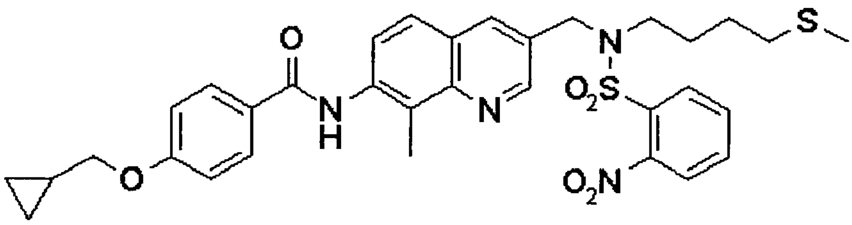

4-(Cyclopropylmethoxy)-N-[8-methyl-3-({[(2-nitrophenyl)sulfonyl]amino}methyl)quinolin-7-yl]benzamide (1.20 g) obtained in Reference Example 46, 4-(methylthio)butanol (0.53 mL) and triphenylphosphine (1.15 g) were dissolved in THF (50 mL), and a solution of diisopropyl azodicarboxylate (purity 90%, 0.96 mL) in THF (10 mL) was added dropwise thereto at 0°C. While allowed to warm to room temperature, the mixture was stirred for 16 hr, and concentrated under reduced pressure. The residue was purified successively by silica gel column chromatography [eluent; hexane:ethyl acetate=7:3 (volume ratio)→hexane:ethyl acetate=3:7 (volume ratio)] and NH silica gel column chromatography [eluent; hexane:ethyl acetate=7:3 (volume ratio)→hexane:ethyl acetate=3:7 (volume ratio)] to give the title compound (1.23 g, yield 87%) as a white solid.
$^1$H NMR (300 MHz, $CDCl_3$) δ: 0.35-0.44 (2 H, m), 0.64-0.74 (2 H, m), 1.26-1.38 (1 H, m), 1.39-1.51 (2 H, m), 1.51-1.65 (2 H, m), 1.99 (3 H, s), 2.35 (2 H, t, J=7.0 Hz), 2.79 (3 H, s), 3.32 (2 H, t, J=7.3 Hz), 3.90 (2 H, d, J=7.0 Hz), 4.74 (2 H, s), 7.02 (2 H, d, J=8.9 Hz), 7.42-7.72 (4 H, m), 7.87-7.95 (3 H, m), 8.01-8.08 (2 H, m), 8.30 (1 H, d, J=9.0 Hz), 8.81 (1 H, d, J=2.3 Hz).

Reference Example 48

4-(cyclopropylmethoxy)-N-[8-methyl-3-({[4-(methylthio)butyl]amino}methyl)quinolin-7-yl]benzamide

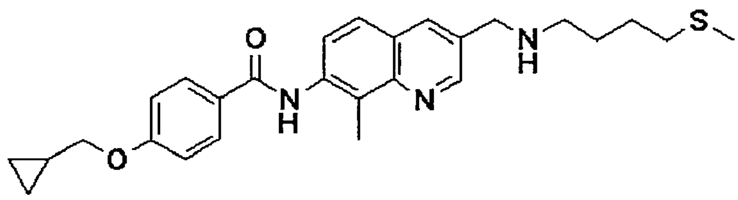

4-(Cyclopropylmethoxy)-N-[8-methyl-3-({[4-(methylthio)butyl][(2-nitrophenyl)sulfonyl]amino}methyl)quinolin-7-yl]benzamide (743 mg) obtained in Reference Example 47 and lithium hydroxide monohydrate (480 mg) were dissolved in DMF (8.0 mL), mercaptoacetic acid (0.50 mL) was added thereto at room temperature, and the mixture was stirred at the same temperature for 5 hr. To the reaction solution were added water and ethyl acetate, and the organic layer was washed with water and saturated brine. The organic layer was purified by silica gel column chromatography [eluent; ethyl acetate:methanol=9:1 (volume ratio)→ethyl acetate:methanol=1:9 (volume ratio)], and the obtained solid was recrystallized from diisopropyl ether-ethyl acetate-methanol to give the title compound (439 mg, yield 83%) as a white solid.
$^1$H NMR (300 MHz, $CDCl_3$) δ: 0.35-0.44 (2 H, m), 0.64-0.74 (2 H, m), 1.25-1.38 (1 H, m), 1.60-1.71 (4 H, m), 2.09 (3 H, s), 2.46-2.56 (2 H, m), 2.70 (2 H, t, J=6.7 Hz), 2.81 (3 H, s), 3.90 (2 H, d, J=7.0 Hz), 3.99 (2 H, s), 7.01 (2 H, d, J=9.0 Hz), 7.71 (1 H, d, J=8.9 Hz), 7.86-7.96 (3 H, m), 8.05 (1 H, d, J=2.1 Hz), 8.26 (1 H, d, J=8.9 Hz), 8.89 (1 H, d, J=2.3 Hz).

Reference Example 49

4-(cyclopropylmethoxy)-N-[8-methyl-3-({[4-(methylsulfonyl)butyl][(2-nitrophenyl)sulfonyl]amino}methyl)quinolin-7-yl] benzamide

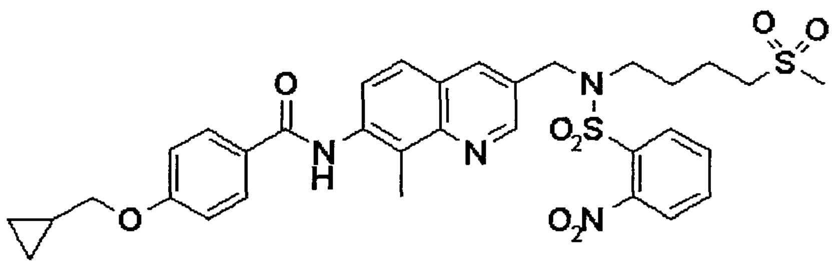

4-(Cyclopropylmethoxy)-N-[8-methyl-3-({[4-(methylthio)butyl][(2-nitrophenyl)sulfonyl]amino}methyl)quinolin-7-yl]benzamide (489 mg) obtained in Reference Example 47 was dissolved in acetone (20 mL), and a solution of oxone (926 mg) in water (10 mL) was added thereto at 0°C. The mixture was stirred at room temperature for 2.5 hr, and concentrated under reduced pressure. The residue was extracted with a mixed solvent of ethyl acetate-THF, and the organic layer was washed with saturated brine, dried over sodium sulfate, and concentrated under reduced pressure. The residue was recrystallized from ethyl acetate-toluene-THF to give the title compound (403 mg, yield 79%) as a white solid.
$^1$H NMR (300 MHz, $CDCl_3$) δ: 0.32-0.40 (2 H, m), 0.56-0.64 (2 H, m), 1.19-1.33 (1 H, m), 1.53 (4 H, t, J=7.1 Hz), 2.63 (3 H, s), 2.84 (3 H, s), 2.98 (2 H, t, J=7.2 Hz), 3.35 (2 H, t, J=5.9 Hz), 3.92 (2 H, d, J=7.0 Hz), 4.77 (2 H, s), 7.07 (2 H, d, J=8.9 Hz), 7.62 (1 H, d, J=8.9 Hz), 7.76 (1 H, d, J=9.0 Hz), 7.84 (1 H, td, J=7.8, 1.5 Hz), 7.91 (1 H, dt, J=7.7, 1.4 Hz), 7.98-8.07 (3 H, m), 8.14 (1 H, dd, J=7.8, 1.4 Hz), 8.17 (1 H, d, J=2.3 Hz), 8.82 (1 H, d, J=2.3 Hz), 10.06 (1 H, s).

Reference Example 50

4-(cyclopropylmethoxy)-N-{3-[1-(1,3-dioxo-1,3-dihydro-2H-isoindol-2-yl)ethyl]-8-methylquinolin-7-yl}benzamide

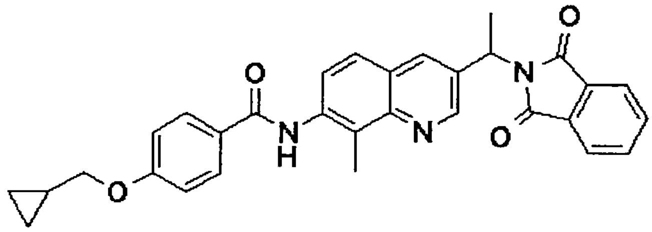

4-(Cyclopropylmethoxy)-N-[3-(1-hydroxyethyl)-8-methylquinolin-7-yl]benzamide (2.0 g) obtained in Reference Example 6 was cooled to 5°C, and the solution was added to thionyl chloride (20 mL). The mixture was allowed to warm to room temperature, stirred for 1 hr, and concentrated under reduced pressure. The obtained solid was dissolved in DMF (20 mL), potassium phthalimide (1.48 g) and potassium carbonate (1.10 g) were added thereto, and the mixture was stirred at room temperature for 5 hr. The reaction mixture was diluted with water (80 mL), and extracted with ethyl acetate. The organic layer was separated, washed with water and saturated brine, dried over magnesium sulfate, and concentrated under reduced pressure. The residue was purified by silica gel column chromatography [eluent; hexane: ethyl acetate=2:1→1:1 (volume ratio)], and the obtained solid was washed with diisopropyl ether, and dried under reduced pressure to give the title compound (1.66 g, yield 62%) as a yellow solid.
$^1$H NMR (300 MHz, $CDCl_3$) δ: 0.24 - 0.47 (2 H, m), 0.59 - 0.76 (2 H, m), 1.18 - 1.43 (1 H, m), 2.05 (3 H, d, J=7.1 Hz), 7.01-7.03 (2 H, m), 7.69 - 7.93 (8 H, m), 8.20 - 8.39 (2 H, m), 9.02 (1 H, s).

Reference Example 51

N-[3-(1-aminoethyl)-8-methylquinolin-7-yl]-4-(cyclopropylmethoxy)benzamide

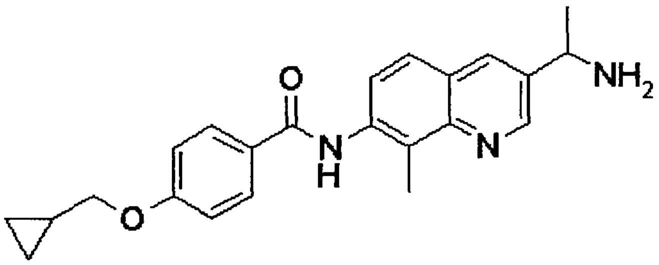

A mixture of 4-(cyclopropylmethoxy)-N-{3-[1-(1,3-dioxo-1,3-dihydro-2H-isoindol-2-yl)ethyl]-8-methylquinolin-7-yl}benzamide (1.60 g) obtained in Reference Example 50, hydrazine monohydrate (3.0 mL) and ethanol (20 mL) was stirred at 90°C for 1 hr. The mixture was allowed to cool to room temperature, the insoluble material was filtered off, and the filtrate was concentrated under reduced pressure. The obtained solid was washed with ether, and dried under reduced pressure to give the title compound (1.13 g, yield 94%) as a pale-brown solid.
$^1$H NMR (300 MHz, $CDCl_3$) δ: 0.31 - 0.47 (2 H, m), 0.58 - 0.77 (2 H, m), 1.17 - 1.41 (1 H, m), 1.51 (3 H, d, J=6.6 Hz), 2.81 (3 H, s), 3.89 (2 H, d, J=6.8 Hz), 4.38 (1 H, q, J=6.6 Hz), 7.01 (2 H, d, J=8.7 Hz), 7.70 (1H, d, J=8.9 Hz), 7.92 (3 H, m), 8.08 (1 H, d, J=2.3 Hz), 8.26 (1 H, d, J=8.9 Hz), 8.93 (1 H, d, J=2.3 Hz).

Reference Example 52

tetrahydro-4H-thiopyran-4-one 1,1-dioxide

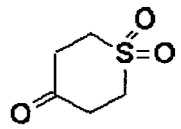

To a solution of tetrahydro-4H-thiopyran-4-one (2.65 g) in ethyl acetate (25 mL) was added peracetic acid (32% acetic acid solution, 13 g) over 1 hr at room temperature. The reaction solution was stirred at the same temperature for 1 hr, and cooled to 0°C. The precipitate was collected by filtration, washed with ethyl acetate (cooled to 0°C in advance), and dried under reduced pressure to give the title compound (3.01 g, yield 89%) as white crystals.
$^1$H NMR (300 MHz, DMSO-$d_6$) δ: 2.03-2.35 (4 H, m), 2.93-3.12 (2 H, m), 3.11-3.29 (2 H, m).

Reference Example 53

4-(cyclopropylmethoxy)-N-{8-methyl-3-[(1R)-1-(tetrahydro-2H-thiopyran-4-ylamino)ethyl]quinolin-7-yl}benzamide

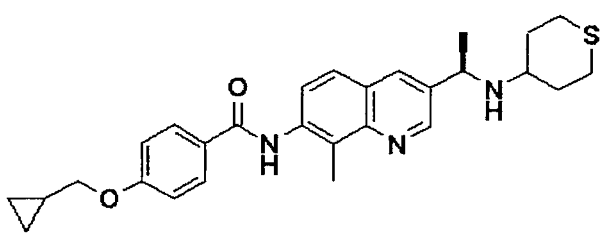

N-{3-[(1R)-1-Aminoethyl]-8-methylquinolin-7-yl}-4-(cyclopropylmethoxy)benzamide (4.00 g) (obtained by subjecting the product obtained in Reference Example 51 to a optical resolution with chiral column) and tetrahydro-4H-thiopyran-4-one 1,1-dioxide (1.59 g) obtained in Reference Example 52 were suspended in a mixed solvent of NMP (30 mL) and acetic acid (3.0 mL), and the mixture was stirred at room temperature for 2.5 hr. Sodium triacetoxyborohydride (5.45 g) was added thereto, and the mixture was stirred at room temperature for 62 hr. The reaction was quenched with aqueous sodium hydroxide solution to basify the mixture. The mixture was extracted with ethyl acetate, and the organic layer was washed with water and saturated brine. The organic layer was purified successively by silica gel column chromatography [eluent; ethyl acetate→ethyl acetate:methanol=93:7 (volume ratio)] and NH silica gel column chromatography [eluent; ethyl acetate→ethyl acetate:methanol=49:1 (volume ratio)] to give the title compound (5.00 g, yield 99%) as a pale-yellow solid.
$^1$H NMR (300 MHz, $CDCl_3$) δ: 0.36-0.43 (2 H, m), 0.65-0.73 (2 H, m), 1.26-1.38 (1 H, m), 1.44 (3 H, d, J=6.6 Hz), 1.46-1.54 (2 H, m), 1.97-2.09 (1 H, m), 2.26-2.40 (2 H, m), 2.45-2.56 (2 H, m), 2.56-2.72 (2 H, m), 2.82 (3 H, s), 3.90 (2 H, d, J=7.0 Hz), 4.20 (1 H, q, J=6.7 Hz), 7.02 (2 H, d, J=8.9 Hz), 7.71 (1 H, d, J=9.0 Hz), 7.87-7.95 (3 H, m), 8.01 (1 H, d, J=2.3 Hz), 8.27 (1 H, d, J=8.9 Hz), 8.91 (1 H, d, J=2.3 Hz).

Reference Example 54

N-[3-(azidomethyl)-8-methylquinolin-7-yl]-4-(cyclopropylmethoxy)benzamide

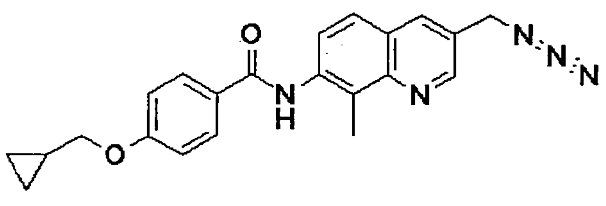

4-(Cyclopropylmethoxy)-N-[3-(hydroxymethyl)-8-methylquinolin-7-yl]benzamide (4.27 g) obtained in Reference Example 4 was dissolved in DMF (110 mL), DBU (2.64 mL) and DPPA (3.81 mL) were added thereto, and the mixture was stirred at room temperature for 67 hr. The reaction solution was partitioned between ethyl acetate and water, and the organic layer was washed with water and saturated brine, dried over sodium sulfate, and concentrated under reduced pressure. The residue was collected by filtration, and dried under reduced pressure to give the title compound (3.44 g, yield 75%) as a white solid.
$^1$H NMR (300 MHz, $CDCl_3$) δ: 0.36-0.43 (2 H, m), 0.65-0.74 (2 H, m), 1.24-1.39 (1 H, m), 2.82 (3 H, s), 3.90 (2 H, d, J=7.0 Hz), 4.57 (2 H, s), 7.02 (2 H, d, J=8.9 Hz), 7.75 (1 H, d, J=9.0 Hz), 7.88-7.96 (3 H, m), 8.08 (1 H, d, J=2.3 Hz), 8.35 (1 H, d, J=9.0 Hz), 8.88 (1 H, d, J=2.3 Hz).

Reference Example 55

N-[3-(aminomethyl)-8-methylquinolin-7-yl]-4-(cyclopropylmethoxy)benzamide

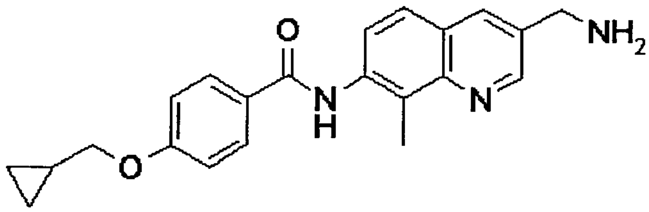

N-[3-(Azidomethyl)-8-methylquinolin-7-yl]-4-(cyclopropylmethoxy)benzamide (3.44 g) obtained in Reference Example 54 was dissolved in a mixed solvent of ethyl acetate (200 mL) and methanol (100 mL), and palladium-activated carbon-ethylenediamine complex (3.9wt%, 0.485 g) was added thereto. The mixture was stirred at room temperature for 17 hr under a hydrogen atmosphere (1 atm), and filtered through celite. The filtrate was concentrated under reduced pressure to give the title compound (2.77 g, yield 86%) as a white solid.
$^1$H NMR (300 MHz, $CDCl_3$) δ: 0.36-0.43 (2 H, m), 0.65-0.74 (2 H, m), 1.26-1.38 (1 H, m), 2.82 (3 H, s), 3.90 (2 H, d, J=7.0 Hz), 4.10 (2 H, s), 7.02 (2 H, d, J=8.9 Hz), 7.71 (1 H, d, J=8.9 Hz), 7.88-7.96 (3 H, m), 8.06 (1 H, d, J=2.3 Hz), 8.26 (1 H, d, J=9.0 Hz), 8.89 (1 H, d, J=2.4 Hz).

Reference Example 56

4-(cyclopropylmethoxy)-N-{8-methyl-3-[(tetrahydro-2H-thiopyran-4-ylamino)methyl]quinolin-7-yl}benzamide

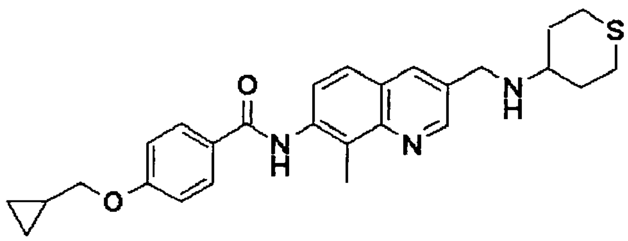

N-[3-(Aminomethyl)-8-methylquinolin-7-yl]-4-(cyclopropylmethoxy)benzamide (1.50 g) obtained in Reference Example 55 and tetrahydro-4H-thiopyran-4-one (627 mg) were added to a mixed solvent of acetic acid (5.0 mL) and NMP (25 mL), and the mixture was stirred at room temperature for 7 hr. Sodium triacetoxyborohydride (2.20 g) was added thereto at the same temperature, and the mixture was stirred for additional 14 hr. The mixture was basified with 1N aqueous sodium hydroxide solution, and extracted with ethyl acetate. The organic layer was washed with water and saturated brine, dried over sodium sulfate, and concentrated under reduced pressure. The residue was purified by silica gel column chromatography [eluent; ethyl acetate→ethyl acetate:methanol=17:3 (volume ratio)], and the obtained solid

was recrystallized from diisopropyl ether-methanol to give the title compound (1.09 g, yield 57%) as white crystals. $^1$H NMR (300 MHz, $CDCl_3$) δ: 0.35-0.44 (2 H, m), 0.64-0.73 (2 H, m), 1.25-1.38 (1 H, m), 1.57-1.70 (2 H, m), 2.18-2.30 (2 H, m), 2.51-2.78 (5 H, m), 2.81 (3 H, s), 3.90 (2 H, d, J=6.8 Hz), 4.02 (2 H, s), 7.01 (2 H, d, J=8.7 Hz), 7.70 (1 H, d, J=8.7 Hz), 7.85-7.96 (3 H, m), 8.05 (1 H, d, J=1.9 Hz), 8.26 (1 H, d, J=8.7 Hz), 8.89 (1 H, d, J=2.3 Hz).

Reference Example 57

ethyl 2-fluoro-4-hydroxybenzoate

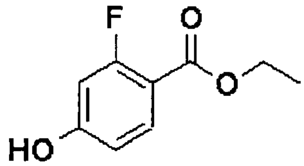

2-Fluoro-4-hydroxybenzoic acid (15.7 g) was suspended in ethanol (100 mL), and concentrated sulfuric acid (3.0 mL) was added thereto at room temperature. The mixture was stirred at 95°C for 26 hr, and concentrated under reduced pressure. The residue was cooled to 0°C, and water (60 mL) was added dropwise thereto. The obtained solid was collected by filtration, washed with water, and dried to give the title compound (16.6 g, yield 90%) as a white solid. $^1$H NMR (300 MHz, $CDCl_3$) δ: 1.38 (3 H, t, J=7.2 Hz), 4.37 (2 H, q, J=7.2 Hz), 6.57-6.70 (2 H, m), 7.87 (1 H, t, J=8.5 Hz).

Reference Example 58

ethyl 2-fluoro-4-(cyclopropylmethoxy)benzoate

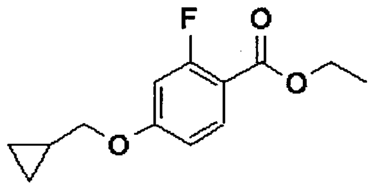

Ethyl 2-fluoro-4-hydroxybenzoate (45.2 g) obtained in Reference Example 57, cyclopropylmethyl bromide (51.8 g) and potassium carbonate (40.7 g) were added to acetonitrile (500 mL), and the mixture was stirred at 80°C for 22 hr. The reaction solution was allowed to cool to room temperature, and filtered, and the residue was washed with ethyl acetate. The filtrate and the washing solution were concentrated under reduced pressure, and the residue was partitioned between ethyl acetate and water. The organic layer was washed with saturated brine, dried over magnesium sulfate, and concentrated under reduced pressure to give the title compound (57.8 g, yield 99%) as a pale-yellow oil. $^1$H NMR (300 MHz, $CDCl_3$) δ: 0.32-0.40 (2 H, m), 0.62-0.72 (2 H, m), 1.21-1.32 (1 H, m), 1.38 (3 H, t, J=7.2 Hz), 3.83 (2 H, d, J=7.0 Hz), 4.36 (2 H, q, J=7.0 Hz), 6.61 (1 H, dd, J=12.8, 2.4 Hz), 6.71 (1 H, dd, J=8.9, 2.4 Hz), 7.88 (1 H, t, J=8.8 Hz).

Reference Example 59

2-fluoro-4-(cyclopropylmethoxy)benzoic acid

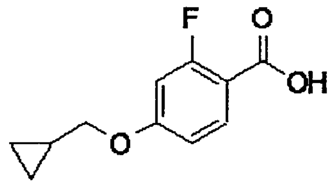

Ethyl 2-fluoro-4-(cyclopropylmethoxy)benzoate (6.53 g) obtained in Reference Example 58 was dissolved in a mixed solvent of methanol (34 mL) and THF (34 mL), 4N aqueous sodium hydroxide solution (34 mL) was added thereto at room temperature. The reaction solution was stirred at 60°C for 1.5 hr, and concentrated under reduced pressure. The residue was dissolved in water (100 mL), and the solution was acidified with concentrated hydrochloric acid at 0°C. The resulting precipitate was collected by filtration, washed with water, and dried to give the title compound (5.60 g, yield 97%) as a white solid.
$^1$H NMR (300 MHz, $CDCl_3$) δ: 0.32-0.42 (2 H, m), 0.64-0.73 (2 H, m), 1.19-1.37 (1 H, m), 3.86 (2 H, d, J=7.0 Hz), 6.65 (1 H, dd, J=12.9, 2.4 Hz), 6.75 (1 H, dd, J=8.9, 2.4 Hz), 7.96 (1 H, t, J=8.8 Hz).

Reference Example 60

4-(cyclopropylmethoxy)-2-fluoro-N-(2-methyl-3-nitrophenyl)benzamide

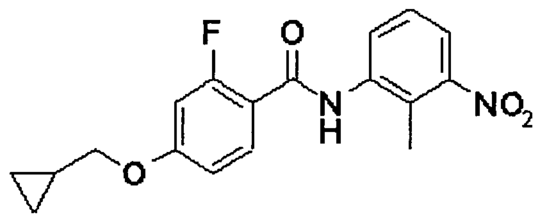

2-Fluoro-4-(cyclopropylmethoxy)benzoic acid (5.00 g) obtained in Reference Example 59, thionyl chloride (2.08 mL) and DMF (3 drops) were dissolved in toluene (50 mL), and the mixture was stirred at 50°C for 5 hr, and concentrated under reduced pressure. The residue was mixed with 2-methyl-3-nitroaniline (3.98 g) and triethylamine (3.65 mL) and NMP (50 mL) at 5°C, and the mixture was stirred at room temperature for 24 hr. The reaction solution was diluted with ethyl acetate, washed with 1N hydrochloric acid, saturated aqueous sodium hydrogen carbonate solution and saturated brine, dried over sodium sulfate, and concentrated under reduced pressure. The residue was washed with ethyl acetate-diisopropyl ether, and dried to give the title compound (5.79 g, yield 71%) as a white solid.
$^1$H NMR (300 MHz, DMSO-$d_6$) δ: 0.32-0.38 (2 H, m), 0.56-0.64 (2 H, m), 1.15-1.32 (1 H, m), 2.31 (3 H, s), 3.92 (2 H, d, J=7.2 Hz), 6.89-7.00 (2 H, m), 7.47 (1 H, t, J=8.0 Hz), 7.68-7.81 (3 H, m), 9.98 (1 H, s).

Reference Example 61

N-(3-amino-2-methylphenyl)-4-(cyclopropylmethoxy)-2-fluorobenzamide

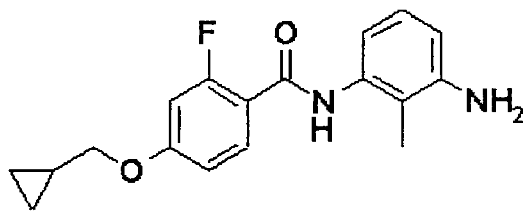

4-(Cyclopropylmethoxy)-2-fluoro-N-(2-methyl-3-nitrophenyl)benzamide (5.79 g) obtained in Reference Example 60 and palladium-carbon (10%,600 mg) were suspended in a mixed solvent of ethanol (400 mL) and THF (400 mL), and the mixture was stirred at room temperature for 24 hr under a hydrogen atmosphere (1 atm). The mixture was filtered through celite, and the filtrate was concentrated under reduced pressure. The obtained residue was washed with diisopropyl ether, and dried to give the title compound (4.68 g, yield 89%) as a white solid.
$^1$H NMR (300 MHz, DMSO-$d_6$) δ: 0.31-0.38 (2 H, m), 0.55-0.63 (2 H, m), 1.17-1.31 (1 H, m), 1.94 (3 H, s), 3.90 (2 H, d, J=7.0 Hz), 4.88 (2 H, s), 6.53 (1 H, d, J=7.7 Hz), 6.61 (1 H, d, J=7.5 Hz), 6.83-6.95 (3 H, m), 7.65 (1 H, t, J=8.5 Hz), 9.43 (1 H, d, J=2. 6 Hz).

Reference Example 62

4-(cyclopropylmethoxy)-2-fluoro-N-(3-formyl-8-methylquinolin-7-yl)benzamide

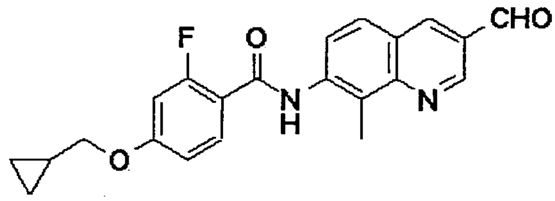

2-Dimethylaminomethylene-1,3-bis(dimethyliminio)propane bistetrafluoroborate (10.6 g) and morpholine (7.81 mL) were suspended in 1-butanol (80 mL), N-(3-amino-2-methylphenyl)-4-(cyclopropylmethoxy)-2-fluorobenzamide (4.68 g) obtained in Reference Example 61 was added thereto, and the mixture was stirred at 80°C for 18 hr. Acetic acid (14 mL) and water (14 mL) were added successively thereto at the same temperature, and the mixture was stirred for 1 hr, and allowed to cool to room temperature. The mixture was filtered, and the obtained solid was washed successively with a mixed solvent of acetic acid-water (1/1) and water, and dried to give the title compound (5.20 g, yield 92%) as a yellow solid.
$^1$H NMR (300 MHz, DMSO-$d_6$) δ: 0.32-0.40 (2 H, m), 0.56-0.64 (2 H, m), 1.18-1.34 (1 H, m), 2.72 (3 H, s), 3.93 (2 H, d, J=6.9 Hz), 6.93 (1 H, dd, J=8.5, 2.2 Hz), 6.98 (1 H, dd, J=13.2, 2.2 Hz), 7.77 (1 H, t, J=8.7 Hz), 7.98 (1 H, d, J=9.1 Hz), 8.06 (1 H, d, J=8.8 Hz), 8.90 (1 H, d, J=1.9 Hz), 9.29 (1 H, d, J=2.2 Hz), 10.00 (1 H, d, J=3.3 Hz), 10.24 (1 H, s).

Reference Example 63

4-(cyclopropylmethoxy)-2-fluoro-N-[3-(hydroxymethyl)-8-methylquinolin-7-yl]benzamide

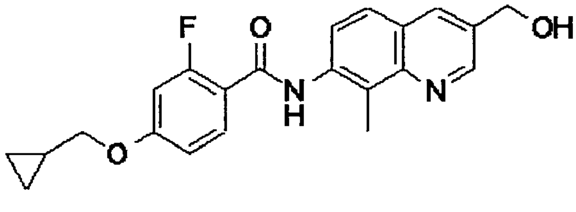

4-(Cyclopropylmethoxy)-2-fluoro-N-(3-formyl-8-methylquinolin-7-yl)benzamide (4.00 g) obtained in Reference Example 62 and sodium borohydride (90%,2.80 g) were suspended in ethanol (40 mL), the mixture was stirred at 0°C for 1 hr, and methanol (20 mL) and THF (20 mL) were added dropwise thereto. While allowed to warm to room temperature, the mixture was stirred for 18 hr, acidified with 1N hydrochloric acid, and basified with 1N aqueous sodium hydroxide solution. The mixture was filtered, and the obtained solid was washed with water, methanol and diisopropyl ether, and dried under reduced pressure to give the title compound (3.76 g, yield 94%) as a white solid.
$^1$H NMR (300 MHz, DMSO-$d_6$) δ: 0.33-0.39 (2 H, m), 0.56-0.64 (2 H, m), 1.18-1.33 (1 H, m), 2.67 (3 H, s), 3.93 (2 H, d, J=6.9 Hz), 4.73 (2 H, d, J=4.9 Hz), 5.42-5.48 (1 H, m), 6.89-7.00 (2 H, m), 7.69-7.83 (3 H, m), 8.18-8.21 (1 H, m), 8.88 (1 H, d, J=2.2 Hz), 9.88 (1 H, s).

Reference Example 64

N-[3-(chloromethyl)-8-methylquinolin-7-yl]-4-(cyclopropylmethoxy)-2-fluorobenzamide hydrochloride

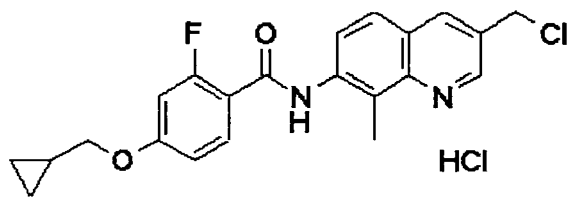

4-(Cyclopropylmethoxy)-2-fluoro-N-[3-(hydroxymethyl)-8-methylquinolin-7-yl]benzamide (27.7 g) obtained in Reference Example 63 was added to thionyl chloride (50 mL) at -78°C. While allowed to warm to room temperature, the mixture was stirred for 45 min. To the reaction solution was added toluene (150 mL), and the mixture was stirred for 15 hr. The resulting crystals were collected by filtration, washed successively with water and diethyl ether, and dried under reduced pressure to give the title compound (29.9 g, yield 94%) as a pale-yellow solid.
$^1$H NMR (300 MHz, DMSO-$d_6$) δ: 0.32-0.39 (2 H, m), 0.56-0.64 (2 H, m), 1.18-1.33 (1 H, m), 2.70 (3 H, s), 3.93 (2 H, d, J=7.1 Hz), 5.07 (2 H, s), 6.89-7.02 (2 H, m), 7.76 (1 H, t, J=8.7 Hz), 7.89-7.98 (2 H, m), 8.64 (1 H, d, J=1.6 Hz), 9.07 (1 H, d, J=2.2 Hz), 10.06 (1 H, d, J=2.5 Hz), 11.18 (1 H, s).

Reference Example 65

4-(cyclopropylmethoxy)-N-[3-{(1,3-dioxido-1,3-dihydro-2H-isoindol-2-yl)methyl}-8-methylquinolin-7-yl]-2-fluorobenzamide

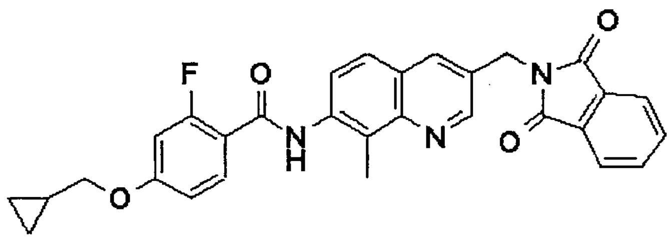

N-[3-(Chloromethyl)-8-methylquinolin-7-yl]-4-(cyclopropylmethoxy)-2-fluorobenzamide hydrochloride (29.9 g) obtained in Reference Example 64 was suspended in NMP (680 mL), and potassium carbonate (20.9 g) was added thereto at 30 - 40°C. The mixture was stirred at room temperature for 1 hr, potassium phthalimide (30.6g) was added thereto, and the mixture was stirred at 50°C for 15 hr. The mixture was allowed to cool to room temperature, water (600 mL) was added dropwise thereto, and the mixture was stirred for 30 min. 1N aqueous potassium hydroxide solution (600 mL) were added dropwise thereto at the same temperature, and the mixture was stirred for 30 min. The precipitate was collected by filtration, washed with water, and dried to give the title compound (32.0 g, yield 91%) as a pale-yellow solid.
$^1$H NMR (300 MHz, DMSO-$d_6$) δ: 0.32-0.39 (2 H, m), 0.55-0.64 (2 H, m), 1.18-1.31 (1 H, m), 2.65 (3 H, s), 3.92 (2 H, d, J=7.1 Hz), 5.00 (2 H, s), 6.86-7.00 (2 H, m), 7.69-7.95 (7 H, m), 8.23 (1 H, d, J=2.2 Hz), 8.93 (1 H, d, J=2.2 Hz), 9.88 (1 H, s).

Reference Example 66

N-[3-(aminomethyl)-8-methylquinolin-7-yl]-4-(cyclopropylmethoxy)-2-fluorobenzamide

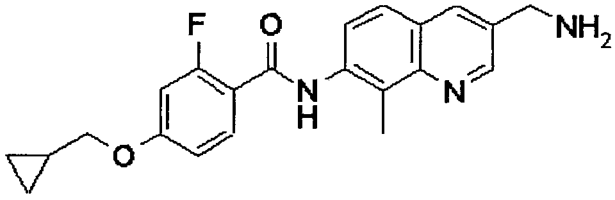

4-(Cyclopropylmethoxy)-N-[3-{(1,3-dioxido-1,3-dihydro-2H-isoindol-2-yl)methyl}-8-methylquinolin-7-yl]-2-fluorobenzamide (32.0 g) obtained in Reference Example 65 was dissolved in NMP (700 mL) at 90°C, hydrazine monohydrate (9.15 mL) was added thereto at 80°C, and the mixture was stirred for 2 hr. The mixture was allowed to cool to room temperature, ethyl acetate (700 mL) was added thereto, and the mixture was stirred for 30 min. The resulting precipitate was filtered off, and washed with ethyl acetate. The filtrate and the washing solution were washed with saturated brine and water, and the aqueous layer was extracted with ethyl acetate. The combined organic layers were washed with saturated brine, and extracted with a mixed solvent of aqueous citric acid solution (10%, 1000 mL) and DMSO (660 mL). The aqueous layer was washed with ethyl acetate (800 mL), 8N aqueous sodium hydroxide solution (250 mL) was added to the aqueous layer, and the mixture was stirred at room temperature for 30 min, and then at 5°C for 2 hr. The resulting precipitate was collected by filtration, washed with water, and dried to give a pale-yellow solid.
On the other hand, the solid collected by filtration from the reaction solution was dissolved in a mixed solvent of THF (250 mL) and 1N aqueous sodium hydroxide solution (450 mL), and the mixture was extracted with ethyl acetate (1000 mL). The organic layer was washed with saturated brine, dried over magnesium sulfate, and concentrated under reduced pressure.
The residue was mixed with the above-mentioned pale-yellow solid, washed with diisopropyl ether-ethyl acetate, and dried to give the title compound (21.3 g, yield 89%) as a pale-yellow solid.
$^1H$ NMR (300 MHz, DMSO-$d_6$) δ: 0.32-0.39 (2 H, m), 0.56-0.64 (2 H, m), 1.18-1.33 (1 H, m), 1.99 (2 H, s), 2.67 (3 H, s), 3.89-3.95 (4 H, m), 6.87-6.99 (2 H, m), 7.67-7.78 (3 H, m), 8.18 (1 H, d, J=1.9 Hz), 8.88 (1 H, d, J=1.9 Hz), 9.86 (1 H, s).

Reference Example 67

4-(cyclopropylmethoxy)-2-fluoro-N-{8-methyl-3-[(tetrahydro-2H-thiopyran-4-ylamino)methyl]quinolin-7-yl}benzamide

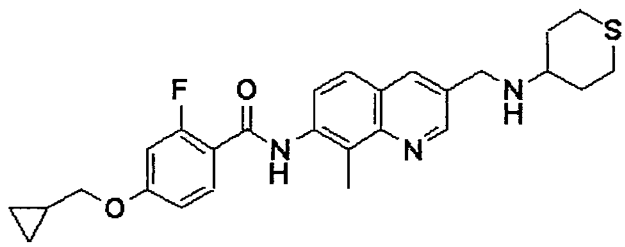

N-[3-(Aminomethyl)-8-methylquinolin-7-yl]-4-(cyclopropylmethoxy)-2-fluorobenzamide (1.37 g) obtained in Reference Example 66 and tetrahydro-4H-thiopyran-4-one (1.26 g) were dissolved in a mixed solvent of acetic acid (9.0 mL) and DMA (30 mL), and sodium triacetoxyborohydride (2.30 g) was added thereto at room temperature. The mixture was stirred at room temperature for 15 hr, and 8N aqueous sodium hydroxide solution (20 mL) was added thereto at 5°C. The mixture was diluted with ethyl acetate, washed with water, 1N aqueous sodium hydroxide solution and saturated brine, dried over sodium sulfate, and concentrated under reduced pressure. The residue was purified by silica gel column chromatography [eluent; ethyl acetate→ethyl acetate:methanol=4:1 (volume ratio)], and the obtained solid was washed with diisopropyl ether-ethyl acetate, and dried to give the title compound (920 mg, yield 53%) as a white solid.
$^1H$ NMR (300 MHz, $CDCl_3$) δ: 0.36-0.43 (2 H, m), 0.66-0.74 (2 H, m), 1.20-1.38 (1 H, m), 1.52-1.68 (2 H, m), 2.20-2.29 (2 H, m), 2.53-2.78 (5 H, m), 2.82 (3 H, s), 3.89 (2 H, d, J=7.0 Hz), 4.02 (2 H, s), 6.73 (1 H, dd, J=14.6, 2.4 Hz), 6.87 (1 H, dd, J=8.9, 2.4 Hz), 7.70 (1 H, d, J=9.0 Hz), 8.05 (1 H, d, J=2.1 Hz), 8.15-8.23 (1 H, m), 8.38 (1 H, d, J=9.0 Hz), 8.65 (1 H, d, J=17.1 Hz), 8.89 (1 H, d, J=2.3 Hz).

Reference Example 68

N-(3-amino-2-fluorophenyl)-4-(cyclopropylmethoxy)benzamide

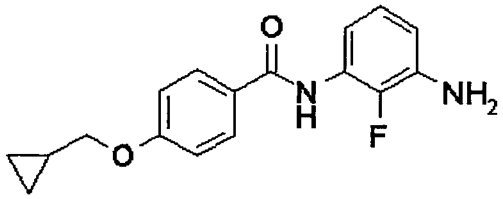

4-(Cyclopropylmethoxy)benzoic acid (27.8 g) obtained in Reference Example 1, thionyl chloride (12.7 mL) and DMF (6 drops) were added to toluene (300 mL), and the mixture was stirred at 50°C for 5 hr, and concentrated under reduced pressure. A solution of the residue in toluene (270 mL) was added dropwise at 5°C to a solution of 2-fluorobenzene-1,3-diamine (18.3 g) and triethylamine (21.2 mL) in a mixed solvent of THF (110 mL), diisopropyl ether (50 mL) and toluene (180 mL), and the mixture was stirred at room temperature for 15 hr. The reaction solution was diluted with acetone (600 mL), water (200 mL) and saturated brine (100 mL), and the separated organic layer was washed with aqueous sodium hydrogen carbonate solution and water. The solvent was evaporated under reduced pressure, and the residue was washed with ethanol (120 mL), collected by filtration, and dried. To the obtained solid were added DMSO, ethyl acetate and 1N hydrochloric acid, the mixture was stirred, and the insoluble material was filtered off. The mother liquor was basified with 1N aqueous sodium hydroxide solution, and the mixture was extracted with ethyl acetate. The organic layer was washed brine, and concentrated under reduced pressure. The residue was washed with ethyl acetate-diisopropyl ether, and dried to give the title compound (19.8 g, yield 45%) as a white solid.
$^1$H NMR (300 MHz, DMSO-$d_6$) δ: 0.31-0.38 (2 H, m), 0.55-0.63 (2 H, m), 1.17-1.32 (1 H, m), 3.90 (2 H, d, J=7.0 Hz), 5.14 (2 H, s), 6. 62 (1 H, td, J=8.1, 1.7 Hz), 6. 69 (1 H, ddd, J=8.1, 6.6, 1.7 Hz), 6.83 (1 H, td, J=7.9, 1.1 Hz), 7.00-7.06 (2 H, m), 7.90-7.96 (2 H, m), 9.70 (1 H, s).

Reference Example 69

4-(cyclopropylmethoxy)-N-(8-fluoro-3-formylquinolin-7-yl)benzamide

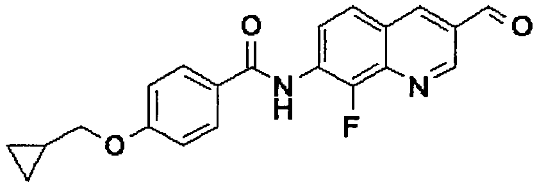

2-Dimethylaminomethylene-1,3-bis(dimethyliminio)propane bistetrafluoroborate (46.9 g) and morpholine (34.5 mL) were suspended in 1-butanol (210 mL), N-(3-amino-2-fluorophenyl)-4-(cyclopropylmethoxy)benzamide (20.0 g) obtained in Reference Example 68 was added thereto, and the mixture was stirred at 80°C for 16 hr. Acetic acid (35 mL) and water (35 mL) were added successively thereto at the same temperature, and the mixture was stirred for additional 1 hr, and allowed to cool to room temperature. The mixture was filtered, and the obtained solid was washed successively with a mixed solvent of acetic acid-water (1/1) and water, and dried to give the title compound (19.9 g, yield 83%) as a pale-brown solid.
$^1$H NMR (300 MHz, DMSO-$d_6$) δ: 0.32-0.40 (2 H, m), 0.56-0.64 (2 H, m), 1.19-1.35 (1 H, m), 3.94 (2 H, d, J=7.2 Hz), 7.09 (2 H, d, J=8.7 Hz), 8.00-8.10 (4 H, m), 9.01 (1 H, s), 9.33 (1 H, d, J=1.9 Hz), 10.26 (1 H, s), 10.42 (1 H, s).

Reference Example 70

4-(cyclopropylmethoxy)-N-[8-fluoro-3-(hydroxymethyl)quinolin-7-yl]benzamide

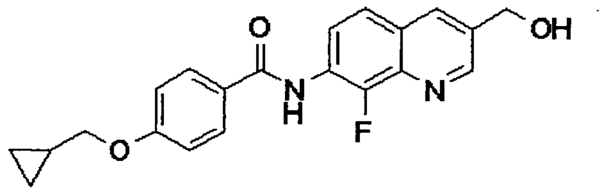

Sodium borohydride (90%,415 mg) and 4-(cyclopropylmethoxy)-N-(8-fluoro-3-formylquinolin-7-yl)benzamide (2.00 g) obtained in Reference Example 69 were suspended in ethanol (20 mL), the mixture was stirred at 5°C for 1 hr, and methanol (10 mL) and THF (10 mL) were added dropwise thereto. While allowed to warm to room temperature, the mixture was stirred for 18 hr, acidified with 1N hydrochloric acid, and basified with 1N aqueous sodium hydroxide solution. The mixture was filtered, and the obtained solid was washed with water, methanol and diisopropyl ether, and dried under reduced pressure to give the title compound (2.00 g, yield 99%) as a white solid.
$^1$H NMR (300 MHz, DMSO-$d_6$) δ: 0.33-0.40 (2 H, m), 0.56-0.64 (2 H, m), 1.19-1.34 (1 H, m), 3.93 (2 H, d, J=7.0 Hz), 4.75 (2 H, s), 5.52 (1 H, s), 7.04-7.11 (2 H, m), 7.77-7.86 (2 H, m), 7.99-8.05 (2 H, m), 8.30 (1 H, s), 8.91 (1 H, d, J=2.1 Hz), 10.25 (1 H, s).

Reference Example 71

4-(cyclopropylmethoxy)-N-{3-[(1,3-dioxo-1,3-dihydro-2H-isoindol-2-yl)methyl]-8-fluoroquinolin-7-yl}benzamide

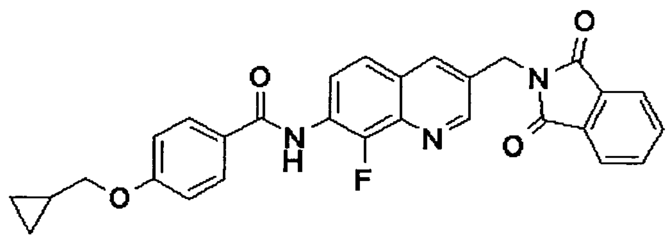

Thionyl chloride (0.438 mL) was added dropwise at -10°C to a solution of 4-(cyclopropylmethoxy)-N-[8-fluoro-3-(hydroxymethyl)quinolin-7-yl]benzamide (2.00 g) obtained in Reference Example 70 in NMP (15 ml), and the mixture was stirred at -10°C for 30 min, and then at room temperature for 2 hr. To the reaction solution was added potassium carbonate (1.66 g), and the mixture was stirred at room temperature for 30 min. Potassium phthalimide (2.43 g) was added thereto at 50°C, and the mixture was stirred for 1.5 hr, and allowed to cool to room temperature. Water (15 mL) was added dropwise thereto, and the mixture was stirred for 30 min. 1N Aqueous potassium hydroxide solution (15 mL) was added dropwise thereto at the same temperature, and the mixture was stirred for 30 min. The precipitate was collected by filtration, washed with water, and dried to give the title compound (2.33 g, yield 86%) as a white solid.
$^1$H NMR (300 MHz, DMSO-$d_6$) δ: 0.33-0.40 (2 H, m), 0.56-0.65 (2 H, m), 1.19-1.33 (1 H, m), 3.93 (2 H, d, J=6.8 Hz), 5.03 (2 H, s), 7.04-7.11 (2 H, m), 7.77-8.05 (8 H, m), 8.35 (1 H, s), 8.98 (1 H, d, J=1.9 Hz), 10.25 (1 H, s).

Reference Example 72

N-[3-(aminomethyl)-8-fluoroquinolin-7-yl]-4-(cyclopropylmethoxy)benzamide

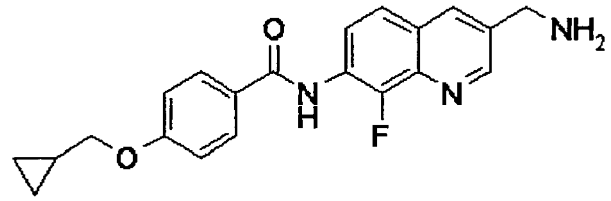

Hydrazine monohydrate (0.684 mL) was added at 80°C to a solution of 4-(cyclopropylmethoxy)-N-{3-[(1,3-dioxo-1,3-dihydro-2H-isoindol-2-yl)methyl]-8-fluoroquinolin-7-yl}benzamide (2.33 g) obtained in Reference Example 71 in NMP (30 mL), and the mixture was stirred for 3 hr, and allowed to cool to room temperature. Ethyl acetate was added thereto, and the resulting precipitate was filtered off, and washed with ethyl acetate. The filtrate and the washing solution were combined, saturated brine and water were added thereto, and the precipitated solid was collected by filtration. The obtained solid was dissolved in a mixed solvent of DMSO and 10% aqueous citric acid solution, and the solution was washed with ethyl acetate. To the aqueous layer was added 1N aqueous sodium hydroxide solution, and the mixture was cooled to 5°C. The resulting precipitate was collected by filtration, washed with water, and dried to give the title compound (1.07 g, yield 62%) as a white solid.
$^1$H NMR (300 MHz, DMSO-$d_6$) δ: 0.33-0.39 (2 H, m), 0.56-0.64 (2 H, m), 1.19-1.34 (1 H, m), 3.93 (2 H, d, J=7.0 Hz), 3.96 (2 H, s), 7.04-7.11 (2 H, m), 7.73-7.84 (2 H, m), 7.98-8.05 (2 H, m), 8.29 (1 H, s), 8.93 (1 H, d, J=2.1 Hz), 10.23 (1 H, s).

Reference Example 73

4-(cyclopropylmethoxy)-N-{8-fluoro-3-[(tetrahydro-2H-thiopyran-4-ylamino)methyl]quinolin-7-yl}benzamide

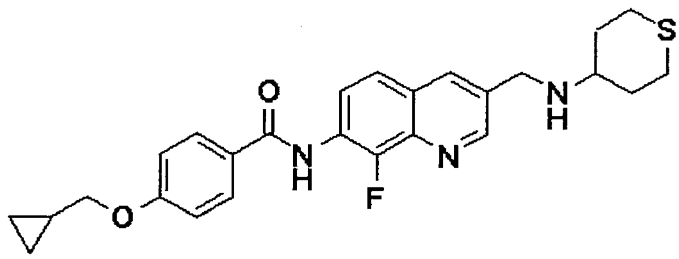

N-[3-(Aminomethyl)-8-fluoroquinolin-7-yl]-4-(cyclopropylmethoxy)benzamide (780 mg) obtained in Reference Example 72 and tetrahydro-4H-thiopyran-4-one (744 mg) were dissolved in a mixed solvent of acetic acid (5.0 mL) and N,N-dimethylacetamide (15 mL), and sodium triacetoxyborohydride (1.36 g) was added thereto at room temperature. The mixture was stirred at room temperature for 15 hr, and 8N aqueous sodium hydroxide solution (15 mL) was added thereto at 5°C. The mixture was diluted with ethyl acetate, washed with water and brine, and concentrated under reduced pressure to give the title compound (501 mg, yield 50%) as a pale-yellow solid.
$^1$H NMR (300 MHz, $CDCl_3$) δ: 0.36-0.43 (2 H, m), 0.65-0.73 (2 H, m), 1.23-1.38 (1 H, m), 1.54-1.68 (2 H, m), 2.20-2.30 (2 H, m), 2.53-2.79 (5 H, m), 3.90 (2 H, d, J=7.0 Hz), 4.03 (2 H, s), 6.99-7.05 (2 H, m), 7.63 (1 H, dd, J=9.0, 1.5 Hz), 7.89-7.95 (2 H, m), 8.10 (1 H, s), 8.27 (1 H, d, J=3.4 Hz), 8.72 (1 H, dd, J=9.1, 7.1 Hz), 8.91 (1 H, d, J=2.1 Hz).

Reference Example 74

methyl 4-(3,3,3-trifluoropropoxy)benzoate

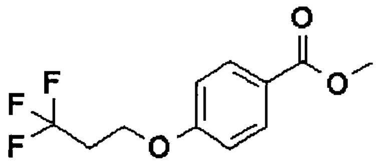

4-Methyl hydroxybenzoate (1.27 g), 3,3,3-trifluoro-1-propanol (1.14 g) and triphenylphosphine (2.63 g) were dissolved in tetrahydrofuran (35 mL), and the solution was cooled to 5°C. A solution of diisopropyl azodicarboxylate (2.25 g) in THF (5 mL) was added dropwise thereto over 10 min, and the mixture was stirred for 17 hr. The reaction mixture was allowed to warm to room temperature, and concentrated. The residue was purified by silica gel column chromatography [eluent; hexane:ethyl acetate=19:1→3:1 (volume ratio)] to give the title compound (668 mg, yield 32%) as a white solid.
$^1$H NMR (300 MHz, $CDCl_3$) δ: 2.65 (2 H, qt, J=10.5, 6.6 Hz), 3.89 (3 H, s), 4.25 (2 H, t, J=6.6 Hz), 6.92 (2 H, d, J=8.9 Hz), 8.00 (2 H, d, J=9.0 Hz).

Reference Example 75

4-(3,3,3-trifluoropropoxy)benzoic acid

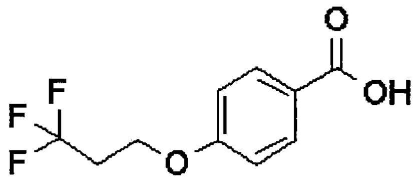

Methyl 4-(3,3,3-trifluoropropoxy)benzoate (2.85 g) obtained in Reference Example 74 was suspended in 2N aqueous sodium hydroxide solution (17.2 mL), and the mixture was stirred with heating at 60°C for 1.5 hr. The reaction solution was concentrated under reduced pressure, ethyl acetate was added thereto, and the mixture was acidified with 1N aqueous hydrochloric acid solution. The organic layer was separated, washed with saturated brine, and dried over sodium sulfate, and the solvent was evaporated under reduced pressure. The obtained solid was washed with a mixed solvent of isopropyl ether and hexane to give the title compound (2.46 g, yield 92%) as a white solid.
$^1$H NMR (300 MHz, DMSO-$d_6$) δ: 2.82 (2 H, qt, J=11.4, 5.9 Hz), 4.29 (2 H, t, J=5.9 Hz), 7.05 (2 H, d, J=8.9 Hz), 7.90 (2 H, d, J=9.0 Hz), 12.68 (1 H, br).

Reference Example 76

N-(2-methyl-3-nitrophenyl)-4-(3,3,3-trifluoropropoxy)benzamide

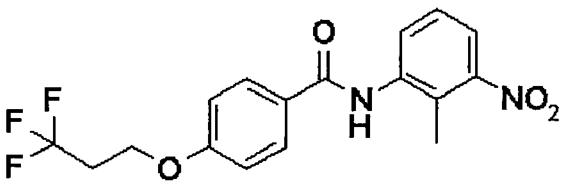

4-(3,3,3-Trifluoropropoxy)benzoic acid (2.34 g) obtained in Reference Example 75 was suspended in toluene (25 mL), thionyl chloride (2.38 g) and DMF (0.75 mL) were added thereto, and the mixture was stirred at 50°C for 1 hr, and concentrated under reduced pressure. The residue was dissolved in THF (30 mL), the solution was added dropwise

to a mixture of 2-methyl-3-nitroaniline (1.52 g) and triethylamine (3.48 mL), toluene (25 mL) and THF (25 mL) at 5°C, and after 10 min, the mixture was allowed to warm to room temperature, and stirred for 2 hr. The reaction solution was diluted with ethyl acetate, and partitioned with water. The organic layer was separated, washed successively with 1N aqueous hydrochloric acid solution, 1N aqueous sodium hydroxide solution, water and saturated brine, dried over sodium sulfate, and dried under reduced pressure. The obtained solid was washed with a mixed solvent of ethyl acetate and isopropyl ether (2:1, volume ratio), and dried under reduced pressure to give the title compound (2.45 g, yield 67%) as a pale-yellow solid.
$^1$H NMR (300 MHz, $CDCl_3$) δ: 2.46 (3 H, s), 2.68 (2 H, qt, J=10.5,6.5 Hz), 4.28 (2 H, t, J=6.5 Hz), 7.02 (2 H, d, J=8.9 Hz), 7.39 (1 H, t, J=8.1 Hz), 7.63 - 7.74 (2 H, m), 7.88 (2 H, d, J=8.9 Hz), 8.12 (1 H, d, J=8.1 Hz).

Reference Example 77

N-(3-amino-2-methylphenyl)-4-(3,3,3-trifluoropropoxy)benzamide

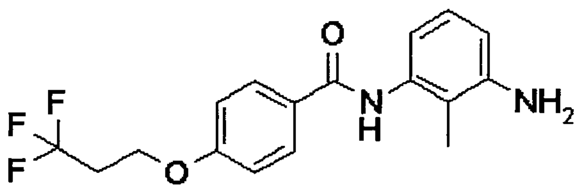

N-(2-Methyl-3-nitrophenyl)-4-(3,3,3-trifluoropropoxy)benzamide (2.44 g) obtained in Reference Example 76 and palladium-carbon (10%,244 mg) were suspended in a mixed solvent of methanol (60 mL) and THF (30 mL), and the mixture was stirred at room temperature for 18 hr under a hydrogen atmosphere (1 atm). The mixture was filtered through celite, and the filtrate was concentrated. The obtained residue was washed with a mixed solvent of ethyl acetate and diisopropyl ether, and dried under reduced pressure to give the title compound (2.04 g, yield 91%) as a white solid.
$^1$H NMR (300 MHz, $CDCl_3$) δ: 2.10 (3 H, s), 2.67 (2 H, qt, J=10.5, 6.6 Hz), 3.67 (2 H, s), 4.27 (2 H, t, J=6.6 Hz), 6.59 - 6.65 (1 H,m), 6.98 (2 H, d, J=8.9 Hz), 7.03 - 7.09 (2 H, m), 7.55 (1 H, s), 7.87 (2 H, d, J=8.9 Hz).

Reference Example 78

N-(3-formyl-8-methylquinolin-7-yl)-4-(3,3,3-trifluoropropoxy)benzamide

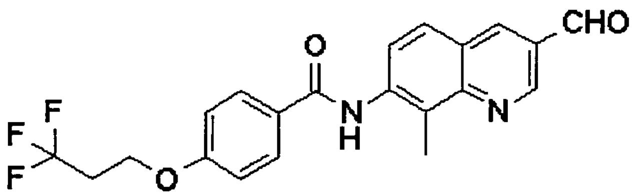

2-Dimethylaminomethylene-1,3-bis(dimethyliminio)propane bistetrafluoroborate (3.26 g) was suspended in 1-butanol (20 mL), and morpholine (2.98 g) was added thereto. To the mixture was added a mixture of N-(3-amino-2-methylphenyl)-4-(3,3,3-trifluoropropoxy)benzamide (1.93 g) obtained in Reference Example 77 and 1-butanol (10 mL), and the mixture was stirred at 80°C for 13 hr. Acetic acid (5.0 mL) and water (5.0 mL) were added successively thereto at the same temperature, and the mixture was stirred for 30 min, and allowed to cool to room temperature. The precipitate was collected by filtration, and the obtained solid was washed successively with a mixed solvent of acetic acid-water (1/2) and water, and dried under reduced pressure to give the title compound (1.81 g, yield 79%) as a pale-yellow solid.
$^1$H NMR (300 MHz, $CDCl_3$) δ: 2.69 (2 H, qt, J=10.4, 6.6 Hz), 2.86 (3 H, s), 4.30 (2 H, t, J=6.6 Hz), 7.04 (2 H, d, J=8.9 Hz), 7.90 (1 H, d, J=8.9 Hz), 7.95 (2 H, d, J=8.9 Hz), 8.04 (1 H, s), 8.53 - 8.62 (2 H, m), 9.35 (1 H, d, J=2.1 Hz), 10.25 (1 H, s).

Reference Example 79

N-{8-methyl-3-[(tetrahydro-2H-thiopyran-4-ylamino)methyl]quinolin-7-yl}-4-(3,3,3-trifluoropropoxy)benzamide

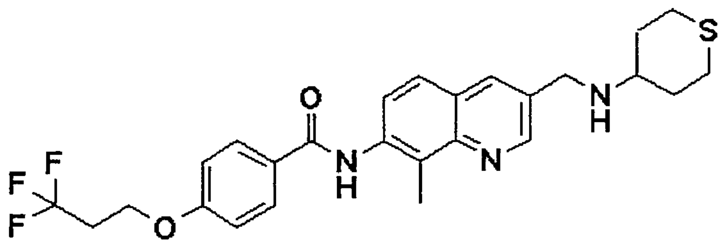

N-(3-Formyl-8-methylquinolin-7-yl)-4-(3,3,3-trifluoropropoxy)benzamide (530 mg) obtained in Reference Example 78 and tetrahydro-2H-thiopyran-4-amine hydrochloride (520 mg) obtained in Reference Example 35 were dissolved in a mixed solvent of DMA (18 mL) and acetic acid (3.0 mL), and the mixture was stirred at room temperature for 10 min, and cooled to 5°C. Sodium triacetoxyborohydride (800 mg) was added thereto, and the mixture was stirred overnight at room temperature. The reaction mixture was cooled to 5°C, basified with 1N aqueous sodium hydroxide solution, and allowed to warm to room temperature. Water was added thereto, and the precipitated solid was collected by filtration, washed with water, and dried under reduced pressure to give the title compound (530 mg, yield 80%) as a pale-yellow solid. FAB(pos): 504 $[MH]^+$

Reference Example 80

methyl 4-[(vinyloxy)methyl]benzoate

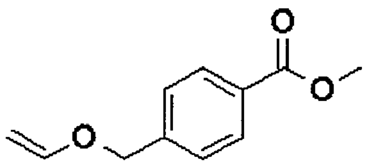

Under a nitrogen atmosphere, methyl 4-(hydroxymethyl)benzoate (10.0 g), iridium chloride-cyclooctadiene complex dimmer (404 mg) and sodium carbonate (2.17 g) were suspended in toluene (45 mL), vinyl acetate (11.1 mL) was added thereto, and the mixture was stirred at 100°C for 18 hr. The mixture was allowed to cool to room temperature, and filtered through celite, and the filtrate was concentrated under reduced pressure. The residue was purified by silica gel column chromatography [eluent; hexane:ethyl acetate=19:1 (volume ratio)→hexane:ethyl acetate=4:1 (volume ratio)] to give the title compound (6.11 g, yield 53%) as a pale-yellow solid.
$^1H$ NMR (300 MHz, $CDCl_3$) δ: 3.92 (3 H, s), 4.12 (1 H, dd, J=6.9, 2.4 Hz), 4.30 (1 H, dd, J=14.3, 2.4 Hz), 4.82 (2 H, s), 6.57 (1 H, dd, J=14.3, 7.0 Hz), 7.43 (2 H, d, J=8.7 Hz), 8.04 (2 H, d, J=8.3 Hz).

Reference Example 81

methyl 4-[(cyclopropyloxy)methyl]benzoate

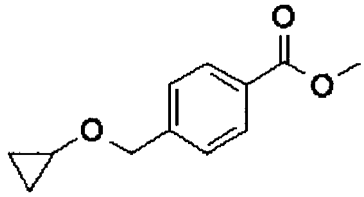

Methyl 4-[(vinyloxy)methyl]benzoate (6.11 g) obtained in Reference Example 80 was dissolved in dichloromethane (200 mL), diethyl zinc (1M hexane solution, 143 mL) and diiodomethane (25.6 mL) were added thereto, and the mixture was stirred at room temperature for 3 hr. 1N Hydrochloric acid was added thereto, and the mixture was extracted with ethyl acetate (twice). The extract was washed with aqueous sodium bisulfite solution and saturated brine, dried over sodium sulfate, and concentrated under reduced pressure. The residue was purified by silica gel column chromatography [eluent; hexane:ethyl acetate=9:1 (volume ratio)→hexane:ethyl acetate=17:3 (volume ratio)] to give the title compound (6.56 g, yield 100%) as a colorless transparent oil.
$^1$H NMR (300 MHz, $CDCl_3$) δ: 0.46 - 0.54 (2 H, m), 0.62 - 0.70 (2 H, m), 3.33 - 3.41 (1 H, m), 3.91 (3 H, s), 4.60 (2 H, s), 7.41 (2 H, d, J=8.5 Hz), 8.01 (2 H, d, J=8.3 Hz).

Reference Example 82

4-[(cyclopropyloxy)methyl]benzoic acid

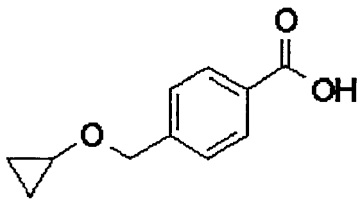

Methyl 4-[(cyclopropyloxy)methyl]benzoate (6.56 g) obtained in Reference Example 81 was diluted with methanol (100 mL), 1N aqueous sodium hydroxide solution (63 mL) was added thereto, and the mixture was stirred at room temperature for 13 hr. 1N Hydrochloric acid (65 mL) was added dropwise thereto at room temperature, and the mixture was stirred for 1 hr. The precipitate was collected by filtration, washed with water, and dried to give the title compound (5.85 g, yield 96%) as a white solid.
$^1$H NMR (300 MHz, DMSO-$d_6$) δ: 0.42 - 0.51 (2 H, m), 0.51 - 0.60 (2 H, m), 3.33 - 3.41 (1 H, m), 4.57 (2 H, s), 7.43 (2 H, d, J=8.1 Hz), 7.91 (2 H, d, J=8.1 Hz).

Reference Example 83

4-[(cyclopropyloxy)methyl]-N-(2-methyl-3-nitrophenyl)benzamide

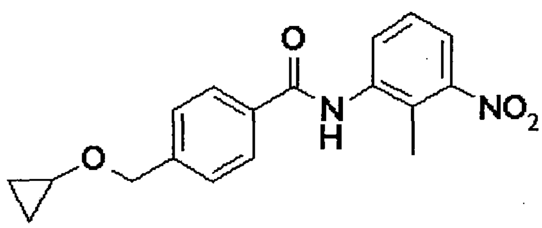

4-[(Cyclopropyloxy)methyl]benzoic acid (3.00 g) obtained in Reference Example 82 was dissolved in THF (30 mL), oxalyl dichloride (2.68 mL) and DMF (one drop) were added thereto, and the mixture was stirred at room temperature for 5 hr, and concentrated under reduced pressure. The residue was azeotropically concentrated with toluene (three times). The residue and 2-methyl-3-nitroaniline (2.61 g) were dissolved in NMP (30 mL), triethylamine (2.39 mL) was added thereto, and the mixture was stirred at room temperature for 16 hr. The mixture was diluted with ethyl acetate, washed with 1N hydrochloric acid, water, saturated aqueous sodium hydrogen carbonate solution and saturated brine, dried over sodium sulfate, and concentrated under reduced pressure. The residue was washed with diisopropyl ether-ethyl acetate, and dried under reduced pressure to give the title compound (4.85 g, yield 95%) as a white solid.
$^1H$ NMR (300 MHz, $CDCl_3$) δ: 0.48 - 0.57 (2 H, m), 0.64 - 0.72 (2 H, m), 2.46 (3 H, s), 3.35 - 3.44 (1 H, m), 4.64 (2 H, s), 7.40 (1 H, t, J=8.3 Hz), 7.50 (2 H, d, J=8.3 Hz), 7.70 (1 H, dd, J=8.1, 1.1 Hz), 7.72 (1 H, br. s.), 7.88 (2 H, d, J=8.3 Hz), 8.16 (1 H, d, J=8.1 Hz).

Reference Example 84

N-(3-amino-2-methylphenyl)-4-[(cyclopropyloxy)methyl]benzamide

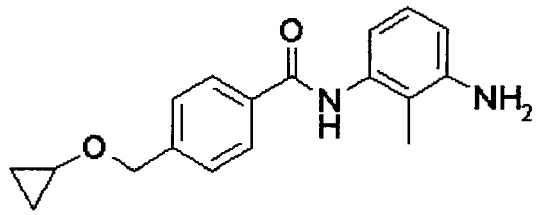

4-[(Cyclopropyloxy)methyl]-N-(2-methyl-3-nitrophenyl)benzamide (4.85 g) obtained in Reference Example 83 was dissolved in THF (100 mL)-ethanol (100 mL), and palladium-carbon (10%-palladium, containing 50% water, 485 mg) was added thereto. The mixture was stirred at room temperature for 17 hr under a hydrogen atmosphere, and filtered through celite. The filtrate was concentrated under reduced pressure, and the residue was washed with diisopropyl ether, and dried under reduced pressure to give the title compound (4.17 g, yield 95%) as a white solid.
$^1H$ NMR (300 MHz, $CDCl_3$) δ: 0.47 - 0.56 (2 H, m), 0.63 - 0.71 (2 H, m), 2.10 (3 H, s), 3.34 - 3.43 (1 H, m), 3.67 (2 H, s), 4.62 (2 H, s), 6.62 (1 H, dd, J=7.1, 2.0 Hz), 7.01 - 7.12 (2 H, m), 7.46 (2 H, d, J=8.3 Hz), 7.60 (1 H, br. s.), 7.87 (2 H, d, J=8.1 Hz).

Reference Example 85

4-[(cyclopropyloxy)methyl]-N-(3-formyl-8-methylquinolin-7-yl)benzamide

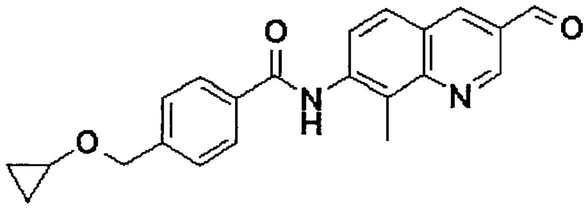

N-(3-Amino-2-methylphenyl)-4-[(cyclopropyloxy)methyl]benzamide (4.17 g) obtained in Reference Example 84, 2-dimethylaminomethylene-1,3-bis(dimethyliminio)propane bistetrafluoroborate (9.68 g) and morpholine (7.34 mL) were suspended in 1-butanol (80 mL), and the mixture was stirred at 80°C for 20 hr. Acetic acid (14 mL) and water (14 mL) were added successively thereto at the same temperature, and the mixture was stirred for 2 hr, and allowed to cool to room temperature. The mixture was filtered, and the obtained solid was washed with acetic acid-water (1/1) and water, and dried to give the title compound (2.97 g, yield 59%) as a yellow solid.
$^1H$ NMR (300 MHz, DMSO-$d_6$) δ: 0.45 - 0.54 (2 H, m), 0.54 - 0.62 (2 H, m), 2.70 (3 H, s), 3.35 - 3.44 (1 H, m), 4.61 (2

H, s), 7.51 (2 H, d, J=8.3 Hz), 7.82 (1 H, d, J=8.7 Hz), 8.04 (2 H, d, J=8.3 Hz), 8.08 (1 H, d, J=8.7 Hz), 8.94 (1 H, d, J=2.1 Hz), 9.32 (1 H, d, J=2.3 Hz), 10.26 (1 H, s), 10.33 (1 H, s).

Reference Example 86

4-[(cyclopropyloxy)methyl]-N-{8-methyl-3-[(tetrahydro-2H-thiopyran-4-ylamino)methyl]quinolin-7-yl}benzamide

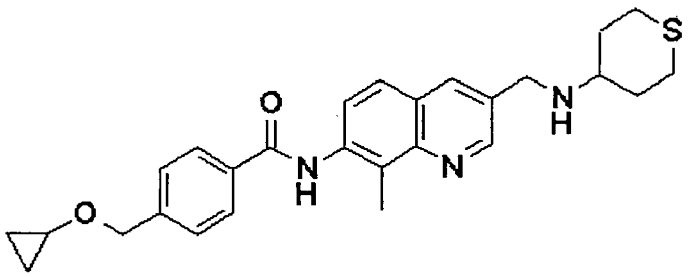

4-[(Cyclopropyloxy)methyl]-N-(3-formyl-8-methylquinolin-7-yl)benzamide (800 mg) obtained in Reference Example 85 and tetrahydro-2H-thiopyran-4-amine hydrochloride (512 mg) obtained in Reference Example 35 were suspended in NMP (12 mL)-acetic acid (2.0 mL), and the mixture was stirred at room temperature for 4 hr. Sodium triacetoxyborohydride (941 mg) was added thereto, and the mixture was stirred at room temperature for 21 hr. The mixture was diluted with ethyl acetate, and basified with 2N aqueous sodium hydroxide solution (40 mL). The organic layer was washed with saturated brine (three times), dried over sodium sulfate, and concentrated under reduced pressure. The residue was purified by silica gel column chromatography [eluent; hexane:ethyl acetate=3:7 (volume ratio)→ethyl acetate: methanol=22:3 (volume ratio)] to give the title compound (842 mg, yield 80%) as a white solid.
$^1$H NMR (300 MHz, $CDCl_3$) δ: 0.48 - 0.58 (2 H, m), 0.64 - 0.73 (2 H, m), 1.54 - 1.69 (2 H, m), 2.18 - 2.31 (2 H, m), 2.52 - 2.79 (5 H, m), 2.81 (3 H, s), 3.35 - 3.44 (1 H, m), 4.03 (2 H, s), 4.65 (2 H, s), 7.51 (2 H, d, J=8.5 Hz), 7.71 (1 H, d, J=8.9 Hz), 7.90 - 7.98 (3 H, m), 8.06 (1 H, d, J=2.1 Hz), 8.27 (1 H, d, J=8.9 Hz), 8.90 (1 H, d, J=2.3 Hz).

Reference Example 87

ethyl 4-(cyclopropylethynyl)benzoate

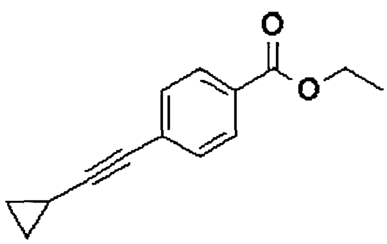

Ethyl 4-bromobenzoate (11.64 g), cyclopropylacetylene (4.23 g), triethylamine (21.3 mL) and copper iodide (97 mg) were dissolved in THF (180 mL), and the solution was degassed under argon gas atmosphere. Palladium acetate (II)(234 mg) was added to the mixture, and the mixture was stirred overnight at room temperature. The solvent was evaporated under reduced pressure. The residue was partitioned between ethyl acetate and 0.1N aqueous hydrochloric acid solution, and the organic layer was washed with water and saturated brine, dried over sodium sulfate, and concentrated under reduced pressure. The residue was purified successively by NH silica gel column chromatography [eluent; ethyl acetate] and silica gel column chromatography [eluent; hexane→hexane:ethyl acetate=97:3 (volume ratio)] to give the title compound (10.64 g, yield 64%) as an oil.
$^1$H NMR (300 MHz, $CDCL_3$) δ: 0.78 - 0.99 (4 H, m), 1.39 (3 H, t, J=7.0 Hz), 1.42 - 1.54 (1 H, m), 4.36 (2 H, q, J=7.2 Hz), 7.41 (2 H, d, J=8.3 Hz), 7.94 (2 H, d, J=8.3 Hz).

Reference Example 88

4-(cyclopropylethynyl)benzoic acid

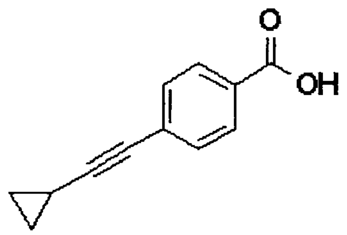

Ethyl 4-(cyclopropylethynyl)benzoate (2.894 g) obtained in Reference Example 87 was dissolved in a mixed solvent of methanol (10 mL), water (10 mL) and THF (15 mL), lithium hydroxide monohydrate (0.70 g) was added thereto, and the mixture was stirred at room temperature for 30 min, and heated under reflux for 3 hr. The reaction mixture was allowed to cool to room temperature, and the solvent was evaporated under reduced pressure. The residue was suspended in water, and the suspension was acidified with 1N aqueous hydrochloric acid solution (20 mL), and stirred at room temperature for 30 min. The obtained suspension was filtered, and the solid was collected by filtration was washed with water, and dried to give the title compound (2.458 g, yield 79%) as a white solid. $^1$H NMR (300 MHz, DMSO-$d_6$) δ: 0.71 - 0.82 (2 H, m) 0.86 - 0.99 (2 H, m) 1.46 - 1.66 (1 H, m) 7.46 (2 H, d, J=8.71 Hz) 7.87 (2 H, d, J=8.33 Hz) 13.08 (1 H, br. s.).

Reference Example 89

4-(cyclopropylethynyl)-N-(2-methyl-3-nitrophenyl)benzamide

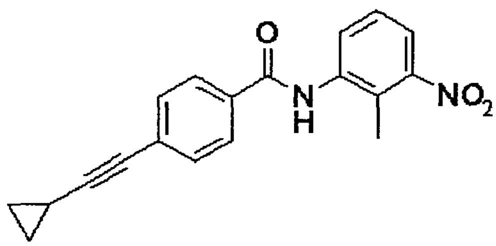

4-(Cyclopropylethynyl)benzoic acid (2.450 g) obtained in Reference Example 88 was suspended in toluene (50 mL), oxalyl chloride (5.0 mL) and DMF (3 drops) were added thereto, and the mixture was stirred at room temperature for 3 hr, and concentrated under reduced pressure. The residue was dissolved in DMA (5 mL), and the solution was added dropwise at 5°C to a mixture of 2-methyl-3-nitroaniline (1.959 g), triethylamine (2.0 mL) and DMA (30 mL), and the mixture was stirred overnight at room temperature. The reaction solution was diluted with water, neutralized with 1N aqueous sodium hydroxide solution, and extracted with ethyl acetate. The organic layer was washed with water and saturated brine, dried over sodium sulfate, and dried under reduced pressure to give the title compound (4.10 g, yield 97%) as a pale-yellow solid. FAB(pos) : 321 $[MH]^+$

Reference Example 90

N-(3-amino-2-methylphenyl)-4-(cyclopropylethynyl)benzamide

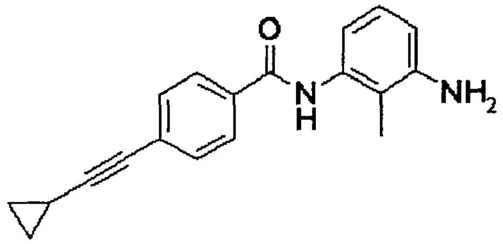

4-(Cyclopropylethynyl)-N-(2-methyl-3-nitrophenyl)benzamide (2.16 g) obtained in Reference Example 89 was suspended in a mixed solvent of ethanol (40 mL), water (5 mL) and acetic acid (5 mL), iron powder (1.83 g) was added thereto, and the mixture was stirred at 85°C for 2 hr. The reaction mixture was allowed to cool to room temperature, neutralized with 1N aqueous sodium hydroxide solution, and filtered through celite. The filtrate was concentrated under reduced pressure. The residue was partitioned between ethyl acetate and water, and the organic layer was washed with saturated brine, dried over sodium sulfate, and concentrated under reduced pressure. The residue was suspended in boiled methanol, and the suspension was allowed to cool to room temperature. The obtained suspension was filtered, and the solid was collected by filtration was washed with methanol, and dried to give the title compound (1.462 g, yield 75%) as a pale-yellow solid.
$^1H$ NMR (300 MHz, DMSO-$d_6$) δ: 0.72 - 0.82 (2 H, m), 0.87 - 0.99 (2 H, m), 1.51 - 1.65 (1 H, m), 1.89 (3 H, s), 4.90 (2 H, s), 6.48 (1 H, d, J=8.0 Hz), 6.55 (1 H, d, J=8.0 Hz), 6.88 (1 H, t, J=8.0 Hz), 7.47 (2 H, d, J=8.3 Hz), 7.91 (2 H, d, J=8.3 Hz), 9.82 (1 H, s).

Reference Example 91

4-(cyclopropylethynyl)-N-(3-formyl-8-methylquinolin-7-yl)benzamide

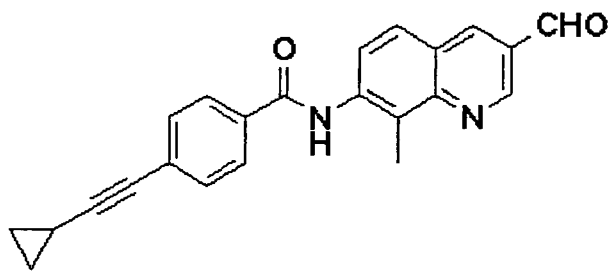

N-(3-Amino-2-methylphenyl)-4-(cyclopropylethynyl)benzamide (1.446 g) obtained in Reference Example 90 and 2-dimethylaminomethylene-1,3-bis(dimethyliminio)propane bistetrafluoroborate (3.70 g) were suspended in 1-butanol (30 mL), morpholine (2.70 mL) was added thereto, and the mixture was stirred overnight at 80°C. Acetic acid (5 mL) and water (5 mL) were added successively thereto at the same temperature, and the mixture was stirred for 1 hr, and allowed to cool to room temperature. The mixture was filtered, and the obtained solid was washed successively with a mixed solvent of acetic acid-water (1/1) and water, and dried to give the title compound (1.487 g, yield 84%) as a yellow solid.
$^1H$ NMR (300 MHz, DMSO-$d_6$) δ: 0.73 - 0.86 (2 H, m), 0.88 - 1.02 (2 H, m), 1.53 - 1.69 (1 H, m), 2.69 (3 H, s), 7.55 (2 H, d, J=8.5 Hz), 7.81 (1 H, d, J=8.7 Hz), 8.01 (2 H, d, J=8.5 Hz), 8.08 (1 H, d, J=8.7 Hz), 8.95 (1 H, d, J=2.1 Hz), 9.32 (1 H, d, J=2.1 Hz), 10.26 (1 H, s), 10.39 (1 H, s).

Reference Example 92

4-(cyclopropylethynyl)-N-{8-methyl-3-[(tetrahydro-2H-thiopyran-4-ylamino)methyl]quinolin-7-yl}benzamide

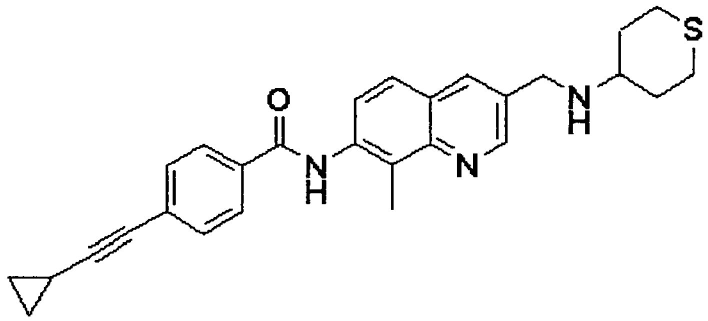

4-(Cyclopropylethynyl)-N-(3-formyl-8-methylquinolin-7-yl)benzamide (550 mg) obtained in Reference Example 91, tetrahydro-2H-thiopyran-4-amine hydrochloride (620 mg) obtained in Reference Example 35 and sodium acetate (0.35 g) were dissolved in a mixed solvent of NMP (22 mL) and acetic acid (2.0 mL), and the mixture was stirred at room temperature for 10 min, and cooled to 5°C. Sodium triacetoxyborohydride (960 mg) was added thereto, and the mixture was stirred overnight at room temperature. The reaction mixture was cooled to 5°C, basified with 8N aqueous sodium hydroxide solution, and extracted with ethyl acetate. The organic layer was washed with water, and concentrated under reduced pressure. The residue was purified by silica gel column chromatography [eluent; ethyl acetate→ethyl acetate: methanol=9:1 (volume ratio)] to give the title compound (426 mg, yield 60%) as a white solid.
$^1$H NMR (300 MHz, DMSO-$d_6$) δ: 0.72 - 0.83 (2 H, m), 0.88 - 1.01 (2 H, m), 1.39 - 1.55 (2 H, m), 1.55 - 1.70 (1 H, m), 2.05 - 2.21 (2 H, m), 2.41 - 2.59 (3 H, m), 2.64 (3 H, s), 2.65 - 2.75 (2 H, m), 3.94 (2 H, s), 7.53 (2 H, d, J=8.3 Hz), 7.57 (1 H, d, J=8.7 Hz), 7.79 (1 H, d, J=8.7 Hz), 8.00 (2 H, d, J=8.3 Hz), 8.21 (1 H, d, J=2.3 Hz), 8.91 (1 H, d, J=2.3 Hz), 10.23 (1 H, s).

Reference Example 93

4-(cyclopropylethynyl)-N-[8-methyl-3-({[3-(methylthio)propyl]amino}methyl)quinolin-7-yl]benzamide

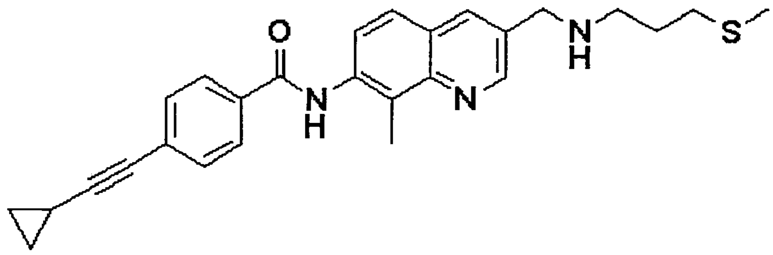

4-(Cyclopropylethynyl)-N-(3-formyl-8-methylquinolin-7-yl)benzamide (463 mg) obtained in Reference Example 91 and 3-methylthiopropylamine (550 mg) were dissolved in a mixed solvent of DMA (18 mL) and acetic acid (3.0 mL), and the mixture was stirred at room temperature for 10 min, and cooled to 5°C. Sodium triacetoxyborohydride (550 mg) was added thereto, and the mixture was stirred overnight at room temperature. The reaction mixture was cooled to 5°C, and basified with 1N aqueous sodium hydroxide solution, and the precipitated solid was collected by filtration, washed with water, and dried under reduced pressure. The residue was purified by silica gel column chromatography [eluent; ethyl acetate→ethyl acetate:methanol=7:3 (volume ratio)], and the obtained solid was washed with methanol, and dried under reduced pressure to give the title compound (492 mg, yield 85%) as a white solid.
$^1$H NMR (300 MHz, DMSO-$d_6$) δ: 0.74 - 0.84 (2 H, m), 0.89 - 1.00 (2 H, m), 1.52 - 1.65 (1 H, m), 1.65 - 1.78 (2 H, m), 2.02 (3 H, s), 2.51 - 2.56 (2 H, m), 2.60 (2 H, t, J=6.8 Hz), 2.64 (3 H, s), 3.90 (2 H, s), 7.53 (2 H, d, J=8.7 Hz), 7.58 (1 H, d, J=8.7 Hz), 7.78 (1 H, d, J=8.7 Hz), 8.00 (2 H, d, J=8.7 Hz), 8.20 (1 H, d, J=1.9 Hz), 8.90 (1 H, d, J=2.3 Hz), 10.23 (1 H, s).

Reference Example 94

4-[(E)-2-cyclopropylvinyl]benzoic acid

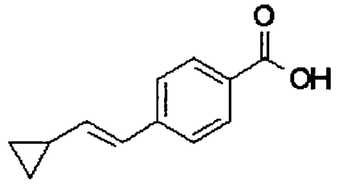

Ethyl 4-iodobenzoate (2.705 g) and (E)-2-cyclopropylvinylboronic acid pinacol ester (2.053 g) were dissolved in 1,4-dioxane (25 mL), 2N aqueous sodium hydroxide solution (5.0 mL) was added dropwise thereto, and the mixture was degasses under a nitrogen atmosphere. Tetrakis(triphenylphosphine)palladium(0) (380 mg) was added thereto, and the mixture was stirred with heating at 100°C for 3 hr, and allowed to cool to room temperature. Water was added thereto, and the mixture was extracted with ethyl acetate. The organic layer was separated, washed with saturated brine, dried over sodium sulfate, and concentrated under reduced pressure to give ethyl 4-[(E)-2-cyclopropylvinyl]benzoate as an oil. The oil was dissolved in a mixed solvent of methanol (10 mL), water (10 mL) and THF (15 mL), lithium hydroxide monohydrate (480 mg) was added thereto, and the mixture was stirred overnight at room temperature. The solvent was evaporated under reduced pressure, and to the residue was added water. The mixture was washed with ethyl acetate, and the aqueous layer was separated. The insoluble material was filtered off, and the filtrate was acidified with 1N aqueous hydrochloric acid solution, and extracted with ethyl acetate. The organic layer was separated, washed with water and saturated brine, dried over sodium sulfate, and concentrated under reduced pressure to give the title compound (993 mg, yield 54%) as a pale-yellow solid.
$^1$H NMR (300 MHz, DMSO-$d_6$) δ: 0.50 - 0.61 (2 H, m) 0.79 - 0.89 (2 H, m) 1.52 - 1.70 (1 H, m) 6.01 (1 H, dd, J=15.90, 9.09 Hz) 6.54 (1 H, d, J=15.90 Hz) 7.44 (2 H, d, J=8.33 Hz) 7.84 (2 H, d, J=8.33 Hz) 12.79 (1 H, br. s.).

Reference Example 95

4-[(E)-2-cyclopropylvinyl]-N-(2-methyl-3-nitrophenyl)benzamide

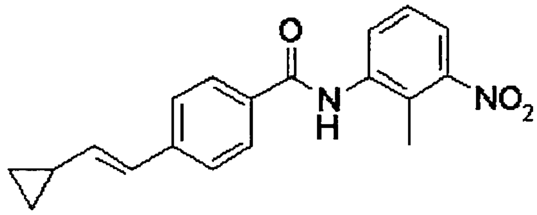

4-[(E)-2-Cyclopropylvinyl]benzoic acid (980 mg) obtained in Reference Example 94 was suspended in toluene (30 mL), oxalyl chloride (3.0 mL) and DMF (2 drops) were added thereto, and the mixture was stirred at room temperature for 3 hr, and concentrated under reduced pressure. The residue was dissolved in DMA (2 mL), and the solution was added dropwise to a mixture of 2-methyl-3-nitroaniline (760 mg), triethylamine (0.8 mL) and DMA (10 mL) at 5°C. The mixture was stirred overnight at room temperature, diluted with water, neutralized with 1N aqueous sodium hydroxide solution, and extracted with ethyl acetate. The organic layer was washed with 1N aqueous hydrochloric acid solution, water and saturated brine, dried over sodium sulfate, and dried under reduced pressure to give the title compound (1.53 g, yield 91%) as a pale-yellow solid. FAB(pos) : 323 $[MH]^+$

Reference Example 96

N-(3-amino-2-methylphenyl)-4-[(E)-2-cyclopropylvinyl]benzamide

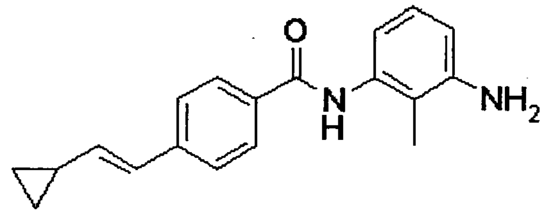

4-[(E)-2-Cyclopropylvinyl]-N-(2-methyl-3-nitrophenyl)benzamide (1.50 g) obtained in Reference Example 95 was suspended in a mixed solvent of ethanol (40 mL), water (5 mL) and acetic acid (5 mL), iron powder (1.3 g) was added thereto, and the mixture was stirred at 85°C for 2 hr. The reaction mixture was allowed to cool to room temperature, neutralized with 1N aqueous sodium hydroxide solution, and filtered through celite, and the filtrate was concentrated under reduced pressure. The residue was partitioned between ethyl acetate and water, and the organic layer was washed with saturated brine, dried over sodium sulfate, and concentrated under reduced pressure to give the title compound (476 mg, yield 35%) as a yellow solid.
$^1$H NMR (300 MHz, DMSO-$d_6$) δ: 0.50 - 0.62 (2 H, m), 0.77 - 0.90 (2 H, m), 1.53 - 1.74 (1 H, m), 1.90 (3 H, s), 4.87 (2 H, br. s.), 6.00 (1 H, dd, J=15.8, 9.2 Hz), 6.45 - 6.61 (3 H, m), 6.88 (1 H, t, J=7.8 Hz), 7.45 (2 H, d, J=8.3 Hz), 7.89 (2 H, d, J=8.5 Hz), 9.70 (1 H, s).

Reference Example 97

4-[(E)-2-cyclopropylvinyl]-N-(3-formyl-8-methylquinolin-7-yl)benzamide

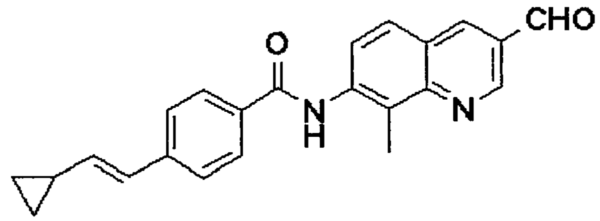

N-(3-Amino-2-methylphenyl)-4-[(E)-2-cyclopropylvinyl]benzamide (474 mg) obtained in Reference Example 96 and 2-dimethylaminomethylene-1,3-bis(dimethyliminio)propane bistetrafluoroborate (1.23 g) were suspended in 1-butanol (10 mL), morpholine (0.87 mL) was added thereto, and the mixture was stirred overnight at 80°C. Acetic acid (2.5 mL) and water (2.5 mL) were added successively thereto at the same temperature, and the mixture was stirred for 1 hr, and allowed to cool to room temperature. The mixture was filtered, and the obtained solid was washed successively with a mixed solvent of acetic acid-water (1/1) and water, and dried to give the title compound (387 mg, yield 67%) as a yellow solid.
$^1$H NMR (300 MHz, DMSO-$d_6$) δ: 0.52 - 0.65 (2 H, m) 0.77 - 0.93 (2 H, m) 1.53 - 1.76 (1 H, m) 2.70 (3 H, s) 6.05 (1 H, dd, J=15.90, 9.28 Hz) 6.58 (1 H, d, J=15.90 Hz) 7.52 (2 H, d, J=8.33 Hz) 7.82 (1 H, d, J=8.71 Hz) 7.99 (2 H, d, J=8.33 Hz) 8.07 (1 H, d, J=8.71 Hz) 8.94 (1 H, d, J=1.89 Hz) 9.32 (1 H, d, J=1.89 Hz) 10.26 (2 H, s).

Reference Example 98

4-[(E)-2-cyclopropylvinyl]-N-{8-methyl-3-[(tetrahydro-2H-thiopyran-4-ylamino)methyl]quinolin-7-yl}benzamide

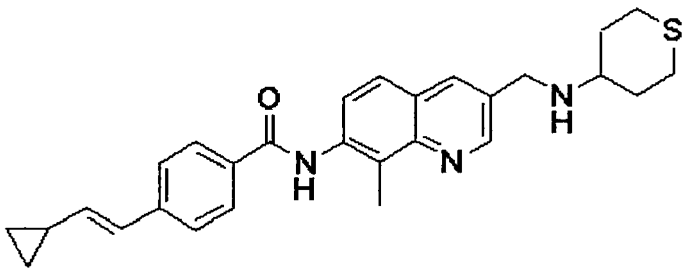

4-[(E)-2-Cyclopropylvinyl]-N-(3-formyl-8-methylquinolin-7-yl)benzamide (370 mg) obtained in Reference Example 97 and tetrahydro-2H-thiopyran-4-amine hydrochloride (501 mg) obtained in Reference Example 35 were dissolved in a mixed solvent of NMP (15 mL) and acetic acid (3.0 mL), and the mixture was stirred at room temperature for 10 min, and cooled to 5°C. Sodium triacetoxyborohydride (350 mg) was added thereto, and the mixture was stirred overnight at room temperature. The reaction mixture was cooled to 5°C, basified with 8N aqueous sodium hydroxide solution, and allowed to warm to room temperature. Water was added thereto, and the precipitated solid was collected by filtration, washed with water, and dried under reduced pressure. The solid was purified by silica gel column chromatography [eluent; ethyl acetate→ethyl acetate:methanol=4:1 (volume ratio)], and the obtained solid was washed with methanol, and dried under reduced pressure to give the title compound (368 mg, yield 77%) as a white solid.
$^1$H NMR (300 MHz, DMSO-$d_6$) δ: 0.52 - 0.63 (2 H, m) 0.79 - 0.91 (2 H, m) 1.39 - 1.56 (2 H, m) 1.57 - 1.71 (1 H, m) 2.04 - 2.24 (2 H, m) 2.42 - 2.58 (3 H, m) 2.64 (3 H, s) 2.65 - 2.76 (2 H, m) 3.94 (2 H, s) 6.03 (1 H, dd, J=15.71, 9.28 Hz) 6.57 (1 H, d, J=15.90 Hz) 7.51 (2 H, d, J=8.33 Hz) 7.58 (1 H, d, J=8.71 Hz) 7.78 (1 H, d, J=8.71 Hz) 7.97 (2 H, d, J=8.33 Hz) 8.21 (1 H, d, J=1.89 Hz) 8.91 (1 H, d, J=2.27 Hz) 10.13 (1 H, s).

Reference Example 99

ethyl 6-amino-2-naphthoate

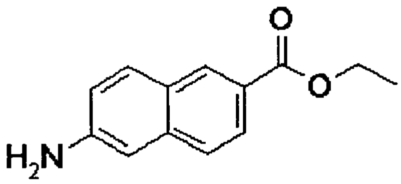

6-Amino-2-naphthoic acid (10.50 g) was suspended in ethanol, thionyl chloride (8.0 mL) was added dropwise thereto, and the mixture was heated under reflux overnight. The reaction mixture was allowed to cool to room temperature, and the solvent was evaporated under reduced pressure. The residue was dissolved in THF, and the solution was diluted with ethyl acetate, and filtered through NH silica gel (50 g). The filtrate was concentrated under reduced pressure. The residue was crystallized from water, and the crystals were collected by filtration, washed with water, and dried under reduced pressure to give the title compound (16.50 g, yield 70%) as a brown solid.
$^1$H NMR (300 MHz, $CDCl_3$) δ: 0.31 - 0.46 (m, 2 H) 0.60 - 0.75 (m, 2 H) 1.21 - 1.39 (m, 1 H) 1.45 (t, J=7.06 Hz, 3 H) 3.88 (d, J=6.97 Hz, 2 H) 4.44 (q, J=7.10 Hz, 2 H) 7.00 (d, J=8.85 Hz, 2 H) 7.63 (dd, J=8.85, 2.07 Hz, 1 H) 7.79 - 8.01 (m, 5 H) 8.06 (dd, J=8.48, 1.70 Hz, 1 H) 8.40 (d, J=2.07 Hz, 1 H) 8.56 (s, 1 H).

Reference Example 100

ethyl 6-{[4-(cyclopropylmethoxy)benzoyl]amino}-2-naphthoate

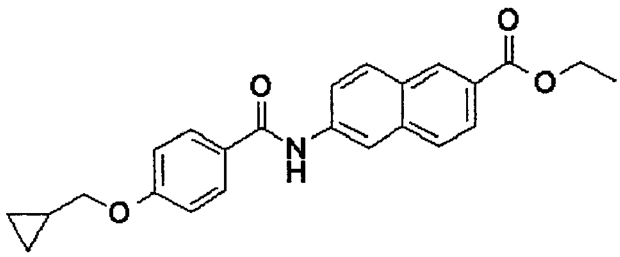

4-Cyclopropylmethoxybenzoic acid (2.130 g) obtained in Reference Example 1 was suspended in toluene (30 mL), thionyl chloride (1.25 mL) and DMF (3 drops) were added thereto, and the mixture was stirred at 50°C for 1 hr, and concentrated under reduced pressure. The residue was dissolved in DMA (3 mL), the solution was added dropwise to a mixture of ethyl 6-amino-2-naphthoate (2.00 g) obtained in Reference Example 99 and DMA (15 mL), and after 10 min, the mixture was allowed to cool to room temperature, and stirred overnight. The reaction solution was diluted with water, and the precipitated solid was collected by filtration, washed with water, and dried under reduced pressure. The solid was dissolved in ethyl acetate while heating, and the solution was filtered through NH silica gel (10 g). The filtrate was concentrated under reduced pressure to give the title compound (1.340 g, yield 37%) as a pale-yellow solid.
$^1$H NMR (300 MHz, $CDCl_3$) δ: 0.31 - 0.46 (2 H, m) 0.60 - 0.75 (2 H, m) 1.21 - 1.39 (1 H, m) 1.45 (3 H, t, J=7.06 Hz) 3.88 (2 H, d, J=6.97 Hz) 4.44 (2 H, q, J=7.10 Hz) 7.00 (2 H, d, J=8.85 Hz) 7.63 (1 H, dd, J=8.85, 2.07 Hz) 7.79 - 8.01 (5 H, m) 8.06 (1 H, dd, J=8.48, 1.70 Hz) 8.40 (1 H, d, J=2.07 Hz) 8.56 (1 H, s).

Reference Example 101

4-(cyclopropylmethoxy)-N-[6-(hydroxymethyl)naphthalen-2-yl]benzamide

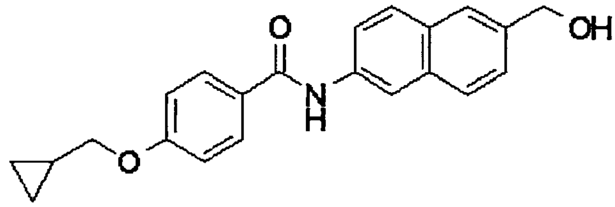

Ethyl 6-{[4-(cyclopropylmethoxy)benzoyl]amino}-2-naphthoate (779 mg) obtained in Reference Example 100 was dissolved in THF (20 mL), lithium aluminum hydride (228 mg) was added thereto under ice-cooling, and the mixture was stirred overnight at room temperature. Sodium sulfate decahydrate was added thereto, and the mixture was partitioned between ethyl acetate and water. The organic layer was washed with water and saturated brine, dried over magnesium sulfate, and concentrated under reduced pressure. The obtained residue was purified by silica gel column chromatography [eluent; hexane:ethyl acetate=7:3 (volume ratio)→hexane:ethyl acetate=0:10 (volume ratio)] to give the title compound (280 mg, yield 40%) as a colorless powder.
$^1$H NMR (300 MHz, DMSO-$d_6$) δ: 0.32-0.40 (2 H, m), 0.57-0.64 (2 H, m), 1.22-1.31 (1 H, m), 3.92 (2 H, d, J=6.8 Hz), 4.64 (2 H, d, J=5.7 Hz), 5.28 (1 H, t, J=5.7 Hz),7.07 (2 H, d, J=9.0 Hz), 7.44 (1 H, d, J=8.3 Hz), 7.75-7.89 (4 H, m), 7.99 (2 H, d, J=9.0 Hz), 8.40 (1 H, s), 10.24 (1 H, s).

Reference Example 102

N-[6-(chloromethyl)naphthalen-2-yl]-4-(cyclopropylmethoxy)benzamide

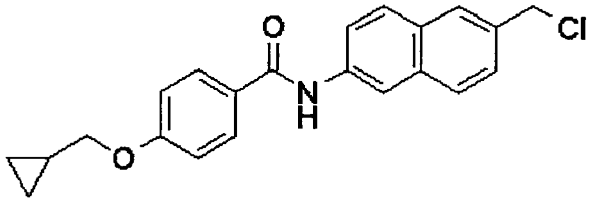

4-(Cyclopropylmethoxy)-N-[6-(hydroxymethyl)naphthalen-2-yl]benzamide (280 mg) obtained in Reference Example 101 was added to thionyl chloride (3 mL) cooled to -78°C. The reaction mixture was allowed to warm to -30°C, and stirred for 30 min. To the reaction mixture was added diethyl ether (20 mL), and the precipitated solid was collected by filtration to give the title compound (220 mg, yield 75%) as a colorless powder.
$^1$H NMR (300 MHz, DMSO-$d_6$) δ: 0.32-0.39 (2 H, m), 0.55-0.64 (2 H, m), 1.19-1.32 (1 H, m), 3.92 (2 H, d, J=6.8 Hz), 4.93 (2 H, s),7.07 (2 H, d, J=8.7 Hz), 7.52 (1 H, dd, J=8.5, 1.7 Hz), 7.81-7.93(4 H, m), 8.00 (2 H, d, J=9.1 Hz), 8.46 (1 H, s), 10.30 (1 H, s).

Reference Example 103

4-(cyclopropylmethoxy)-N-[6-({(1,1-dioxidotetrahydro-2H-thiopyran-4-yl)[(2-nitrophenyl)sulfonyl]amino}methyl)naphthalen-2-yl]benzamide

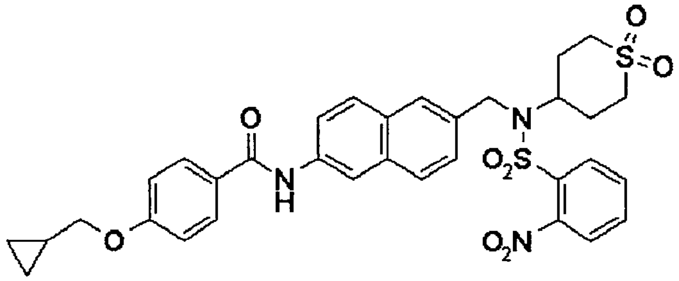

N-[6-(Chloromethyl)naphthalen-2-yl]-4-(cyclopropylmethoxy)benzamide (150 mg) obtained in Reference Example 102 and N-(1,1-dioxidotetrahydro-2H-thiopyran-4-yl)-2-nitrobenzenesulfonamide (165 mg) were dissolved in DMF (5 mL), potassium carbonate (138 mg) was added thereto, and the mixture was stirred overnight. The reaction solution was partitioned between ethyl acetate and water, and the organic layer was washed with water and saturated brine, dried over magnesium sulfate, and concentrated under reduced pressure.
The obtained residue was washed with ethyl acetate:hexane=1:1 (volume ratio) to give the title compound (190 mg, yield 70%) as a colorless powder.
$^1$H NMR (300 MHz, DMSO-$d_6$) δ: 0.31-0.40 (2 H, m), 0.54-0.65 (2 H, m), 1.21-1.36 (1 H, m), 1.74-2.07(4 H, m),2.93-2.99 (2 H, m),3.22-3.31(2 H, m),3.92 (2 H, d, J=7.2 Hz), 4.25 (1 H, br),4.72 (2 H,s),7.07 (2 H, d, J=9.1 Hz), 7.50 (1 H, dd, J=8.3, 1.5 Hz), 7.77-8.07(9 H, m), 8.17 (1 H, d, J=8.0 Hz), 8.42 (1 H, s), 10.28 (1 H, s).

Reference Example 104

4-(cyclopropylmethoxy)-N-(5-oxo-5,6,7,8-tetrahydronaphthalen-2-yl)benzamide

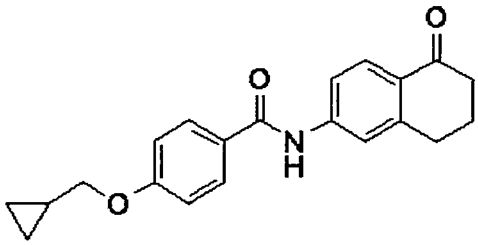

6-Amino-3,4-dihydronaphthalen-1(2H)-one (9.67 g), obtained in Reference Example 1, 4-(cyclopropylmethoxy) benzoic acid (11.53 g), N-(3-dimethylaminopropyl)-N'-ethylcarbodiimide hydrochloride (13.80 g), 1-hydroxybenzotriazole (11.02 g) and 4-dimethylaminopyridine (7.33 g) were dissolved in DMF (60 mL), and the solution was stirred overnight at room temperature. The reaction solution was partitioned between ethyl acetate and water, and the organic layer was washed with water and saturated brine, dried over magnesium sulfate, and concentrated under reduced pressure. The obtained residue was washed with ethyl acetate (50 mL) to give the title compound (9.34 g, yield 46%) as a pale-yellow powder.
$^1$H NMR (300 MHz, DMSO-$d_6$) δ: 0.30-0.39 (2 H, m), 0.54-0.64 (2 H, m), 1.17-1.34 (1 H, m), 1.99-2.10 (2 H, m),2.56 (2 H, t, J=6.4 Hz), 2.93 (2 H, t, J=5.7 Hz),3.92 (2 H, d, J=6.8 Hz), 7.06 (2 H, d, J=8.7 Hz), 7.69-7.76 (1 H, m), 7.82-7.88 (2 H, m), 7.96 (2 H, d, J=8.7 Hz),10.32 (1 H, s).

Reference Example 105

4-(cyclopropylmethoxy)-N-(5-methyl-7,8-dihydronaphthalen-2-yl)benzamide

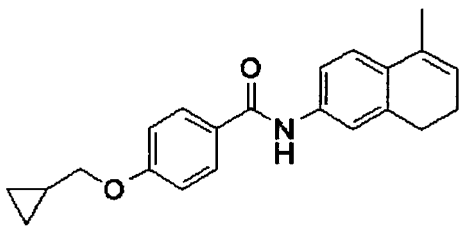

4-(Cyclopropylmethoxy)-N-(5-oxo-5,6,7,8-tetrahydronaphthalen-2-yl)benzamide (3.3 g) obtained in Reference Example 104 was dissolved in THF (100 mL), 1N MeMgBr-THF solution (40 mL) was added thereto, and the mixture was stirred at room temperature for 3 hr. The reaction solution was poured into saturated aqueous ammonium chloride solution, and the mixture was extracted with ethyl acetate.
The organic layer was washed with water and saturated brine, dried over magnesium sulfate, and concentrated under reduced pressure. The obtained residue (3.8 g) was dissolved in toluene (50 mL), p-toluenesulfonic acid monohydrate (172 mg) was added thereto, and the mixture was stirred at 60°C for 1 hr. The reaction solution was partitioned between ethyl acetate and water, and the organic layer was washed with water and saturated brine, dried over magnesium sulfate, and concentrated under reduced pressure. The obtained residue was purified by silica gel column chromatography [eluent; hexane:ethyl acetate=8:2 (volume ratio)→hexane:ethyl acetate=3:7 (volume ratio)] to give the title compound (1.05 g, yield 63%) as a colorless powder.
$^1$H NMR (300 MHz, DMSO-$d_6$) δ: 0.31-0.40 (2 H, m), 0.55-0.66 (2 H, m), 1.17-1.33 (1 H, m), 2.00 (3 H, s), 2.20 (2 H, br), 2.69 (2 H, t, J=7.9 Hz), 3.91 (2 H, d, J=7.2 Hz),5.79(1H,br), 7.04 (2 H, d, J=9.0 Hz), 7.17 (1 H, d, J=9.0 Hz),7.56-7.66 (2 H, m), 7.94 (2 H, d, J=9.0 Hz),10.02 (1 H, s).

Reference Example 106

4-(cyclopropylmethoxy)-N-(6-formyl-5-methyl-7,8-dihydronaphthalen-2-yl)benzamide

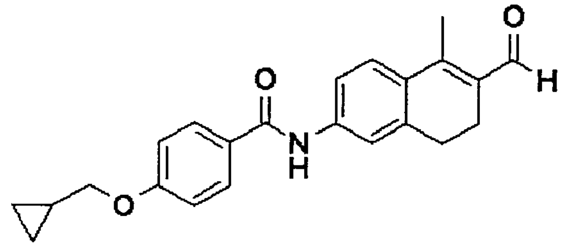

4-(Cyclopropylmethoxy)-N-(5-methyl-7,8-dihydronaphthalen-2-yl)benzamide (333 mg) obtained in Reference Example 105 and (chloromethylene)dimethylammonium chloride (384 mg) were dissolved in dichloromethane (15 mL), and the solution was stirred overnight at 50°C under a nitrogen atmosphere. The reaction solution was poured into aqueous sodium hydrogen carbonate solution, and the mixture was extracted with ethyl acetate. The organic layer was washed with water and saturated brine. The organic layer was filtered through acidic silica gel, and concentrated under reduced pressure to give the title compound (210 mg, yield 58%) as a pale-yellow powder.
$^1$H NMR (300 MHz, DMSO-$d_6$) δ: 0.30-0.39 (2 H, m), 0.53-0.64 (2 H, m), 1.22-1.30 (1 H, m), 1.99 (3 H, s), 2.33-2.43 (2 H, m), 2.65-2.73 (1 H, m), 2.94 (1 H, s),3.91 (2 H, d, J=7.0 Hz), 7.06 (2 H, d, J=8.9 Hz), 7.63 (1 H, d, J=8.7 Hz),7.70-7.80 (2 H, m), 7.91-7.98 (2 H, m),10.22 (1 H, s),10.29 (1 H, s).

Reference Example 107

4-(cyclopropylmethoxy)-N-(6-formyl-5-methylnaphthalen-2-yl)benzamide

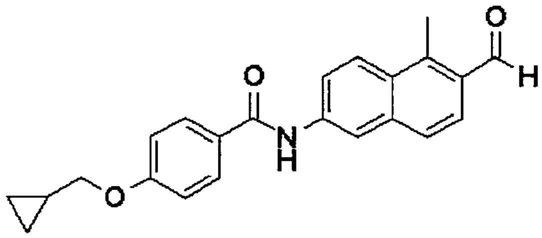

4-(Cyclopropylmethoxy)-N-(6-formyl-5-methyl-7,8-dihydronaphthalen-2-yl)benzamide (120 mg) obtained in Reference Example 106 was dissolved in dioxane (10 mL), 2,3-dichloro-5,6-dicyano-1,4-benzoquinone (113 mg) was added thereto, and the mixture was stirred at 80°C for 4 hr under a nitrogen atmosphere. The reaction solution was partitioned between ethyl acetate and water, and the organic layer was washed with 0.2N aqueous sodium hydroxide solution and saturated brine, dried over magnesium sulfate, and concentrated under reduced pressure. The obtained residue was purified by silica gel column chromatography [eluent; hexane:ethyl acetate=8:2 (volume ratio)→hexane: ethyl acetate=4:6 (volume ratio)] to give the title compound (80 mg, yield 66%) as a pale-yellow powder.
$^1$H NMR (300 MHz, DMSO-$d_6$) δ: 0.32-0.40 (2 H, m), 0.57-0.66 (2 H, m), 1.21-1.33 (1 H, m), 3.01 (3 H, s), 3.93 (2 H, d, J=6.8 Hz),7.09 (2 H, d, J=8.7 Hz), 7.75-8.06 (5 H, m), 8.33 (1 H, d, J=9.5 Hz), 8.52 (1 H, d, J=1.9 Hz) 10.46 (1 H, s), 10.59 (1 H, s).

Example 1

4-(cyclopropylmethoxy)-N-[8-methyl-3-({[2-(methylsulfonyl)ethyl]amino}methyl)quinolin-7-yl]benzamide

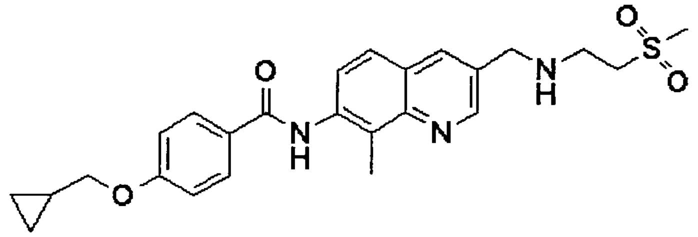

N-[3-(Chloromethyl)-8-methylquinolin-7-yl]-4-(cyclopropylmethoxy)benzamide hydrochloride (168 mg) obtained in Reference Example 5, 2-(methylsulfonyl)ethanamine hydrochloride (109 mg) and diisopropylethylamine (0.20 mL) were added to DMF (5.0 mL), and the mixture was stirred at 50°C for 5 hr. The solvent was evaporated under reduced pressure, and the residue was partitioned between ethyl acetate and saturated aqueous sodium hydrogen carbonate solution. The organic layer was separated, washed with water and saturated brine, dried over sodium sulfate, and concentrated under reduced pressure. The residue was washed with ethyl acetate, and recrystallized from methanol to give the title compound (92 mg, yield 45%) as a white solid.
$^1$H NMR (300 MHz, DMSO-$d_6$) δ: 0.25 - 0.43 (m, 2 H) 0.53 - 0.70 (m, 2 H) 1.15 - 1.39 (m, 1 H) 2.64 (s, 3 H) 3.06 (s, 3 H) 3.10 (br. s., 2 H) 3.34 - 3.42 (m, 2 H) 3.92 (d, J=7.16 Hz, 2 H) 4.05 (br. s., 2 H) 7.07 (d, J=8.85 Hz, 2 H) 7.62 (d, J=8.85 Hz, 1 H) 7.80 (d, J=8.67 Hz, 1 H) 8.02 (d, J=8.85 Hz, 2 H) 8.27 (d, J=1.51 Hz, 1 H) 8.93 (d, J=2.26 Hz, 1 H) 10.06 (s, 1 H). FAB(pos) : 468 $[MH]^+$

Example 2

4-(cyclopropylmethoxy)-N-[8-methyl-3-({[3-(methylsulfonyl)propyl]amino}methyl)quinolin-7-yl]benzamide

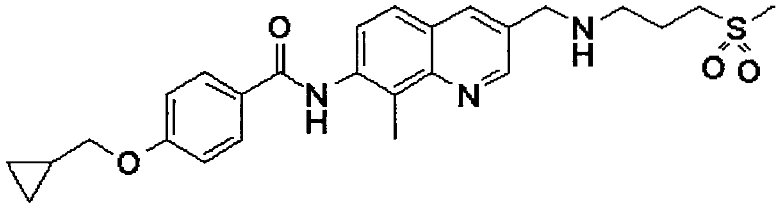

N-[3-(Chloromethyl)-8-methylquinolin-7-yl]-4-(cyclopropylmethoxy)benzamide hydrochloride (240 mg) obtained in Reference Example 5, 3-(methylsulfonyl)propan-1-amine hydrochloride (300 mg) and diisopropylethylamine (0.60 mL) were added to NMP (6.0 mL), and the mixture was stirred at 60°C for 4 hr. To the reaction solution was added ethyl acetate, and the mixture was washed with water and saturated brine, dried over sodium sulfate, and concentrated under reduced pressure. The residue was recrystallized from ethyl acetate to give the title compound (163 mg, yield 59%) as pale-orange crystals.
$^1$H NMR (300 MHz, $CDCl_3$) δ: 0.66-0.73 (2 H, m), 1.28-1.36 (1 H, m), 2.01-2.11 (2 H, m), 2.81 (3 H, s), 2.85 (2 H, t, J=6. 6 Hz), 2.91 (3 H, s), 3.13-3.22 (2 H, m), 3.90 (2 H, d, J=7.0 Hz), 3.99 (2 H, s), 7.02 (2 H, d, J=8.9 Hz), 7.70 (1 H, d, J=8.9 Hz), 7.90 (1 H, s), 7.92 (2 H, d, J=8.9 Hz), 8.02 (1 H, d, J=2.1 Hz), 8.27 (1 H, d, J=8.9 Hz), 8.89 (1 H, d, J=2.3 Hz). FAB (pos): 482 $[MH]^+$
elemental analysis value ($C_{26}H_{31}N_3O_4S$)
Calculated: C, 64.84; H, 6.49; N, 8.72.
Found: C, 64.57; H, 6.76; N, 8.68.

Example 3

4-(cyclopropylmethoxy)-N-[8-methyl-3-(1-{[3-(methylsulfonyl)propyl]amino}ethyl)quinolin-7-yl]benzamide

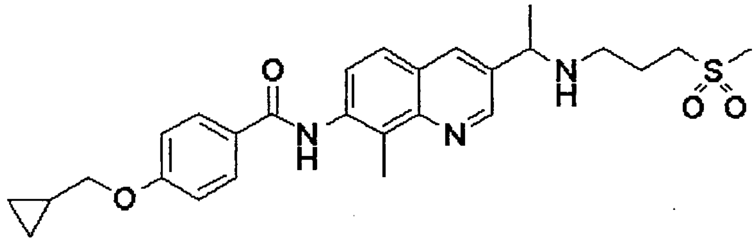

N-[3-(1-Chloroethyl)-8-methylquinolin-7-yl]-4-(cyclopropylmethoxy)benzamide hydrochloride (306 mg) obtained in Reference Example 7, 3-(methylsulfonyl)propan-1-amine hydrochloride (493 mg) and diisopropylethylamine (0.99 mL) were added to NMP (7.0 mL), and the mixture was stirred at 60°C for 5 days. To the reaction solution was added ethyl acetate, and the mixture was washed with water and saturated brine, dried over sodium sulfate, and concentrated under reduced pressure. The residue was purified by NH silica gel column chromatography [eluent; hexane:ethyl acetate=4:1 (volume ratio)→ethyl acetate:methanol=17:3 (volume ratio)], and the obtained solid was recrystallized from ethyl acetate to give the title compound (129 mg, yield 37%) as white crystals.
$^1$H NMR (300 MHz, $CDCl_3$) δ: 0.36-0.43 (2 H, m), 0.65-0.73 (2 H, m), 1.27-1.37 (1 H, m), 1.47 (3 H, d, J=6.6 Hz), 1.98 (2 H, quintet, J=7.3 Hz), 2.55 (1 H, dt, J=11.9, 6.9 Hz), 2.77 (1 H, t, J=6.2 Hz), 2.81 (3 H, s), 2.88 (3 H, s), 2.98-3.10 (1 H, m), 3.14-3.26 (1 H, m), 3.90 (2 H, d, J=6.8 Hz), 4.00 (1 H, q, J=6.6 Hz), 7.02 (2 H, d, J=8.9 Hz), 7.71 (1 H, d, J=9.0 Hz), 7.91 (1 H, s), 7.92 (2 H, d, J=9.0 Hz), 7.99 (1 H, d, J=2.1 Hz), 8.27 (1 H, d, J=8.9 Hz), 8.91 (1 H, d, J=2.1 Hz). FAB (pos): 496 $[MH]^+$
elemental analysis value ($C_{27}H_{33}N_3O_4S$)
Calculated: C, 65.43; H, 6.71; N, 8.48.
Found: C, 65.22; H, 6.74; N, 8.45.

Example 4

4-(cyclopropylmethoxy)-N-[3-({[2,2-dimethyl-3-(methylsulfonyl)propyl]amino}methyl)-8-methylquinolin-7-yl]benzamide

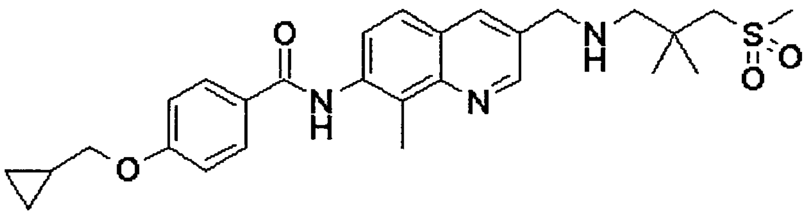

N-[3-(Chloromethyl)-8-methylquinolin-7-yl]-4-(cyclopropylmethoxy)benzamide hydrochloride (418 mg) obtained in Reference Example 5, 2,2-dimethyl-3-(methylsulfonyl)propan-1-amine hydrochloride (505 mg) obtained in Reference Example 15 and ethyldiisopropylamine (0.96 mL) were added to NMP (10 mL), and the mixture was stirred at 60°C for 6 hr. The mixture was diluted with ethyl acetate, washed successively with water (three times) and saturated brine, dried over sodium sulfate, and concentrated under reduced pressure. The residue was purified by silica gel column chromatography [eluent; hexane:ethyl acetate=4:1 (volume ratio)→ethyl acetate], and the obtained solid was recrystallized from ethyl acetate to give the title compound (296 mg, yield 58%) as white crystals. $^1$H NMR (300 MHz, $CDCl_3$) δ: 0.36-0.43 (2 H, m), 0.65-0.73 (2 H, m), 1.18 (6 H, s), 1.26-1.36 (1 H, m), 2.68 (2 H, s), 2.81 (3 H, s), 2.91 (3 H, s), 3.15 (2 H, s), 3.90 (2 H, d, J=6.8 Hz), 4.00 (2 H, s), 7.02 (2 H, d, J=8.7 Hz), 7.71 (1 H, d, J=8.7 Hz), 7.90 (1 H, s), 7.92 (2 H, d, J=9.0 Hz), 8.01 (1 H, d, J=1.9 Hz), 8.26 (1 H, d, J=8.7 Hz), 8.92 (1 H, d, J=2.3 Hz). FAB(pos): 510 $[MH]^+$
elemental analysis value ($C_{28}H_{35}N_3O_4$)
Calculated: C, 65.99; H, 6.92; N, 8.24.
Found: C, 65.91; H, 6.90; N, 8.17.

Example 5

4-(cyclopropylmethoxy)-N-[3-(1-{[2,2-dimethyl-3-(methylsulfonyl)propyl]amino}ethyl)-8-methylquinolin-7-yl]benzamide

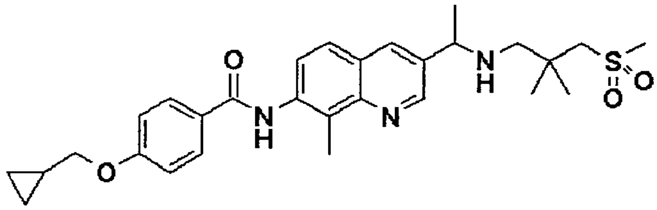

N-[3-(1-Chloroethyl)-8-methylquinolin-7-yl]-4-(cyclopropylmethoxy)benzamide hydrochloride (1.45 g) obtained in Reference Example 7, 2,2-dimethyl-3-(methylsulfonyl)propan-1-amine hydrochloride (3.39 g) obtained in Reference Example 15 and ethyldiisopropylamine (4.4 mL) were added to NMP (35 mL), and the mixture was stirred at 70°C for 47 hr. The mixture was diluted with ethyl acetate, washed successively with water (three times) and saturated brine, dried over sodium sulfate, and concentrated under reduced pressure. The residue was purified by silica gel column chromatography [eluent; hexane:ethyl acetate=3:1 (volume ratio)→ethyl acetate:methanol→4:1 (volume ratio)] to give the title compound (1.38 g, yield 78%) as a brown amorphous solid.
$^1$H NMR (300 MHz, $CDCl_3$) δ: 0.36-0.43 (2 H, m), 0.65-0.73 (2 H, m), 1.10 (3 H, s), 1.18 (3 H, s), 1.28-1.38 (1 H, m), 1.47 (3 H, d, J=6.8 Hz), 2.33 (1 H, d, J=12.2 Hz), 2.67 (1 H, d, J=12.2 Hz), 2.81 (3 H, s), 2.90 (3 H, s), 2.99 (1 H, d, J=13.9 Hz), 3.23 (1 H, d, J=13.9 Hz), 3.90 (2 H, d, J=7.0 Hz), 3.95 (1 H, q, J=6.7 Hz), 7.02 (2 H, d, J=8.9 Hz), 7.70 (1 H, d, J=8.9 Hz), 7.90 (1 H, s), 7.92 (2 H, d, J=8.9 Hz), 7.98 (1 H, d, J=2.3 Hz), 8.26 (1 H, d, J=8.9 Hz), 8.93 (1 H, d, J=2.3 Hz). FAB(pos): 524 $[MH]^+$

Example 6

4-(cyclopropylmethoxy)-N-{8-methyl-3-[({2-[(methylsulfonyl)amino]ethyl}amino)methyl]quinolin-7-yl}benzamide

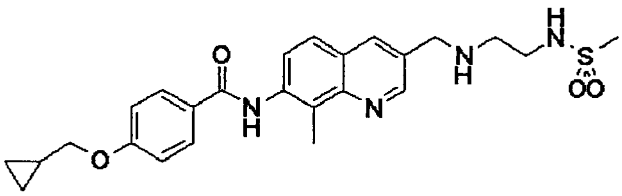

N-[3-(Chloromethyl)-8-methylquinolin-7-yl]-4-(cyclopropylmethoxy)benzamide hydrochloride (418 mg) obtained in Reference Example 5, N-(2-aminoethyl)methanesulfonamide hydrochloride (437 mg) obtained in Reference Example 17 and ethyldiisopropylamine (0.87 mL) were added to NMP (10 mL), and the mixture was stirred at 70°C for 2 hr. The mixture was diluted with ethyl acetate, washed with water (three times), dried over sodium sulfate, and concentrated under reduced pressure. The residue was recrystallized from ethyl acetate to give the title compound (283 mg, yield 59%) as pale-yellow crystals.
$^1$H NMR (300 MHz, $CDCl_3$) δ: 0.36-0.43 (2 H, m), 0.66-0.73 (2 H, m), 1.29-1.37 (1 H, m), 2.81 (3 H, s), 2.90 (2 H, dd, J=6.3, 4.8 Hz), 2.95 (3 H, s), 3.24 (2 H, t, J=5.4 Hz), 3.90 (2 H, d, J=7.0 Hz), 3.99 (2 H, s), 4.88 (1 H, br. s.), 7.02 (2 H, d, J=8.9 Hz), 7.70 (1 H, d, J=9.0 Hz), 7.91 (1 H, s), 7.92 (2 H, d, J=8.9 Hz), 8.02 (1 H, d, J=2.1 Hz), 8.27 (1 H, d, J=8.9 Hz), 8.87 (1 H, d, J=2.3 Hz).
FAB(pos): 483 $[MH]^+$

Example 7

4-(cyclopropylmethoxy)-N-{8-methyl-3-[1-({2-[(methylsulfonyl)amino]ethyl}amino)ethyl]quinolin-7-yl}benzamide

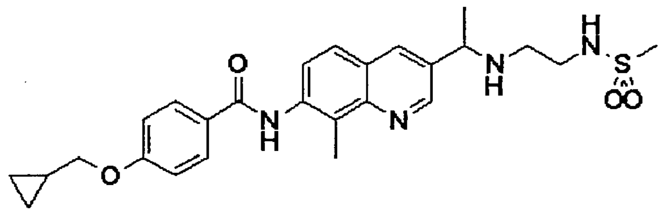

N-[3-(1-Chloroethyl)-8-methylquinolin-7-yl]-4-(cyclopropylmethoxy)benzamide hydrochloride (432 mg) obtained in Reference Example 7, N-(2-aminoethyl)methanesulfonamide hydrochloride (3.39 g) obtained in Reference Example 17 and ethyldiisopropylamine (1.3 mL) were added to NMP (10 mL), and the mixture was stirred at 70°C for 28 hr. The mixture was diluted with ethyl acetate, washed with water (three times), dried over sodium sulfate, and concentrated under reduced pressure. The residue was purified by NH silica gel column chromatography [eluent; ethyl acetate→ethyl acetate:methanol→4:1 (volume ratio)], and the obtained solid was recrystallized from ethyl acetate to give the title compound (324 mg, yield 65%) as pale-yellow crystals.
$^1$H NMR (300 MHz, $CDCl_3$) δ: 0.36-0.43 (2 H, m), 0.65-0.73 (2 H, m), 1.27-1.37 (1 H, m), 1.49 (3 H, d, J=6.6 Hz), 2.62-2.71 (1 H, m), 2.76-2.86 (1 H, m), 2.81 (3 H, s), 2.91 (3 H, s), 3.05-3.14 (1 H, m), 3.15-3.25 (1 H, m), 3.90 (2 H, d, J=7.0 Hz), 4.00 (1 H, q, J=6.6 Hz), 4.83 (1 H, br), 7.01 (2 H, d, J=8.9 Hz), 7.71 (1 H, d, J=9.0 Hz), 7.91 (1 H, s), 7.92 (2 H, d, J=8.9 Hz), 8.00 (1 H, d, J=2.3 Hz), 8.27 (1 H, d, J=8.9 Hz), 8.89 (1 H, d, J=2.3 Hz).
FAB(pos): 497 $[MH]^+$
elemental analysis value ($C_{26}H_{32}N_4O_4S$)
Calculated: C, 62.88; H, 6.49; N, 11.28.
Found: C, 62.62; H, 6.56; N, 11.02.

Example 8

4-(cyclopropylmethoxy)-N-[8-methyl-3-({[1-(methylsulfonyl)piperidin-4-yl]amino}methyl)quinolin-7-yl]benzamide

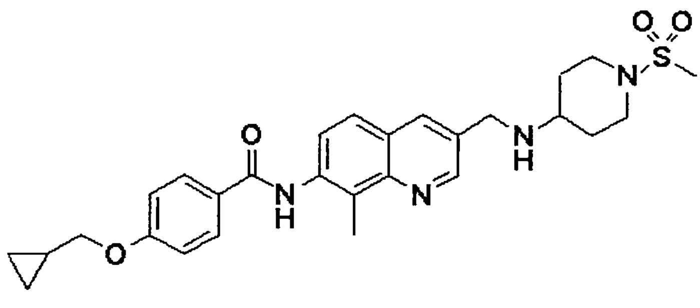

N-[3-(Chloromethyl)-8-methylquinolin-7-yl]-4-(cyclopropylmethoxy)benzamide hydrochloride (1200 mg) obtained in Reference Example 5, 1-(methylsulfonyl)piperidin-4-amine hydrochloride (1930 mg) obtained in Reference Example 18 and diisopropylethylamine (2.59 mL) were added to DMF (50.0 mL), and the mixture was stirred overnight at 60°C. To the reaction solution was added ethyl acetate, and the mixture was washed with water and saturated brine, dried over sodium sulfate, and concentrated under reduced pressure. The residue was purified by silica gel column chromatography [eluent; ethyl acetate:methanol=90:10 (volume ratio)→ethyl acetate:methanol=80:20 (volume ratio)], and the obtained solid was recrystallized from ethyl acetate to give the title compound (803 mg, yield 53%) as white crystals.

$^1$H NMR (300 MHz, DMSO-$d_6$) δ: 0.33-0.38 (2 H, m), 0.57-0.63 (2 H, m), 1.23-1.37 (1 H, m), 1.41-1.45 (2 H, m), 1.92-1.97 (2 H, m),2.46-2.54 (2 H, m),2.63 (3 H, s), 2.73-2.77 (2 H, m),2.82 (3 H, s), 3.44-3.48 (2 H, m), 3.90-3.95 (4 H, m), 7.06 (2 H, d, J=8.7 Hz), 7.57 (1 H, d, J=8.7 Hz), 7.77 (1 H, d, J=8.7 Hz), 8.01 (2 H, d, J=8.7 Hz), 8.21 (1 H, d, J=2.1 Hz), 8.91 (1 H, d, J=2.1 Hz), 10.04 (1 H, s).
FAB(pos): 523 $[MH]^+$
elemental analysis value ($C_{28}H_{34}N_4O_4S \cdot 0.5H_2O$)
Calculated: C, 63.25; H, 6.64; N, 10.54.
Found: C, 63.55; H, 6.47; N, 10.70.

Example 9

4-(cyclopropylmethoxy)-N-[3-({[2-(1,1-dioxidoisothiazolidin-2-yl)ethyl]amino}methyl)-8-methylquinolin-7-yl]benzamide

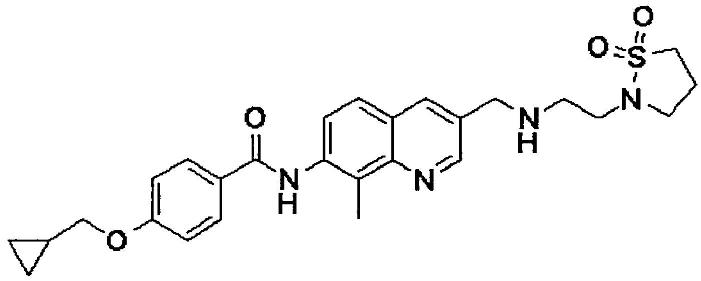

N-[3-(Chloromethyl)-8-methylquinolin-7-yl]-4-(cyclopropylmethoxy)benzamide hydrochloride (1200 mg) obtained in Reference Example 5, 2-(1,1-dioxidoisothiazolidin-2-yl)ethanamine hydrochloride (2000 mg) obtained in Reference Example 19 and diisopropylethylamine (2.59 mL) were added to DMF (50.0 mL), and the mixture was stirred at 60°C for 4 hr.
To the reaction solution was added ethyl acetate, and the mixture was washed with water and saturated brine, dried over sodium sulfate, and concentrated under reduced pressure. The residue was purified by silica gel column chromatography [eluent; ethyl acetate:methanol=90:10 (volume ratio)→ethyl acetate:methanol=80:20 (volume ratio)], and the obtained solid was recrystallized from ethyl acetate to give the title compound (422 mg, yield 29%) as white crystals.
$^1$H NMR (300 MHz, DMSO-$d_6$) δ: 0.35-0.37 (2 H, m), 0.57-0.61 (2 H, m), 1.24-1.26 (1 H, m),2.18-2.27 (2 H, m), 2.65 (3 H, s), 2.92-2.93 (2 H, m),3.13-3.25 (6 H, m),3.36 (1 H, br),3.92 (2 H, d, J=6.9 Hz), 4.13 (2 H, s), 7.05 (2 H, d, J=8.7 Hz), 7.62 (1 H, d, J=8.7 Hz), 7.79 (1 H, d, J=8.7 Hz), 8.02 (2 H, d, J=8.4 Hz), 8.33 (1 H, s), 8.97 (1 H, d, J=1.8 Hz), 10.11 (1 H, s). FAB(pos): 509 $[MH]^+$

Example 10

4-(cyclopropylmethoxy)-N-(8-methyl-3-{[(2-{[(trifluoromethyl)sulfonyl]amino}ethyl)amino]methyl}quinolin-7-yl)benzamide

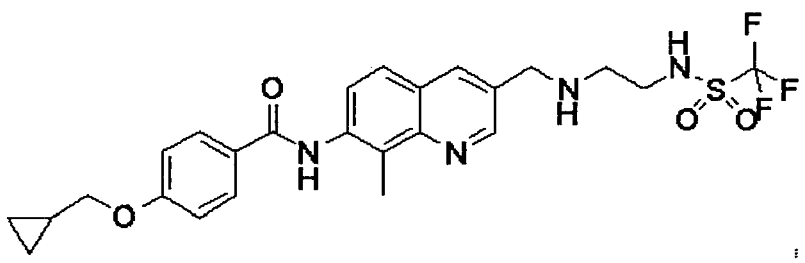

N-[3-(Chloromethyl)-8-methylquinolin-7-yl]-4-(cyclopropylmethoxy)benzamide (1000 mg) obtained in Reference Example 5, N-(2-aminoethyl)-1,1,1-trifluoromethanesulfonamide hydrochloride (1370 mg) obtained in Reference Example 20 and triethylamine (1.39 mL) were added to DMF (30.0 mL), and the mixture was stirred at 80°C for 4 hr. To the reaction solution was added ethyl acetate, and the mixture was washed with water and saturated brine, dried over sodium sulfate, and concentrated under reduced pressure. The residue was purified using preparative HPLC [Apparatus: Gilson Inc. high throughput purification system, column: YMC Combiprep ODS-A, S-5 μm, 50x20mm, solvent: SOLUTION A; 0.1% trifluoroacetic acid-containing water, SOLUTION B; 0.1% trifluoroacetic acid-containing acetonitrile, gradient cycle: 0.00 min (SOLUTION A/SOLUTION B=90/10), 1.00 min (SOLUTION A/SOLUTION B=90/10), 4.20 min (SOLUTION A/SOLUTION B=10/90), 5.40 min (SOLUTION A/SOLUTION B=10/90), 5.50 min (SOLUTION A/SOLUTION B=90/10), 5.60 min (SOLUTION A/SOLUTION B=90/10), flow rate: 25 mL/min, detection method: UV 220nm], and the fractions were concentrated under reduced pressure. To the residue was added aqueous potassium carbonate solution, and the mixture was extracted with ethyl acetate. The extract was washed with water and saturated brine, dried over sodium sulfate, and concentrated under reduced pressure to give the title compound (424 mg, yield 30%) as white crystals.
$^1$H NMR (300 MHz, DMSO-$d_6$) δ: 0.34-0.37 (2 H, m), 0.58-0.61 (2 H, m), 1.26-1.28 (1 H, m),2.64 (3 H, s), 2.72-2.78 (2 H, m),3.16 (2 H, br),3.92 (2 H, d, J=7.2 Hz), 4.04 (2 H, s), 3.82-4.10 (2 H, m),7.04-7.07 (2 H, m),7.58-7.61 (1 H, m), 7.78 (1 H, d, J=8.7 Hz), 7.99-8.02 (2 H, m), 8.27 (1 H, s), 8.91 (1 H, s), 10.03 (1 H, s).
FAB(pos): 537 $[MH]^+$

Example 11

4-(cyclopropylmethoxy)-N-{8-methyl-3-[({1-[(trifluoromethyl)sulfonyl]piperidin-4-yl}amino)methyl]quinolin-7-yl}benzamide

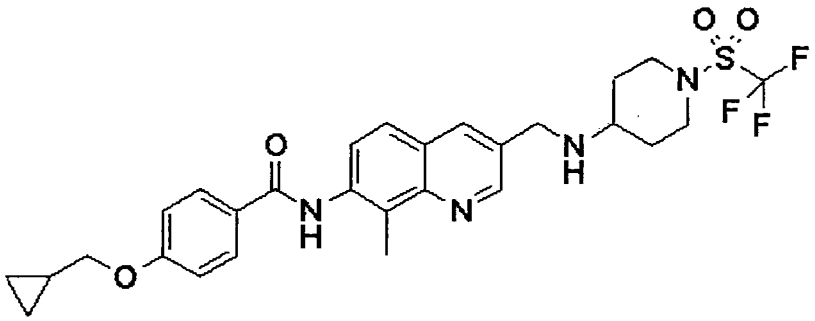

N-[3-(Chloromethyl)-8-methylquinolin-7-yl]-4-(cyclopropylmethoxy)benzamide (800 mg) obtained in Reference Example 5, 1-[(trifluoromethyl)sulfonyl]piperidin-4-amine hydrochloride (1160 mg) obtained in Reference Example 21 and triethylamine (1.39 mL) were added to DMF (15.0 mL), and the mixture was stirred at 80°C for 3 hr. To the reaction solution was added ethyl acetate, and the mixture was washed with water and saturated brine, dried over sodium sulfate, and concentrated under reduced pressure. The residue was purified by silica gel column chromatography [eluent; ethyl acetate:methanol=90:10 (volume ratio)→ethyl acetate:methanol=80:20 (volume ratio)], and the obtained solid was recrystallized from ethyl acetate to give the title compound (143 mg, yield 12%) as white crystals.
$^1$H NMR (300 MHz, DMSO-$d_6$) δ: 0.35-0.37 (2 H, m), 0.59-0.61 (2 H, m), 1.24-1.45 (1 H, m),1.95-2.00 (2 H, m), 2.51-2.61 (1 H, m), 2.63 (3 H, s), 3.26-3.32 (3 H, m),3.73-3.77 (2 H, m),3.90-3.94 (4 H, m), 7.05 (2 H, d, J=8.1 Hz), 7.57 (1 H, d, J=8.4 Hz), 7.76 (1 H, d, J=7.8 Hz), 7.99 (2 H, d, J=8.4 Hz), 8.20 (1 H, s), 8.90 (1 H, s), 10.02 (1 H, s).
FAB(pos): 577 $[MH]^+$

Example 12

4-(cyclopropylmethoxy)-N-[8-methyl-3-({[1-(methylsulfonyl)pyrrolidin-3-yl]amino}methyl)quinolin-7-yl]benzamide

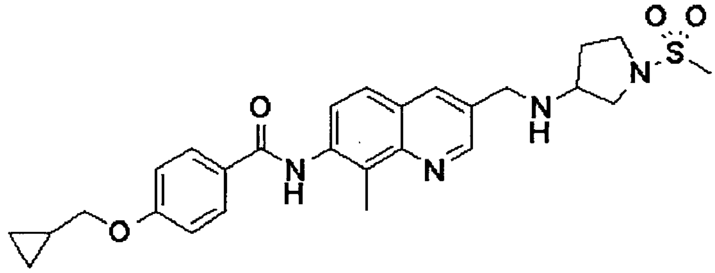

N-[3-(Chloromethyl)-8-methylquinolin-7-yl]-4-(cyclopropylmethoxy)benzamide (800 mg) obtained in Reference Example 5, 1-(methylsulfonyl)pyrrolidin-3-amine hydrochloride (863 mg) obtained in Reference Example 22 and triethylamine (1.39 mL) were added to DMF (15.0 mL), and the mixture was stirred at 80°C for 3 hr. To the reaction solution was added ethyl acetate, and the mixture was washed with water and saturated brine, dried over sodium sulfate, and concentrated under reduced pressure. The residue was purified by silica gel column chromatography [eluent; ethyl acetate:methanol=100:0 (volume ratio)→ethyl acetate:methanol=80:20 (volume ratio)], and the obtained solid was recrystallized from ethyl acetate to give the title compound (526.9 mg, yield 49%) as white crystals.
$^1$H NMR (300 MHz, DMSO-$d_6$) δ: 0.35-0.38 (2 H, m), 0.59-0.61 (2 H, m), 1.25-1.29 (1 H, m),1.82-1.84 (1 H, m), 1.98-2.00 (1 H, m), 2.51-2.62 (1 H, m), 2.64 (3 H, s), 2.89 (3 H, s),3.11-3.32 (5 H, m),3.90-3.94 (4 H, m), 7.06 (2 H, d, J=8.7 Hz), 7.57 (1 H, d, J=8.7 Hz), 7.77 (1 H, d, J=8.7 Hz), 8.00 (2 H, d, J=8.1 Hz), 8.21 (1 H, s), 8.90 (1 H, s), 10.02 (1 H, s).
FAB(pos): 509 [MH]$^+$

Example 13

4-(cyclopropylmethoxy)-N-{8-methyl-3-[({3-[(methylsulfonyl)amino]propyl}amino)methyl]quinolin-7-yl}benzamide

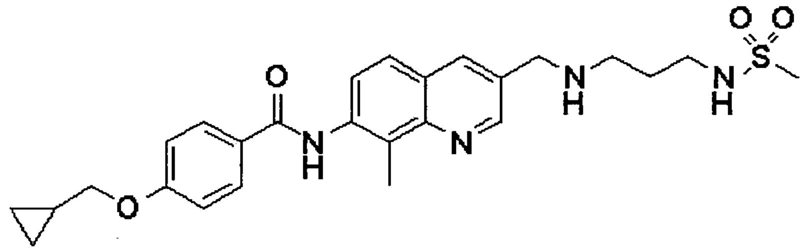

4-(Cyclopropylmethoxy)-N-(3-formyl-8-methylquinolin-7-yl)benzamide (2.00 g) obtained in Reference Example 3 and N-(3-aminopropyl)methanesulfonamide hydrochloride (1470 mg) obtained in Reference Example 23 were added to a mixed solvent of acetic acid (7.0 mL) and DMA (20 mL), and the mixture was stirred at room temperature for 1 hr. Sodium triacetoxyborohydride (2.35 g) was added thereto at the same temperature, and the mixture was stirred for additional 2 hr, and basified with 1N aqueous sodium hydroxide solution. The mixture was extracted with ethyl acetate, and the organic layer was washed with water and saturated brine, dried over sodium sulfate, and concentrated under reduced pressure. The residue was dissolved in a mixed solvent of DMSO (20 mL) and water (40 mL), citric acid (4.0 g) was added thereto, and the mixture was washed with ethyl acetate, and basified with 1N aqueous sodium hydroxide solution. The mixture was extracted with ethyl acetate, and the extract was washed with water and saturated brine, dried over sodium sulfate, and concentrated under reduced pressure. The residue was recrystallized from diisopropyl ether-methanol to give the title compound (1.71 g, yield 62%) as white crystals.
$^1$H NMR (300 MHz, DMSO-$d_6$) δ: 0.33-0.38 (2 H, m), 0.57-0.63 (2 H, m), 1.24-1.28 (1 H, m),1.60-1.69 (2 H, m), 2.34 (1 H, br), 2.59 (2 H, t, J=6.6 Hz), 2.64 (3 H, s), 2.87 (3 H, s), 3.00-3.02 (2 H, m), 3.89-3.93 (4 H, m), 6.98 (1 H, br), 7.06 (2 H, d, J=8.7 Hz), 7.57 (1 H, d, J=8.4 Hz), 7.77 (1 H, d, J=8.7 Hz), 8.01 (2 H, d, J=9.0 Hz), 8.19 (1 H, d, J=1.8 Hz), 8.88 (1 H, d, J=1.8 Hz), 10.23 (1 H, s).
FAB (pos) : 497 [MH]$^+$

Example 14

4-(cyclopropylmethoxy)-N-{8-methyl-3-[({[1-(methylsulfonyl)piperidin-4-yl]methyl}amino)methyl]quinolin-7-yl}benzamide

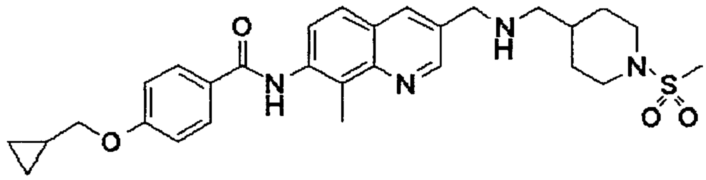

4-(Cyclopropylmethoxy)-N-(3-formyl-8-methylquinolin-7-yl)benzamide (2.00 g) obtained in Reference Example 3 and 1-[1-(methylsulfonyl)piperidin-4-yl]methanamine hydrochloride (1780 mg) obtained in Reference Example 24 were added to a mixed solvent of acetic acid (7.0 mL) and DMA (20 mL), and the mixture was stirred at room temperature for 1 hr. Sodium triacetoxyborohydride (2.35 g) was added thereto at the same temperature, and the mixture was stirred for 2 hr, and basified with 1N aqueous sodium hydroxide solution. The mixture was extracted with ethyl acetate, and the extract was washed with water and saturated brine, dried over sodium sulfate, and concentrated under reduced pressure. The residue was dissolved in a mixed solvent of DMSO (20 mL) and water (40 mL), citric acid (4.0 g) was added thereto, and the mixture was washed with ethyl acetate, and basified with 1N aqueous sodium hydroxide solution. The mixture was extracted with ethyl acetate, and the extract was washed with water and saturated brine, dried over sodium sulfate, and concentrated under reduced pressure. The residue was recrystallized from diisopropyl ether-methanol to give the title compound (1.87 g, yield 63%) as white crystals.
$^1$H NMR (300 MHz, DMSO-$d_6$) δ: 0.35-0.37 (2 H, m), 0.59-0.61 (2 H, m), 1.11-1.26 (3 H, m), 1.53-1.58 (1 H, m),1.82-1.86 (2 H, m), 2.34 (1 H, br), 2.44 (2 H, d, J=6.3 Hz), 2.64 (3 H, s), 2.63-2.70 (2 H, m), 2.83 (3 H, s), 3.52-3.56 (2 H, m), 3.89-3.93 (4 H, m), 7.06 (2 H, d, J=8.7 Hz), 7.57 (1 H, d, J=8.7 Hz), 7.77 (1 H, d, J=9.0 Hz), 8.01 (2 H, d, J=8.4 Hz), 8.19 (1 H, s), 8.89 (1 H, s), 10.03 (1 H, s).
FAB(pos): 537 $[MH]^+$

Example 15

4-(cyclopropylmethoxy)-N-[3-({[(4-hydroxy-1,1-dioxidotetrahydro-2H-thiopyran-4-yl)methyl]amino}methyl)-8-methylquinolin-7-yl]benzamide

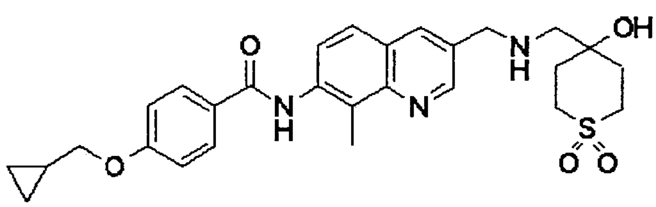

4-(Cyclopropylmethoxy)-N-(3-formyl-8-methylquinolin-7-yl)benzamide (84.7 mg) obtained in Reference Example 3 and 4-(aminomethyl)tetrahydro-2H-thiopyran-4-ol 1,1-dioxide hydrochloride (48.0 mg) obtained in Reference Example 28 were suspended in NMP (4.0 mL), and diisopropylethylamine (0.04 mL) and acetic acid (2.0 mL) were added thereto. The mixture was stirred at room temperature for 8.5 hr, and sodium triacetoxyborohydride (250 mg) was added thereto.
On the other hand, 4-(cyclopropylmethoxy)-N-(3-formyl-8-methylquinolin-7-yl)benzamide (200 mg) obtained in Reference Example 3 and 4-(aminomethyl)tetrahydro-2H-thiopyran-4-ol 1,1-dioxide hydrochloride (144 mg) obtained in Reference Example 28 were suspended in NMP (5.0 mL), and diisopropylethylamine (0.11 mL) and acetic acid (2.0 mL) were added thereto. The mixture was stirred at room temperature for 2 hr, and sodium triacetoxyborohydride (589 mg)

was added thereto.
Both of the reaction solutions were each stirred for 14 hr, combined, and basified with 8N aqueous sodium hydroxide solution. The mixture was extracted with ethyl acetate, and the organic layer was washed with water and saturated brine. The organic layer was purified by NH silica gel column chromatography [eluent; ethyl acetate→ethyl acetate:methanol=9:1 (volume ratio)], and the obtained solid was recrystallized from diisopropyl ether-ethyl acetate-methanol to give the title compound (307 mg, yield 74%) as a flesh-colored solid.
$^1$H NMR (300 MHz, $CDCl_3$) δ: 0.36-0.43 (2 H, m), 0.65-0.74 (2 H, m), 1.26-1.36 (1 H, m), 1.90-2.17 (2 H, m), 2.69 (2 H, s), 2.81 (3 H, s), 2.83-2.93 (2 H, m), 3.28 (1 H, br. s.), 3.37-3.53 (4 H, m), 3.90 (2 H, d, J=6.8 Hz), 4.05 (2 H, s), 7.02 (2 H, d, J=8.7 Hz), 7.71 (1 H, d, J=8.7 Hz), 7.88-7.95 (3 H, m), 8.01 (1 H, d, J=2.3 Hz), 8.31 (1 H, d, J=8.7 Hz), 8.89 (1 H, d, J=2.3 Hz).
FAB(pos): 524 $[MH]^+$

Example 16

4-(cyclopropylmethoxy)-N-[3-({[(1,1-dioxidotetrahydro-2H-thiopyran-4-yl)methyl]amino}methyl)-8-methylquinolin-7-yl] benzamide

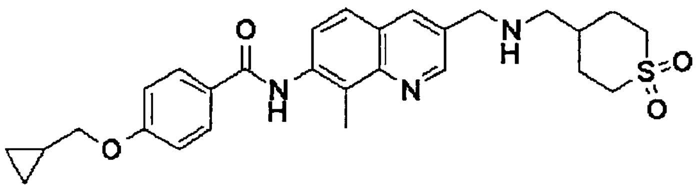

4-(Cyclopropylmethoxy)-N-(8-methyl-3-{[(tetrahydro-2H-thiopyran-4-ylmethyl)amino]methyl}quinolin-7-yl) benzamide (200 mg) obtained in Reference Example 31 was dissolved in acetone (10 mL)-methanol (10 mL)-water (2.0 mL), oxone (388 mg) was added thereto, and the mixture was stirred at room temperature for 86 hr. The reaction solution was concentrated under reduced pressure, the residue was suspended in ethyl acetate-THF, and the suspension was basified with 1N aqueous sodium hydroxide solution. The organic layer was washed with saturated brine, dried over sodium sulfate, and concentrated under reduced pressure. The residue was purified by silica gel column chromatography [eluent; ethyl acetate:methanol=17:3 (volume ratio)→ethyl acetate:methanol=1:1 (volume ratio)], and the obtained solid was recrystallized from diisopropyl ether-ethyl acetate-methanol to give the title compound (148 mg, yield 69%) as a white solid.
$^1$H NMR (300 MHz, $CDCl_3$) δ: 0.36-0.43 (2 H, m), 0.64-0.74 (2 H, m), 1.25-1.39 (1 H, m), 1.61-1.74 (1 H, m), 1.78-1.96 (2 H, m), 2.23 (2 H, dd, J=14.0, 2.5 Hz), 2.63 (2 H, d, J=6.6 Hz), 2.82 (3 H, s), 2.88-3.01 (2 H, m), 3.01-3.12 (2 H, m), 3.90 (2 H, d, J=7.0 Hz), 3.99 (2 H, s), 7.02 (2 H, d, J=8.9 Hz), 7.70 (1 H, d, J=8.9 Hz), 7.87 - 7.96 (3 H, m), 8.02 (1 H, d, J=2.3 Hz), 8.28 (1 H, d, J=8.9 Hz), 8.89 (1 H, d, J=2.3 Hz).
FAB(pos): 508 $[MH]^+$
elemental analysis value ($C_{28}H_{33}N_3O_4S$)
Calculated: C, 66.25; H, 6.55; N, 8.28.
Found: C, 66.02; H, 6.56; N, 8.21.

Example 17

4-(cyclopropylmethoxy)-N-[8-methyl-3-({[(1-oxidotetrahydro-2H-thiopyran-4-yl)methyl]amino}methyl)quinolin-7-yl]benzamide

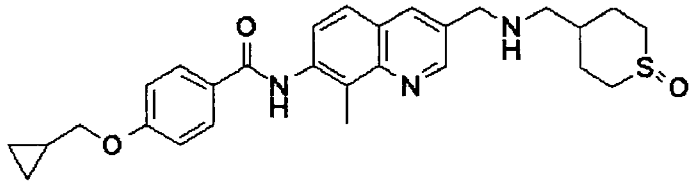

4-(Cyclopropylmethoxy)-N-(8-methyl-3-{[(tetrahydro-2H-thiopyran-4-ylmethyl)amino]methyl}quinolin-7-yl) benzamide (200 mg) obtained in Reference Example 31 was dissolved in methanol (30 mL)-water (3.0 mL), sodium periodate (247 mg) was added thereto, and the mixture was stirred at room temperature for 7 hr. The mixture was basified with saturated aqueous sodium hydrogen carbonate solution, and concentrated under reduced pressure. The residue was dissolved in water-DMSO, and the solution was acidified with citric acid, and washed with ethyl acetate. The solution was added to ethyl acetate-THF, and the mixture was basified with 1N aqueous sodium hydroxide solution. The organic layer was washed with saturated brine (three times), dried over sodium sulfate, and concentrated under reduced pressure. The residue was washed with ethyl acetate, and dried under reduced pressure to give the title compound (441 mg, geometric isomer ratio=35/65, yield 89%) as a white solid.
$^1$H NMR (300 MHz, $CDCl_3$) δ: 0.35-0.44 (2 H, m), 0.64-0.74 (2 H, m), 1.22-1.51 (3 H, m), 1.58-1.74 (1 H, m), 1.77-1.90 (0.65 x 2 H, m), 2.02-2.28 (2 H, m), 2.44 (0.65 x 2 H, td, J=13.5, 3.6 Hz), 2.52-2.68 (2 H, m), 2.81 (3 H, s), 2.97-3.08 (0.65 x 2 H, m), 3.27-3.37 (0.35 x 2 H, m), 3.90 (2 H, d, J=7.2 Hz), 3.98 (0.35 x 2 H, s), 3.99 (0.65 x 1 H, s), 7.02 (2 H, d, J=8.7 Hz), 7.70 (1 H, d, J=9.1 Hz), 7.88-7.96 (3 H, m), 8.01 (0.35 x 1 H, d, J=1.9 Hz), 8.04 (0.65 x 1 H, d, J=1.9 Hz), 8.27 (1 H, d, J=8.7 Hz), 8.87-8.91 (1 H, m).
FAB(pos): 492 $[MH]^+$
elemental analysis value ($C_{28}H_{33}N_3O_3S$)
Calculated: C, 68.40; H, 6.77; N, 8.55.
Found: C, 68.13; H, 6.77; N, 8.47.

Example 18

4-(2-cyclopropylethoxy)-N-[8-methyl-3-({[3-(methylsulfonyl)propyl]amino}methyl)quinolin-7-yl]benzamide

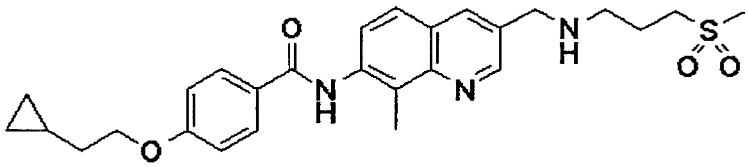

4-(2-Cyclopropylethoxy)-N-[8-methyl-3-({[3-(methylthio)propyl]amino}methyl)quinolin-7-yl]benzamide (170 mg) obtained in Reference Example 32 was dissolved in methanol (6.0 mL), a solution of oxone (452 mg) in water (3.0 mL) was added thereto at 0°C. While allowed to warm to room temperature, the mixture was stirred for 18 hr. The reaction solution was basified with aqueous sodium hydroxide solution, and extracted with ethyl acetate. The organic layer was washed with saturated brine, and purified by silica gel column chromatography [eluent; ethyl acetate→ethyl acetate: methanol=91:9 (volume ratio)], and the obtained solid was recrystallized from methanol-diisopropyl ether to give the title compound (95.6 mg, yield 53%) as a white solid.
$^1$H NMR (300 MHz, $CDCl_3$) δ: 0.11 - 0.19 (2 H, m), 0.47 - 0.57 (2 H, m), 0.80 - 0.95 (1 H, m), 1.73 (2 H, q, J=6.8 Hz), 2.00-2.13 (2 H, m), 2.81 (3 H, s), 2.85 (2 H, t, J=6.6 Hz), 2.91 (3 H, s), 3.12 - 3.23 (2 H, m), 3.99 (2 H, s), 4.13 (2 H, t, J=6.7 Hz), 7.02 (2 H, d, J=8.9 Hz), 7.70 (1 H, d, J=8.9 Hz), 7.87 - 7.96 (3 H, m), 8.02 (1 H, d, J=2.3 Hz), 8.28 (1 H, d, J=9.0 Hz), 8.89 (1 H, d, J=2.1 Hz).
FAB(pos): 496 $[MH]^+$

Example 19

4-(2-cyclopropylethoxy)-N-[8-methyl-3-(1-{[3-(methylsulfonyl)propyl]amino}ethyl)quinolin-7-yl]benzamide

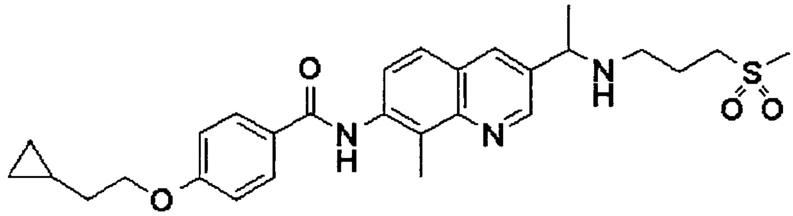

4-(2-Cyclopropylethoxy)-N-[8-methyl-3-(1-{[3-(methylthio)propyl]amino}ethyl)quinolin-7-yl]benzamide (170 mg) obtained in Reference Example 33 was dissolved in methanol (6.0 mL), a solution of oxone (438 mg) in water (3.0 mL) was added thereto at 0°C. While allowed to warm to room temperature, the mixture was stirred for 19 hr. The reaction solution was basified with aqueous sodium hydroxide solution, and extracted with ethyl acetate. The organic layer was washed with water and saturated brine, and purified by silica gel column chromatography, and the obtained solid was recrystallized from ethyl acetate-diisopropyl ether to give the title compound (74.8 mg, yield 41%) as a pale-yellow solid.
$^1$H NMR (300 MHz, $CDCl_3$) δ: 0.11-0.19 (2 H, m), 0.47-0.56 (2 H, m), 0.80-0.94 (1 H, m), 1.47 (3 H, d, J=6.4 Hz), 1.73 (2 H, q, J=6.7 Hz), 1.93-2.03 (2 H, m), 2.51-2.61 (1 H, m), 2.73-2.80 (1 H, m), 2.82 (3 H, s), 2.88 (3 H, s), 2.97-3.09 (1 H, m), 3.13-3.27 (1 H, m), 3.99 (1 H, q, J=6.4 Hz), 4.13 (2 H, t, J=6.7 Hz), 7.03 (2 H, d, J=8.9 Hz), 7.71 (1 H, d, J=9.0 Hz), 7.88-7.95 (3 H, m), 7.99 (1 H, d, J=2.3 Hz), 8.28 (1 H, d, J=9.0 Hz), 8.91 (1 H, d, J=2.3 Hz).
FAB(pos): 510 $[MH]^+$

Example 20

4-(2-cyclopropylethoxy)-N-(3-{[(1,1-dioxidotetrahydro-2H-thiopyran-4-yl)amino]methyl}-8-methylquinolin-7-yl)benzamide

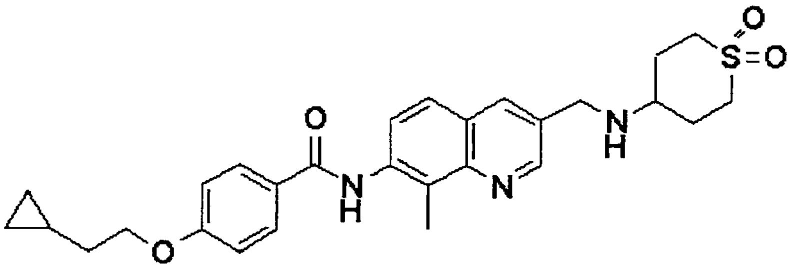

N-[3-(Chloromethyl)-8-methylquinolin-7-yl]-4-(2-cyclopropylethoxy)benzamide hydrochloride (276 mg) obtained in Reference Example 10, tetrahydro-2H-thiopyran-4-amine 1,1-dioxide hydrochloride (238 mg) obtained in Reference Example 38 and ethyldiisopropylamine (0.87 mL) were suspended in NMP (10 mL), and the mixture was stirred at 50°C for 100 hr. The reaction solution was added to a mixture of water and ethyl acetate. The organic layer was washed successively with water (twice) and saturated brine, dried over sodium sulfate, and concentrated under reduced pressure. The residue was purified successively by silica gel column chromatography [eluent; ethyl acetate→methyl acetate:methanol=23:2 (volume ratio)] and NH silica gel column chromatography [eluent; hexane:ethyl acetate=1:1 (volume ratio)→ethyl acetate:methanol=19:1 (volume ratio)], and the obtained solid was recrystallized from diisopropyl ether-ethyl acetate-methanol to give the title compound (116 mg, yield 36%) as pale-yellow crystals.
$^1$H NMR (300 MHz, $CDCl_3$) δ: 0.15 (2 H, q, J=5.0 Hz), 0.47-0.57 (2 H, m), 0.80-0.95 (1 H, m), 1.73 (2 H, q, J=6.6 Hz), 2.06-2.22 (2 H, m), 2.23-2.40 (2 H, m), 2.82 (3 H, s), 2.84-2.95 (2 H, m), 2.96-3.07 (1 H, m), 3.26-3.42 (2 H, m), 3.99 (2 H, s), 4.13 (2 H, t, J=6.6 Hz), 7.03 (2 H, d, J=8.7 Hz), 7.71 (1 H, d, J=8.7 Hz), 7.88-7.96 (3 H, m), 8.03 (1 H, d, J=2.3 Hz), 8.31 (1 H, d, J=9.1 Hz), 8.89 (1 H, d, J=2.3 Hz).

FAB(pos): 508 $[MH]^+$
elemental analysis value ($C_{28}H_{33}N_3O_4S$)
Calculated: C, 66.25; H, 6.55; N, 8.28.
Found: C, 66.20; H, 6.50; N, 8.20.

Example 21

4-(2-cyclopropylethoxy)-N-(8-methyl-3-{[(trans-1-oxidotetrahydro-2H-thiopyran-4-yl)amino]methyl}quinolin-7-yl)benzamide

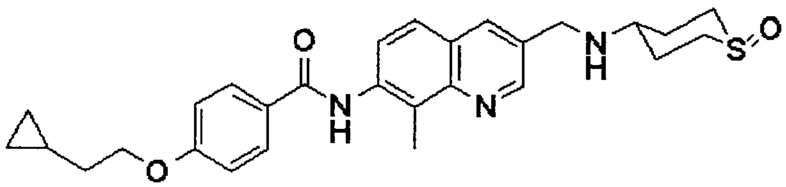

Example 22

4-(2-cyclopropylethoxy)-N-(8-methyl-3-{[(cis-1-oxidotetrahydro-2H-thiopyran-4-yl)amino]methyl}quinolin-7-yl)benzamide

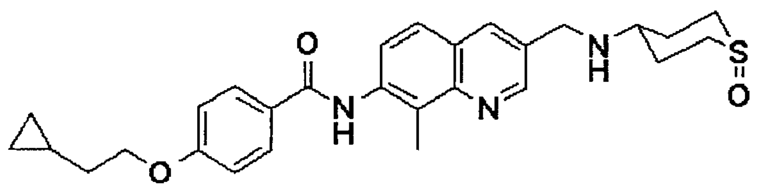

4-(2-Cyclopropylethoxy)-N-{8-methyl-3-[(tetrahydro-2H-thiopyran-4-ylamino)methyl]quinolin-7-yl}benzamide (760 mg) obtained in Reference Example 39 was dissolved in a mixed solvent of methanol (50 mL) and acetone (50 mL), and a solution of sodium periodate (683 mg) in water (20 mL) was added thereto at room temperature. The mixture was stirred at the same temperature for 15 hr, the insoluble material was filtered off, and the filtrate was concentrated under reduced pressure. The residue was dissolved in THF-ethyl acetate, and the solution was basified with 1N aqueous sodium hydroxide solution. The organic layer was washed with water and saturated brine, dried over sodium sulfate, and concentrated under reduced pressure. The residue was recrystallized from diisopropyl ether-ethyl acetate-methanol. The obtained crystals were purified by silica gel column chromatography [eluent; ethyl acetate:methanol=4:1 (volume ratio)→ethyl acetate:methanol=3:7 (volume ratio)] and the obtained solid was recrystallized from diisopropyl ether-ethyl acetate-methanol to give a trans form of the title compound ($R_f$=0.60:NH silica gel, AcOEt/MeOH=10/1) as white crystals (198 mg, yield 25%) and a cis form of the title compound ($R_f$=0.49:NH silica gel, AcOEt/MeOH=10/1) as white crystals (446 mg, yield 57%). trans form :
$^1$H NMR (300 MHz, $CDCl_3$) δ: 0.11-0.19 (2 H, m), 0.47-0.56 (2 H, m), 0.83-0.94 (1 H, m), 1.63-1.77 (4 H, m), 2.45-2.58 (2 H, m), 2.63-2.76 (2 H, m), 2.82 (3 H, s), 2.97-3.07 (1 H, m), 3.14 (2 H, t, J=13.3 Hz), 3.99 (2 H, s), 4.13 (2 H, t, J=6.6 Hz), 7.03 (2 H, d, J=8.9 Hz), 7.71 (1 H, d, J=8.9 Hz), 7.87-7.97 (3 H, m), 8.03 (1 H, d, J=2.1 Hz), 8.30 (1 H, d, J=8.9 Hz), 8.90 (1 H, d, J=2.1 Hz).
FAB(pos): 492 $[MH]^+$
elemental analysis value ($C_{28}H_{33}N_3O_3S\cdot 1.5H_2O$)
Calculated: C, 64.84; H, 7.00; N, 8.10.
Found: C, 64.89; H, 6.89; N, 7.81.
cis form :
$^1$H NMR (300 MHz, $CDCl_3$) δ: 0.11-0.19 (2 H, m), 0.48-0.56 (2 H, m), 0.80-0.95 (1 H, m), 1.73 (2 H, q, J=6.8 Hz), 1.94-2.04

(2 H, m), 2.15-2.30 (2 H, m), 2.48-2.61 (2 H, m), 2.65-2.75 (1 H, m), 2.82 (3 H, s), 3.06-3.18 (2 H, m), 3.49 (1 H, s), 4.06 (2 H, s), 4.13 (2 H, t, J=6.6 Hz), 7.03 (2 H, d, J=8.9 Hz), 7.71 (1 H, d, J=9.0 Hz), 7.87-7.96 (3 H, m), 8.07 (1 H, d, J=2.1 Hz), 8.27 (1 H, d, J=9.0 Hz), 8.90 (1 H, d, J=2.3 Hz).
FAB(pos): 492 $[MH]^+$
elemental analysis value ($C_{28}H_{33}N_3O_3S$)
Calculated: C, 68.40; H, 6.77; N, 8.55.
Found: C, 68.16; H, 6.75; N, 8.52.

Example 23

4-(2-cyclopropylethoxy)-N-(3-{[(1,1-dioxidotetrahydro-2H-thiopyran-4-yl)amino]methyl}-8-methylquinolin-7-yl)-2-fluorobenzamide

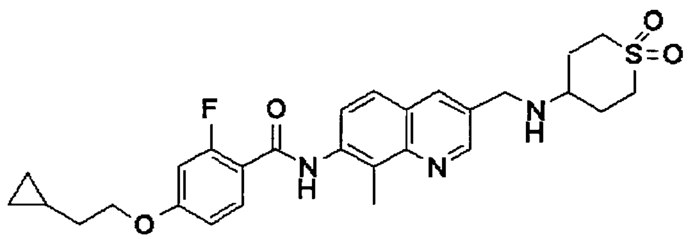

N-[3-(Chloromethyl)-8-methylquinolin-7-yl]-4-(2-cyclopropylethoxy)-2-fluorobenzamide hydrochloride (288 mg) obtained in Reference Example 43, tetrahydro-2H-thiopyran-4-amine 1,1-dioxide hydrochloride (238 mg) obtained in Reference Example 38 and ethyldiisopropylamine (0.87 mL) were suspended in NMP (10 mL), and the mixture was stirred at 50°C for 100 hr. The reaction solution was added to a mixture of ethyl acetate-water, and the organic layer was washed successively with water (twice) and saturated brine, dried over sodium sulfate, and concentrated under reduced pressure. The residue was purified successively by silica gel column chromatography [eluent; ethyl acetate→methyl acetate:methanol=23:2 (volume ratio)] and NH silica gel column chromatography [eluent; hexane:ethyl acetate=1:1 (volume ratio)→ethyl acetate:methanol=19:1 (volume ratio)], and the obtained solid was recrystallized from ethyl acetate-methanol to give the title compound (123 mg, yield 37%) as pale-yellow crystals.
$^1H$ NMR (300 MHz, $CDCl_3$) δ: 0.11-0.19 (2 H, m), 0.48-0.58 (2 H, m), 0.79-0.94 (1 H, m), 1.73 (2 H, q, J=6.7 Hz), 2.06-2.21 (2 H, m), 2.24-2.39 (2 H, m), 2.82 (3 H, s), 2.84-2.96 (2 H, m), 2.96-3.06 (1 H, m), 3.28-3.42 (2 H, m), 3.99 (2 H, s), 4.12 (2 H, t, J=6.7 Hz), 6.74 (1 H, dd, J=14.6, 2.4 Hz), 6.88 (1 H, dd, J=8.9, 2.4 Hz), 7.71 (1 H, d, J=8.9 Hz), 8.03 (1 H, d, J=2.3 Hz), 8.19 (1 H, t, J=9.2 Hz), 8.42 (1 H, d, J=8.9 Hz), 8.67 (1 H, d, J=17.3 Hz), 8.89 (1 H, d, J=2.1 Hz).
FAB(pos): 526 $[MH]^+$
elemental analysis value ($C_{28}H_{32}N_3O_4SF$)
Calculated: C, 63.98; H, 6.14; N, 7.99.
Found: C, 63.90; H, 6.12; N, 7.88.

Example 24

4-(2-cyclopropylethoxy)-2-fluoro-N-(8-methyl-3-{[(trans-1-oxidotetrahydro-2H-thiopyran-4-yl)amino]methyl}quinolin-7-yl)benzamide

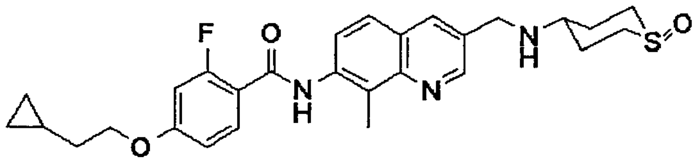

Example 25

4-(2-cyclopropylethoxy)-2-fluoro-N-(8-methyl-3-{[(cis-1-oxidotetrahydro-2H-thiopyran-4-yl)amino]methyl}quinolin-7-yl) benzamide

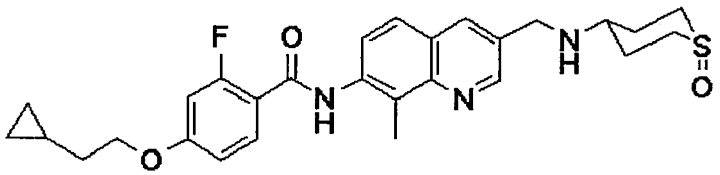

4-(2-Cyclopropylethoxy)-2-fluoro-N-{8-methyl-3-[(tetrahydro-2H-thiopyran-4-ylamino)methyl]quinolin-7-yl} benzamide (867 mg) obtained in Reference Example 44 was dissolved in a mixed solvent of methanol (50 mL) and acetone (50 mL), and a solution of sodium periodate (752 mg) in water (20 mL) was added thereto at room temperature. The mixture was stirred at the same temperature for 14 hr, the insoluble material was filtered off, and the filtrate was concentrated under reduced pressure. The residue was dissolved in THF-ethyl acetate, and the solution was basified with 1N aqueous sodium hydroxide solution. The organic layer was washed successively with water and saturated brine, dried over sodium sulfate, and concentrated under reduced pressure. The residue was purified by silica gel column chromatography [eluent; ethyl acetate:methanol=24:1 (volume ratio)→ethyl acetate:methanol=17:3 (volume ratio)], and the obtained solid was recrystallized from diisopropyl ether-ethyl acetate-methanol. The obtained crystals were purified by silica gel column chromatography [eluent; ethyl acetate:methanol=17:3 (volume ratio)→ethyl acetate:methanol=1:2 (volume ratio)], and the obtained solid was recrystallized from diisopropyl ether-ethyl acetate-methanol to give a trans form of the title compound ($R_f$=0.35:NH silica gel, AcOEt/MeOH=20/1) as white crystals (190 mg, yield 21%) and a cis form of the title compound ($R_f$=0.26:NH silica gel, AcOEt/MeOH=20/1) as white crystals (552 mg, yield 62%).
trans form :
$^1$H NMR (300 MHz, $CDCl_3$) δ: 0.15 (2 H, q, J=5.0 Hz), 0.48-0.57 (2 H, m), 0.79-0.94 (1 H, m), 1.63-1.78 (4 H, m), 2.45-2.58 (2 H, m), 2.63-2.75 (2 H, m), 2.82 (3 H, s), 2.96-3.06 (1 H, m), 3.07-3.20 (2 H, m), 3.98 (2 H, s), 4.12 (2 H, t, J=6.7 Hz), 6.74 (1 H, dd, J=14.6, 2.4 Hz), 6.88 (1 H, dd, J=8.9, 2.4 Hz), 7.70 (1 H, d, J=9.0 Hz), 8.02 (1 H, d, J=2.1 Hz), 8.19 (1 H, t, J=9.2 Hz), 8.41 (1 H, d, J=8.9 Hz), 8.66 (1 H, d, J=17.1 Hz), 8.90 (1 H, d, J=2.3 Hz).
FAB(pos): 510 $[MH]^+$
elemental analysis value ($C_{28}H_{32}N_3O_3SF$)
Calculated: C, 65.99; H, 6.33; N, 8.25.
Found: C, 65.80; H, 6.36; N, 8.18.
cis form :
$^1$H NMR (300 MHz, $CDCl_3$) δ: 0.12-0.19 (2 H, m), 0.48-0.57 (2 H, m), 0.80-0.94 (1 H, m), 1.73 (2 H, q, J=6.7 Hz), 1.94-2.04 (2 H, m), 2.14-2.31 (2 H, m), 2.48-2.61 (2 H, m), 2.64-2.76 (1 H, m), 2.82 (3 H, s), 3.06-3.19 (2 H, m), 3.49 (1 H, s), 4.06 (2 H, s), 4.12 (2 H, t, J=6.6 Hz), 6.74 (1 H, dd, J=14.6, 2.4 Hz), 6.88 (1 H, dd, J=8.9, 2.4 Hz), 7.71 (1 H, d, J=8.9 Hz), 8.07 (1 H, d, J=2.1 Hz), 8.19 (1 H, t, J=9.2 Hz), 8.39 (1 H, d, J=8.9 Hz), 8.66 (1 H, d, J=17.3 Hz), 8.90 (1 H, d, J=2.3 Hz).
FAB(pos): 510 $[MH]^+$
elemental analysis value ($C_{28}H_{32}N_3O_3SF \cdot 0.1H_2O$)
Calculated: C, 65.76; H, 6.35; N, 8.22.
Found: C, 65.56; H, 6.34; N, 8.20.

Example 26

4-(cyclopropylmethoxy)-N-[8-methyl-3-({[4-(methylsulfinyl)butyl]amino}methyl)quinolin-7-yl]benzamide

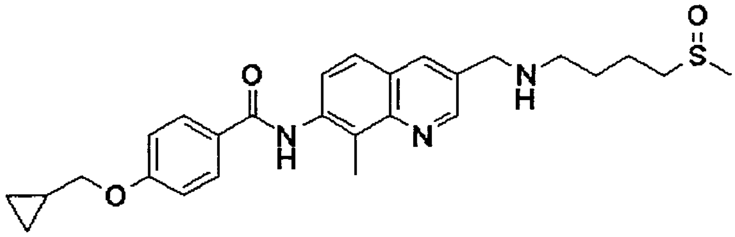

4-(Cyclopropylmethoxy)-N-[8-methyl-3-({[4-(methylthio)butyl]amino}methyl)quinolin-7-yl]benzamide (290 mg) obtained in Reference Example 48 was dissolved in methanol (30 mL), and a solution of sodium periodate (267 mg) in water (10 mL) was added thereto at room temperature. The mixture was stirred at the same temperature for 14 hr, and concentrated under reduced pressure. To the residue was added an aqueous solution of sodium bisulfite (0.5 g), and the mixture was washed with ethyl acetate. The aqueous layer was basified with aqueous sodium hydroxide solution, and extracted with ethyl acetate. The organic layer was washed with water and saturated brine, and purified by silica gel column chromatography [eluent; ethyl acetate:methanol=1:1 (volume ratio)→methanol], and the obtained solid was recrystallized from diisopropyl ether-methanol to give the title compound (144 mg, yield 48%) as a white solid.
$^1$H NMR (300 MHz, $CDCl_3$) δ: 0.36-0.43 (2 H, m), 0.65-0.74 (2 H, m), 1.26-1.37 (1 H, m), 1.64-1.79 (2 H, m), 1.80-1.93 (2 H, m), 2.56 (3 H, s), 2.63-2.80 (4 H, m), 2.81 (3 H, s), 3.90 (2 H, d, J=7.0 Hz), 3.99 (2 H, s), 7.02 (2 H, d, J=8.7 Hz), 7.71 (1 H, d, J=9.0 Hz), 7.87-7.95 (3 H, m), 8.04 (1 H, d, J=2.1 Hz), 8.26 (1 H, d, J=8.9 Hz), 8.89 (1 H, d, J=2.3 Hz).
FAB(pos): 480 $[MH]^+$

Example 27

4-(cyclopropylmethoxy)-N-[8-methyl-3-({[4-(methylsulfonyl)butyl]amino}methyl)quinolin-7-yl]benzamide

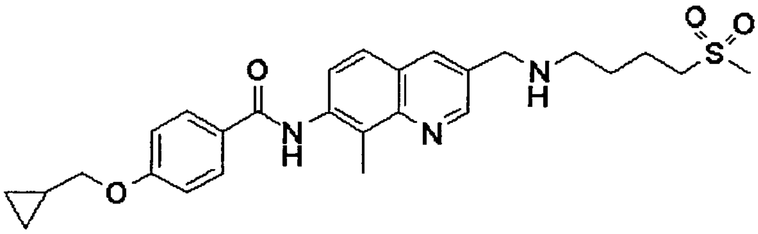

4-(Cyclopropylmethoxy)-N-[8-methyl-3-({[4-(methylsulfonyl)butyl][(2-nitrophenyl)sulfonyl]amino}methyl)quinolin-7-yl]benzamide (403 mg) obtained in Reference Example 49 was dissolved in DMF (4.0 mL), lithium hydroxide monohydrate (248 mg) and mercaptoacetic acid (0.26 mL) were added thereto, and the mixture was stirred at room temperature for 16 hr. To the reaction solution was added water, and the mixture was extracted with THF-ethyl acetate. The organic layer was washed successively with water and saturated brine, dried over sodium sulfate, and concentrated under reduced pressure to give the title compound (169 mg, yield 58%) as a white solid.
$^1$H NMR (300 MHz, $CDCl_3$) δ: 0.35-0.43 (2 H, m), 0.65-0.73 (2 H, m), 1.26-1.38 (1 H, m), 1.63-1.76 (2 H, m), 1.88-2.02 (2 H, m), 2.73 (2 H, t, J=6.9 Hz), 2.81 (3 H, s), 2.90 (3 H, s), 2.98-3.08 (2 H, m), 3.90 (2 H, d, J=7.0 Hz), 3.99 (2 H, s), 7.02 (2 H, d, J=8.9 Hz), 7.71 (1 H, d, J=8.9 Hz), 7.87-7.96 (3 H, m), 8.04 (1 H, d, J=2.1 Hz), 8.26 (1 H, d, J=8.9 Hz), 8.89 (1 H, d, J=2.3 Hz).
FAB(pos): 496 $[MH]^+$

Example 28

4-(cyclopropylmethoxy)-N-(3-{1-[(1,1-dioxidotetrahydro-2H-thiopyran-4-yl)amino]ethyl}-8-methylquinolin-7-yl)benzamide

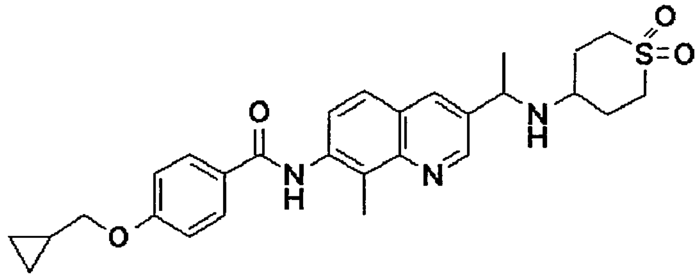

N-[3-(1-Aminoethyl)-8-methylquinolin-7-yl]-4-(cyclopropylmethoxy)benzamide (375 mg) obtained in Reference Example 51 and tetrahydro-4H-thiopyran-4-one 1,1-dioxide (593 mg) obtained in Reference Example 52 were suspended in a mixed solvent of NMP (20 mL) and acetic acid (2.0 mL), and the mixture was stirred at room temperature for 1.75 hr. Sodium triacetoxyborohydride (1.06 g) was added thereto at room temperature, and the mixture was stirred for 90 hr. The reaction was quenched with 1N aqueous sodium hydroxide solution to basify the mixture. The mixture was extracted with ethyl acetate, and the organic layer was washed with water and saturated brine. The organic layer was purified successively by NH silica gel column chromatography [eluent; ethyl acetate→ethyl acetate:methanol=19:1 (volume ratio)] and silica gel column chromatography [eluent; ethyl acetate:methanol=99:1 (volume ratio)→ethyl acetate: methanol=9:1 (volume ratio)], and the obtained solid was recrystallized from ethyl acetate-diisopropyl ether to give the title compound (202 mg, yield 40%) as a white solid.

$^1$H NMR (300 MHz, $CDCl_3$) δ: 0.35-0.43 (2 H, m), 0.65-0.74 (2 H, m), 1.24-1.39 (1 H, m), 1.50 (3 H, d, J=6.6 Hz), 1.85-2.21 (3 H, m), 2.28 (1 H, dd, J=15.3, 8.9 Hz), 2.70-2.91 (6 H, m), 3.12-3.32 (2 H, m), 3.90 (2 H, d, J=7.0 Hz), 4.07 (1 H, q, J=6.3 Hz), 7.02 (2 H, d, J=8.9 Hz), 7.71 (1 H, d, J=9.0 Hz), 7.88-7.96 (3 H, m), 7.98 (1 H, s), 8.31 (1 H, d, J=9.0 Hz), 8.90 (1 H, d, J=2.3 Hz).

FAB(pos): 508 $[MH]^+$

elemental analysis value ($C_{28}H_{33}N_3O_4S{\cdot}0.25H_2O$)

Calculated: C, 65.66; H, 6.59; N, 8.20.

Found: C, 65.70; H, 6.78; N, 7.99.

Example 29

4-(cyclopropylmethoxy)-N-(8-methyl-3-{(1R)-1-[trans-(1-oxidotetrahydro-2H-thiopyran-4-yl)amino]ethyl}quinolin-7-yl) benzamide

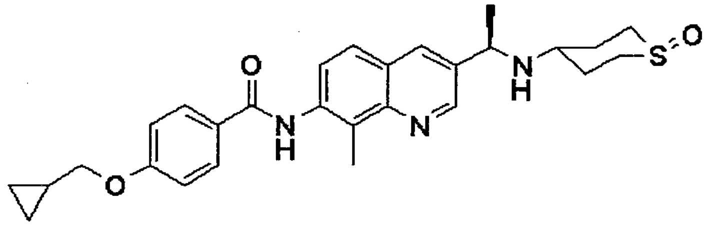

Example 30

4-(cyclopropylmethoxy)-N-(8-methyl-3-{(1R)-1-[cis-(1-oxidotetrahydro-2H-thiopyran-4-yl)amino]ethyl}quinolin-7-yl) benzamide

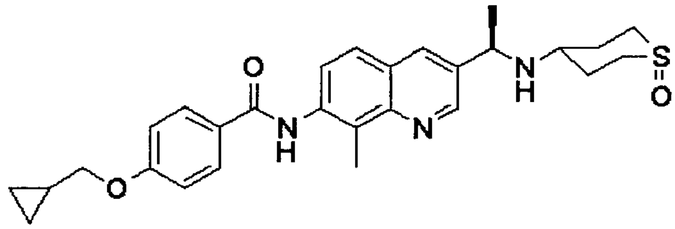

4-(Cyclopropylmethoxy)-N-{8-methyl-3-[(1R)-1-(tetrahydro-2H-thiopyran-4-ylamino)ethyl]quinolin-7-yl}benzamide (600 mg) obtained in Reference Example 53 was dissolved in methanol (70 mL), and a solution of sodium periodate (810 mg) in water (12 mL) was added thereto at room temperature. The mixture was stirred at the same temperature for 17 hr, and concentrated under reduced pressure, and to the residue was added sodium bisulfite aqueous solution. The mixture was washed with ethyl acetate, and the aqueous layer was basified with 8N aqueous sodium hydroxide solution. The mixture was extracted with ethyl acetate, and the organic layer was washed with saturated brine. The organic layer was purified successively by NH silica gel column chromatography [eluent; ethyl acetate→ethyl acetate: methanol=9:1 (volume ratio)] and silica gel column chromatography [eluent; ethyl acetate:methanol=19:1 (volume ratio) →ethyl acetate:methanol=3:2 (volume ratio)], and the obtained solid was recrystallized from methanol-ethyl acetate-diisopropyl ether to give a white solid (420 mg). The solid (248 mg) was purified by HPLC (column; CHIRALPAK AD 50 mmID×500 mmL, mobile phase; hexane:ethanol=1:1), and the obtained solid was recrystallized from ethyl acetate-diisopropyl ether to give a trans form of the title compound (retention time: shorter) as a white solid (38.8 mg, yield 11%) and a cis form of the title compound (186.8 mg, yield 51%) as a white solid.
trans form
$^1$H NMR (300 MHz, $CDCl_3$) δ: 0.40 (2 H, q, J=4.9 Hz), 0.65-0.73 (2 H, m), 1.26-1.37 (1 H, m), 1.48 (3 H, d, J=6.4 Hz), 1.52-1.69 (2 H, m), 2.21-2.35 (1 H, m), 2.38-2.69 (3 H, m), 2.73-2.81 (1 H, m), 2.82 (3 H, s), 2.98-3.20 (2 H, m), 3.90 (2 H, d, J=6.8 Hz), 4.05 (1 H, q, J=6.9 Hz), 7.02 (2 H, d, J=8.7 Hz), 7.70 (1 H, d, J=9.1 Hz), 7.88-7.95 (3 H, m), 7.97 (1 H, d, J=2.3 Hz), 8.27 (1 H, d, J=8.7 Hz), 8.90 (1 H, d, J=2.3 Hz).
FAB(pos): 492 $[MH]^+$
cis form
$^1$H NMR (300 MHz, $CDCl_3$) δ: 0.35-0.43 (2 H, m), 0.65-0.73 (2 H, m), 1.26-1.37 (1 H, m), 1.46 (3 H, d, J=6.4 Hz), 1.79 (1 H, dd, J=15.0, 4.0 Hz), 2.02-2.21 (3 H, m), 2.31-2.50 (3 H, m), 2.82 (3 H, s), 2.95-3.11 (2 H, m), 3.90 (2 H, d, J=7.2 Hz), 4.24 (1 H, q, J=6.6 Hz), 7.02 (2 H, d, J=9.1 Hz), 7.71 (1 H, d, J=8.7 Hz), 7.87-7.96 (3 H, m), 8.03 (1 H, d, J=1.9 Hz), 8.26 (1 H, d, J=9.1 Hz), 8.93 (1 H, d, J=2.3 Hz).
FAB(pos): 492 $[MH]^+$
elemental analysis value ($C_{28}H_{33}N_3O_3S \cdot 0.1H_2O$)
Calculated: C, 68.15; H, 6.78; N, 8.52.
Found: C, 67.95; H, 6.77; N, 8.52.

Example 31

4-(cyclopropylmethoxy)-N-(3-{[(1,1-dioxidotetrahydro-2H-thiopyran-4-yl)amino]methyl}-8-methylquinolin-7-yl)benzamide

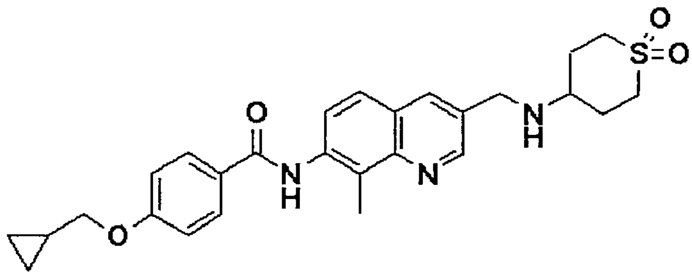

N-[3-(Aminomethyl)-8-methylquinolin-7-yl]-4-(cyclopropylmethoxy)benzamide (361 mg) obtained in Reference Example 55 and tetrahydro-4H-thiopyran-4-one 1,1-dioxide (193 mg) obtained in Reference Example 52 were added

to a mixed solvent of acetic acid (2.0 mL) and NMP (10 mL), and the mixture was stirred at room temperature for 7 hr. Sodium triacetoxyborohydride (530 mg) was added thereto at the same temperature, and the mixture was stirred for 13 hr, and basified with 1N aqueous sodium hydroxide solution. The mixture was extracted with ethyl acetate, and the extract was washed with water (three times) and saturated brine, dried over sodium sulfate, and concentrated under reduced pressure. The residue was purified by silica gel column chromatography [eluent; ethyl acetate→ethyl acetate: methanol=17:3 (volume ratio)] to give the title compound (124 mg, yield 25%) as pale-yellow crystals.
$^1$H NMR (300 MHz, $CDCl_3$) δ: 0.40 (2 H, q, J=4.9 Hz), 0.64-0.73 (2 H, m), 1.27-1.36 (1 H, m), 2.06-2.20 (2 H, m), 2.25-2.38 (2 H, m), 2.81 (3 H, s), 2.83-2.95 (2 H, m), 2.97-3.05 (1 H, m), 3.27-3.41 (2 H, m), 3.90 (2 H, d, J=6.8 Hz), 3.99 (2 H, s), 7.02 (2 H, d, J=8.7 Hz), 7.71 (1 H, d, J=9.1 Hz), 7.87-7.96 (3 H, m), 8.03 (1 H, d, J=1.9 Hz), 8.30 (1 H, d, J=9.1 Hz), 8.88 (1 H, d, J=2.3 Hz).
FAB(pos): 494 $[MH]^+$
elemental analysis value ($C_{27}H_{31}N_3O_4S$)
Calculated: C, 65.70; H, 6.33; N, 8.51.
Found: C, 65.45; H, 6.32; N, 8.37.

Example 32

4-(cyclopropylmethoxy)-N-(8-methyl-3-{[(trans-1-oxidotetrahydro-2H-thiopyran-4-yl)amino]methyl}quinolin-7-yl)benzamide

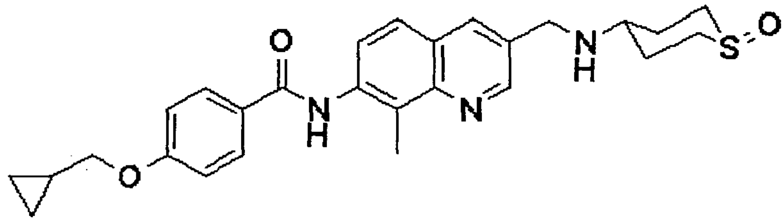

Example 33

4-(cyclopropylmethoxy)-N-(8-methyl-3-{[(cis-1-oxidotetrahydro-2H-thiopyran-4-yl)amino]methyl}quinolin-7-yl)benzamide

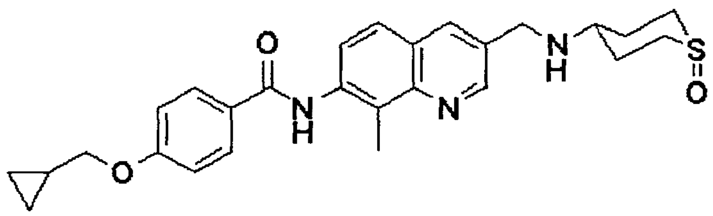

4-(Cyclopropylmethoxy)-N-{8-methyl-3-[(tetrahydro-2H-thiopyran-4-ylamino)methyl]quinolin-7-yl}benzamide (900 mg) obtained in Reference Example 56 was dissolved in methanol (150 mL), and a solution of sodium periodate (834 mg) in water (30 mL) was added thereto at room temperature. The mixture was stirred at the same temperature for 13 hr, and concentrated under reduced pressure. To the residue was added an aqueous solution of sodium bisulfite (1.6 g), and the mixture was stirred, and basified with 1N aqueous sodium hydroxide solution. The mixture was extracted with ethyl acetate-methanol, and the organic layer was washed with saturated brine, dried over sodium sulfate, and concentrated under reduced pressure. The residue was purified by silica gel column chromatography [eluent; ethyl acetate:methanol=9:1 (volume ratio)→ethyl acetate:methanol=3:7 (volume ratio)], and the obtained solid was recrystallized from diisopropyl ether-methanol to give a trans form of the title compound ($R_f$=0.42:NH silica gel, AcOEt/MeOH=10/1) as white crystals (295 mg, yield32%) and a cis form of the title compound ($R_f$=0.34:NH silica gel, AcOEt/MeOH=10/1) as white crystals (449 mg, yield48%).

trans form:
$^1$H NMR (300 MHz, $CDCl_3$) δ: 0.36-0.44 (2 H, m), 0.64-0.73 (2 H, m), 1.25-1.37 (1 H, m), 1.61-1.76 (2 H, m), 2.44-2.59 (2 H, m), 2.63-2.74 (2 H, m), 2.81 (3 H, s), 2.97-3.05 (1 H, m), 3.07-3.20 (2 H, m), 3.90 (2 H, d, J=6.8 Hz), 3.98 (2 H, s), 7.02 (2 H, d, J=8.7 Hz), 7.70 (1 H, d, J=8.7 Hz), 7.87-7.95 (3 H, m), 8.02 (1 H, d, J=2.3 Hz), 8.29 (1 H, d, J=9.1 Hz), 8.90 (1 H, d, J=2.3 Hz).
FAB(pos): 478 $[MH]^+$
elemental analysis value ($C_{27}H_{31}N_3O_3S \cdot 0.35H_2O$)
Calculated: C, 67.01; H, 6.60; N, 8.68.
Found: C, 66.99; H, 6.65; N, 8.52.
cis form:
$^1$H NMR (300 MHz, $CDCl_3$) δ: 0.36-0.43 (2 H, m), 0.65-0.73 (2 H, m), 1.25-1.37 (1 H, m), 1.92-2.05 (2 H, m), 2.14-2.31 (2 H, m), 2.47-2.61 (2 H, m), 2.64-2.76 (1 H, m), 2.81 (3 H, s), 3.06-3.18 (2 H, m), 3.90 (2 H, d, J=6.8 Hz), 4.06 (2 H, s), 7.02 (2 H, d, J=8.7 Hz), 7.71 (1 H, d, J=8.3 Hz), 7.86-7.95 (3 H, m), 8.07 (1 H, d, J=1.9 Hz), 8.28 (1 H, d, J=9.1 Hz), 8.90 (1 H, d, J=2.3 Hz).
FAB(pos): 478 $[MH]^+$
elemental analysis value ($C_{27}H_{31}N_3O_3S \cdot 0.9MeOH$)
Calculated: C, 66.17; H, 6.89; N, 8.30.
Found: C, 66.19; H, 6.99; N, 8.56.

Example 34

4-(cyclopropylmethoxy)-N-(3-{[(1,1-dioxidotetrahydro-2H-thiopyran-4-yl)amino]methyl}-8-methylquinolin-7-yl)-2-fluorobenzamide

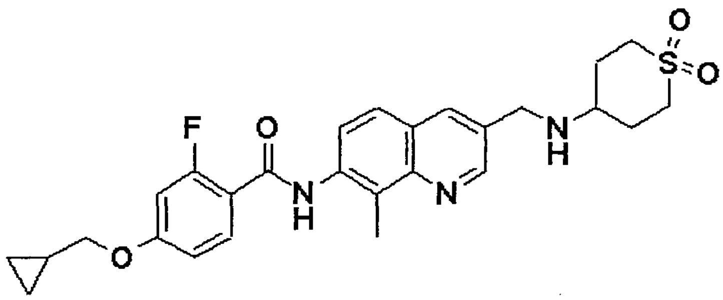

4-(Cyclopropylmethoxy)-2-fluoro-N-{8-methyl-3-[(tetrahydro-2H-thiopyran-4-ylamino)methyl]quinolin-7-yl}benzamide (6.59 g) obtained in Reference Example 67 was suspended in methanol (1000 mL), and the suspension was cooled to 0°C. A solution of oxone (8.87 g) in water (100 mL) at the same temperature was added dropwise thereto, and the mixture was stirred at room temperature for 18 hr. Methanol (500 mL) was added thereto, and the mixture was heated to 50°C. A solution of oxone (4.22 g) in water (50 mL) was added dropwise thereto, and the mixture was stirred at room temperature for 1 hr, and concentrated under reduced pressure. The residue was suspended in ethyl acetate, and the suspension was washed with water, saturated brine and saturated aqueous sodium hydrogen carbonate solution, dried over sodium sulfate, and concentrated under reduced pressure. The residue was purified by silica gel column chromatography [eluent; ethyl acetate→ethyl acetate:methanol=1:1 (volume ratio)], and the obtained solid was recrystallized from diisopropyl ether-ethyl acetate to give the title compound (4.07 g, yield 58%) as a pale-yellow solid.
$^1$H NMR (300 MHz, $CDCl_3$) δ: 0.36-0.44 (2 H, m), 0.66-0.75 (2 H, m), 1.22-1.39 (1 H, m), 2.06-2.21 (2 H, m), 2.24-2.39 (2 H, m), 2.82 (3 H, s), 2.84-2.95 (2 H, m), 2.96-3.05 (1 H, m), 3.28-3.41 (2 H, m), 3.89 (2 H, d, J=6.8 Hz), 3.98 (2 H, s), 6.73 (1 H, dd, J=14.8, 2.3 Hz), 6.87 (1 H, dd, J=9.1, 2.3 Hz), 7.70 (1 H, d, J=9.1 Hz), 8.03 (1 H, d, J=1.9 Hz), 8.19 (1 H, t, J=9.3 Hz), 8.41 (1 H, d, J=9.1 Hz), 8.66 (1 H, d, J=17.0 Hz), 8.88 (1 H, d, J=2.3 Hz).
FAB(pos): 512 $[MH]^+$
elemental analysis value ($C_{27}H_{30}N_3O_4SF$)
Calculated: C, 63.39; H, 5.91; N, 8.21.
Found: C, 63.12; H, 5.81; N, 8.04.

Example 35

4-(cyclopropylmethoxy)-2-fluoro-N-(8-methyl-3-{[(trans-1-oxidotetrahydro-2H-thiopyran-4-yl)amino]methyl}quinolin-7-yl)benzamide

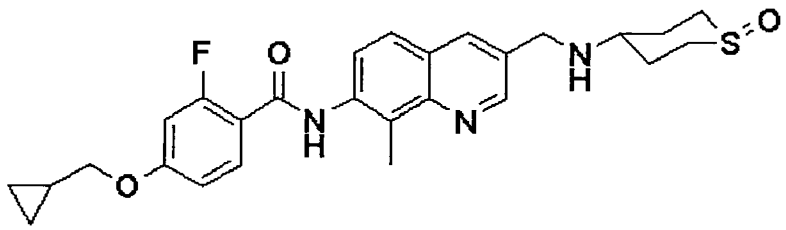

Example 36

4-(cyclopropylmethoxy)-2-fluoro-N-(8-methyl-3-{[(cis-1-oxidotetrahydro-2H-thiopyran-4-yl)amino]methyl}quinolin-7-yl) benzamide

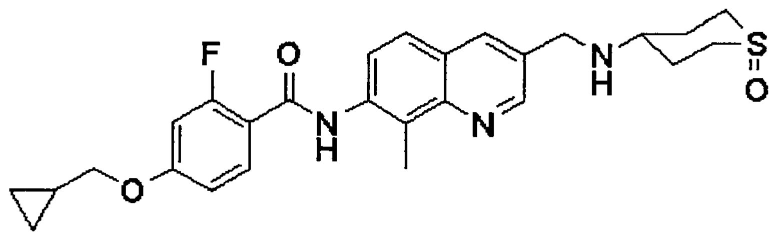

4-(Cyclopropylmethoxy)-2-fluoro-N-{8-methyl-3-[(tetrahydro-2H-thiopyran-4-ylamino)methyl]quinolin-7-yl} benzamide (790 mg) obtained in Reference Example 67 and sodium periodate (705 mg) were suspended in methanol (273 mL), and water (81 mL) was added thereto at room temperature. The mixture was stirred at the same temperature for 15 hr, and concentrated under reduced pressure. The residue was suspended in ethyl acetate, and the suspension was washed with saturated aqueous sodium hydrogen carbonate solution, saturated brine and water, dried over sodium sulfate, and concentrated under reduced pressure. The residue was purified by silica gel column chromatography [eluent; ethyl acetate:methanol=4:1 (volume ratio)→ethyl acetate:methanol=3:7 (volume ratio)], and the obtained solid was recrystallized from diisopropyl ether-ethyl acetate to give a trans form of the title compound (larger $R_f$) as white crystals (331 mg, yield 41%) and a cis form of the title compound (smaller $R_f$) as white crystals (450 mg, yield 55%).
trans form :
$^1$H NMR (300 MHz, $CDCl_3$) δ: 0.36-0.43 (2 H, m), 0.66-0.74 (2 H, m), 1.23-1.38 (1 H, m), 1.63-1.76 (2 H, m), 2.45-2.57 (2 H, m), 2.64-2.74 (2 H, m), 2.82 (3 H, s), 2.97-3.05 (1 H, m), 3.07-3.19 (2 H, m), 3.89 (2 H, d, J=7.0 Hz), 3.98 (2 H, s), 6.73 (1 H, dd, J=14.6, 2.4 Hz), 6.87 (1 H, dd, J=8.9, 2.4 Hz), 7.70 (1 H, d, J=8.9 Hz), 8.02 (1 H, d, J=2.1 Hz), 8.15-8.22 (1 H, m), 8.41 (1 H, d, J=8.9 Hz), 8.66 (1 H, d, J=17.3 Hz), 8.90 (1 H, d, J=2.3 Hz).
FAB(pos): 496 $[MH]^+$
elemental analysis value ($C_{27}H_{30}N_3O_3SF \cdot H_2O$)
Calculated: C, 63.14; H, 6.28; N, 8.18.
Found: C, 63.04; H, 5.91; N, 8.22.
cis form :
$^1$H NMR (300 MHz, $CDCl_3$) δ: 0.36-0.43 (2 H, m, J=4.9, 4.9, 4.9 Hz), 0.66-0.75 (2 H, m), 1.23-1.38 (1 H, m), 1.93-2.05 (2 H, m), 2.14-2.30 (2 H, m), 2.48-2.61 (2 H, m), 2.65-2.75 (1 H, m), 2.82 (3 H, s), 3.06-3.16 (2 H, m), 3.89 (2 H, d, J=7.2 Hz), 4.06 (2 H, s), 6.73 (1 H, dd, J=14.7, 2.3 Hz), 6.86 (1 H, dd, J=8.9, 2.4 Hz), 7.70 (1 H, d, J=9.0 Hz), 8.06 (1 H, d, J=2.1 Hz), 8.18 (1 H, t, J=9.2 Hz), 8.39 (1 H, d, J=8.9 Hz), 8.65 (1 H, d, J=17.3 Hz), 8.90 (1 H, d, J=2.1 Hz).
FAB(pos): 496 $[MH]^+$

elemental analysis value ($C_{27}H_{30}N_3O_3SF \cdot 0.4H_2O$)
Calculated: C, 64.49; H, 6.17; N, 8.36.
Found: C, 64.49; H, 5.97; N, 8.14.

Example 37

4-(cyclopropylmethoxy)-N-(8-fluoro-3-{[(trans-1-oxidotetrahydro-2H-thiopyran-4-yl)amino]methyl}quinolin-7-yl)benzamide

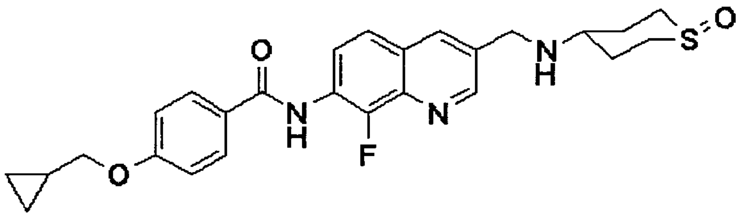

Example 38

4-(cyclopropylmethoxy)-N-(8-fluoro-3-{[(cis-1-oxidotetrahydro-2H-thiopyran-4-yl)amino]methyl}quinolin-7-yl)benzamide

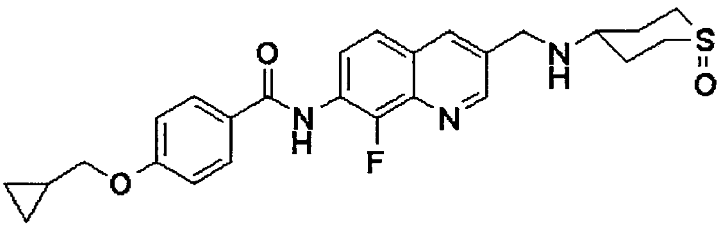

An aqueous solution (10 mL) of sodium periodate (276 mg) was added dropwise to a suspension of 4-(cyclopropylmethoxy)-N-{8-fluoro-3-[(tetrahydro-2H-thiopyran-4-ylamino)methyl]quinolin-7-yl}benzamide (300 mg) obtained in Reference Example 73 in methanol (30 mL) at room temperature, and the mixture was stirred at room temperature for 5 hr. The reaction solution was concentrated under reduced pressure, and the residue was dissolved in ethyl acetate, and the solution was washed with aqueous sodium hydrogen carbonate solution and saturated brine, and concentrated under reduced pressure. The residue was purified successively by NH-silica gel column chromatography [eluent; ethyl acetate→ethyl acetate:methanol=93:7 (volume ratio)] and silica gel column chromatography [eluent; ethyl acetate:methanol=70:30→ethyl acetate:methanol=30:70 (volume ratio)], and the obtained solid was recrystallized from diisopropyl ether-ethyl acetate to give a trans form of the title compound (larger $R_f$) as white crystals (67.7 mg, yield 22%) and a cis form of the title compound (smaller $R_f$) as white crystals (109 mg, yield 35%). trans form :
$^1H$ NMR (300 MHz, $CDCl_3$) δ: 0.36 - 0.43 (2 H, m), 0.66 - 0.73 (2 H, m), 1.20 - 1.39 (1 H, m), 1.62 - 1.75 (2 H, m), 2.45 - 2.59 (2 H, m), 2.64 - 2.75 (2 H, m), 2.97 - 3.06 (1 H, m), 3.07 - 3.18 (2 H, m), 3.90 (2 H, d, J=7.2 Hz), 4.00 (2 H, s), 6.99 - 7.06 (2 H, m), 7.64 (1 H, dd, J=9.1, 1.5 Hz), 7.89 - 7.95 (2 H, m), 8.07 (1 H, s), 8.27 (1 H, d, J=3.4 Hz), 8.75 (1 H, dd, J=9.1, 7.2 Hz), 8.92 (1 H, d, J=1.9 Hz).
FAB(pos): 482 $[MH]^+$
cis form :
$^1H$ NMR (300 MHz, $CDCl_3$) δ: 0.36 - 0.43 (2 H, m), 0.65 - 0.73 (2 H, m), 1.23 - 1.39 (1 H, m), 1.95 - 2.06 (2 H, m), 2.16 - 2.31 (2 H, m), 2.49 - 2.61 (2 H, m), 2.66 - 2.77 (1 H, m), 3.07-3.18 (2 H, m), 3.90 (2 H, d, J=6.8 Hz), 4.07 (2 H, s), 6.99-7.05 (2 H, m), 7.65 (1 H, dd, J=9.1, 1.5 Hz), 7. 90 - 7.95 (2 H, m), 8.12 (1 H, s), 8.27 (1 H, d, J=3.4 Hz), 8.73 (1 H, dd, J=8.9, 7.0 Hz), 8.92 (1 H, d, J=1.9 Hz).
FAB(pos): 482 $[MH]^+$

Example 39

N-(3-{[(1,1-dioxidotetrahydro-2H-thiopyran-4-yl)amino]methyl}-8-methylquinolin-7-yl)-4-(3,3,3-trifluoropropoxy)benzamide

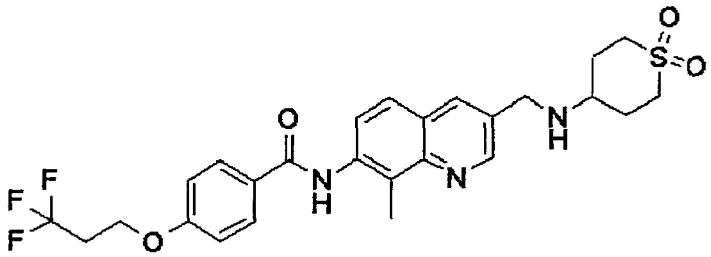

N-{8-Methyl-3-[(tetrahydro-2H-thiopyran-4-ylamino)methyl]quinolin-7-yl}-4-(3,3,3-trifluoropropoxy)benzamide (530 mg) obtained in Reference Example 79 was dissolved in methanol (50 mL) with heating, and the solution was allowed to cool to room temperature. A solution of oxone (1.21 g) in water (10 mL) was added dropwise thereto, and the mixture was stirred overnight. The reaction mixture was diluted with water, and basified with 1N aqueous sodium hydroxide solution. The precipitated solid was collected by filtration, washed with water, and dried under reduced pressure. The obtained solid was purified by silica gel column chromatography [eluent; ethyl acetate→ethyl acetate:methanol=9:1 (volume ratio)], and the obtained solid was washed with methanol, and dried under reduced pressure to give the title compound (130 mg, yield 23%) as a white solid.
$^1$H NMR (300 MHz, DMSO-$d_6$) δ: 1.84 - 2.02 (2 H, m) 2.02 - 2.21 (2 H, m) 2.64 (3 H, s) 2.72 - 2.95 (3 H, m) 2.95 - 3.09 (2 H, m) 3.09 - 3.27 (2 H, m) 3.92 (2 H, s) 4.32 (2 H, t, J=5.87 Hz) 7.12 (2 H, d, J=8.71 Hz) 7.58 (1 H, d, J=8.71 Hz) 7.79 (1 H, d, J=8.71 Hz) 8.04 (2 H, d, J=8.33 Hz) 8.24 (1 H, s) 8.92 (1 H, s) 10.08 (1 H, s).
FAB(pos): 536 $[MH]^+$

Example 40

4-[(cyclopropyloxy)methyl]-N-(3-{[(1,1-dioxidotetrahydro-2H-thiopyran-4-yl)amino]methyl}-8-methylquinolin-7-yl)benzamide

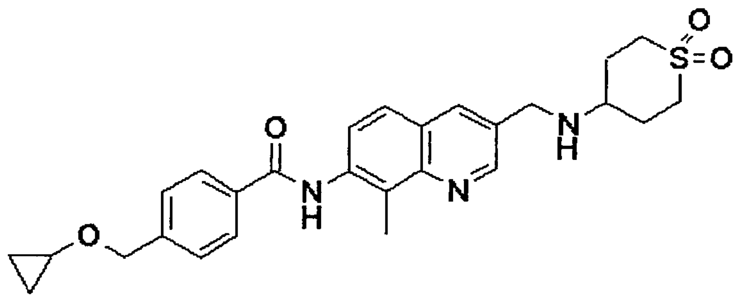

4-[(Cyclopropyloxy)methyl]-N-{8-methyl-3-[(tetrahydro-2H-thiopyran-4-ylamino)methyl]quinolin-7-yl}benzamide (200 mg) obtained in Reference Example 86 was dissolved in acetone (6.0 mL)-methanol (6.0 mL)-water (1.0 mL), oxone (400 mg) was added thereto, and the mixture was stirred at room temperature for 64 hr. The reaction solution was concentrated under reduced pressure, the residue was dissolved in ethyl acetate-water, and the solution was basified with 1N aqueous sodium hydroxide solution. The organic layer was washed twice with saturated brine, dried over sodium sulfate, and concentrated under reduced pressure. The residue was purified by silica gel column chromatography [eluent; ethyl acetate:methanol=19:1 (volume ratio)→ethyl acetate:methanol=3:1 (volume ratio)], and the obtained solid was recrystallized from ethyl acetate to give the title compound (135 mg, yield 63%) as a white solid.
$^1$H NMR (300 MHz, $CDCl_3$) δ: 0.48 - 0.57 (2 H, m), 0.65 - 0.72 (2 H, m), 2.06 - 2.21 (2 H, m), 2.25 - 2.39 (2 H, m), 2.82 (3 H, s), 2.84 - 2.96 (2 H, m), 2.97 - 3.06 (1 H, m), 3.28 - 3.45 (3 H, m), 3.99 (2 H, s), 4.65 (2 H, s), 7.51 (2 H, d, J=8.5 Hz), 7.72 (1 H, d, J=8.9 Hz), 7.91 - 7.99 (3 H, m), 8.04 (1 H, d, J=2.3 Hz), 8.30 (1 H, d, J=8.9 Hz), 8.89 (1 H, d, J=2.3 Hz).
FAB(pos): 494 $[MH]^+$

Example 41

4-(cyclopropylethynyl)-N-(3-{[(1,1-dioxidotetrahydro-2H-thiopyran-4-yl)amino]methyl}-8-methylquinolin-7-yl)benzamide

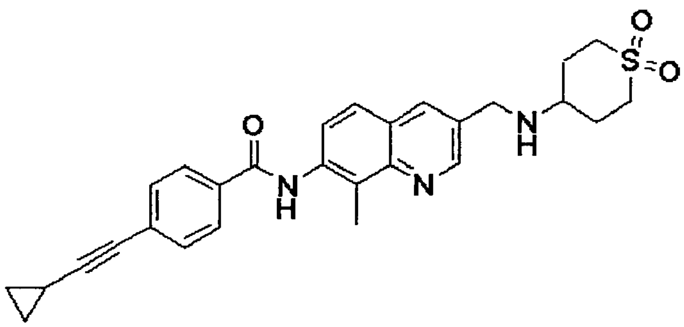

A mixture of sodium hydrogen carbonate (0.5 g), water (1.0 mL) and acetone (1.0 mL) was cooled to 5°C, oxone (1.0 g) was added thereto, and the mixture was stirred at the same temperature for 5 min, and then at room temperature for 10 min. Acetone (5 mL) was added thereto to prepare a solution of dimethyldioxirane in acetone. The solution was added to a suspension of 4-(cyclopropylethynyl)-N-{8-methyl-3-[(tetrahydro-2H-thiopyran-4-ylamino)methyl]quinolin-7-yl}benzamide (313 mg) obtained in Reference Example 92 in acetone (15 mL), and the mixture was stirred overnight at room temperature. The solvent was evaporated under reduced pressure, and the residue was dissolved in NMP (10 mL). 10% Aqueous citric acid solution (100 mL) was added thereto, and the mixture was washed twice with ethyl acetate. The aqueous layer was filtered to remove the insoluble material, and the filtrate was neutralized with 4N aqueous sodium hydroxide solution. The obtained suspension was filtered, and the solid was collected by filtration was washed with water, dried under reduced pressure, and suspended in boiled methanol. The solution was allowed to cool to room temperature, and filtered, and the obtained solid was dried under reduced pressure to give the title compound (157 mg, yield 47%) as a white solid.
$^1$H NMR (300 MHz, DMSO-$d_6$) δ: 0.73 - 0.85 (2 H, m), 0.89 - 1.01 (2 H, m), 1.51 - 1.70 (1 H, m), 1.88 - 2.09 (2 H, m), 2.10 - 2.29 (2 H, m), 2.51 - 2.69 (1 H, m), 2.64 (3 H, s), 2.86 - 3.23 (5 H, m), 4.04 (2 H, br. s.), 7.53 (2 H, d, J=8.5 Hz), 7.61 (1 H, d, J=8.7 Hz), 7.81 (1 H, d, J=8.7 Hz), 8.00 (2 H, d, J=8.5 Hz), 8.29 (1 H, d, J=2.1 Hz), 8.95 (1 H, d, J=2.1 Hz), 10.25 (1 H, s).
FAB(pos): 488 $[MH]^+$

Example 42

4-(cyclopropylethynyl)-N-[8-methyl-3-({[1-(methylsulfonyl)piperidin-4-yl]amino}methyl)quinolin-7-yl]benzamide

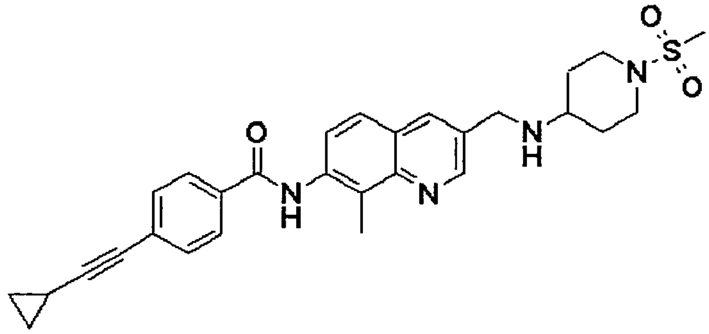

4-(Cyclopropylethynyl)-N-(3-formyl-8-methylquinolin-7-yl)benzamide (254 mg) obtained in Reference Example 91 and 1-(methylsulfonyl)piperidin-4-amine (460 mg) obtained in Reference Example 18 were dissolved in a mixed solvent of DMA (10 mL) and acetic acid (2.0 mL), and the mixture was stirred at room temperature for 10 min. Sodium triacetoxyborohydride (210 mg) was added thereto, and the mixture was stirred overnight. The reaction mixture was cooled to 5°C, basified with 1N aqueous sodium hydroxide solution, and extracted with ethyl acetate. The insoluble material was filtered off, and the organic layer was separated, washed with water and saturated brine, dried over sodium sulfate, and concentrated under reduced pressure. The residue was purified by silica gel column chromatography [eluent; ethyl acetate→ethyl acetate:methanol=4:1 (volume ratio)], and the obtained solid was washed with methanol, and dried under reduced pressure to give the title compound (137 mg, yield 37%) as a white solid.
$^1$H NMR (300 MHz, DMSO-$d_6$) δ: 0.74 - 0.83 (2 H, m) 0.89 - 0.98 (2 H, m) 1.31 - 1.49 (2 H, m) 1.54 - 1.66 (1 H, m) 1.87 - 2.02 (2 H, m) 2.39 - 2.60 (2 H, m) 2.64 (3 H, s) 2.71 - 2.82 (2 H, m) 2.82 (3 H, s) 3.40 - 3.53 (2 H, m) 3.95 (2 H, s) 7.53 (2 H, d, J=8.33 Hz) 7.58 (1 H, d, J=9.09 Hz) 7.79 (1 H, d, J=8.71 Hz) 8.00 (2 H, d, J=8.33 Hz) 8.23 (1 H, d, J=1.89 Hz) 8.93 (1 H, d, J=1.89 Hz) 10.24 (1 H, s).
FAB(pos): 517 $[MH]^+$
elemental analysis value ($C_{29}H_{32}N_4O_3S$)
Calculated: C, 67.42; H, 6.24; N, 10.84.
Found: C, 67.38; H, 6.34; N, 10.93.

Example 43

4-(cyclopropylethynyl)-N-[3-({[(4-hydroxy-1,1-dioxidotetrahydro-2H-thiopyran-4-yl)methyl]amino}methyl)-8-methylquinolin-7-yl]benzamide

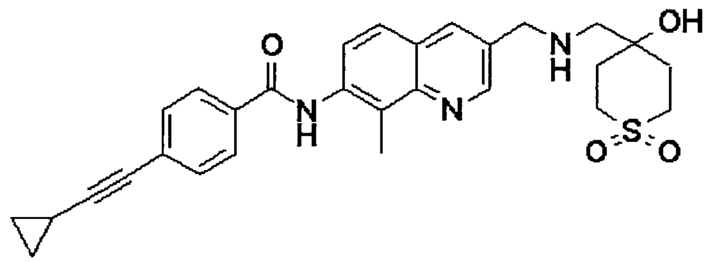

4-(Cyclopropylethynyl)-N-(3-formyl-8-methylquinolin-7-yl)benzamide (224 mg) obtained in Reference Example 91 and 4-(aminomethyl)tetrahydro-2H-thiopyran-4-ol 1,1-dioxide hydrochloride (406 mg) obtained in Reference Example 28 were dissolved in a mixed solvent of DMA (15 mL) and acetic acid (3.0 mL), and the mixture was stirred at room temperature for 10 min. Sodium triacetoxyborohydride (200 mg) was added thereto, and the mixture was stirred overnight. The reaction mixture was cooled to 5°C, basified with 1N aqueous sodium hydroxide solution, and extracted with ethyl acetate. The insoluble material was filtered off, and the organic layer was separated, washed with water and saturated brine, dried over sodium sulfate, and concentrated under reduced pressure. The residue was purified by silica gel column chromatography [eluent; ethyl acetate→ethyl acetate:methanol=4:1 (volume ratio)], and the obtained solid was washed with ethyl acetate, and dried under reduced pressure to give the title compound (213 mg, yield 65%) as a white solid.
$^1$H NMR (300 MHz, DMSO-$d_6$) δ: 0.73 - 0.83 (2 H, m) 0.89 - 0.99 (2 H, m) 1.54 - 1.67 (1 H, m) 1.87 - 2.10 (4 H, m) 2.64

(3 H, s) 2.86 - 3.00 (2 H, m) 3.08 - 3.26 (2 H, m) 3.95 (2 H, s) 4.75 (1 H, s) 7.53 (2 H, d, J=8.33 Hz) 7.58 (1 H, d, J=8.71 Hz) 7.79 (1 H, d, J=8.71 Hz) 8.00 (2 H, d, J=8.33 Hz) 8.21 (1 H, d, J=1.89 Hz) 8.92 (1 H, d, J=2.27 Hz) 10.24 (1 H, s).
FAB(pos): 518 $[MH]^+$

Example 44

4-(cyclopropylethynyl)-N-[8-methyl-3-({[3-(methylsulfonyl)propyl]amino}methyl)quinolin-7-yl]benzamide

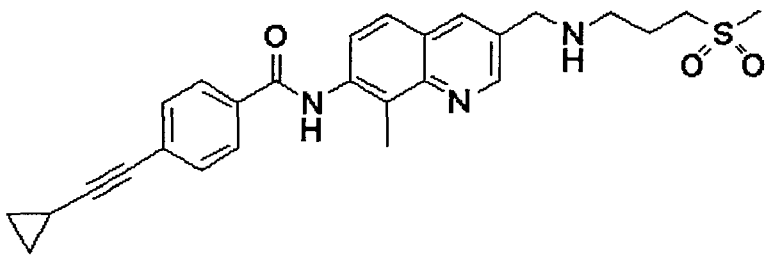

4-(Cyclopropylethynyl)-N-[8-methyl-3-({[3-(methylthio)propyl]amino}methyl)quinolin-7-yl]benzamide (436 mg) obtained in Reference Example 93 was suspended in acetone (30 mL), and the suspension was cooled to 5°C. A solution of oxone (720 mg) in water (2 mL) was added dropwise thereto, and the mixture was allowed to warm to room temperature, and stirred overnight. The reaction mixture was basified with 1N aqueous sodium hydroxide solution, and the precipitated solid was collected by filtration, washed with water, dried under reduced pressure, and recrystallized from acetone and water to give the title compound (376 mg, yield 80%) as a white solid. $^1H$ NMR (300 MHz, DMSO-$d_6$) δ: 0.74 - 0.84 (2 H, m) 0.89 - 0.98 (2 H, m) 1.53 - 1.67 (1 H, m) 1.79 - 1.95 (2 H, m) 2.59 - 2.70 (5 H, m) 2.95 (3 H, s) 3.13 - 3.24 (2 H, m) 3.90 (2 H, s) 7.53 (2 H, d, J=8.33 Hz) 7.58 (1 H, d, J=8.71 Hz) 7.79 (1 H, d, J=8.71 Hz) 8.00 (2 H, d, J=8.33 Hz) 8.21 (1 H, d, J=1.89 Hz) 8.91 (1 H, d, J=2.27 Hz) 10.24 (1 H, s).
FAB(pos): 476 $[MH]^+$

Example 45

4-[(E)-2-cyclopropylvinyl]-N-(3-{[(1,1-dioxidotetrahydro-2H-thiopyran-4-yl)amino]methyl}-8-methylquinolin-7-yl)benzamide

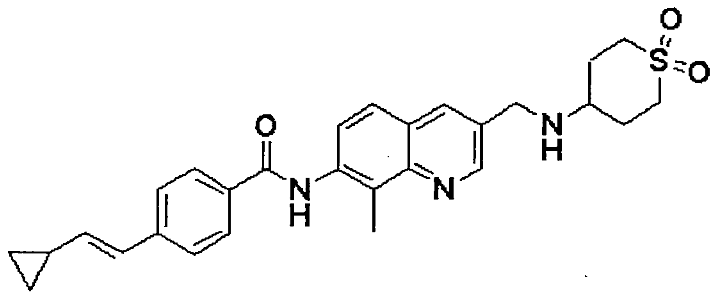

4-[(E)-2-Cyclopropylvinyl]-N-{8-methyl-3-[(tetrahydro-2H-thiopyran-4-ylamino)methyl]quinolin-7-yl}benzamide (330 mg) obtained in Reference Example 98 was suspended in acetone (35 mL), and the suspension was cooled to 5°C. An aqueous solution of oxone (550 mg) in water (3 mL) was added dropwise thereto, and the mixture was allowed to warm to room temperature, and stirred for 2 hr. To the reaction mixture was added 10% aqueous citric acid solution (100 mL), and the mixture was extracted with ethyl acetate. The aqueous layer was separated, and the insoluble material was filtered off, and the filtrate was basified with 8N aqueous sodium hydroxide solution. The precipitated solid was collected by filtration, washed with water, dried under reduced pressure, and recrystallized from acetone and water to give the title compound (203 mg, yield 58%) as a white solid.

$^1H$ NMR (300 MHz, DMSO-$d_6$) δ: 0.51 - 0.67 (2 H, m) 0.79 - 0.93 (2 H, m) 1.82 - 2.03 (2 H, m) 2.02 - 2.22 (2 H, m) 2.55 - 2.70 (1 H, m) 2.64 (3 H, s) 2.73 - 2.88 (1 H, m) 2.95 - 3.09 (2 H, m) 3.11 - 3.25 (2 H, m) 3.93 (2 H, s) 6.03 (1 H, dd, J=15.90, 9.47 Hz) 6.57 (1 H, d, J=15.90 Hz) 7.51 (2 H, d, J=8.33 Hz) 7.59 (1 H, d, J=8.71 Hz) 7.79 (1 H, d, J=8.71 Hz) 7.97 (2 H, d, J=8.33 Hz) 8.24 (1 H, d, J=1.89 Hz) 8.92 (1 H, d, J=2.27 Hz) 10.13 (1 H, s).
FAB(pos): 490 $[MH]^+$
elemental analysis value ($C_{28}H_{32}N_3O_3S$)
Calculated: C, 68.68; H, 6.38; N, 8.59.
Found: C, 68.38; H, 6.33; N, 8.54.

Example 46

4-(cyclopropylmethoxy)-N-(6-{[(1,1-dioxidotetrahydro-2H-thiopyran-4-yl)amino]methyl}naphthalen-2-yl)benzamide

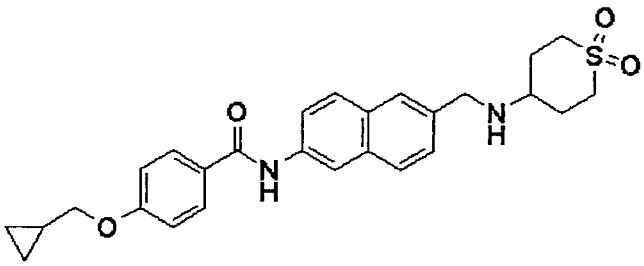

4-(Cyclopropylmethoxy)-N-[6-({(1,1-dioxidotetrahydro-2H-thiopyran-4-yl)[(2-nitrophenyl)sulfonyl]amino}methyl)naphthalen-2-yl]benzamide (190 mg) obtained in Reference Example 103 was dissolved in DMF (2 mL), 2N lithium hydroxide (2 mL) and thioglycol acid (52 mg) were added thereto, and the mixture was stirred overnight. To the reaction mixture was added water (2 mL), and the precipitated solid was collected by filtration to give the title compound (130 mg, yield 95%) as a colorless powder.

$^1H$ NMR (300 MHz, DMSO-$d_6$) δ: 0.32-0.40 (2 H, m), 0.56-0.64 (2 H, m), 1.21-1.32 (1 H, m), 1.89-2.14 (4 H, m), 2.76-2.85 (1 H, m),2.94-3.05 (2 H, m),3.11-3.23 (2 H, m),3.84(2H,br),3.92 (2 H, d, J=7.0 Hz),7.07 (2 H, d, J=8.9 Hz), 7.50 (1 H, dd, J=8.4, 1.6 Hz), 7.76-7.86 (4 H, m),7.99 (2 H, d, J=8.9 Hz), 8.40 (1 H, s),10.23 (1 H, s).
FAB(pos): 479 $[MH]^+$

Example 47

4-(cyclopropylmethoxy)-N-(6-{[(1,1-dioxidotetrahydro-2H-thiopyran-4-yl)amino]methyl}-5-methyl-7,8-dihydronaphthalen-2-yl)benzamide

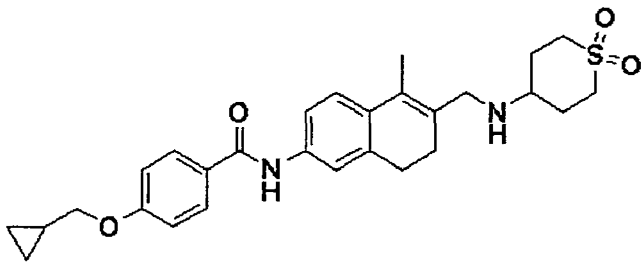

4-(Cyclopropylmethoxy)-N-(6-formyl-5-methyl-7,8-dihydronaphthalen-2-yl)benzamide (181 mg) obtained in Reference Example 106, tetrahydro-2H-thiopyran-4-amine 1,1-dioxide hydrochloride (186 mg) obtained in Reference Example 38, triethylamine (152 mg) and acetic acid (400 μL) were added to methanol (20 mL), and the mixture was stirred for 30 min. Sodium triacetoxyborohydride (424 mg) was added thereto, and the mixture was stirred overnight.

The reaction solution was partitioned between aqueous sodium hydrogen carbonate solution and ethyl acetate, and the organic layer was washed with water and saturated brine, dried over magnesium sulfate, and concentrated under reduced pressure. The obtained residue was purified by silica gel column chromatography [eluent; hexane:ethyl acetate=5:5 (volume ratio)→hexane:ethyl acetate=0:10 (volume ratio)] to give the title compound (60 mg, yield 24%) as a pale-yellow powder.
$^1$H NMR (300 MHz, DMSO-$d_6$) δ: 0.28-0.40 (2 H, m), 0.54-0.66 (2 H, m), 1.11-1.33 (1 H, m), 1.84-2.15 (9 H, m),2.22-2.35 (2 H, m), 2.60-2.82 (3 H, m),2.94-3.22 (4 H, m),3.90 (2 H, d, J=7.2 Hz), 7.04 (2 H, d, J=9.0 Hz), 7.22 (1 H, d, J=8.3 Hz), 7.52-7.67 (2 H, m),7.94 (2 H, d, J=8.7 Hz),10.01 (1 H, s).

Example 48

4-(cyclopropylmethoxy)-N-(6-{[(1,1-dioxidotetrahydro-2H-thiopyran-4-yl)amino]methyl}-5-methylnaphthalen-2-yl)benzamide

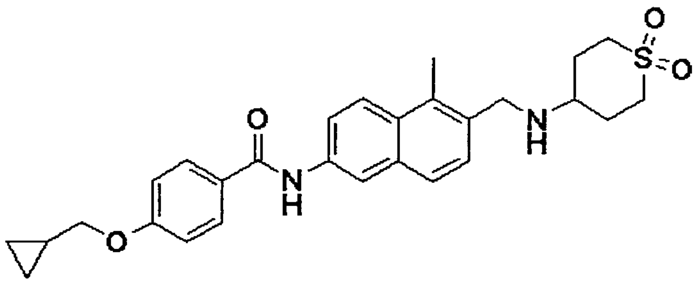

4-(Cyclopropylmethoxy)-N-(6-formyl-5-methylnaphthalen-2-yl)benzamide (100 mg) obtained in Reference Example 107, tetrahydro-2H-thiopyran-4-amine 1,1-dioxide hydrochloride (77 mg) obtained in Reference Example 38, triethylamine (42 mg) and acetic acid (200 μL) were added to DMF (21 mL)-THF (4 mL), and the mixture was stirred for 30 min. Sodium triacetoxyborohydride (236 mg) was added thereto, and the mixture was stirred overnight. The reaction solution was partitioned between ethyl acetate and water, and the organic layer was washed with water and saturated brine, dried over magnesium sulfate, and concentrated under reduced pressure, The obtained residue was purified by silica gel column chromatography [eluent; ethyl acetate:methanol=10:0 (volume ratio)→ethyl acetate:methanol=8:2 (volume ratio)] to give the title compound (55 mg, yield 39%) as a colorless powder.
$^1$H NMR (300 MHz, DMSO-$d_6$) δ: 0.33-0.39 (2 H, m), 0.56-0.63 (2 H, m), 1.21-1.31 (1 H, m), 1.94-2.17 (4 H, m),2.62 (3 H, s), 2.81-2.90 (1 H, m),2.97-3.08 (2 H, m),3.11-3.23 (2 H, m),3.82-3.94 (4 H, m),7.07 (2 H, d, J=8.7 Hz), 7.49 (1 H, d, J=8.3 Hz), 7.65 (1 H, d, J=8.7 Hz),7.84 (1 H, dd, J=9.1, 2.3 Hz),8.00 (2 H, d, J=8.7 Hz), 8.07 (1 H, d, J=9.1 Hz), 8.39 (1 H, d, J=1.9 Hz),10.24 (1 H, s).
FAB(pos): 493 $[MH]^+$

Formulation Example 1

| | | |
|---|---|---|
| (1) | compound obtained in Example 1 | 50 mg |
| (2) | lactose | 34 mg |
| (3) | cornstarch | 10.6 mg |
| (4) | corn starch (paste) | 5 mg |
| (5) | magnesium stearate | 0.4 mg |
| (6) | calcium carboxymethylcellulose | 20 mg |
| | | Total 120 mg |

According to a conventional method, the above-mentioned (1) to (6) are mixed, and the mixture is tableted by a tableting machine to give a tablet.

Experimental Example 1 Determination of MCH receptor antagonistic activity of test compound using GTP γ S binding assay

Using human SLC-1 expression CHO cell clone 57 and rat SLC-1 expression CHO cell clone 44 described in WO01/82925, membrane fractions of SLC-1 expression CHO cells were prepared by the following method. In phosphate buffered saline (pH 7.4) supplemented with 5 mM EDTA (ethylenediaminetetraacetic acid) were suspended human and rat SLC-1 expression CHO cells ($1x10^8$) and centrifuged. Homogenate buffer (10 ml, 10 mM $NaHCO_3$, 5 mM EDTA, pH 7.5) was added to the pellets of the cells and, using Polytron Homogenizer, the mixture was homogenated. The supernatant obtained after centrifugation at 400xg for 15 min was further centrifuged at 100,000xg for 1 hr to give precipitate of the membrane fraction. The precipitate was suspended in 2 ml of an assay buffer [50 mM Tris-HCl (pH 7.5), 1 mM EDTA, 0.1% BSA (bovine serum albumin), 10 mM $MgCl_2$, 100 mM NaCl, 1 μM GDP (guanosine 5'-diphosphate), 0.25 mM PMSF (phenylmethylsulfonyl fluoride), 1 mg/ml pepstatin, 20 mg/ml leupeptin, 10 mg/ml phosphoramidon] and centrifuged at 100,000xg for 1 hr. The membrane fraction recovered as precipitate was suspended again in 20 ml of an assay buffer, and after dispensing, preserved at -80°C and used upon thawing each time when in use.
The MCH receptor antagonistic activity of the test compound was determined as follows. The membrane fractions of SLC-1 expression CHO cells (171 μl) diluted with an assay buffer was dispensed to a polypropylene 96 well plate and $3x10^{-10}$ M MCH (2 μl) diluted with DMSO solution, test compound solution (2 μl) diluted to various concentrations and [$^{35}$S]-Guanosine 5'-(γ-thio)triphosphate (25 μl, Daiichi Pure Chemicals Co., Ltd.) were respectively added (cellular membrane final concentration: 20 μg/ml, [$^{35}$S]-Guanosine 5'-(γ-thio)triphosphate final concentration: 0.33 nM). The reaction mixture was reacted at 25°C for 1 hr with stirring, suction filtered with a glass filter (GF-C) and washed 3 times with a wash solution (300 μl, 50 mM Tris-HCl buffer, pH 7.5). Liquid scintillator (50 ml) was added to the glass filter and the residual radioactivity was determined by a liquid scintillation counter.

Binding inhibition (%) = (radioactivity upon addition of test compound and MCH - radioactivity upon addition of DMSO solution)/(radioactivity upon addition of MCH - radioactivity upon addition of DMSO solution) x 100

From the binding inhibition (%), $IC_{50}$ of the test compound was calculated.
The results are shown below.
Table 1

| Compound No. | Inhibitory Activity ($IC_{50}$ value: nM) |
|---|---|
| Ex. 2 | 2.3 |
| Ex. 3 | 2.2 |
| Ex. 4 | 2.8 |
| Ex. 6 | 4.0 |
| Ex. 7 | 3.7 |
| Ex. 8 | 3.9 |
| Ex. 13 | 2.8 |
| Ex. 14 | 3.0 |
| Ex. 15 | 5.6 |
| Ex. 16 | 1.8 |
| Ex. 18 | 1.1 |
| Ex. 20 | 1.4 |
| Ex. 21 | 2.7 |

(continued)

| Compound No. | Inhibitory Activity ($IC_{50}$ value: nM) |
|---|---|
| Ex. 22 | 1.8 |
| Ex. 23 | 1.5 |
| Ex. 24 | 1.5 |
| Ex. 25 | 1.2 |
| Ex. 26 | 4.6 |
| Ex. 27 | 1.6 |
| Ex. 28 | 4.6 |
| Ex. 30 | 3.9 |
| Ex. 31 | 5.1 |
| Ex. 32 | 6.5 |
| Ex. 33 | 4.1 |
| Ex. 34 | 2.7 |
| Ex. 35 | 5.6 |
| Ex. 36 | 3.4 |
| Ex. 41 | 2.2 |
| Ex. 42 | 2.3 |
| Ex. 43 | 4.7 |
| Ex. 44 | 2.2 |
| Ex. 45 | 4.9 |
| Ex. 46 | 4.4 |
| Ex. 47 | 6.7 |

As is clear from Table 1, the compound of the present invention has a superior MCH receptor antagonistic activity.

Experimental Example 2 Evaluation of HERG inhibitory activity

MEM medium, MEM non-essential amino acid solution, sodium pyruvate solution and G418 sulfate solution (Geneticin) were purchased from Invitrogen (Carlsbad, CA). Bovine serum albumin (BSA, Fatty Acid Free) used was the product of Wako Pure Chemical Industries, Ltd. (Osaka, Japan). As fetal calf serum (FCS), a product of Trau Scientific Ltd. (Melbourne, Australia) was used.
HERG expression cell HERG.T.HEK was obtained from Wisconsin ALUMNI Research Foundation. HERG.T.HEK was maintained and passaged in an MEM medium containing 10% FCS, 1 mM MEM non-essential amino acid, 1 mM sodium pyruvate and 500 μg/ml Geneticin at 37°C, 5% $CO_2$.
The cells at 80-90% confluent were collected by a trypsin treatment and plated in IVF dish (Falcon, Franklin Lakes, NJ). After 2-3 hr, the cells were adhered to a glass electrode (resistance value 2-3 MΩ) filled with an inner electrode solution (7 mM NaCl, 130 mM KCl, 1 mM $MgCl_2$, 5 mM HEPES, 5 mM EGTA, 5 mM ATP-Na: pH 7.2) while perfusing with an extracellular solution (137 mM NaCl, 4 mM KCl, 1 mM $MgCl_2$, 1.8 mM $CaCl_2$, 10 mM HEPES, 11 mM dextrose: pH 7.4), and a whole-cell configuration was formed and stimulation by voltage-clamp protocol were performed using a patch clamp amplifier AXOPATCH 200B (Axon Instruments, Foster City, CA) (holding potential - 75 mV, primary voltage 10 mV: 0.5 sec, secondary voltage -40 mV: 0.5 sec, stimulation frequency 10 sec). Preliminary stimulation was applied, and when the electric wave profile became stable, the HERG electric current value (peak tail current) was measured.
For measurement of HERG electric current with addition of a test compound, the cells were perfused with an extracellular solution, and when the wave profile became stable, the cells were perfused with an extracellular solution containing 10 μM of the test compound. When the electric current wave profile became stable under each perfusation condition, the HERG electric current was measured.

HERG electric current inhibitory rate (%) by the test compound was obtained based on the HERG electric current value without addition of test compound as 100%. As a result, the compound of the present invention showed low HERG inhibitory activity and low toxicity.

Experimental Example 3 Evaluation of PLsis

DMEM medium, L-glutamine, penicillin-streptomycin, pyruvic acid, and N-(7-nitrobenz-2-oxa-1,3-diazol-4-yl)-1,2-hexadecanoyl-sn-glycerol-3-phosphoethanolamine triethylammonium salt (NBD-PE) were purchased from Invitrogen. As bovine serum albumin (BSA), the product of Thermo Trace Ltd. (Melbourne, Australia) was used and as Amiodarone, the product of ICN (Costa Mesa, CA) was used. A test substance was used as 10 mM DMSO solution.
To DMEM medium supplemented with L-glutamine, pyruvic acid, and penicillin-streptomycin was added FBS at a final concentration of 5 vol% and the medium was subjected to an experiment. The cells were cultured in a $CO_2$ incubator set to 37°C and using 5% carbon dioxide gas - 95% air as a gaseous phase. HepG2 cells were suspended in the culture medium in a 96 well plate to $50 \times 10^4$ cells/mL, seeded at 50 μL/well, and precultured for 24 hr. After preculture, the culture medium was removed, a culture medium containing 60 μm NBD-PE was added at 50 μL/well and a culture medium containing 0, 6 μm or 20 μm test substance was added to HepG2 cells at 50 μL/well, and the cells were cultured for 24 hr. As a positive control, Amiodarone at a final concentration of 10 μM was used.
After exposure to the test substance for 24 hr, the fluorescence intensity (Ex. 485 nm, Em. 538 nm) of NBD-PE taken up into the cells was measured by a fluorophotometer. The measurement value of the solution containing 0 μM test substance was subtracted as a blank, and the relative value to the measurement value with addition of 10 μM Amiodarone was calculated. The maximum value per unit concentration of the test substance was obtained as a phospholipidosis (PLsis)-induced potential. As a result, the PLsis induced potential of the compound of the present invention was low and the compound was shown to be low toxic.

## Industrial Applicability

Since compound (I) has a melanin-concentrating hormone receptor antagonistic action and low toxicity, the compound is very useful as an agent for the prophylaxis or treatment of obesity and the like.
This application is based on patent application No. 094805/2008 filed in Japan, the contents of which are hereby incorporated by reference.

## Claims

1. A compound represented by the formula (I):

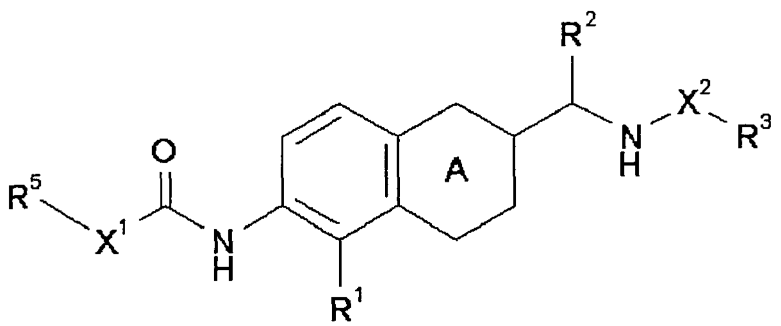

(I)

wherein

ring A is an optionally further substituted 6-membered ring;
$R^1$ is a hydrogen atom, a halogen atom or a $C_{1-6}$ alkyl group;
$R^2$ is a hydrogen atom or a $C_{1-6}$ alkyl group;
$R^3$ is
a group represented by the formula: $-Y-S(O)_{m1}-R^{4a}$
wherein

Y is a bond or NH;
ml is an integer of 1 or 2; and
$R^{4a}$ is a $C_{1-6}$ alkyl group optionally substituted by 1 to 3 halogen atoms, or

a cyclic group represented by the formula:

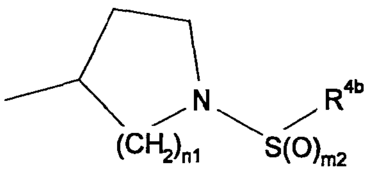 , 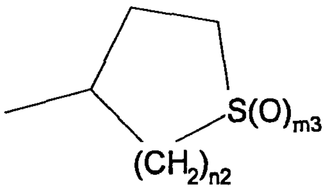 , or 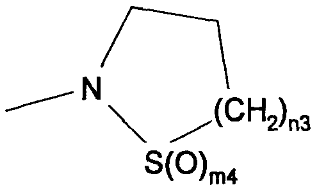

wherein

m2, m3, m4, n1, n2 and n3 are each independently an integer of 1 or 2; and
$R^{4b}$ is a $C_{1-6}$ alkyl group optionally substituted by 1 to 3 halogen atoms
(the ring moiety of the cyclic group is optionally further substituted);

$R^5$ is an optionally further substituted 5- or 6-membered cyclic group;
$X^1$ is a bond or a $C_{1-6}$ alkylene group; and
$x^2$ is a bond or a $C_{1-6}$ alkylene group,

or a salt thereof.

2. The compound of claim 1, wherein ring A is an optionally further substituted 6-membered aromatic heterocycle.

3. The compound of claim 1, wherein $R^3$ is a group represented by the formula: -Y-S(O)$_{m1}$-$R^{4a}$ wherein Y is a bond; ml is an integer of 1 or 2; and $R^{4a}$ is a $C_{1-6}$ alkyl group optionally substituted by 1 to 3 halogen atoms, or a cyclic group represented by the formula:

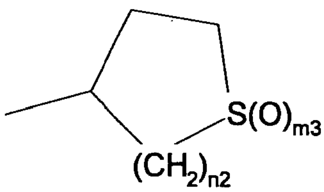

wherein m3 and n2 are each independently an integer of 1 or 2 (the ring moiety of the cyclic group is optionally further substituted).

4. The compound of claim 1, wherein $R^5$ is an optionally further substituted phenyl group.

5. The compound of claim 1, wherein $X^1$ is a bond.

6. The compound of claim 1, wherein
ring A is an optionally further substituted 6-membered aromatic heterocycle;
$R^1$ is a hydrogen atom, a halogen atom or a $C_{1-6}$ alkyl group; $R^2$ is a hydrogen atom or a $C_{1-6}$ alkyl group;
$R^3$ is a group represented by the formula: -Y-S(O)$_{m1}$-$R^{4a}$ wherein Y is a bond; ml is an integer of 1 or 2; and $R^{4a}$ is a $C_{1-6}$ alkyl group optionally substituted by 1 to 3 halogen atoms, or a cyclic group represented by the formula:

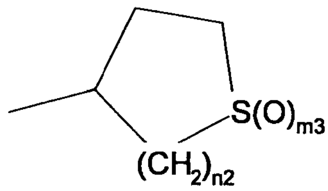

wherein m3 and n2 are each independently an integer of 1 or 2 (the ring moiety of the cyclic group is optionally further substituted);
$R^5$ is an optionally further substituted phenyl group;
$X^1$ is a bond; and
$X^2$ is a bond or a $C_{1-6}$ alkylene group.

**7.** 4-(Cyclopropylmethoxy)-N-(3-{[(1,1-dioxidotetrahydro-2H-thiopyran-4-yl)amino]methyl}-8-methylquinolin-7-yl)benzamide or a salt thereof.

**8.** 4-(Cyclopropylmethoxy)-N-(8-methyl-3-{[(trans-1-oxidotetrahydro-2H-thiopyran-4-yl)amino]methyl}quinolin-7-yl) benzamide or a salt thereof.

**9.** 4-(Cyclopropylmethoxy)-N-(3-{[(1,1-dioxidotetrahydro-2H-thiopyran-4-yl)amino]methyl}-8-methylquinolin-7-yl)-2-fluorobenzamide or a salt thereof.

**10.** 4-(Cyclopropylmethoxy)-2-fluoro-N-(8-methyl-3-{[(trans-1-oxidotetrahydro-2H-thiopyran-4-yl)amino]methyl}quinolin-7-yl)benzamide or a salt thereof.

**11.** 4-(Cyclopropylethynyl)-N-(3-{[(1,1-dioxidotetrahydro-2H-thiopyran-4-yl)amino]methyl}-8-methylquinolin-7-yl)benzamide or a salt thereof.

**12.** A prodrug of the compound of claim 1.

**13.** A pharmaceutical agent comprising the compound of claim 1.

**14.** The pharmaceutical agent of claim 13, which is a melanin-concentrating hormone receptor antagonist.

**15.** The pharmaceutical agent of claim 13, which is an anorexigenic agent.

**16.** The pharmaceutical agent of claim 13, which is an agent for the prophylaxis or treatment of obesity.

**17.** A method for the prophylaxis or treatment of obesity in a mammal, which comprises administering an effective amount of the compound of claim 1 or a prodrug thereof to the mammal.]

**18.** Use of the compound of claim 1 or a prodrug thereof for the production of an agent for the prophylaxis or treatment of obesity.

**INTERNATIONAL SEARCH REPORT**

International application No.
PCT/JP2009/056663

A. CLASSIFICATION OF SUBJECT MATTER
*C07D215/38*(2006.01)i, *A61K31/382*(2006.01)i, *A61K31/47*(2006.01)i, *A61K31/4709*(2006.01)i, *A61P3/04*(2006.01)i, *A61P43/00*(2006.01)i, *C07D335/02*(2006.01)i, *C07D401/12*(2006.01)i, *C07D409/12*(2006.01)i,
According to International Patent Classification (IPC) or to both national classification and IPC

B. FIELDS SEARCHED
Minimum documentation searched (classification system followed by classification symbols)
C07D215/38, A61K31/382, A61K31/47, A61K31/4709, A61P3/04, A61P43/00, C07D335/02, C07D401/12, C07D409/12, C07D417/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
WPI, CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2001/021577 A2 (TAKEDA CHEM IND LTD.), 29 March, 2001 (29.03.01), Claims; pages 51 to 52 & JP 2002-003370 A & EP 1218336 A2 & US 7115750 B1 & US 2007/173498 A1 | 1-6,12-16,18 |
| X | WO 2001/082925 A1 (TAKEDA CHEM IND LTD.), 08 November, 2001 (08.11.01), Claims; pages 41 to 42 & JP 2002-241274 A & EP 1285651 A1 & US 2004/077628 A1 & US 6930185 B2 | 1-6,12-16,18 |
| X | WO 2003/035624 A1 (TAKEDA CHEM IND LTD.), 01 May, 2003 (01.05.03), Claims; pages 36 to 39 & JP 2004-059567 A & EP 1447402 A1 & US 2005/209213 A1 & US 7183415 B2 | 1-6,12-16,18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 April, 2009 (17.04.09) | 28 April, 2009 (28.04.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2009/056663

C (Continuation). DOCUMENTS CONSIDERED TO BE RELEVANT

| Category* | Citation of document, with indication, where appropriate, of the relevant passages | Relevant to claim No. |
|---|---|---|
| A | WO 2003/045313 A2 (MERCK & CO. INC.),<br>05 June, 2003 (05.06.03),<br>Claims; examples 28, 324, 333<br>& EP 1450801 A2 & US 2005/026915 A1<br>& JP 2005-519876 A & US 7084156 B2 | 1-16,18 |
| A | WO 2003/087046 A1 (7TM PHARMA AS),<br>23 October, 2003 (23.10.03),<br>Claims<br>& AU 2003226928 A1 & NO 200404131 A | 1-16,18 |
| A | WO 2003/015769 A1 (AVENTIS PHARMA DEUT GMBH),<br>27 February, 2003 (27.02.03),<br>Claims<br>& US 2003/212070 A1 & EP 1418906 A1<br>& US 2004/192693 A1 & US 2004/198731 A1<br>& US 2004/198733 A1 & US 2004/198732 A1<br>& JP 2005-505530 A | 1-16,18 |
| P,A | WO 2009/021740 A2 (SANOFIS-AVENTIS),<br>19 February, 2009 (19.02.09),<br>Claims; pages 11 to 12; Bsp.Nos.3-64, 3-81<br>& EP 2025674 A1 | 1-16,18 |

Form PCT/ISA/210 (continuation of second sheet) (April 2007)

**INTERNATIONAL SEARCH REPORT**

International application No.
PCT/JP2009/056663

Continuation of A. CLASSIFICATION OF SUBJECT MATTER
(International Patent Classification (IPC))

*C07D417/12*(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2009/056663

**Box No. II Observations where certain claims were found unsearchable (Continuation of item 2 of first sheet)**

This international search report has not been established in respect of certain claims under Article 17(2)(a) for the following reasons:

1. [x] Claims Nos.: 17
because they relate to subject matter not required to be searched by this Authority, namely:
Claim 17 includes the methods for treatment of the human body or animal body by surgery or therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0121577 A
- WO 0182925 A
- WO 01087834 A
- WO 03035624 A
- WO 2006118320 A
- WO 2001082925 A
- WO 2003035624 A
- WO 0114372 A
- WO 9710224 A
- JP 2008094805 A


### Non-patent literature cited in the description


- *Nature,* 1998, vol. 396, 670
- Design of Molecules. IYAKUHIN no KAIHATSU. HIROKAWA SHOTEN, 1990, vol. 7, 163-198
- Organic Functional Group Preparations. Academic Press Inc, 1989
- Comprehensive Organic Transformations. VCH Publishers Inc, 1989
- Mitsunobu reaction. *Synthesis,* 1981, 1-27
- Protective Groups in Organic Synthesis. John Wiley and Sons, 1980